# EUROPEAN PATENT APPLICATION

(11) **EP 2 543 368 A1**
(43) Date of publication of application: **09.01.2013**
(21) Application number: 12187104.0
(22) Date of filing: 03.12.2008
(51) Int. Cl.: A61K 31/10, A61K 31/122, A61K 31/35, A61K 31/37, A61K 31/381, A61K 31/382, A61K 31/40, A61K 31/404, A61K 31/431, A61K 31/437, A61K 31/44, A61K 31/55, A61K 31/5513

(54) **Metalloenzyme inhibitors using metal binding moieties in combination with targeting moieties**

(30) Priority: 11.12.2007 US 7206 P
(62) Divisional of application: 08872570.0
(71) Applicant: Viamet Pharmaceuticals, Inc., Morrisville, NC 27560 (US)
(72) Inventor: Schotzinger, Robert, J., Raleigh, NC North Carolina 27613 (US); Hoekstra, William, J., Chapel Hill, NC North Carolina 27514 (US)
(74) Representative: Silcock, Peter James

(57) **Abstract**

The presently disclosed subject matter is described to metalloenzyme inhibitors having metal binding moieties linked to a targeting moiety through a linking group or a direct bond, method of screening for metalloenzyme inhibitors, and methods for treating a metalloenzyme related disorder by administering a metalloenzyme inhibitor to a subject in need of treatment thereof.

## Description

### TECHNICAL FIELD

The presently disclosed subject matter is directed to metalloenzyme inhibitors having metal binding moieties linked to a targeting moiety through a linking group or a direct bond, methods of screening for metalloenzyme inhibitors, and methods for treating a metalloenzyme related disorder.

### BACKGROUND

Metalloenzymes are enzymes that include one or more metal atoms, e.g., one or more metal ions, as functional components of their structure. These metal ions typically form part of the active site of the enzyme. Because the metal ions can be multicoordinated, they can be bound to a protein while having a high affinity for a substrate, for example, through a lone pair of electrons. Such metal ions are often involved in enzyme catalysis.

Despite the importance of the metal ions to metalloenzyme activity, the current evaluation and development of metalloenzyme inhibitors typically ignores the activity of the metal ions in the design of the inhibitors. As described in more detail herein, certain candidate metalloenzyme inhibitors are known in the art. While some of these candidates have shown promise, there is a need for novel selective inhibitors of metalloenzymes, such as carbonic anhydrase, catechol O-methyl transferase (COMT), farnesyl diphosphate synthase, farnesyl transferase, neprilysin (NEP), thromboxane synthase, anthrax lethal factor, endothelin converting enzyme, methionine aminopeptidase-2, peptide deformylase, HIV integrase, TNF-alpha converting enzyme (TACE), UDP-(3-O-acyl)-N-acetylglucosamine deacetylase (LpxC), histone deacetylase (HDAC), and matrix metalloproteinase (MMP).

The presently disclosed subject matter addresses, in whole or in part, these and other needs in the art.

### SUMMARY

The presently disclosed subject matter is directed to a two-prong approach to inhibiting metalloenzymes. More particularly, the presently disclosed subject matter is directed to the combination of a metal binding moiety (MBM) in conjunction with a targeting moiety (TM), optionally linked through a linker. Thus, the presently disclosed subject matter results in more efficacious inhibitors by combining the affinity and specificity of two different but proximal sites of the metalloprotein.

In this way, both additive and synergistic binding effects, including both binding affinity and binding specificity, can be utilized. As will be appreciated by those in the art, the binding affinity and binding specificity can work in a variety of ways. Some metal binding moieties, such as the hydroxamates, bind tightly to zinc ions, for example. However, these inhibitors tend to be not very specific, and can exhibit toxic effects from binding zinc in a variety of metalloproteins. The presently disclosed subject matter provides for enhanced specificity of tight metal binding moieties by using specificity to the region of the metalloprotein in proximity to the metal binding site to allow for better targeting and a reduction in toxicity due to non-specific binding. Similarly, the addition of two moieties with low affinity and/or low specificity can result in an inhibitor with high affinity and/or high specificity. Thus, any combination of "good" and "poor" metal binding moieties can be linked to either "good" or "poor" targeting moieties to result in "good" inhibitors.

In one embodiment, the presently disclosed subject matter provides metalloenzyme inhibitors comprising a metal binding moiety and a targeting moiety, optionally with linkers. In some embodiments, the metalloenzyme inhibitor includes a carbonic anhydrase inhibitor, a catechol *O*-methyl transferase (COMT) inhibitor, a farnesyl diphosphate synthase inhibitor, a farnesyl transferase inhibitor, a neprilysin (NEP) inhibitor, a thromboxane synthase inhibitor, an anthrax lethal factor inhibitor, an endothelin converting enzyme inhibitor, a methionine aminopeptidase-2, peptide deformylase inhibitor, an HIV integrase inhibitor, a TNF-alpha converting enzyme (TACE) inhibitor, a UDP-(3-O-acyl)-N-acetylglucosamine deacetylase (LpxC) inhibitor, a histone deacetylase (HDAC) inhibitor, and a matrix metalloproteinase (MMP) inhibitor.

The presently disclosed subject matter also includes pharmaceutical compositions comprising a pharmaceutical carrier and one or more of the presently disclosed metalloenzyme inhibitors, or a prodrug or salt thereof.

In some embodiments, the metal binding moiety is selected from the group consisting of a sulfonyl moiety, a carbonyl moiety, a boronic acid or boronic ester moiety, a sulfur-containing moiety, a nitrogen-containing moiety, a phosphorous-containing moiety, a 5-membered heteroaromatic ring having one heteroatom, a 5-membered aromatic ring having two heteroatoms, a 5-membered heteroaromatic ring having three heteroatoms, a 5-membered heteroaromatic ring having four heteroatoms, a 5-membered heteroaromatic ring having five heteroatoms, a 5-membered saturated or partially unsaturated heteroalkyl ring having one heteroatom, a 5-membered saturated or partially unsaturated heteroalkyl ring having two heteroatoms, a six-membered aromatic ring, a 6-membered heteroaromatic ring having one heteroatom, a 6-membered aromatic ring having two heteroatoms, a 6-membered heteroaromatic ring having three heteroatoms, a 6-membered heteroaromatic ring having four heteroatoms, a 6-membered unsaturated or partially saturated heteroalkyl ring having one heteroatom, and a 6-membered unsaturated or partially saturated heteroalkyl ring having two heteroatoms, with the proviso that certain combinations of metal binding moieties, targeting moieties, and optional linkers are excluded from the presently disclosed subject matter.

In an additional aspect, the presently disclosed subject matter provides a method for screening for inhibitors of metalloenzymes, the method including (a) providing a candidate inhibitor comprising: (i) a targeting moiety; (ii) a metal binding moiety; and (iii) an optional linker; (b) contacting the inhibitor candidate with a metalloenzyme; and (c) determining the activity of the metalloenzyme.

In some embodiments, the presently disclosed subject matter provides a method of treating a metalloenzyme related disorder comprising administering a composition of any of the presently disclosed metalloenzyme inhibitors or a prodrug or salt thereof to a patient in need thereof, wherein the disorder is selected from disorders associated with carbonic anhydrase, catechol O-methyl transferase (COMT), farnesyl diphosphate synthase, farnesyl transferase, neprilysin (NEP), thromboxane synthase, anthrax lethal factor, endothelin converting enzyme, methionine aminopeptidase-2, peptide deformylase, HIV integrase, TNF-alpha converting enzyme (TACE), UDP-(3-O-acyl)-N-acetylglucosamine deacetylase (LpxC), histone deacetylase (HDAC), and matrix metalloproteinase (MMP).

In an additional aspect, the presently disclosed subject matter provides a method of inhibiting a metalloenzyme, the method including contacting a metalloenzyme with a presently disclosed inhibitor, wherein the metalloenzyme is selected from the group consisting of carbonic anhydrase, catechol O-methyl transferase (COMT), farnesyl diphosphate synthase, farnesyl transferase, neprilysin (NEP), thromboxane synthase, anthrax lethal factor, endothelin converting enzyme, methionine aminopeptidase-2, peptide deformylase, HIV integrase, TNF-alpha converting enzyme (TACE), UDP-(3-O-acyl)-N-acetylglucosamine deacetylase (LpxC), histone deacetylase (HDAC), and matrix metalloproteinase (MMP).

Certain aspects of the presently disclosed subject matter having been stated hereinabove, which are addressed in whole or in part by the presently disclosed subject matter, other aspects will become evident as the description proceeds when taken in connection with the accompanying Drawings as best described herein below.

### BRIEF DESCRIPTION OF THE DRAWINGS

Having thus described the presently disclosed subject matter in general terms, reference will now be made to the accompanying Drawings, which are not necessarily drawn to scale, and wherein:

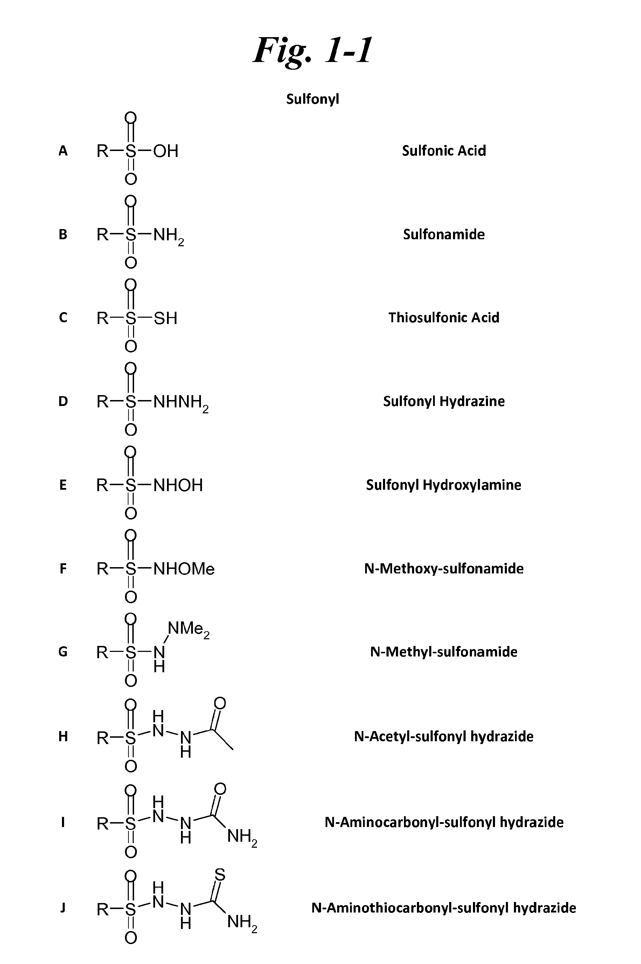

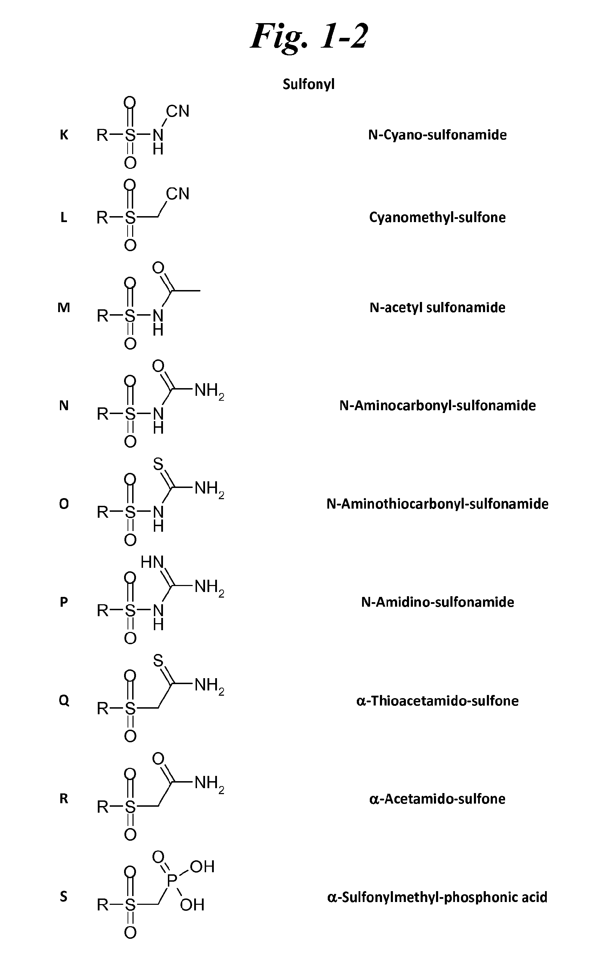

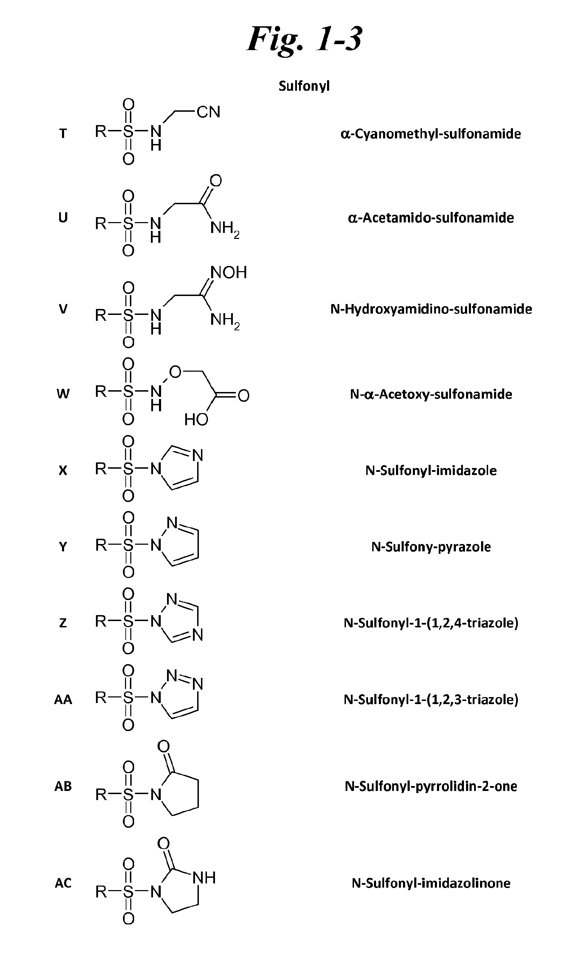

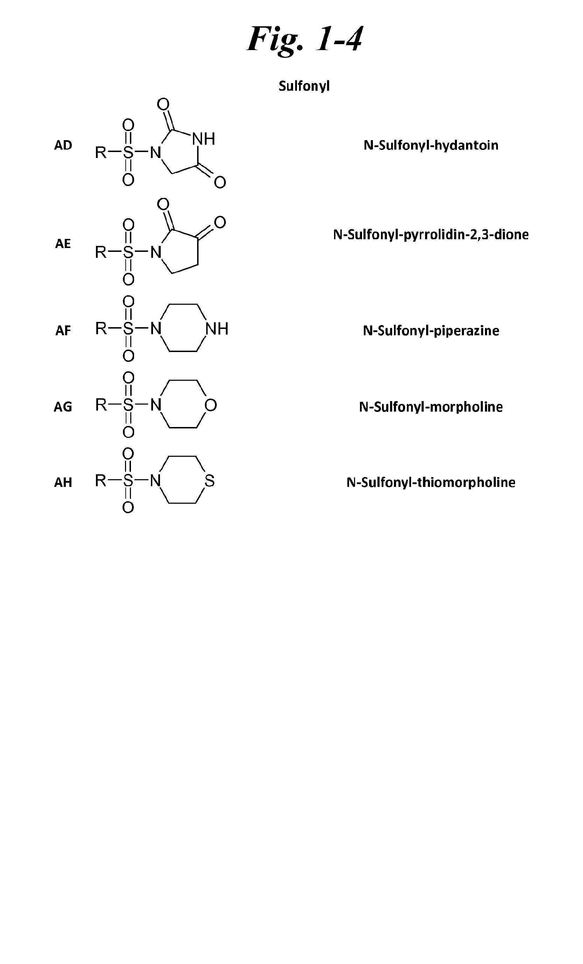

Figures 1-15 depict representative metal binding moieties and derivatives thereof including attachment points to the presently disclosed targeting moieties. In each of Figures 1-15, R represents the attachment of the targeting moiety, with an optional linker as described herein. X represents optional individually selected substitution groups, as outlined herein. Z is a heteroatom selected from the group of oxygen, nitrogen, e.g., NH or N-alkyl, and sulfur. As will be appreciated by those in the art, in some cases, the X groups are hydrogen and are generally not depicted. In addition, when non-hydrogen X substitution groups are used, in general, only one X
   group is preferred. In some cases, and for all the structures herein, as outlined below, two adjacent X groups can be joined to form cyclic structures (including 1 or more cyclic and/or heterocyclic structures, including cyclic aromatic) structures.

For all classes of metal binding moieties recited herein, the presently disclosed subject matter can include or exclude any member of the class individually; for example, the depiction of the sulfonyl-based metal binding moieties in Figure 1 can, in some embodiments, exclude any member; e.g., "sulfonyl-based metal binding moieties except sulfonamide." Each member can be specifically and independently included or excluded.
Figures 1A - 1AH depict sulfonyl-based metal binding moieties.
Figures 2A - 2AJ depict carbonyl-based metal binding moieties.
Figures 3A - 3AD depict various metal binding moieties of the presently disclosed subject matter. Figures 3A-3B depict boronic acid-based metal binding moieties. Figures 3C-3J depict sulfur-based metal binding moieties. Figures 3K - 3V depict nitrogen-based metal binding moieties. Figures 3W to 3AD depict phosphorus-based metal binding moieties.
Figures 4A-C depict metal binding moieties based on 5-membered aromatic heterocycles having one heteroatom, wherein R and X are as described herein.
Figures 5A-5I depict metal binding moieties based on 5-membered aromatic heterocycles having two heteroatoms, wherein R and X are as described herein.
Figures 6A-6O depict metal binding moieties based on 5-membered aromatic heterocycles having three heteroatoms, wherein R and X are as described herein.
Figures 7A-7I depict metal binding moieties based on 5-membered aromatic heterocycles having four or five heteroatoms, wherein R and X are as described herein.
Figures 8A-8I depict metal-binding moieties based on 5-membered non-aromatic rings having one heteroatom, wherein R and X are as described herein.
Figures 9A-9K depict metal-binding moieties based on 5-membered non-aromatic rings having two heteroatoms, wherein R and X are as described herein.
Figure 10A depicts metal binding moieties based on 6-membered aromatic heterocycles having no heteroatoms, wherein R and X are as described herein.
Figure 11A depicts metal binding moieties based on 6-membered aromatic heterocycles having one heteroatom, wherein R and X are as described herein.
Figures 12A-12C depict metal binding moieties based on 6-membered aromatic heterocycles having two heteroatoms, wherein R and X are as described herein.
Figures 13A-13C depict metal binding moieties based on 6-membered aromatic heterocycles having three or four heteroatoms, wherein R and X are as described herein.
Figures 14A-14E depict metal binding moieties based on 6-membered non-aromatic rings having one heteroatom, wherein R and X are as described herein.
Figures 15A-15-L depict metal binding moieties based on 6-membered non-aromatic rings having two heteroatoms, wherein R and X are as described herein.
Figure 16 depicts structures useful as targeting moieties for carbonic anhydrase.
Figure 17 depicts structures useful as targeting moieties for catechol *O*-methyl transferase (COMT).
Figure 18 depicts structures useful as targeting moieties for farnesyl diphosphate synthase.
Figure 19 depicts structures useful as targeting moieties for farnesyl transferase.
Figure 20 depicts structures useful as targeting moieties for neprilysin (NEP).
Figure 21 depicts structures useful as targeting moieties for thromboxane synthase.
Figure 22 depicts structures useful as targeting moieties for anthrax lethal factor.
Figure 23 depicts structures useful as targeting moieties for endothelin converting enzyme.
Figure 24 depicts structures useful as targeting moieties for methionine aminopeptidase-2.
Figure 25 depicts structures useful as targeting moieties for peptide deformylase.
Figure 26 depicts structures useful as targeting moieties for HIV integrase.
Figure 27 depicts structures useful as targeting moieties for TNF-alpha converting enzyme (TACE).
Figure 28 depicts structures useful as targeting moieties for UDP-(3-O-acyl)-N-acetylglucosamine deacetylase (LpxC).
Figure 29 depicts structures useful as targeting moieties for histone deacetylases (HDAC).
Figure 30 depicts structures useful as targeting moieties for matrix metalloproteinases (MMP).

### DETAILED DESCRIPTION

The presently disclosed subject matter now will be described more fully hereinafter with reference to the accompanying Drawings, in which some, but not all embodiments of the presently disclosed subject matter are shown. Many modifications and other embodiments of the presently disclosed subject matter set forth herein will come to mind to one skilled in the art to which the presently disclosed subject matter pertains having the benefit of the teachings presented in the foregoing descriptions and the associated Drawings. Therefore, it is to be understood that the presently disclosed subject matter is not to be limited to the specific embodiments disclosed and that modifications and other embodiments are intended to be included within the scope of the appended claims. Although specific terms are employed herein, they are used in a generic and descriptive sense only and not for purposes of limitation.

The terms "a," "an," and "the" refer to "one or more" when used in this application, including the claims. Thus, for example, reference to "a sample" includes a plurality of samples, unless the context clearly is to the contrary (e.g., a plurality of samples), and so forth.

Throughout this specification and the claims, the words "comprise," "comprises," and "comprising" are used in a non-exclusive sense, except where the context requires otherwise.

As used herein, the term "about," when referring to a value is meant to encompass variations of, in some embodiments ± 20%, in some embodiments ± 10%, in some embodiments ± 5%, in some embodiments ± 1%, in some embodiments ± 0.5%, and in some embodiments ± 0.1% from the specified amount, as such variations are appropriate to perform the disclosed methods or employ the disclosed compositions.

### I. Inhibitors of Selected Metalloenzymes

The presently disclosed subject matter provides inhibitors of metalloenzymes, wherein the inhibitor comprises one or more metal binding moieties, a targeting moiety, and optionally a linker.

By "inhibitor" herein is meant a molecule that is capable of inhibiting a metalloenzyme. By "inhibit" herein is meant to decrease the activity of the metalloenzyme, as compared to the activity of the metalloenzyme in the absence of the inhibitor. In some embodiments, the term "inhibit" represents at least a 5% to 25% decrease in the activity; in some embodiments, a 50% to 75% decrease in the activity; and, in some embodiments, a 95% to 100% decrease in the activity, e.g., a 95%, 96%, 97%, 98%, 99%, or 100% decrease of the activity. The activity of each metalloenzyme can vary, and is described in more detail herein. An assay for measuring individual activity is described below.

### A. Metal binding moieties

By "metal binding moiety (MBM)" herein is meant a moiety that is capable of binding to one or more metal ions through one or more coordination atoms of the MBM, resulting in a coordinate/covalent attachment of the metal to the coordination atom(s). In general, this binding is accomplished through at least one pair of unpaired electrons. As is appreciated by those in the art, the nature of the coordination bond can have covalent characteristics, but is generally referred to as a "coordinate" or "coordinate/covalent" bond.

In some embodiments, the metal binding moieties provides a single coordination atom for binding to the metal ion of a metalloenzyme, such as the heme iron ion of the lanosterol demethylase molecule. In other embodiments, two or more coordination atoms are provided by the metal binding moieties. When two or more coordination atoms are provided by the metal binding moieties, the metal binding moieties can be referred to as a "chelator" or a "ligand". The number of coordination sites is an intrinsic characteristic of the metal being bound: those molecules that use two different atoms to form two bonds to a metal are said to be bidentate. The terms tridentate, tetradentate, pentadentate, and the like then refer to metal binding moieties that use three, four or five atoms, and the like, to form the same number of bonds, respectively.

In general, the nature of the coordination atom depends on the metal to be bound. In general, useful heteroatoms for use as coordination atoms include nitrogen, oxygen and sulfur.

As will be appreciated by those in the art, a wide variety of suitable metal binding moieties can be used. The metal binding moieties can be macrocyclic or non-macrocyclic in structure. "Macrocyclic" in this context includes at least 12 atoms in a cyclic structure, frequently containing heteroatoms, binding of a metal in the interior of the cycle and generally planar.

In many embodiments, the metal binding moieties are not macrocyclic, but can contain cyclic structures.

Generally, in some embodiments, the metal binding moiety is selected from the group consisting of a sulfonyl moiety, a carbonyl moiety, a boronic acid or boronic ester moiety, a sulfur-containing moiety, a nitrogen-containing moiety, a phosphorous-containing moiety, a 5-membered heteroaromatic ring having one heteroatom, a 5-membered aromatic ring having two heteroatoms, a 5-membered heteroaromatic ring having three heteroatoms, a 5-membered heteroaromatic ring having four or five heteroatoms, a 5-membered saturated or partially unsaturated heteroalkyl ring having one heteroatom, a 5-membered saturated or partially unsaturated heteroalkyl ring having two heteroatoms, a six-membered aromatic ring, a 6-membered heteroaromatic ring having one heteroatom, a 6-membered aromatic ring having two heteroatoms, a 6-membered heteroaromatic ring having three or four heteroatoms, a 6-membered unsaturated or partially saturated heteroalkyl ring having one heteroatom, and a 6-membered unsaturated or partially saturated heteroalkyl ring having two heteroatoms; wherein, in some embodiments, the metal binding moiety is not a metal binding moiety selected from the group consisting of: 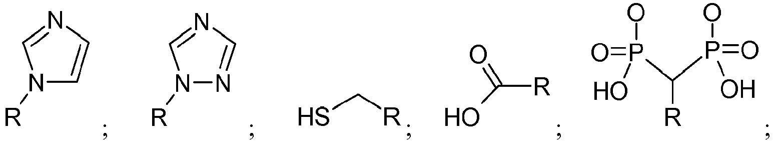 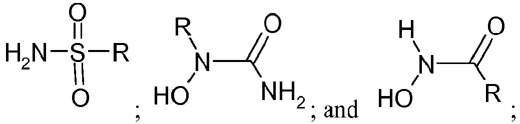 wherein: R is an attachment site through which the metal binding moiety can be attached to the targeting moiety, optionally through a linker, Lₙ.

One class of suitable metal binding moieties includes five-membered ring structures having at least one heteroatom and can be aromatic or non-aromatic. Subclasses of this class include, but are not limited to, five membered rings having one heteroatom (51HA), including five-membered aromatic rings having one heteroatom (5A1HA) and five-membered non-aromatic rings having one heteroatom (5NA1HA); five-membered rings having two heteroatoms (again, either aromatic or not: 5A2HA and 5NA2HA); five-membered rings having three heteroatoms (either aromatic or not, 5A3HA and 5NA3HA) and five-membered aromatic rings having four or five heteroatoms (5A4HA, 5A5HA). As outlined above, each class or subclass can include or exclude any member of the class or subclass individually. Additionally, each heteroatom can be included or excluded independently and individually as well; for example, the five membered aromatic rings having one heteroatom can exclude nitrogen as the heteroatom.

Another class of suitable metal binding moieties includes six-membered ring structures having none or at least one heteroatom that can be aromatic or non-aromatic. Subclasses of this class include, but are not limited to, six-membered aromatic rings having no heteroatoms (6A), six-membered rings having one heteroatom (61HA), including six-membered aromatic rings having one heteroatom (6A1HA) and six-membered non-aromatic rings having one heteroatom (6NA1HA); six membered rings having two heteroatoms (again, either aromatic or not: 6A2HA and 6NA2HA); six-membered rings having three or four heteroatoms (either aromatic or not, 6A3HA and 6NA3HA or 6A4HA and 6NA4HA). As outlined above, each class or subclass can include or exclude any member of the class or subclass individually. Additionally, as for the five-membered ring structures, each heteroatom can be included or excluded independently, as well.

It should be noted that in embodiments where adjacent substitution groups form a cyclic structure, the actual metal binding moiety can be based on a 5- or 6-membered ring, and can include additional ring structures.

Figures 1A-1AH depict representative sulfonyl-based metal binding moieties including, but not limited to, sulfonic acid, sulfonamide, thiosulfonic acid, sulfonyl hydrazine, sulfonyl hydroxylamine, N-methoxy-sulfonamide, N-methyl-sulfonamide, N-acetyl-sulfonyl hydrazide, N-aminocarbonyl-sulfonyl hydrazide, N-aminothiocarbonyl-sulfonyl hydrazide, N-cyano-sulfonamide, cyanomethyl-sulfone, N-acetyl sulfonamide, N-aminocarbonyl-sulfonamide, N-aminothiocarbonyl-sulfonamide, N-amidino-sulfonamide, α-thioacetamido-sulfone, α-acetamido-sulfone, α-sulfonylmethyl-phosphonic acid, α-cyanomethyl-sulfonamide, α-acetamido-sulfonamide, N-hydroxyamidino-sulfonamide, N-α-acetoxy-sulfonamide, N-sulfonyl-imidazole, N-sulfonyl-pyrazole, N-sulfonyl-1-(1,2,4-triazole), N-sulfonyl-1-(1,2,3-triazole), N-sulfonyl-pyrrolidin-2-one, N-sulfonyl-imidazolinone, N-sulfonyl-hydantoin, N-sulfonyl-pyrrolidin-2,3-dione, N-sulfonyl-piperazine, N-sulfonyl-morpholine, and N-sulfonyl-thiomorpholine.

In some embodiments, the sulfonyl moiety has the following general formula 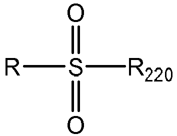 wherein: R is an attachment site through which the metal binding moiety can be attached to a targeting moiety, optionally through a linker, Lₙ, wherein n can be 0 or 1; and R₂₂₀ is selected from the group consisting of -OH, -SH, -NHNH₂, -NHOH, - NHOCH₃, -NHN(CH₃)₂, -NHNHC(=O)CH₃, -NHNHC(=O)NH₂, -NHNHC(=S)NH₂, - NHC≡N, -CH₂C≡N, -NHC(=O)CH₃, -NHC(=O)NH₂, -NHC(=S)NH₂,-NHC(=NH)NH₂, -CH₂C(=S)NH₂, -CH₂C(=O)NH₂, -CH₂P(=O)(OH)₂, -NHCH₂C≡N, - NHCH₂C(=O)-NH₂, -NHCH₂C(-NOH)-NH₂, -NHOCH₂C(=O)OH, 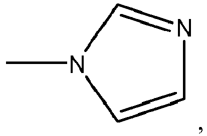 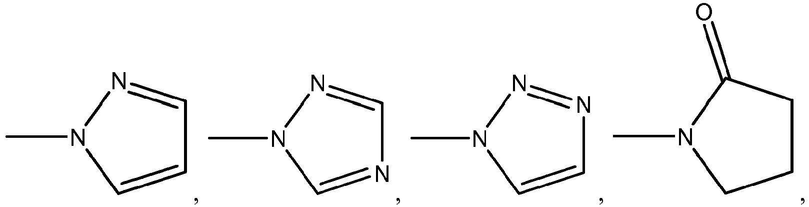 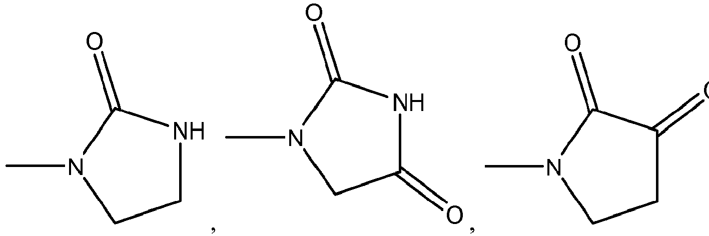 and 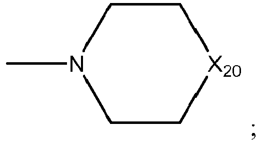 wherein X₂₀ is NH, O, or S.

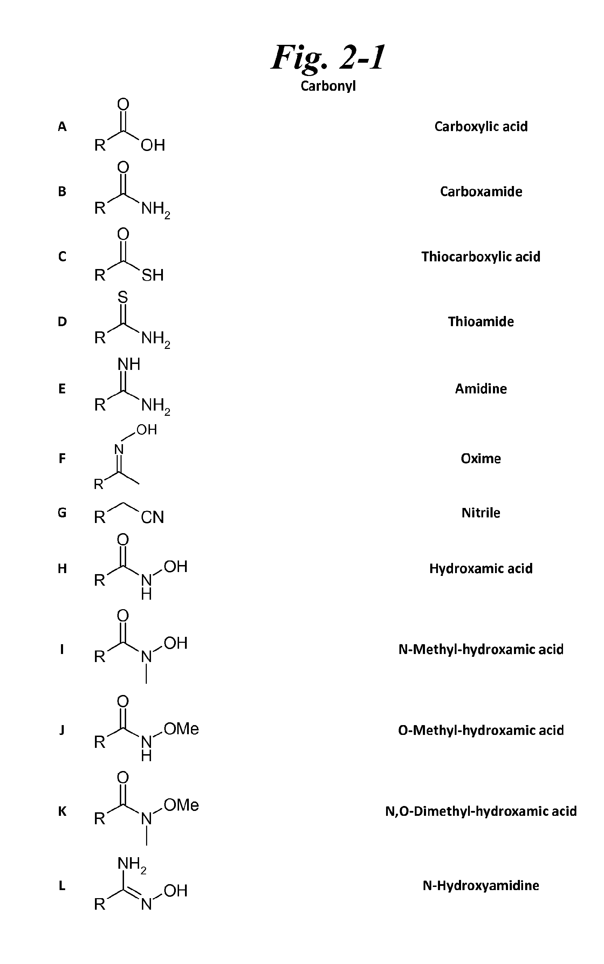

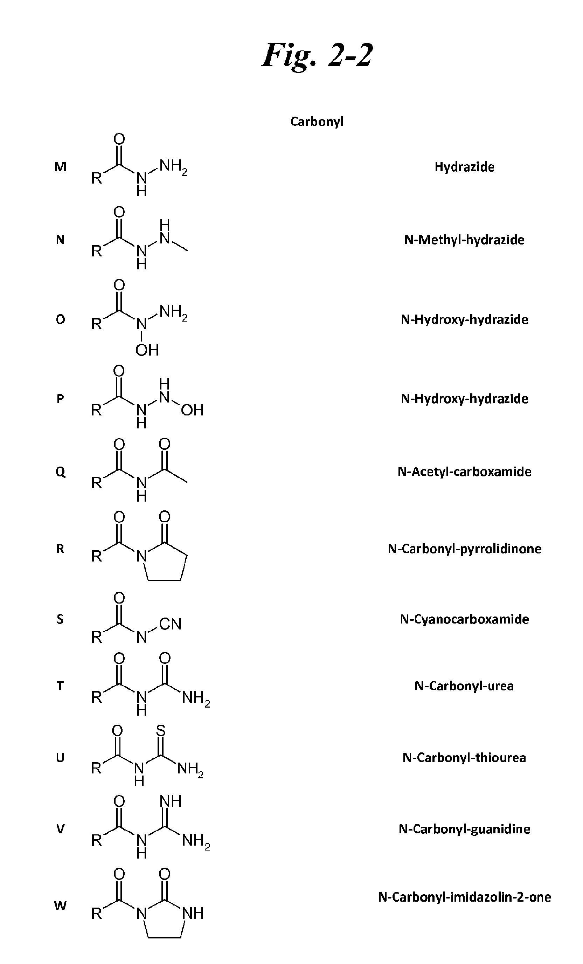

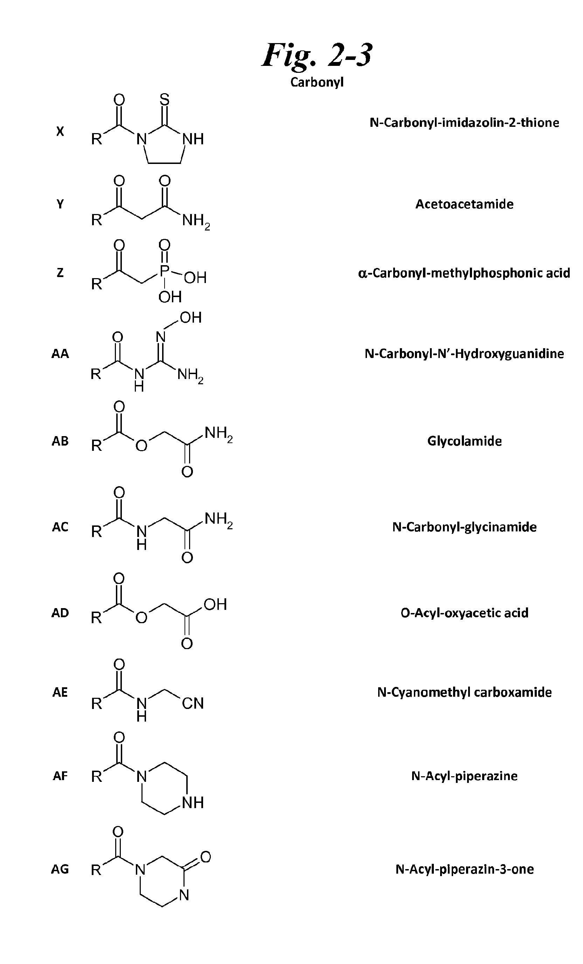

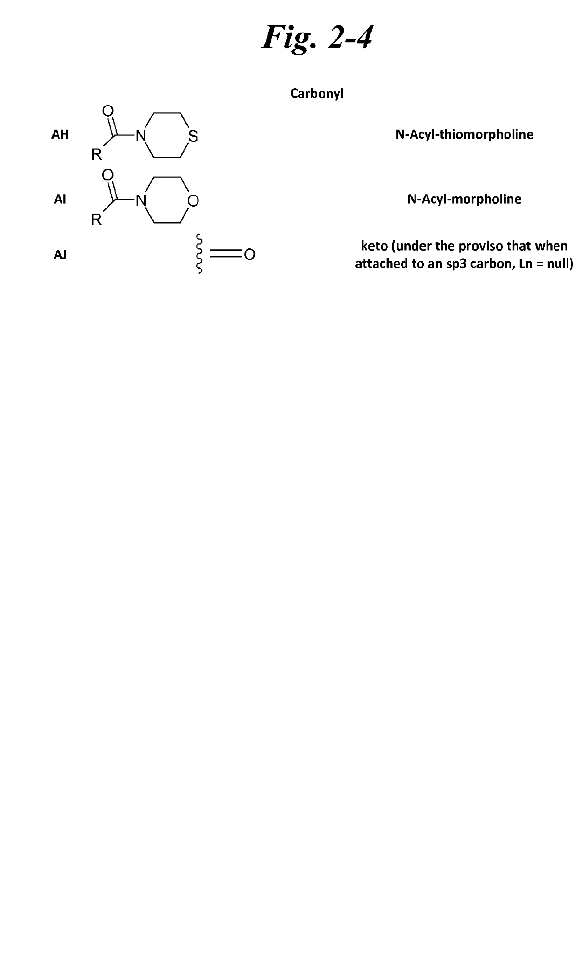

Figures 2A-2AJ depict representative carbonyl-based metal binding moieties including, but not limited to, carboxylic acid, carboxmide, thiocarboxylic acid, thioamide, amidine, oxime, nitrile, hydroxamic acid, N-methyl-hydroxamic acid, O-methyl-hydroxamic acid, N,O-dimethyl-hydroxamic acid, N-hydroxyamidine, hydrazide, N-methyl-hydrazide, N-hydroxy-hydrazide, N-hydroxy-hydrazide, N-acetyl-carboxamide, N-carbonyl-pyrrolidinone, N-cyanocarboxamide, N-carbonyl-urea, N-carbonyl-thiourea, N-carbonyl-guanidine, N-carbonyl-imidazolin-2-one, N-carbonyl-imidazolin-2-thione, acetoacetamide, α-carbonyl-methylphosphonic acid, N-carbonyl-N'-hydroxyguanidine, glycolamide, N-carbonyl-glycinamide, O-acyl-oxyacetic acid, N-cyanomethyl carboxamide, N-acyl-piperazine, N-acyl-piperazin-3-one, N-acyl-thiomorpholine, N-acyl-morpholine, and a ketone moiety, under the proviso that if the metal binding moiety is a ketone, as depicted in Figure 2-AJ, Lₙ is null with the ketone is attached to an sp3 carbon atom.

In some embodiments, the carbonyl moiety has the following general formula: 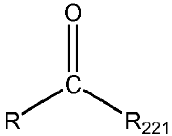 wherein: R is an attachment site through which the metal binding moiety can be attached to a targeting moiety, optionally through a linker, Lₙ, wherein n can be 0 or 1; and R₂₂₁ is selected from the group consisting of -NH₂, -SH, -NHNH₂, -N(OH)NH₂, - NHOH, -NCH₃OH, -NHOCH₃, -NCH₃OCH₃, -NHNHCH₃, -NHNHOH, - NHNHC(=O)CH₃, -NHNHC(=O)NH₂, -NHNHC(=S)NH₂, -NHC≡N, -NHC(=NH)NH₂, -CH₂C(=O)NH₂, -CH₂P(=O)(OH)₂, -NHCH₂C≡N, -NHCH₂C(=O)-NH₂, - NHC(=NOH)-NH₂, -OCH₂C(=O)-NH₂, -OCH₂C(=O)-OH, 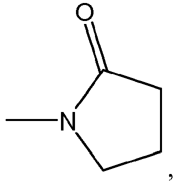 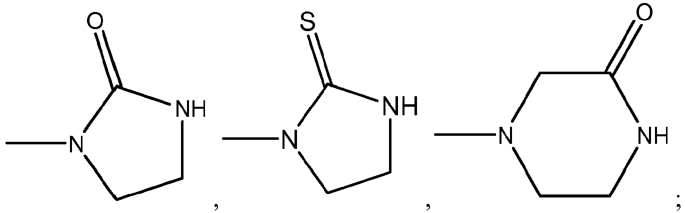 and 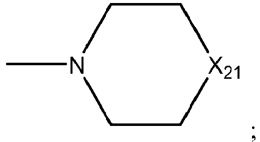 wherein X₂₁ is NH, S, or O; or R₂₂₁ is selected from the group consisting of:

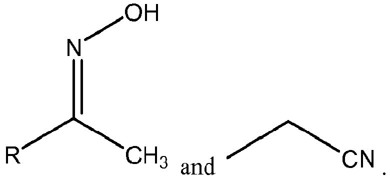

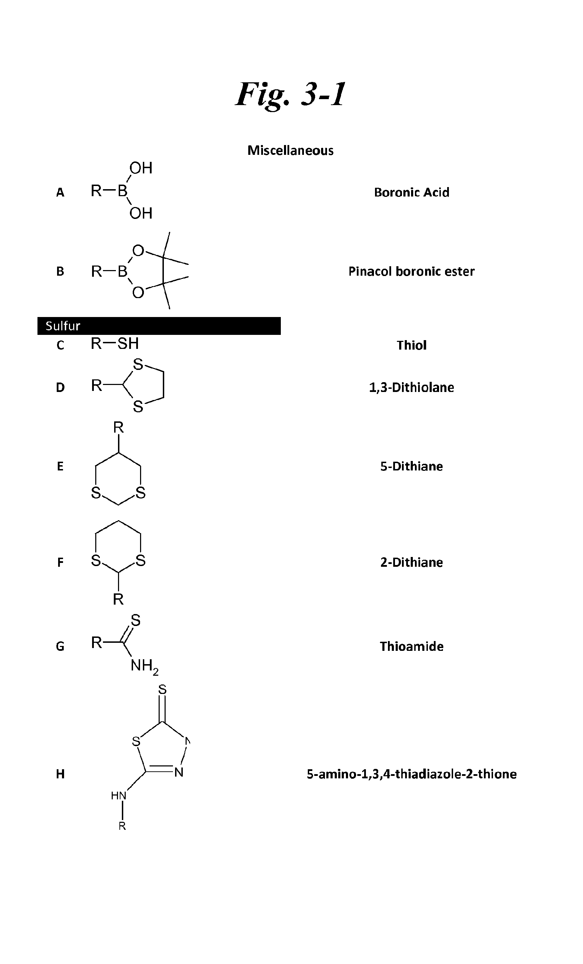

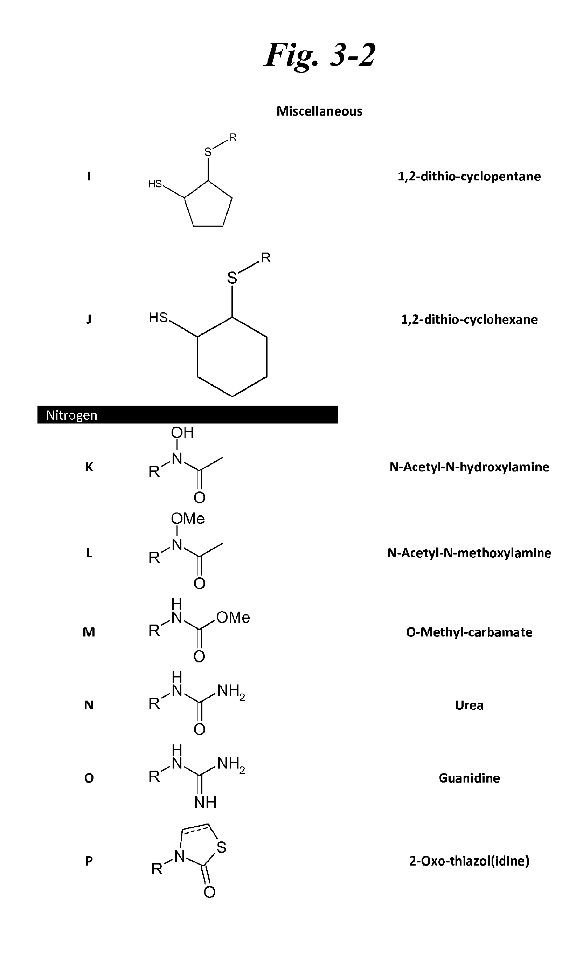

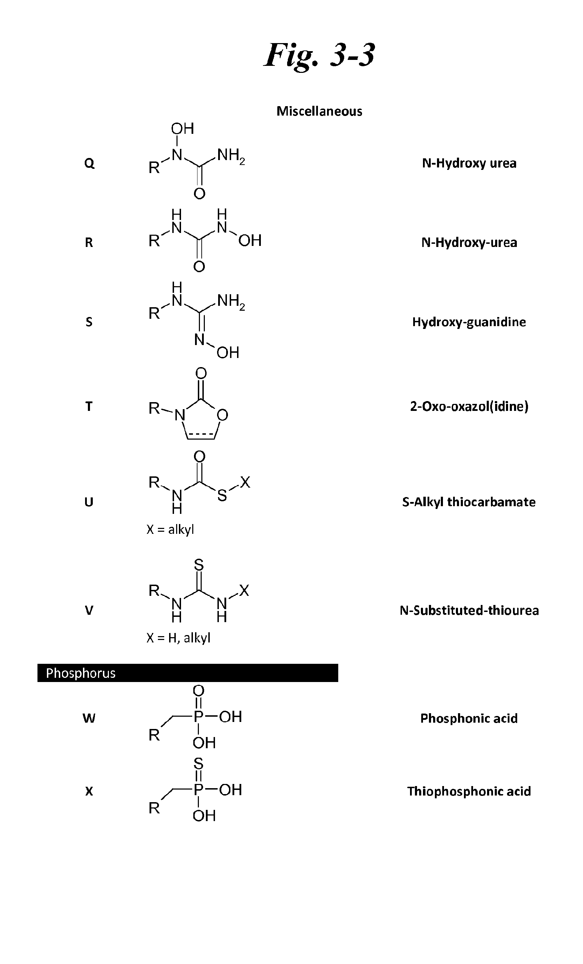

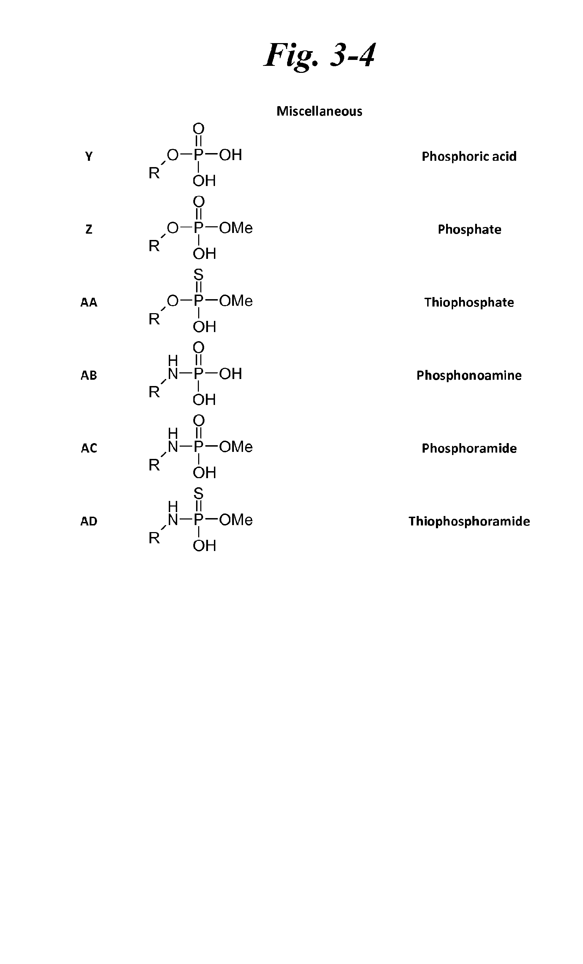

Figures 3A and 3B depict representative boron-based metal binding moieties including, but not limited to, boronic acid and pinacol boronic ester.

In some embodiments, the boronic acid or boronic ester moiety is selected from the group consisting of: 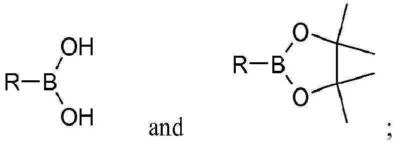 wherein: R is an attachment site through which the metal binding moiety can be attached to a targeting moiety, optionally through a linker, Lₙ, wherein n can be 0 or 1.

Figures 3C-3J depict representative sulfur-based metal binding moieties including, but not limited to, thiol, 1,3-dithiolane, 5-dithiane, 2-dithiane, thioamide, 5-amino-1,3,4-thiadiazole-2-thione, 1,2-dithio-cyclopentane, and 1,2-dithio-cyclohexane. In some embodiments, the sulfur-containing moiety is selected from the group consisting of: R-SH, R-C(=S)-NH₂, 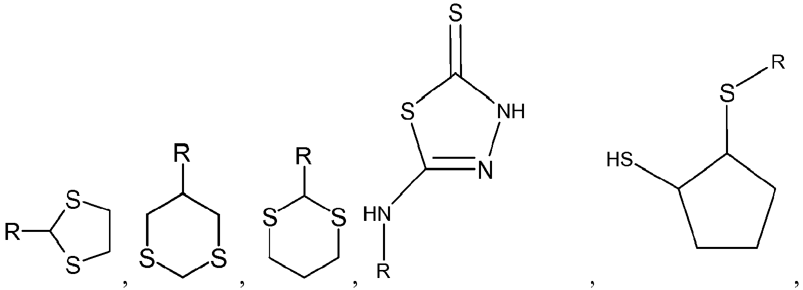 and 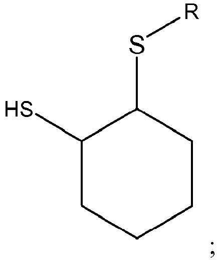 wherein: R is an attachment site through which the metal binding moiety can be attached to a targeting moiety, optionally through a linker, Lₙ, wherein n can be 0 or 1.

Figures 3K-3V depict representative nitrogen-based metal binding moieties including, but not limited to, N-acetyl-N-hydroxylamine, N-acetyl-N-methoxylamine, O-methyl-carbamate, urea, guanidine, 2-oxo-thiazol(idine), N-hydroxy urea, N-hydroxy-urea, hydroxy-guanidine, 2-oxo-oxazol(idine), S-alkyl thiocarbamate, and N-substituted-thiourea. In some embodiments, the nitrogen-containing moiety has the following formula: 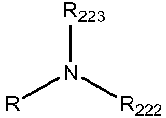 wherein: R is an attachment site through which the metal binding moiety can be attached to a targeting moiety, optionally through a linker, Lₙ, wherein n can be 0 or 1; and R₂₂₂ is selected from the group consisting of: -C(=O)-CH₃, -C(=O)-OCH₃, -C(=O)-NH₂, -C(=NH)-NH₂, -C(=O)-NHOH, -C(=NOH)-NH₂, -C(=O)-S-R₂₂₄, wherein R₂₂₄ is H or alkyl, -C(=S)-NH-R₂₂₅, wherein R₂₂₅ is H or alkyl, and; R₂₂₃ is selected from the group consisting of: -H, -OH, and -OCH₃;provided that when R₂₂₃ is OH, R₂₂₂ is not -C(=O)-NH₂ or -C(=O)-CH₃; or R₂₂₃ and R₂₂₂ together combine to form: 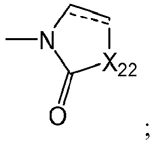 wherein: X₂₂ is S or O; and a dashed line indicates that a bond can be present or absent.

Figures 3W-3AD depict representative phosphorous-based metal binding moieties including, but not limited to, phosphonic acid, thiophosphonic acid, phosphoric acid, phosphate, thiophosphate, phosphonoamine, phosphoramide, and thiophosphoramide. In some embodiments, the phosphorous-containing moiety has the following formula:

R-R₂₂₆-P(=X₂₃)(OH)(R₂₂₇)

wherein: R is an attachment site through which the metal binding moiety can be attached to a targeting moiety, optionally through a linker, Lₙ, wherein n can be 0 or 1; and X₂₃ is O or S; R₂₂₆ is selected from the group consisting of -CH₂-, -O-, and -NH-; and R₂₂₇ is selected from the group consisting of -OH and -OCH₃.

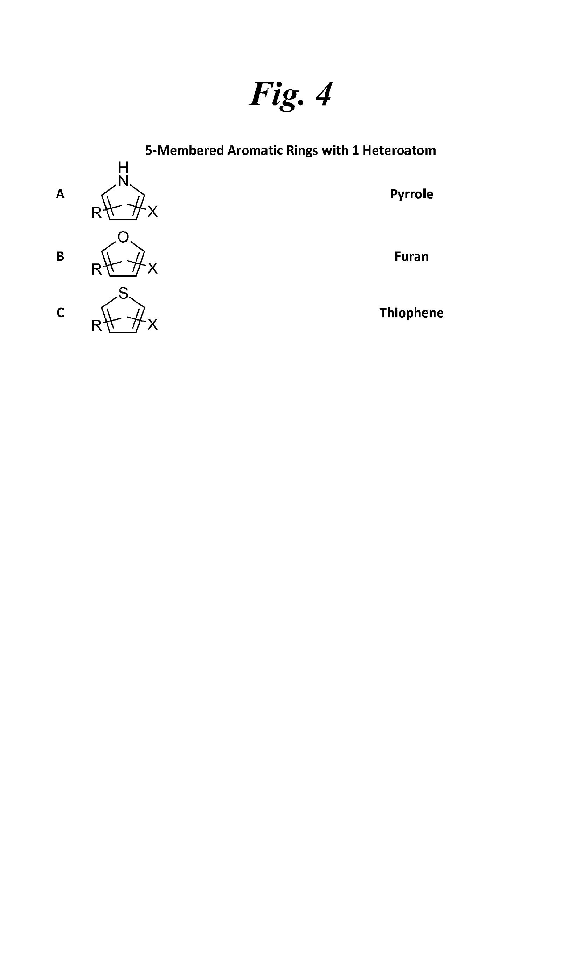

Figures 4A-4C depict representative 5-membered aromatic rings having one heteroatom including, but not limited to, pyrrole, furan, and thiophene. In some embodiments, the 5-membered heteroaromatic ring having one heteroatom has the following formula: 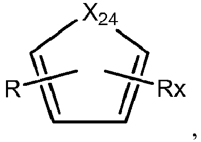 wherein: X₂₄ is selected from the group consisting of NH, O, and S; R is an attachment site through which the metal binding moiety can be attached to a targeting moiety, optionally through a linker, Lₙ, wherein n can be 0 or 1; and Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol.

In some embodiments, the heteroatom in the 5-membered aromatic ring having one heteroatom is not oxygen.

In some embodiments, the heteroatom in the 5-membered aromatic ring having one heteroatom is not nitrogen.

In some embodiments, the heteroatom in the 5-membered aromatic ring having one heteroatom is not sulfur.

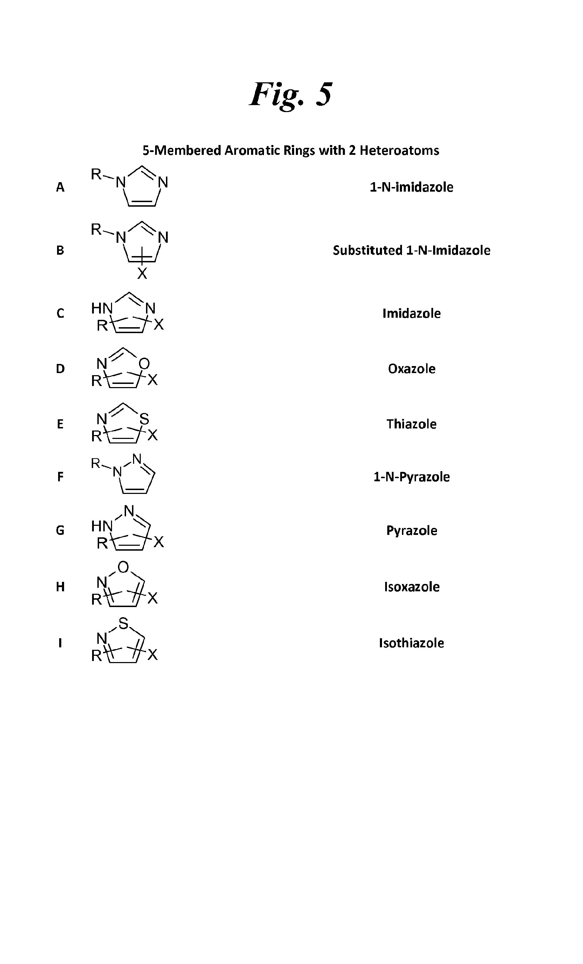

Figures 5A-5I depict representative 5-membered aromatic rings having two heteroatoms including, but not limited to, 1-N-imidazole, substituted 1-N-imidazole, imidazole, oxazole, thiazole, 1-N-pyrazole, pyrazole, isoxazole, and isothiazole.

In some embodiments, the 5-membered aromatic ring having two heteroatoms is selected from the group consisting of: 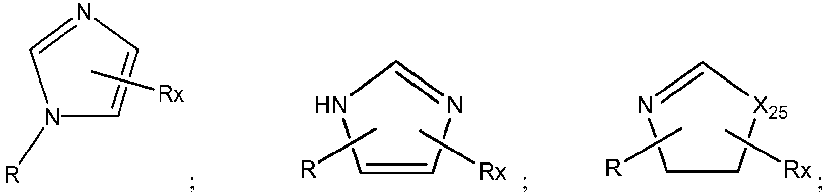 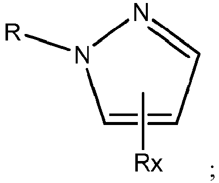 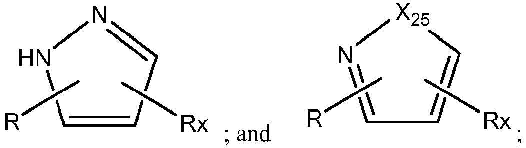 wherein: each X₂₅ is O or S; and R is an attachment site through which the metal binding moiety can be attached to a targeting moiety, optionally through a linker, Lₙ, wherein n can be 0 or 1; and Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol; under the proviso that the metal binding moiety is not:

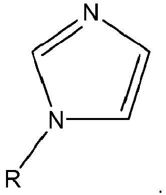

In some embodiments, one of the heteroatoms in the 5-membered aromatic ring having two heteroatoms is not oxygen.

In some embodiments, neither heteroatom in the 5-membered aromatic ring having two heteroatoms is oxygen.

In some embodiments, one of the heteroatoms in the 5-membered aromatic ring having two heteroatoms is not nitrogen.

In some embodiments, neither heteroatom in the 5-membered aromatic ring having two heteroatoms is nitrogen.

In some embodiments, one of the heteroatoms in the 5-membered aromatic ring having two heteroatoms is not sulfur.

In some embodiments, neither heteroatom in the 5-membered aromatic rings having two heteroatoms is sulfur.

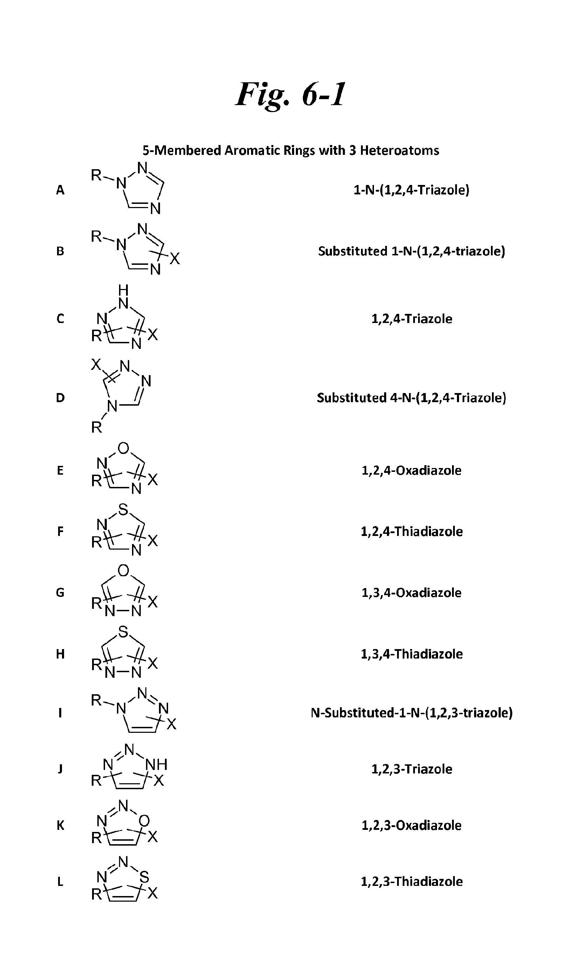

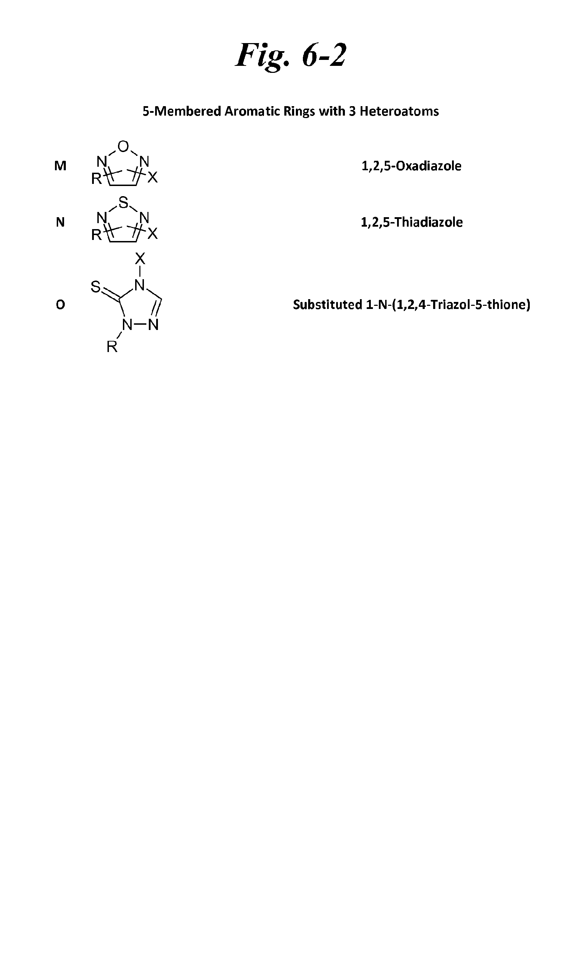

Figures 6A-60 depict representative 5-membered aromatic rings having three heteroatoms including, but not limited to, 1-N-(1,2,4-triazole), substituted 1-N-(1,2,4-triazole), 1,2,4-triazole, substituted 4-N-(1,2,4-triazole), 1,2,4-oxadiazole, 1,2,4-thiadiazole, 1,3,4-oxadiazole, 1,3,4-thiadiazole, N-substituted-1-N-(1,2,3-triazole), 1,2,3-triazole, 1,2,3-oxadiazole, 1,2,3-thiadiazole, 1,2,5-oxadiazole, 1,2,5-thiadiazole, and substituted 1-N-(1,2,4-triazol-5-thione).

In some embodiments, the 5-membered heteroaromatic ring having three heteroatoms is selected from the group consisting of: 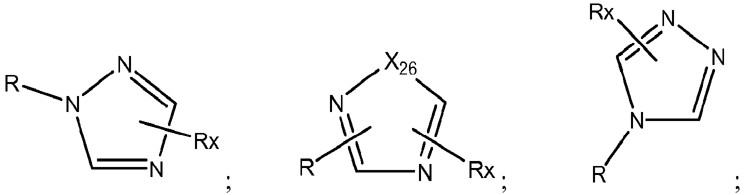 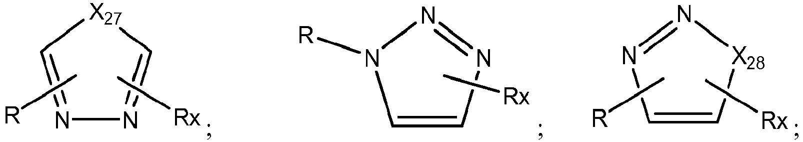 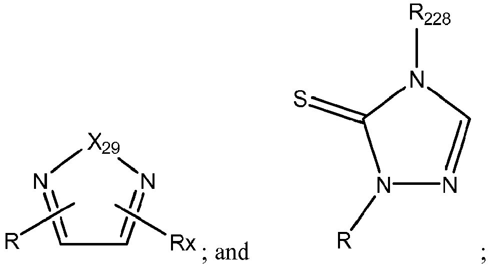 wherein: X₂₆ and X₂₈ are each independently selected from the group consisting of NH, O, and S; X₂₇ and X₂₉ are each independently O or S; R is an attachment site through which the metal binding moiety can be attached to a targeting moiety, optionally through a linker, Lₙ, wherein n can be 0 or 1; and Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol; and R₂₂₈ is H or alkyl; under the proviso that the metal binding moiety is not:

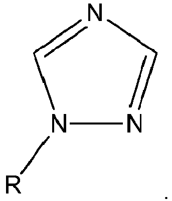

In some embodiments, one of the heteroatoms in the 5-membered aromatic ring having three heteroatoms is not oxygen.

In some embodiments, two of the heteroatoms in the 5-membered aromatic ring having three heteroatoms are not oxygen.

In some embodiments, none of the heteroatoms in the 5-membered aromatic ring having three heteroatoms is oxygen.

In some embodiments, one of the heteroatoms in the 5-membered aromatic ring having three heteroatoms is not nitrogen.

In some embodiments, two of the heteroatoms in the 5-membered aromatic ring having three heteroatoms are not nitrogen.

In some embodiments, none of the heteroatoms in the 5-membered aromatic ring having three heteroatoms is nitrogen.

In some embodiments, one of the heteroatoms in the 5-membered aromatic ring having three heteroatoms is not sulfur.

In some embodiments, two of the heteroatoms in the 5-membered aromatic ring having three heteroatoms are not sulfur.

In some embodiments, none of the heteroatoms in the 5-membered aromatic ring having three heteroatoms is not sulfur.

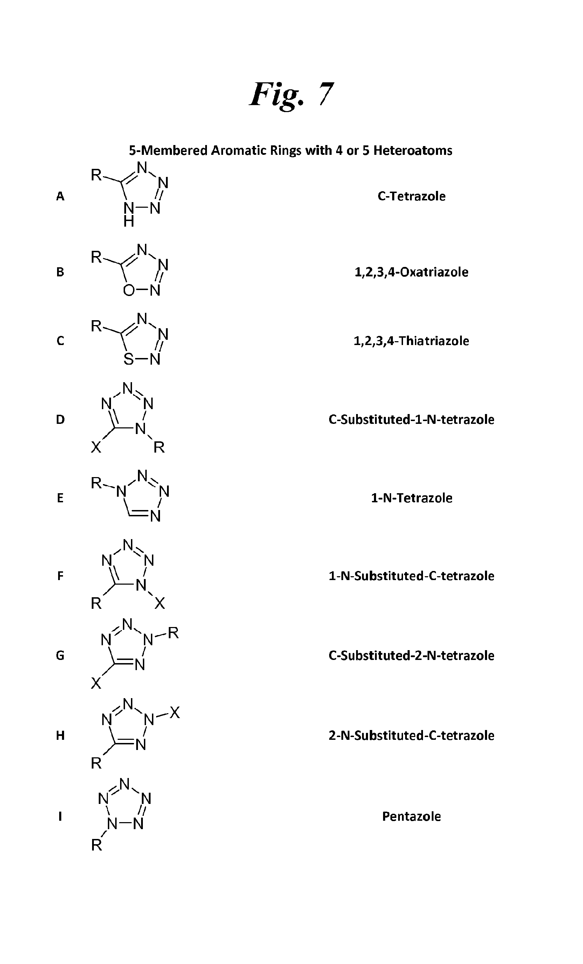

Figures 7A-7I depict representative 5-membered aromatic rings having four or five heteroatoms including, but not limited to, C-tetrazole, 1,2,3,4-oxatriazole, 1,2,3,4-thiatriazole, C-substituted-1-N-tetrazole, 1-N-tetrazole, 1-N-substituted-C-tetrazole, C-substituted-2-N-tetrazole, 2-N-substituted-C-tetrazole, and pentazole.

In some embodiments, the 5-membered heteroaromatic ring having four or five heteroatoms is selected from the group consisting of: 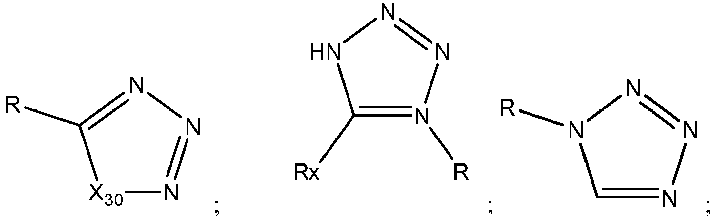 and 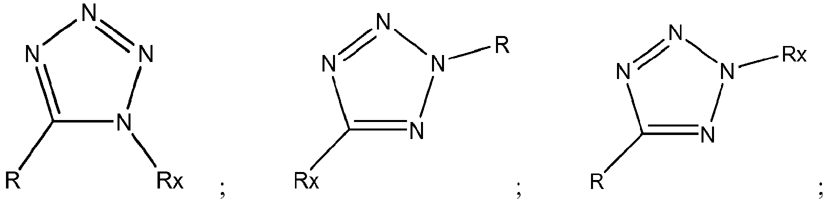 and 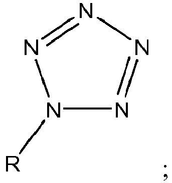 wherein: X₃₀ is selected from the group consisting of NH, O, and S; R is an attachment site through which the metal binding moiety can be attached to a targeting moiety, optionally through a linker, Lₙ, wherein n can be 0 or 1; and Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol.

In some embodiments, one of the heteroatoms in the 5-membered aromatic ring having four or five heteroatoms is not oxygen.

In some embodiments, two of the heteroatoms in the 5-membered aromatic ring having four or five heteroatoms are not oxygen.

In some embodiments, three of the heteroatoms in the 5-membered aromatic ring having four or five heteroatoms are not oxygen.

In some embodiments, none of the heteroatoms in the 5-membered aromatic ring having four or five heteroatoms is oxygen.

In some embodiments, one of the heteroatoms in the 5-membered aromatic ring having four or five heteroatoms is not nitrogen.

In some embodiments, two of the heteroatoms in the 5-membered aromatic ring having four or five heteroatoms are not nitrogen.

In some embodiments, three of the heteroatoms in the 5-membered aromatic ring having four or five heteroatoms are not nitrogen.

In some embodiments, none of the heteroatoms in the 5-membered aromatic ring having four or five heteroatoms is nitrogen.

In some embodiments, one of the heteroatoms in the 5-membered aromatic ring having four or five heteroatoms is not sulfur.

In some embodiments, two of the heteroatoms in the 5-membered aromatic ring having four or five heteroatoms are not sulfur.

In some embodiments, three of the heteroatoms in the 5-membered aromatic ring having four or five heteroatoms are not sulfur.

In some embodiments, none of the heteroatoms in the 5-membered aromatic ring having four or five heteroatoms is sulfur.

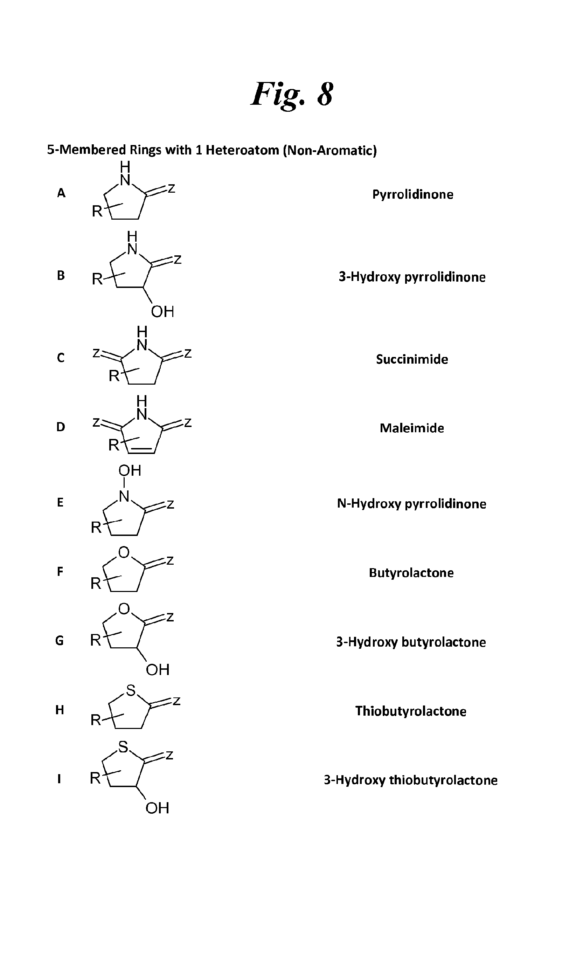

Figures 8A-8I depict representative 5-membered non-aromatic rings having one heteroatom including, but not limited to, pyrrolidinone, 3-hydroxy pyrrolidinone, succinimide, maleimide, N-hydroxy pyrrolidinone, butyrolactone, 3-hydroxy butyrolactone, thiobutyrolactone, and 3-hydroxy thiobutyrolactone.

In some embodiments, the 5-membered saturated or partially unsaturated heteroalkyl ring having one heteroatom is selected from the group consisting of: 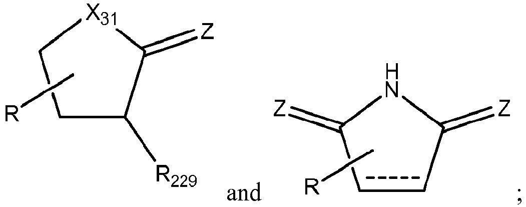 wherein: X₃₁ is selected from the group consisting of NH, NOH, O, and S; and each Z is independently selected from the group consisting of O, S, and NR₂₃₀, wherein R₂₃₀ is H or alkyl; R is an attachment site through which the metal binding moiety can be attached to a targeting moiety, optionally through a linker, Lₙ, wherein n can be 0 or 1; and R₂₂₉ is selected from the group consisting of H and OH; and wherein a dashed line indicates that a bond can be present or absent.

In some embodiments, the heteroatom in the 5-membered non-aromatic ring having one heteroatom is not oxygen.

In some embodiments, the heteroatom in the 5-membered non-aromatic ring having one heteroatom is not nitrogen.

In some embodiments, the heteroatom in the 5-membered non-aromatic ring having one heteroatom is not sulfur.

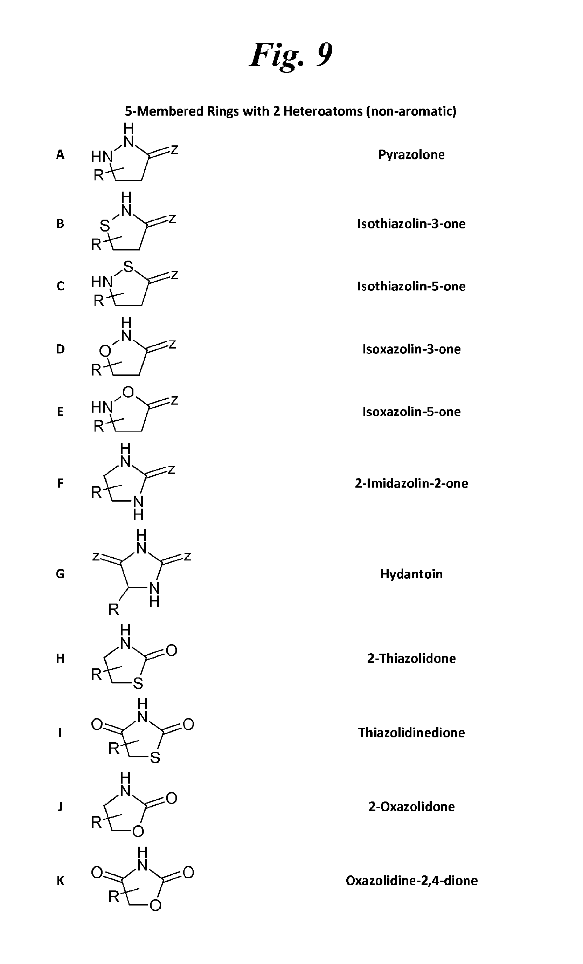

Figures 9A-9K depict representative 5-membered non-aromatic rings having two heteroatoms including, but not limited to, pyrazolone, isothiazolin-3-one, isothiazolin-5-one, isoxazolin-3-one, isoxazolin-5-one, 2-imidazolin-2-one, hydantoin, 2-thiazolidone, thiazolidinedione, 2-oxazolidone, and oxazolidine-2,4-dione.

In some embodiments, the 5-membered saturated or partially unsaturated heteroalkyl ring having two heteroatoms is selected from the group consisting of: 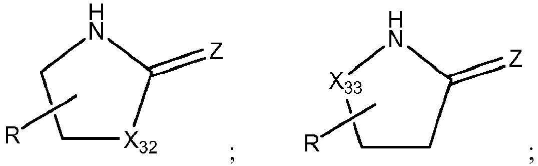 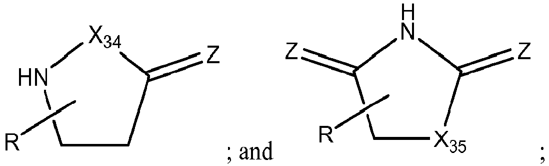 wherein: R is an attachment site through which the metal binding moiety can be attached to a targeting moiety, optionally through a linker, Lₙ, wherein n can be 0 or 1; X₃₂, X₃₃, and X₃₅ are each independently selected from the group consisting of NH, O, and S; X₃₄ is O or S; and each Z is independently selected from the group consisting of O, S, and NR₂₃₁, wherein R₂₃₁ is H or alkyl.

In some embodiments, one of the heteroatoms in the 5-membered non-aromatic ring having two heteroatoms is not oxygen.

In some embodiments, neither heteroatom in the 5-membered non-aromatic ring having two heteroatoms is oxygen.

In some embodiments, one of the heteroatoms in the 5-membered non-aromatic ring having two heteroatoms is not nitrogen.

In some embodiments, neither heteroatom in the 5-membered non-aromatic ring having two heteroatoms is nitrogen.

In some embodiments, one of the heteroatoms in the 5-membered non-aromatic ring having two heteroatoms is not sulfur.

In some embodiments, neither heteroatom in the 5-membered non-aromatic ring having two heteroatoms is sulfur.

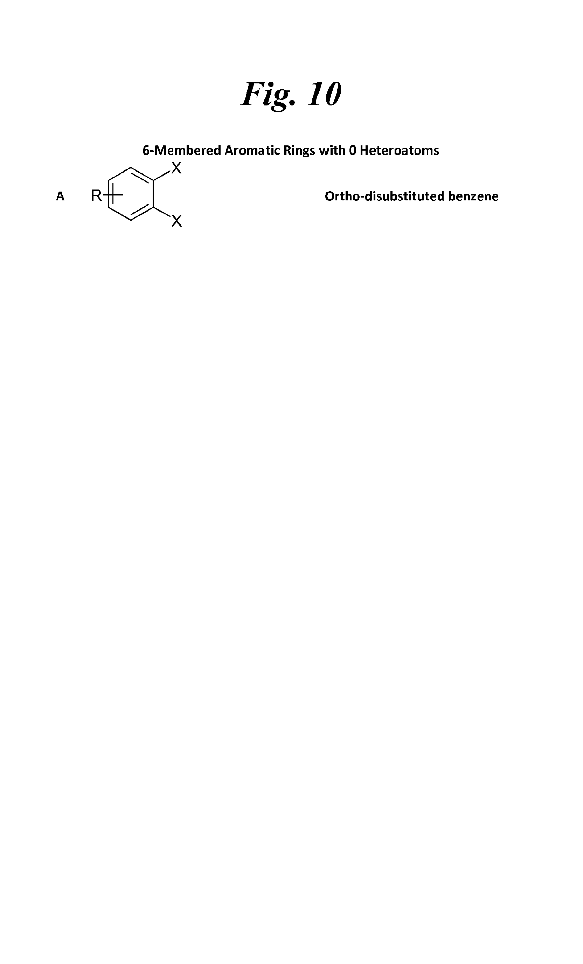

Figure 10A depicts representative 6-membered aromatic rings having no heteroatoms including, but not limited to, ortho-disubstituted benzene.

In some embodiments, the six-membered aromatic ring has the following formula: 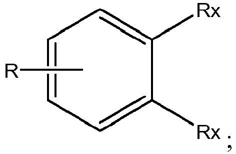 wherein: R is an attachment site through which the metal binding moiety can be attached to a targeting moiety, optionally through a linker, Lₙ, wherein n can be 0 or 1; and each Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol.

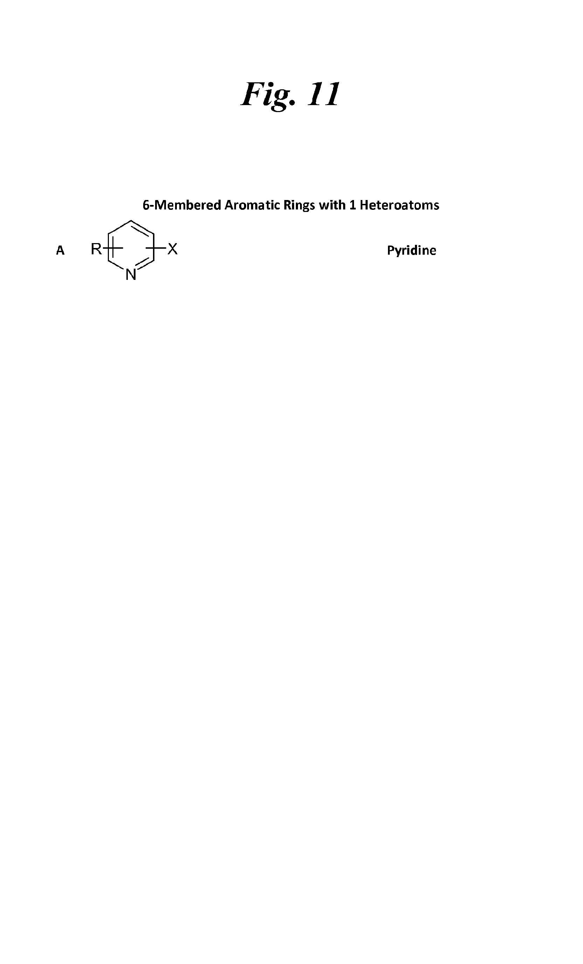

Figure 11A depicts representative 6-membered aromatic rings having one heteroatom including, but not limited to, pyridine.

In some embodiments, the 6-membered heteroaromatic ring having one heteroatom has the following formula: 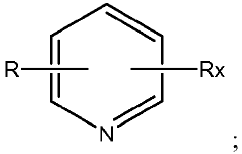 wherein: R is an attachment site through which the metal binding moiety can be attached to a targeting moiety, optionally through a linker, Lₙ, wherein n can be 0 or 1; and Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol.

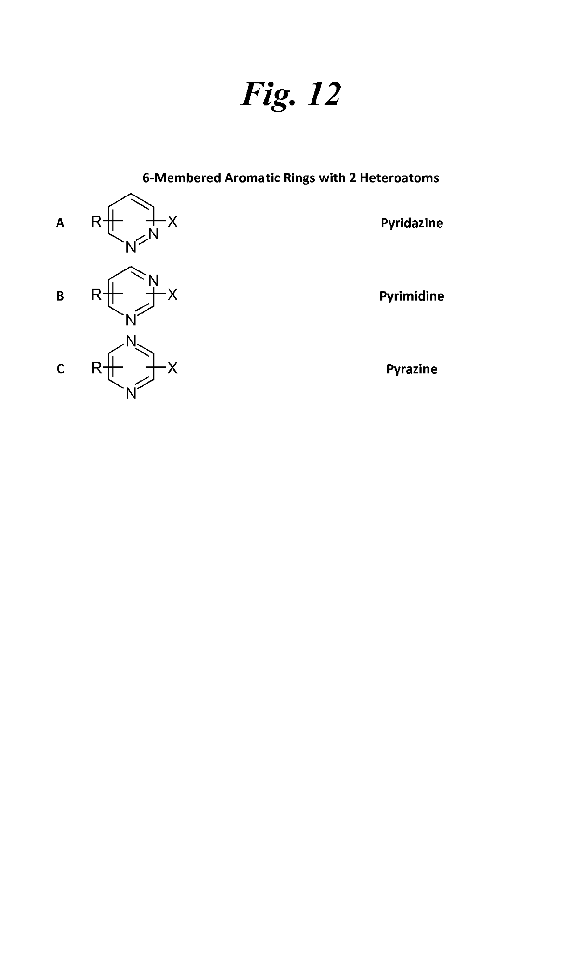

Figures 12A-12C depict representative 6-membered aromatic rings having two heteroatoms including, but not limited to, pyridazine, pyrimidine, and pyrazine.

In some embodiments, the 6-membered aromatic ring having two heteroatoms is selected from the group consisting of: 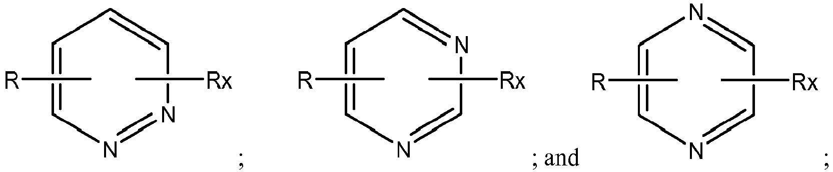 wherein: R is an attachment site through which the metal binding moiety can be attached to a targeting moiety, optionally through a linker, Lₙ, wherein n can be 0 or 1; and Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol.

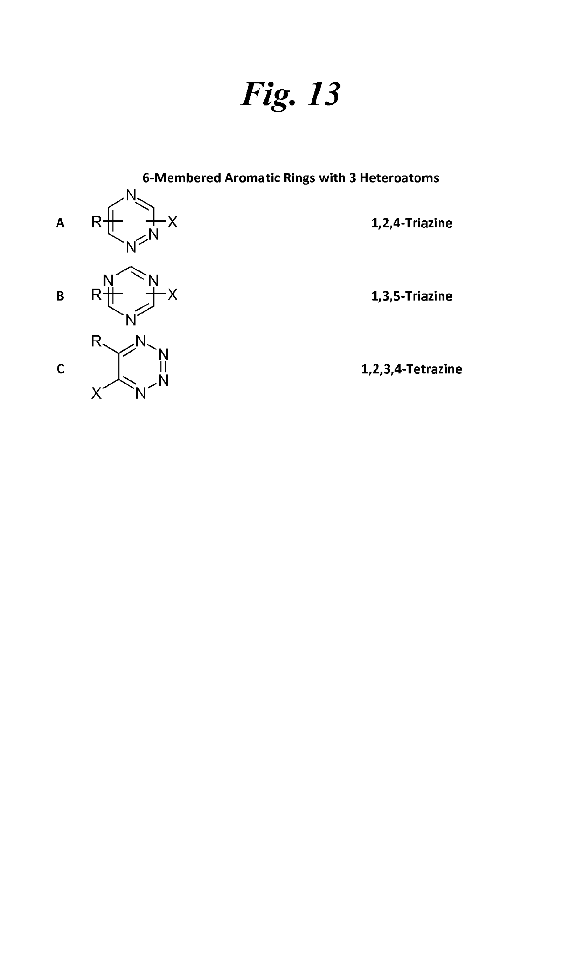

Figures 13A-13C depict representative 6-membered aromatic rings having three or four heteroatoms including, but not limited to, 1,2,4-triazine, 1,3,5-triazine, and 1,2,3,4-tetrazine.

In some embodiments, the 6-membered heteroaromatic ring having three or four heteroatoms is selected from the group consisting of: 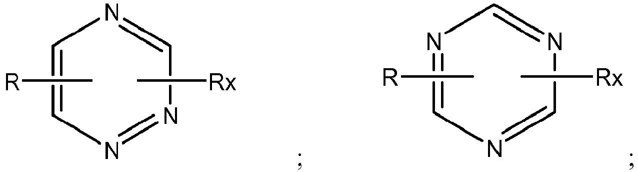 and 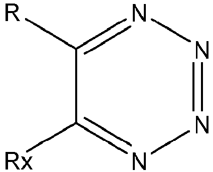 wherein: R is an attachment site through which the metal binding moiety can be attached to a targeting moiety, optionally through a linker, Lₙ, wherein n can be 0 or 1; and Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol.

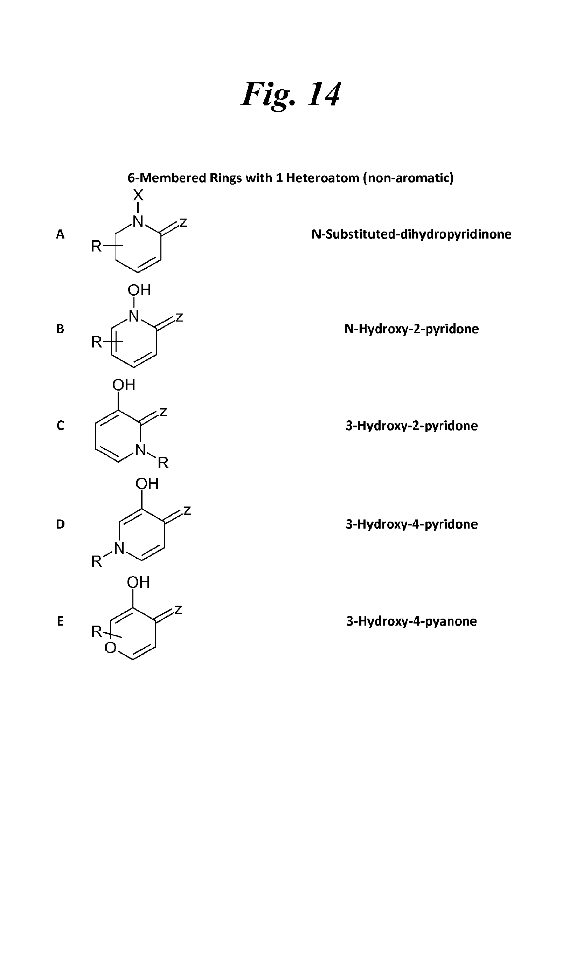

Figures 14A-14E depict representative 6-membered non-aromatic rings having one heteroatom including, but not limited to, N-substituted-dihydropyridinone, N-hydroxy-2-pyridone, 3-hydroxy-2-pyridone, 3-hydroxy-4-pyridone, and 3-hydroxy-4-pyanone.

In some embodiments, the 6-membered unsaturated or partially saturated heteroalkyl ring having one heteroatom is selected from the group consisting of: 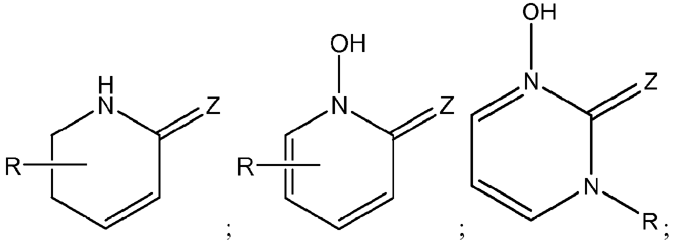 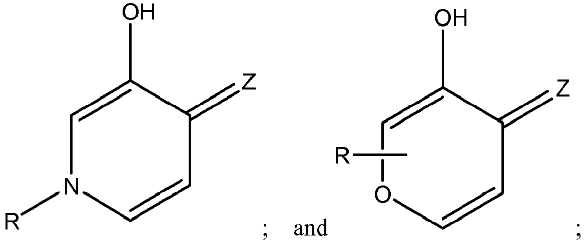 wherein: R is an attachment site through which the metal binding moiety can be attached to a targeting moiety, optionally through a linker, Lₙ, wherein n can be 0 or 1; and Z is selected from the group consisting of O, S, and NR₂₃₂, wherein R₂₃₂ is H or alkyl.

In some embodiments, the heteroatom in the 6-membered non-aromatic ring having one heteroatom is not oxygen.

In some embodiments, the heteroatom in the 6-membered non-aromatic ring having one heteroatom is not nitrogen.

In some embodiments, the heteroatom in the 6-membered non-aromatic ring having one heteroatom is not sulfur.

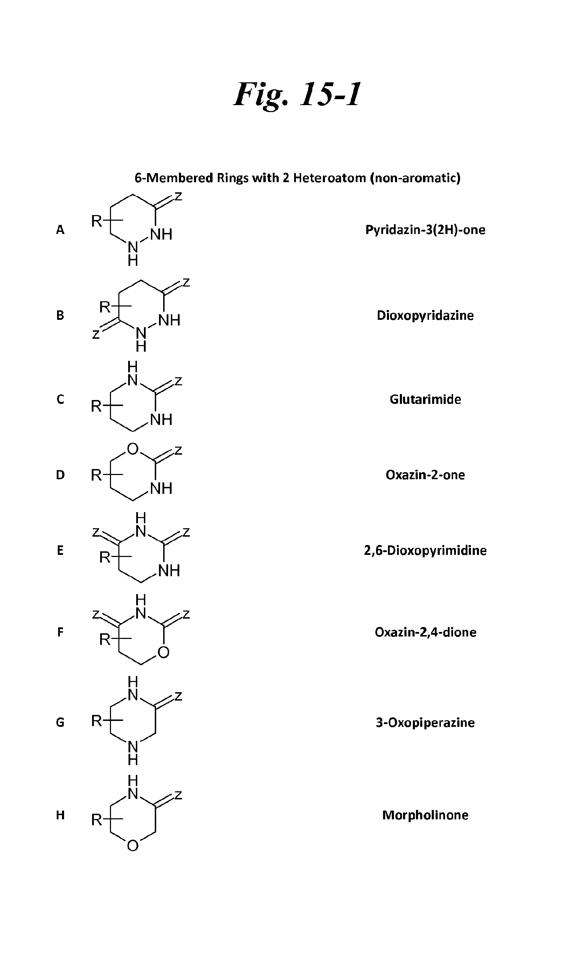

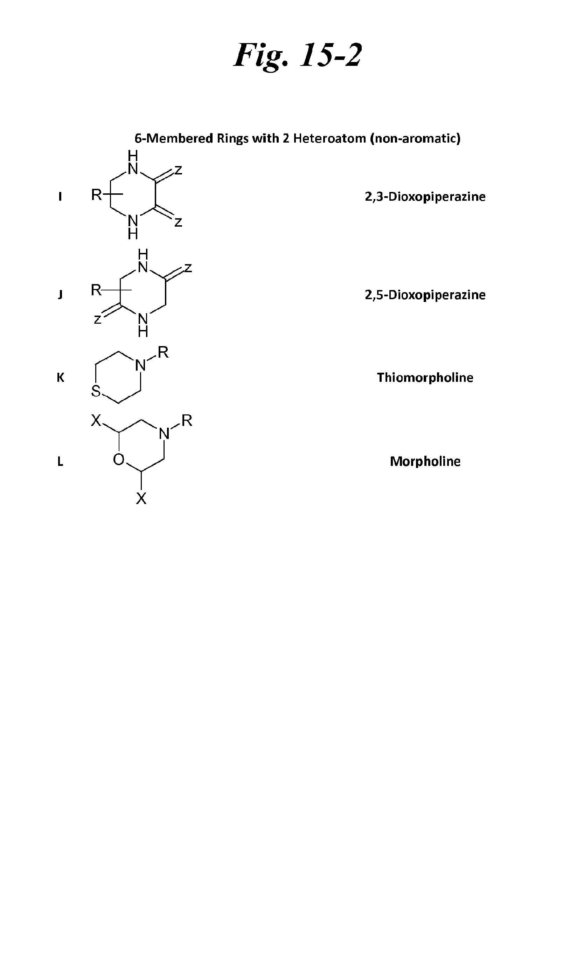

Figures 15A-15L depict representative 6-membered non-aromatic rings having two heteroatoms including, but not limited to, pyridazin-3(2H)-one, dioxopyridazine, glutarimide, oxazin-2-one, 2,6-dioxopyrimidine, oxazin-2,4-dione, 3-oxopiperazine, morpholinone, 2,3-dioxopiperazine, 2,5-dioxopiperazine, thiomorpholine, and morpholine.

In some embodiments, the 6-membered unsaturated or partially saturated heteroalkyl ring having two heteroatoms is selected from the group consisting of: 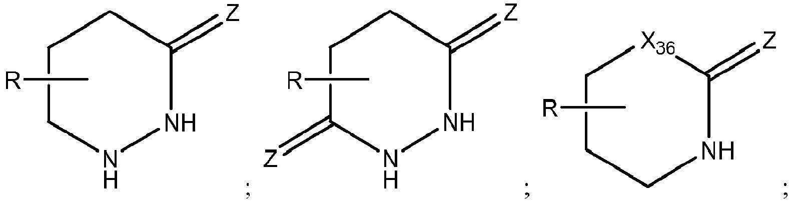 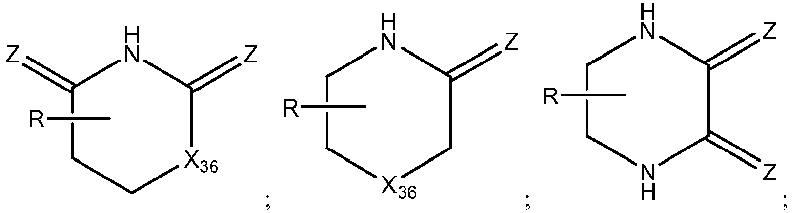 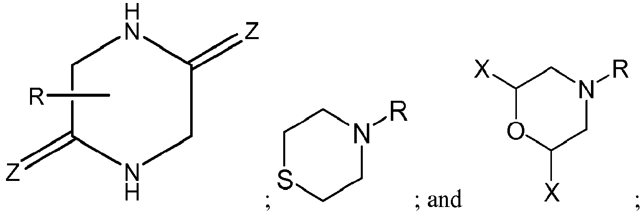 wherein: R is an attachment site through which the metal binding moiety can be attached to a targeting moiety, optionally through a linker, Lₙ, wherein n can be 0 or 1; each X₃₆ is NR₂₃₃, wherein R₂₃₃ is H or alkyl; and each Z is independently selected from the group consisting of O, S, and NR₂₃₄, wherein R₂₃₄ is H or alkyl.

In some embodiments, one of the heteroatoms in the 6-membered non-aromatic ring having two heteroatoms is not oxygen.

In some embodiments, neither heteroatom in the 6-membered non-aromatic ring having two heteroatoms is oxygen.

In some embodiments, one of the heteroatoms in the 6-membered non-aromatic ring having two heteroatoms is not nitrogen.

In some embodiments, neither heteroatom in the 6-membered non-aromatic ring having two heteroatoms is nitrogen.

In some embodiments, one of the heteroatoms in the 6-membered non-aromatic ring having two heteroatoms is not sulfur.

In some embodiments, neither heteroatom in the 6-membered non-aromatic ring having two heteroatoms is sulfur.

As shown in the Figures, the metal binding moieties have an attachment site, generally depicted as "R", which is used to attach the targeting moiety, described below, optionally using a linker, wherein the linker can be present or absent.

As depicted in the Figures, in addition to the attachment site, many of the metal binding moieties can be optionally derivatized, for example as depicted using an "X" substitution group, which also can be referred to as an "Rₓ" group as depicted in the appended claims. In some cases these X groups, or Rₓ groups, can provide additional coordination atoms. Suitable substitution groups are known in the art and include, but are not limited to, hydrogen, linkers (which can be depicted herein as "L" or "Lₙ", with n being 0 or 1) alkyl, alcohol, aromatic, amino, amido, carbonyl, carboxyl, cyano, nitro, ethers, esters, aldehydes, sulfonyl, silicon moieties, halogens, sulfur containing moieties, phosphorus containing moieties, and ethylene glycols. In the structures depicted herein, X is hydrogen when the position is unsubstituted. It should be noted that some positions can allow two substitution groups, X and X', in which case the X and X' groups can be either the same or different. Generally, in some embodiments, only a single non-hydrogen X group is attached at any particular position; that is, preferably at least one of the X groups at each position is not hydrogen. Thus, if X is an alkyl or aryl group, there is generally an additional hydrogen attached to the carbon, although not necessarily depicted herein. In addition, X groups on adjacent carbons can be joined to form ring structures (including heterocycles, aryl and heteroaryls), which can be further derivatized as outlined herein.

By "alkyl group" or grammatical equivalents herein is meant a straight or branched chain alkyl group, with straight chain alkyl groups being preferred. If branched, it can be branched at one or more positions, and unless specified, at any position. "Alkyl" in this context includes alkenyl and alkynyl, and any combination of single, double and triple bonds. The alkyl group can range from about 1 to about 30 carbon atoms (C₁ -C₃₀), with a preferred embodiment utilizing from about 1 to about 20 carbon atoms (C₁ -C₂₀), with about C₁ through about C₁₂ to about C₁₅ being preferred, and C₁ to C₅ being particularly preferred, although in some embodiments the alkyl group can be much larger. Also included within the definition of an alkyl group are cycloalkyl groups such as C₅ and C₆ rings, and heterocyclic rings with nitrogen, oxygen, sulfur or phosphorus, as well as cycloalkyl and heterocycloalkyl groups with unsaturated bonds. Alkyl also includes heteroalkyl, with heteroatoms of sulfur, oxygen, nitrogen, and silicone being preferred. Alkyl includes substituted alkyl groups. Thus, a substituent group referred to herein as "alkyl" also encompasses substituted alkyl groups. By "substituted alkyl group" herein is meant an alkyl group as defined herein further comprising one or more substitution moieties "X", as defined herein.

By "amino groups" or grammatical equivalents herein is meant -NH₂, -NHX and -NX₂ groups, with X being as defined herein.

By "nitro group" herein is meant an -NO₂ group.

By "sulfur containing moieties" herein is meant compounds containing sulfur atoms, including but not limited to, thia-, thio- and sulfo- compounds, thiols (-SH and - SX), and sulfides (-XSX-). By "phosphorus containing moieties" herein is meant compounds containing phosphorus, including, but not limited to, phosphines and phosphates. By "silicon containing moieties" herein is meant compounds containing silicon.

By "ether" herein is meant an -O-X group. Preferred ethers include alkoxy groups, with -O-(CH₂)₂CH₃ and -O-(CH₂)₄CH₃ being preferred.

By "ester" herein is meant a -COOX group, including carboxyl groups. By "carboxyl" herein is meant a -COOH group.

By "halogen" herein is meant bromine, iodine, chlorine, or fluorine. Preferred substituted alkyls are partially or fully halogenated alkyls such as CF₃, and the like.

By "aldehyde" herein is meant -XCOH groups.

By "alcohol" herein is meant -OH groups, and alkyl alcohols -XOH.

By "amido" herein is meant -XCONH- or XCONX- groups.

By "ethylene glycol" or "(poly)ethylene glycol" herein is meant a -(O-CH₂-CH₂)n- group, although each carbon atom of the ethylene group also can be singly or doubly substituted, i.e. -(O-CX₂-CX₂)ₙ-, with X as described above. Ethylene glycol derivatives with other heteroatoms in place of oxygen (i.e., -(N-CH₂-CH₂)ₙ- or -(S-CH₂-CH₂)ₙ-, or with substitution groups) are also useful.

By "aryl group" or grammatical equivalents herein is meant an aromatic monocyclic or polycyclic hydrocarbon moiety generally containing 5 to 14 carbon atoms (although larger polycyclic rings structures can be made) and any carbocylic ketone or thioketone derivative thereof, wherein the carbon atom with the free valence is a member of an aromatic ring. Aromatic groups include arylene groups and aromatic groups with more than two atoms removed. For the purposes of this application aromatic includes heteroaryl. "Heteroaryl" means an aromatic group wherein 1 to 5 of the indicated carbon atoms are replaced by a heteroatom chosen from nitrogen, oxygen, sulfur, phosphorus, boron and silicon wherein the atom with the free valence is a member of an aromatic ring, and any heterocyclic ketone and thioketone derivative thereof. Thus, heteroaryl includes for example pyrrolyl, pyridyl, thienyl, or furanyl (single ring, single heteroatom); oxazolyl, isoxazolyl, oxadiazolyl, or imidazolyl (single ring, multiple heteroatoms); benzoxazolyl, benzothiazolyl, or benzimidazolyl, (multi-ring, multiple heteroatoms); quinolyl, benzofuranyl or indolyl (multi-ring, single heteroatom). "Aryl" includes substituted aryl and substituted heteroaryl groups as well, with one or more X groups as defined herein.

X substituents can be used to modify the solubility of the candidate inhibitors, or alter the electronic environment of the metal binding moiety. For example, additional selected ring substituents are utilized to alter the solubility of the resulting candidate inhibitor in either aqueous or organic solvents. Typically, the substitution of halogen, alkyl, alkoxy, perfluoroalkyl, CN, amino, alkylamino, dialkylamino, 1-(acyloxy)alkyl ester of carboxy, aryl or heteroaryl onto the metal binding moiety results in an candidate inhibitor that is more soluble in non-polar solvents. Alternatively, substitution is by a "water solubilizing group", i.e., a sulfonic acid, salt of sulfonic acid, salt of amine, carboxy, carboxyalkyl, carboxyalkoxy, carboxyalkylamino, or carboxyalkylthio or other substituent that results in a candidate inhibitor that is more soluble in aqueous solution. Similarly, careful selection of the identity of linker and targeting moiety is also used to modify the solubility of the final candidate inhibitor with those candidate inhibitors containing charged or ionizable groups usually enhancing water solubility.

Alternatively, a ring substituent is used as a reactive site to further modify candidate inhibitors to attach the candidate inhibitors to a carrier or substrate as is more fully outlined below.

A number of suitable metal binding moieties are depicted in Figures 1-15. It should be noted, that in some combinations of metal binding moieties, targeting moieties, and optional linkers, the metal binding moiety is not a metal binding moiety selected from the group consisting of: 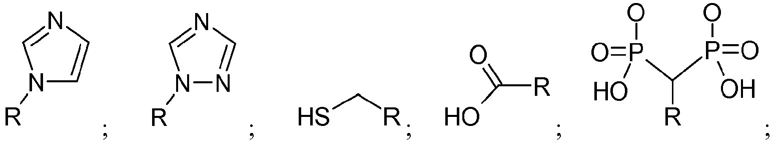 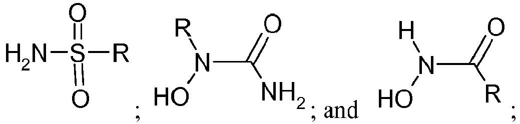 wherein: R is an attachment site through which the metal binding moiety can be attached to the targeting moiety, optionally through linker, Lₙ.

### B. Targeting moieties

In addition to the metal binding moieties, the presently disclosed inhibitors comprise targeting moieties. By "targeting moiety" herein is meant a functional group that serves to target or direct the inhibitor to a particular location or association. Thus, for example, a targeting moiety can be used to bring the metal binding moiety to the vicinity of a metal ion that is essential to the function of metalloenzymes. That is, the targeting moiety has binding affinity and/or binding specificity for a particular metalloenzyme, preferably in proximity of the metal binding site, such that the metal binding moieties can bind the metal ion. As described below, optional linkers are used to provide proper spacing. All inhibitors listed in the sections bellow find use as targeting moieties; in some case, moieties of the inhibitor or targeting moiety are removed to be replaced with metal binding moieties and optional linkers.

### 1. Carbonic Anhydrase

The carbonic anhydrase (CA) enzymes are a family of zinc metalloenzymes found throughout the prokaryotes and eukaryotes. In humans, fourteen different CA isozymes have been described with very different subcellular localization and tissue distribution. There are several cytosolic forms (CA I-III, CA VII), four membrane-bound isozymes (CA IV, CA IX, CA XII and CA XIV), one mitochondrial form (CA V), as well as a secreted CA isozyme, CA VI. These enzymes catalyze the interconversion between carbon dioxide and the bicarbonate ion, and are thus involved in crucial physiological processes connected with respiration and transport of CO₂/bicarbonate between metabolizing tissues and the lungs, pH and CO₂ homeostasis, electrolyte secretion in a variety of tissues, biosynthetic reactions such as the gluconeo-genesis, lipogenesis and ureagenesis, bone resorption, calcification, tumorigenicity, and many other physiologic or pathologic processes. As such, CA has been the target for the design of inhibitors with various therapeutic applications, such as glaucoma, migraine, hypertension, congestive heart failure, and cancer. Inhibitors of carbonic anyhydrase are among the most commonly utilized agents in the treatment of glaucoma. Chegwidden, W.R.; Edwards, Y., Carter, N. (Eds.) *The Carbonic Anhydrases - New Horizons,* Birkhäuser Verlag, Basel, 2000.

A number of CA inhibitors have been approved for human use and have demonstrated clinical utility across a broad range of conditions. For example, acetazolamide is used in the treatment of congestive heart failure. Dorzolamide and brinzolamide are effective in the treatment of glaucoma. Topiramate is effective in the treatment of epilepsy and migraine. Supuran, C. T., Curr. Top. Med. Chem., 7:825-33 (2007).

A series of sulfamate derivatives are described as inhibitors of CA and have been show to be useful in the treatment of convulsive disorders. Maryanoff et al., J. Med. Chem., 30:880-7 (1987). See also U.S. Patent Nos. 4,513,006 and 6,503,884, each of which is incorporated herein by reference in its entirety.

A series of substituted sulfonamide derivatives are described as inhibitors of CA and have been found to be useful as anti-glaucoma agents. Wang et al., Arch. Ophthalmol., 108:511 (1990). See also U.S. Patent No. 4,863,922, which is incorporated herein by reference in its entirety.

A series of bicyclic heterocyclic sulfonamide and sulfonic ester derivatives have been disclosed as carbonic anhydrase inhibitors with potential applications in oncology. See U.S. 5,721,246, which is incorporated herein by reference in its entirety.

A series of thiophene sulfonamides have been shown to be useful as carbonic anhydrase inhibitors with applications in the treatment of glaucoma. See U.S. Patent Nos. 5,240,923 and 5,378,703, each of which is incorporated herein by reference in its entirety.

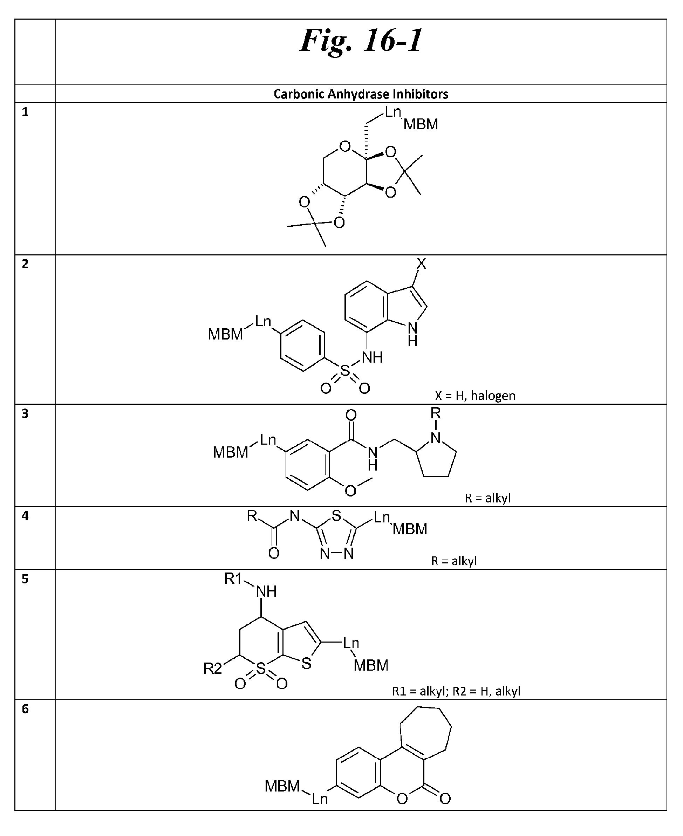

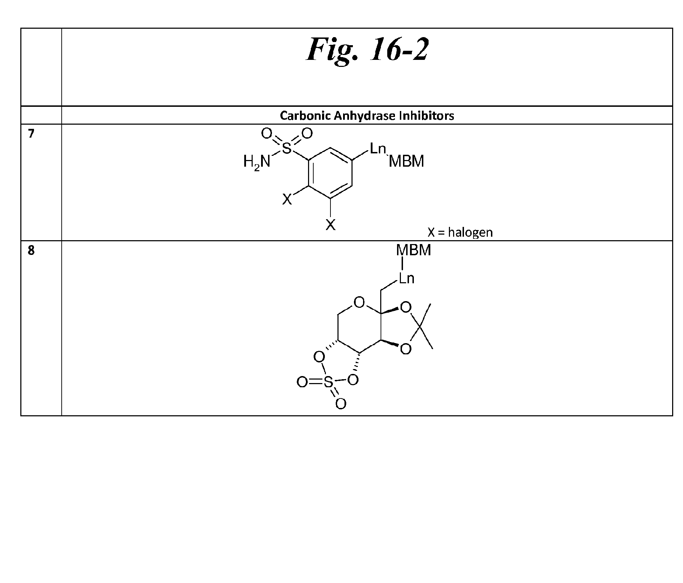

Figure 16 depicts a number of inhibitors of CA, which are suitable for use as targeting moieties in the presently disclosed subject matter. Figure 16 shows the structure of these known inhibitors along with possible sites of attachment of the linkers and metal binding moieties ("R"), as well as possible derivatives.

Accordingly, in some embodiments, the metalloenzyme inhibitor is a carbonic anhydrase inhibitor comprising a targeting moiety, a metal binding moiety, and optionally a linker, and wherein the inhibitor has a formula selected from the group consisting of:
(a) 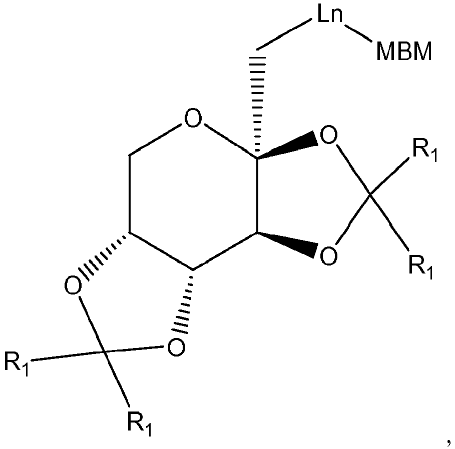 wherein each R₁ is independently alkyl or substituted alkyl;
(b) 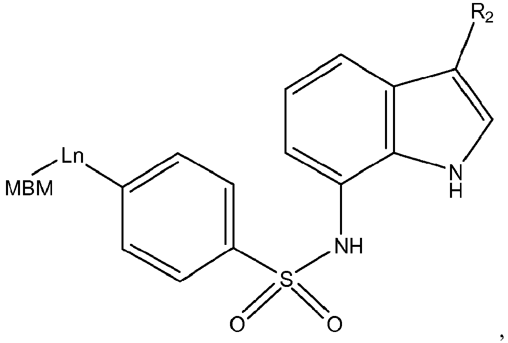 wherein R₂ is H or halogen;
(c) 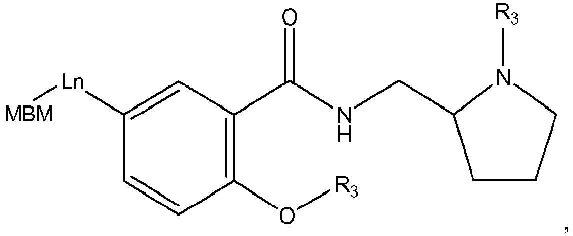 wherein each R₃ is independently alkyl or substituted alkyl;
(d) 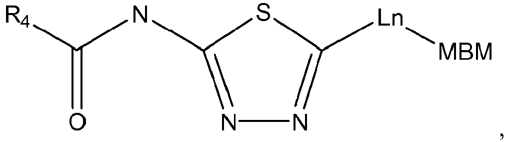 wherein R₄ is alkyl or substituted alkyl;
(e) 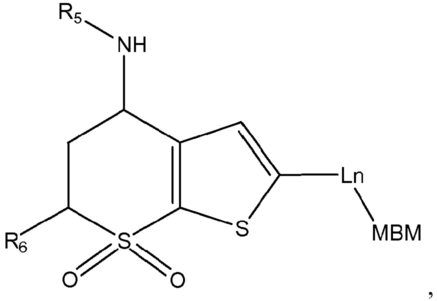 wherein:
   R₅ is alkyl or substituted alkyl; and
   R₆ is selected from the group consisting of H, alkyl, and substituted alkyl;
(f) 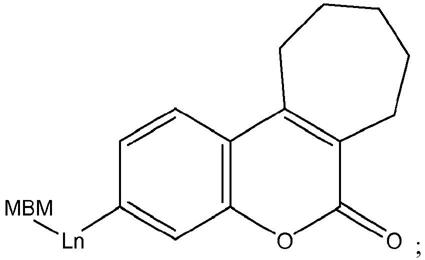
(g) 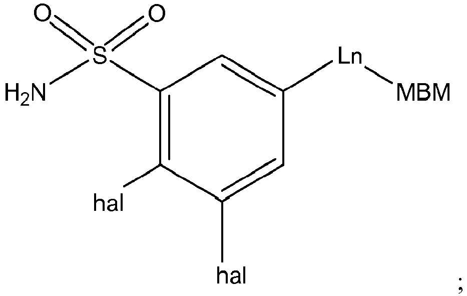 and
(h) 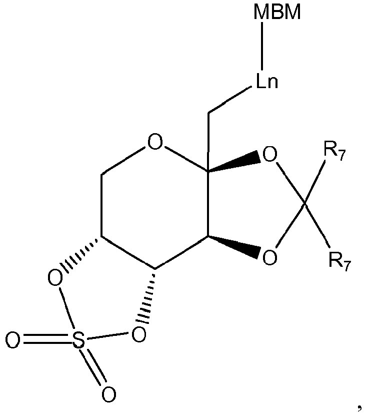 wherein each R₇ is independently alkyl or substituted alkyl; and
   wherein:
   MBM is a metal binding moiety;
   Lₙ is a linker, wherein n is an integer from 0 to 1;
   hal is halogen;
   under the proviso that the metal binding moiety is not a metal binding moiety selected from the group consisting of: 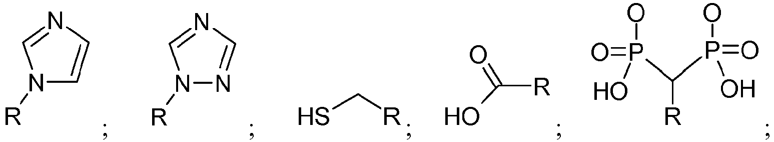 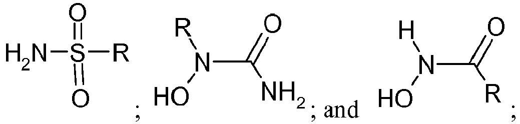
   wherein:
   R is an attachment site through which the metal binding moiety can be attached to the targeting moiety, optionally through linker, Lₙ; and
   pharmaceutically acceptable salts thereof.

In addition to these targeting moieties, other known targeting moieties, identified by the screening methods outlined below or shown to bind to CA can be used. Thus, suitable targeting moieties include, but are not limited to, small organic molecules, including known drugs and drug candidates, polysaccharides, fatty acids, vaccines, polypeptides, proteins (including peptides, as described herein), nucleic acids, carbohydrates, lipids, hormones, including proteinaceous and steroid hormones, growth factors, receptor ligands, antigens, antibodies and enzymes, (as outlined below, collectively "candidate agents"), and the like.

CA activity can be measured using established methods. See Wilbur, K. M. and Anderson, N. G., J. of Biol. Chem., 176:147-154 (1948) and McIntosh, J. E., Biochem. J., 109, 203-207 (1968).

### 2. Catechol-O-methyl transferase (COMT)

Catechol-O-methyl transferase (COMT) is a magnesium-containing metalloenzyme responsible for the inactivation of endogenous catecholamine neurotransmitters such as dopamine, epinephrine, and norepinephrine. The enzyme introduces a methyl group to the catecholamine, which is donated by S-adenosyl methionine. COMT is an intracellular enzyme located in the postsynaptic neuronal cell and is found in two forms, a soluble form and a membrane-bound form. Any compound having a catechol structure, like catechol estrogens and catechol-containing flavonoids, also are efficient substrates of COMT. Levodopa, a precursor of catecholamines and a commonly utilized drug in Parkinson's Disease, also is an important substrate of COMT. A functional single nucleotide polymorphism of the gene for COMT has been shown to affect cognitive tasks broadly related to executive function, such as set shifting, response inhibition, abstract thought, and the acquisition of rules or task structure. This polymorphism in the COMT gene results in the substitution of the amino acid valine for methionine. It has been shown that this valine variant catabolizes dopamine at up to four times the rate of its methionine counterpart, resulting in a significant reduction of synaptic dopamine following neurotransmitter release. COMT inhibitors save levodopa from COMT and prolong the action of levodopa. As such, inhibitors of COMT have found use in the treatment of Parkinson's disease. Bonifacio et al., CNS Drug Rev., 13:352-79 (2007).

COMT inhibitors that are approved for use in humans include tolcapone and entacapone. These agents are typically given in combination with levodopa in the treatment of Parkinson's Disease. Schrag A., Lancet Neurol., 4:366-70 (2005); Keating GM et al., CNS Drugs, 19:165-84 (2005).

A series of nitro-substituted catechols have been demonstrated to be inhibitors of COMT with potential use in the treatment of Parkinson's Disease. See U.S. Patent Nos. 4,963,590, 5,112,861, and 5,446,194, each of which is incorporated herein by reference in its entirety.

A series of 3,5-disubstituted pyrocatechol derivatives have been disclosed as inhibitors of COMT. See U.S. Patent Nos. 5,236,952, 5,476,875, and 5,389,653, each of which is incorporated herein by reference in its entirety.

A series of substituted 2-phenyl-1-(3,4-dihydroxy-5-nitrophenyl)-1-ethanones have been demonstrated to be effective inhibitors of COMT. See European Patent No. 101688 and WO 2000/037423.

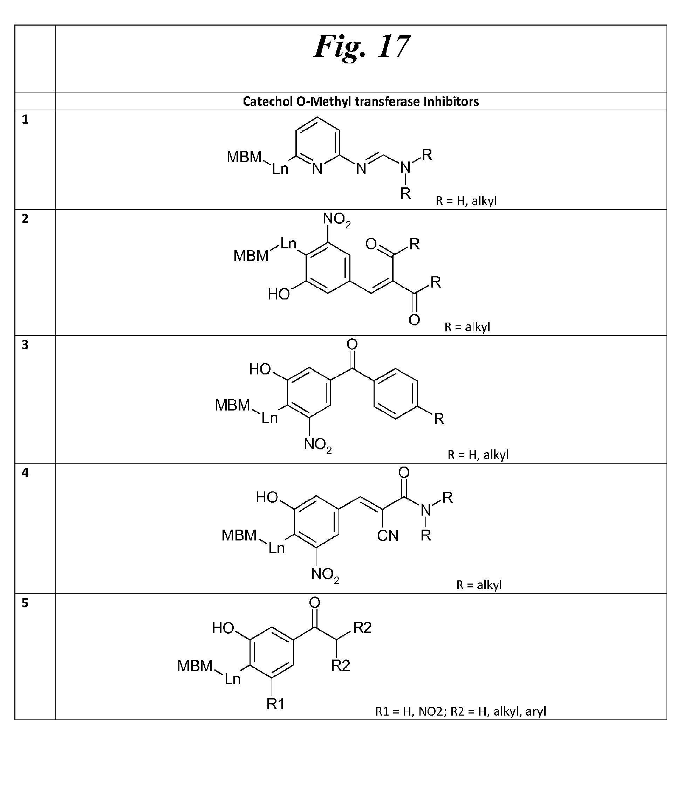

Figure 17 depicts a number of inhibitors of COMT, which are suitable for use as targeting moieties in the present invention. Figure 17 shows the structure of these known inhibitors along with possible sites of attachment of the linkers and metal binding moieties ("R"), as well as possible derivatives.

Accordingly, in some embodiments, the metalloenzyme inhibitor is a catechol *O-*methyl transferase inhibitor comprising a targeting moiety, a metal binding moiety, and optionally a linker, and wherein the inhibitor has a formula selected from the group consisting of:
(a) 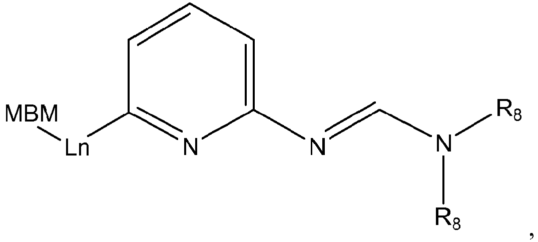 wherein each R₈ is independently selected from the group consisting of H, alkyl, and substituted alkyl;
(b) 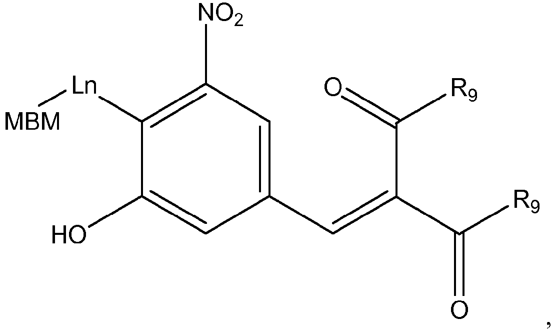 wherein each R₉ is independently alkyl or substituted alkyl;
(c) 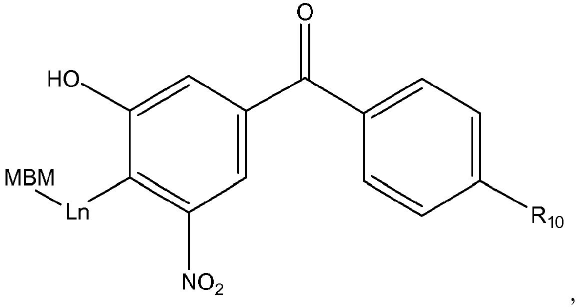 wherein R₁₀ is selected from the group consisting of H, alkyl, and substituted alkyl;
(d) 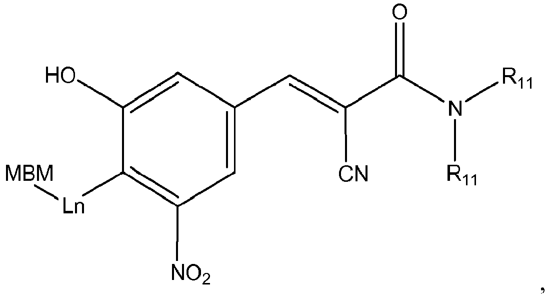 wherein each R₁₁ is independently selected from the group consisting of alkyl and substituted alkyl; and
(e) 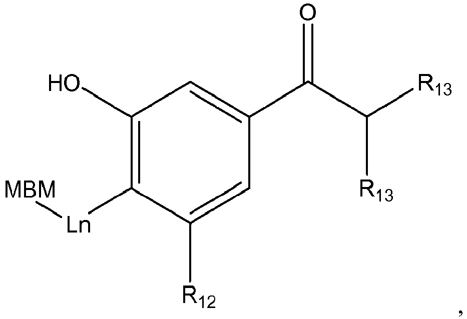 wherein:
   R₁₂ is H or NO₂; and
   R₁₃ is selected from the group consisting of H, alkyl, substituted alkyl, aryl, and substituted aryl; and
   wherein:
   MBM is a metal binding moiety;
   Lₙ is a linker, wherein n is an integer from 0 to 1;
   under the proviso that the metal binding moiety is not a metal binding moiety selected from the group consisting of: 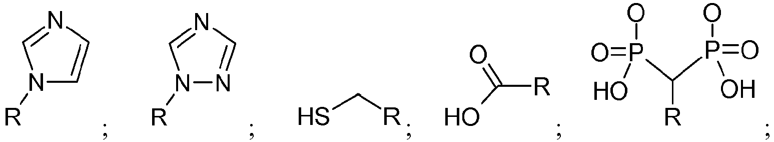 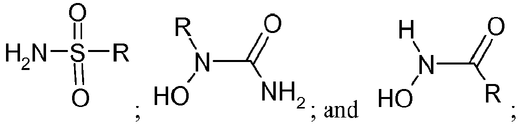 wherein:
      R is an attachment site through which the metal binding moiety can be attached to the targeting moiety, optionally through linker, Lₙ.

In addition to these targeting moieties, other known targeting moieties, identified by the screens outlined below or shown to bind to COMT can be used. Thus, suitable targeting moieties include, but are not limited to, small organic molecules including known drugs and drug candidates, polysaccharides, fatty acids, vaccines, polypeptides, proteins (including peptides, as described herein), nucleic acids, carbohydrates, lipids, hormones including proteinaceous and steroid hormones, growth factors, receptor ligands, antigens, antibodies and enzymes, (as outlined below, collectively "candidate agents"), and the like.

COMT activity can be measured using established methods. Hirano et al., Journal of Chromatography, 819:41-46 (2005); Bailey et al., Bioorganic & Medicinal Chemistry, 12:595-601 (2004).

### 3. Farnesyl Diphosphate Synthase

Farnesyl diphosphate synthase (FDS) is a key enzyme in the mevalonate pathway. Although there is general agreement that the enzyme is a metalloenzyme, some debate exists over the exact identity of the active site metal *in vivo* (i.e., zinc vs. magnesium vs. manganese). FDS is responsible for the synthesis of farnesyl diphosphate and its subsequent metabolite geranylgeranyl diphosphate. These isoprenoid lipids are the building blocks for the production of a variety of metabolites, such as dolichol and ubiquinone, and are also required for post-translational modification (prenylation) of proteins, including small GTPases. Prenylated GTPases, such as those of the Ras, Rho, and Rab families, are important signaling proteins that regulate a variety of cell processes important for cell function, particularly in osteoclasts. As such, inhibitors of FDS have found clinical utility in the treatment of osteoporosis. The most commonly utilized inhibitors of FDS are the bisphosphonates, which are stable pyrophosphate analogs capable of inhibiting osteoclast function *in vivo.* Inhibitors of FDS have been shown to be useful in the treatment of osteoporosis, Paget's disease, hypercalcemia caused by malignancy, tumor metastases in bone, and other bone diseases. Roelofs et al., Clinical Cancer Research, 12:6222- 6230 (2006).

Inhibitors of FDS that are approved for human use include pamidronate, etidronate, tiludronate, zolendronate and alendronate. These agents are approved for various bone diseases such as osteoporosis, Paget's Disease and hypercalcemia secondary to bone metastases. Russel, R. G., Ann. N.Y. Acad. Sci., 1068:367-401 (2006).

A series of phenyl-substituted diphosphonic acid derivatives have been shown to be effective in reducing bone turnover. See U.S. Patent No. 4,980,171, which is incorporated herein by reference in its entirety.

Aminoalkyl disphosphonate derivatives have been disclosed for use in bone disease and disorders of calcium metabolism. See U.S. Patent Nos. 4,639,339 and 4,711,880, each of which is incorporated herein by reference in its entirety.

A series of amino-hydroxy-alkylidene bisphosphonic acids have been disclosed as inhibitors of FDS with potential application in bone disease. See U.S. Patent No. 4,621,077, which is incorporated herein by reference in its entirety.

A series of substituted alkanediphosphonic acids have been demonstrated to be inhibitors of FDS. See U.S. Patent Nos. 4,777,163 and 4,939,130, each of which is incorporated herein by reference in its entirety.

A series of cycloalkylamino methylenebisphosphonic acids have been disclosed as inhibitors of bone turnover. See U.S. Patent No. 5,041,428, which is incorporated herein by reference in its entirety.

A series of novel alky-substituted diphosphonic-acid derivatives have been disclosed as inhibitors of FDS. See U.S. Patent Nos. 4,927,814 and 4,942,157, each of which is incorporated herein by reference in its entirety.

A series of geminal diphosphonates have been disclosed as useful in the treatment of bone disorders. See U.S. Patent No. 5,583,122, which is incorporated herein by reference in its entirety.

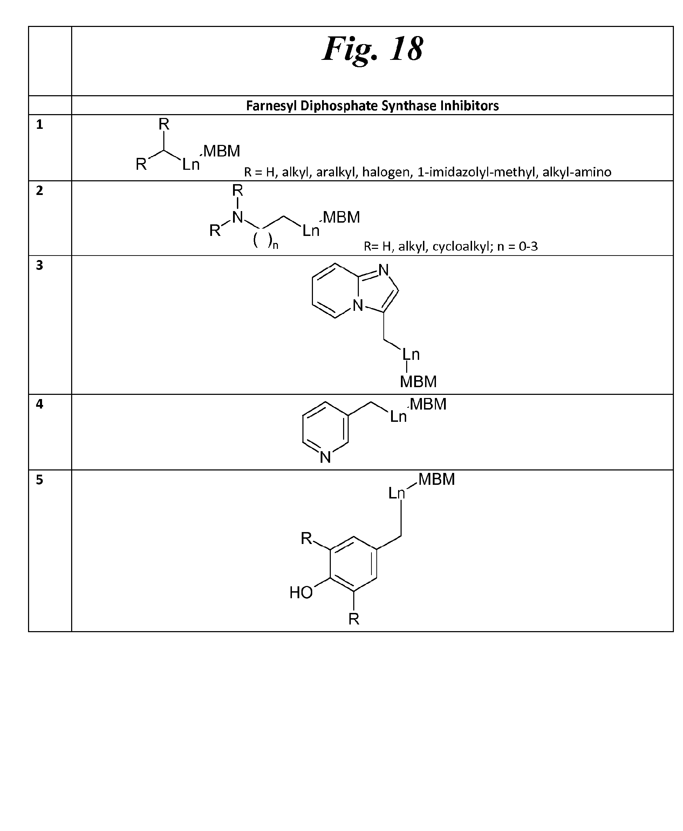

Figure 18 depicts a number of inhibitors of FDS, which are suitable for use as targeting moieties in the present invention. Figure 18 shows the structure of these known inhibitors along with possible sites of attachment of the linkers and metal binding moieties ("R"), as well as possible derivatives.

Accordingly, in some embodiments, the metalloenzyme inhibitor is a farnesyl diphosphate synthase inhibitor comprising a targeting moiety, a metal binding moiety, and optionally a linker, and wherein the inhibitor has a formula selected from the group consisting of:
(a) 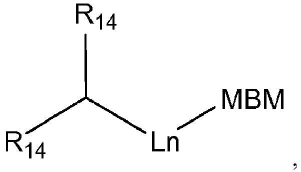 wherein each R₁₄ is independently selected from the group consiting of H, alkyl, substituted alkyl, aralkyl, halogen, 1-imidazolyl-methyl, and alkyl-amino;
(b) 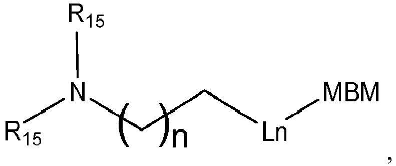 wherein:
   n is an integer from 0 to 3; and
   each R₁₅ is selected from the group consisting of H, alkyl, substituted alkyl, cycloalkyl, substituted cycloalkyl, heterocycloalkyl, and substituted heterocycloalkyl;
(c) 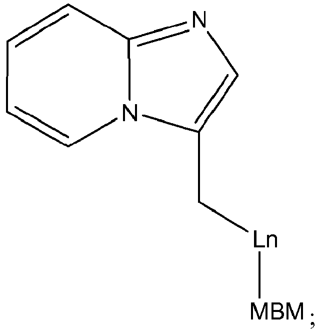
(d) 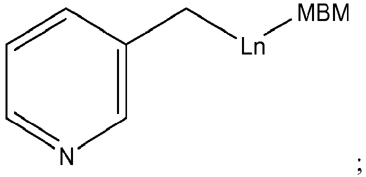 and
(e) 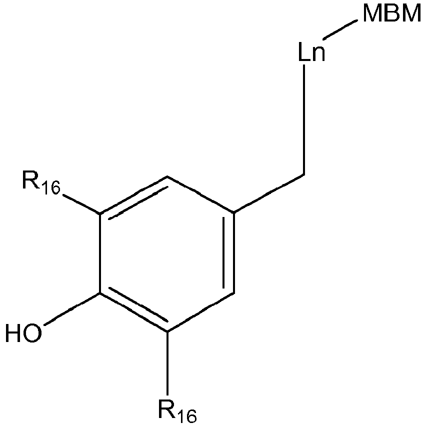 wherein each R₁₆ is independently selected from the group consisting of H, alkyl, and substituted alkyl; and
   wherein:
   MBM is a metal binding moiety;
   Lₙ is a linker, wherein n is an integer from 0 to 1;
   under the proviso that the metal binding moiety is not a metal binding moiety selected from the group consisting of: 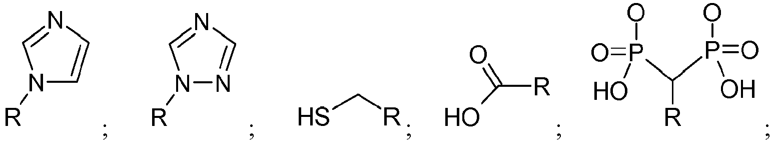 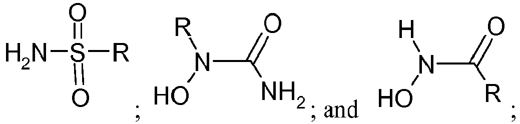 wherein:
      R is an attachment site through which the metal binding moiety can be attached to the targeting moiety, optionally through linker, Lₙ.

In addition to these targeting moieties, other known targeting moieties, identified by the screens outlined below or shown to bind to FDS can be used. Thus, suitable targeting moieties include, but are not limited to, small organic molecules including known drugs and drug candidates, polysaccharides, fatty acids, vaccines, polypeptides, proteins (including peptides, as described herein), nucleic acids, carbohydrates, lipids, hormones including proteinaceous and steroid hormones, growth factors, receptor ligands, antigens, antibodies and enzymes, (as outlined below, collectively "candidate agents"), and the like.

FDS activity can be measured using established methods. Glickman, J. F. and Schmid, A., Assay and Drug Development Technologies, 5:205-214 (2007); Dunford, J. E. et al., J. Pharm. Exp. Therapeut., 296:235-242 (2001).

### 4. Farnesyl Transferase

Farnesyl transferase (FT) is a zinc metalloenzyme and is one of the three enzymes in the prenyltransferase group. FT post-translationally modifies proteins by adding an isoprenoid lipid called a farnesyl group to the carboxyl terminus of the target protein. This process, called farnesylation (more generally prenylation), causes farnesylated proteins to become membrane-associated due to the hydrophobic nature of the farnesyl group. All FT substrates invariably have a cysteine as their fourth to last residue. This cysteine engages in an SN2 type attack, coordinated by the zinc and a transient stabilizing magnesium ion on the farnesyl diphosphate, displacing the diphosphate. The product remains bound to farnesyltransferase until displaced by new substrates. The last three amino acids of the CaaX motif are removed later. Most farnesylated proteins are involved in cellular signaling where membrane association is critical for function. Targets of FT include members of the Ras superfamily of small GTP-binding proteins critical to cell cycle progression. As such, inhibitors of FT have been examined in the treatment of a variety of cancers. Basso et al., Journal of Lipid Research, 47:15-31 (2006).

A number of FT inhibitors, including tipifarnib, lonafarnib and BMS-214662, are currently in clinical development for the treatment of both solid and hematologic cancers. Appels, N. et al., The Oncologist, 10:565-578 (2005).

A series of peptidomimetic, piperazine-containing compounds have been disclosed as selective inhibitors of FT. See U.S. Patent No. 5,856,326, which is incorporated herein by reference in its entirety.

A series of imidazol-5-yl, methyl-2-quinolinone derivatives have been demonstrated to be potent inhibitors of FT. See U.S. Patent Nos. 6,420,387 and 6,037,350, each of which is incorporated herein by reference in its entirety.

A series of tricyclic amides have been disclosed as inhibitors of FT. See U.S. Patent No. 5,874,442, which is incorporated herein by reference in its entirety.

A series of benzodiazepine compounds have been disclosed as inhibitors of FT. See U.S. Patent No. 6,011,029, which is incorporated herein by reference in its entirety.

A series of cyclic and acyclic imidazolylalkyl derivatives have been demonstrated to be selective and potent inhibitors of FT. See WO 2006/065828, U.S. Patent Application Publication Nos. 2006/205,775 and 2006/211,706, each of which is incorporated herein by reference in its entirety.

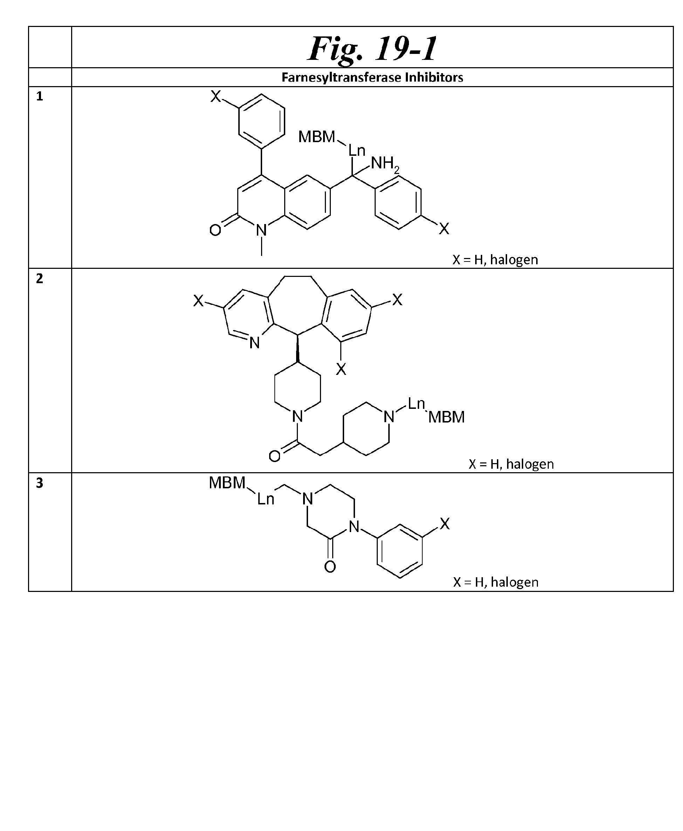

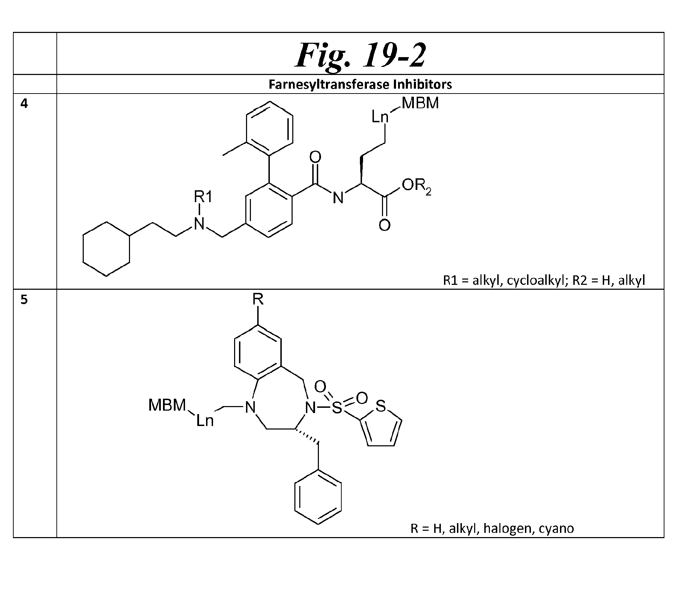

Figure 19 depicts a number of inhibitors of FT, which are suitable for use as targeting moieties in the present invention. Figure 19 shows the structure of these known inhibitors along with possible sites of attachment of the linkers and metal binding moieties ("R"), as well as possible derivatives.

Accordingly, in some embodiments, the metalloenzyme inhibitor is a farnesyl transferase inhibitor comprising a targeting moiety, a metal binding moiety, and optionally a linker, and wherein the inhibitor has a formula selected from the group consisting of:
(a) 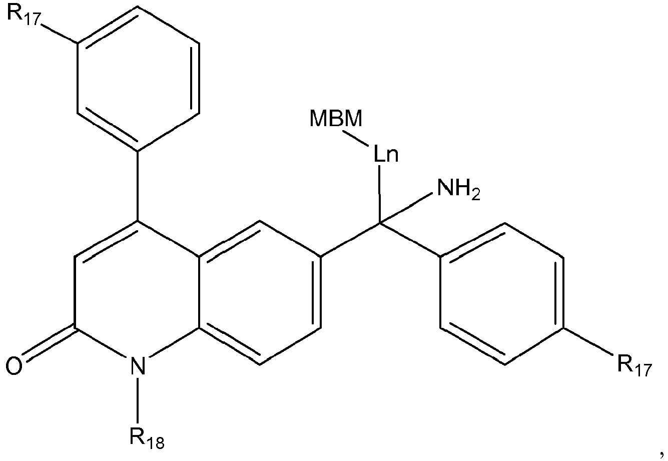 wherein:
   each R₁₇ is independently H or halogen; and
   R₁₈ is alkyl or substituted alkyl;
(b) 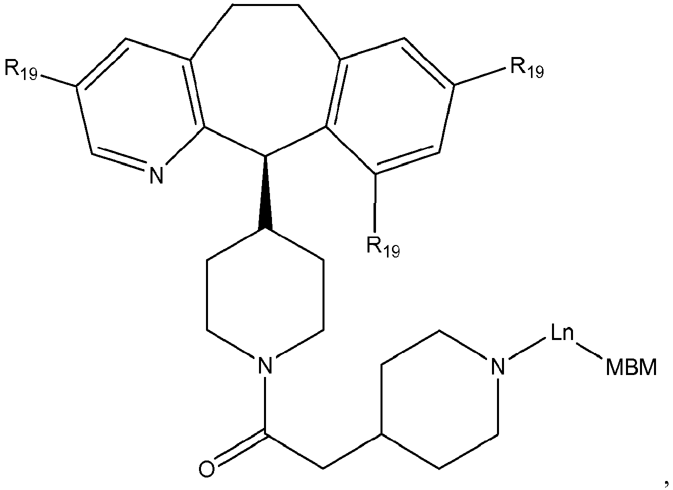 wherein each R₁₉ is independently H or halogen;
(c) 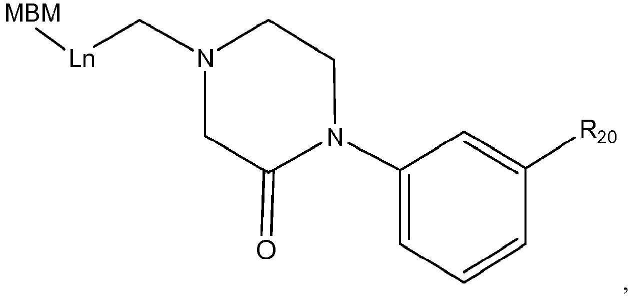 wherein R₂₀ H or halogen;
(d) 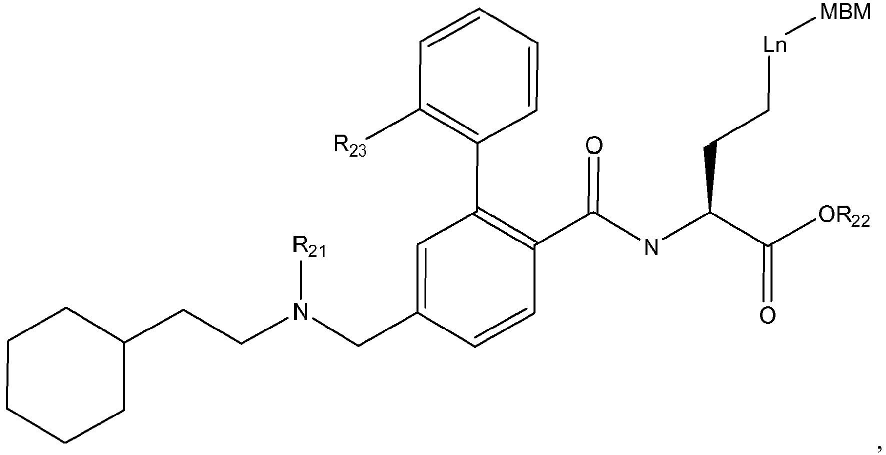 wherein:
   R₂₁ is selected from the group consisting of alkyl, substituted alkyl, cycloalkyl, substituted cycloalkyl, heterocycloalkyl, and substituted heterocycloalkyl;
   R₂₂ is selected from the group consisting of H, alkyl, and substituted alkyl; and
   R₂₃ is alkyl or substituted alkyl;
(e) 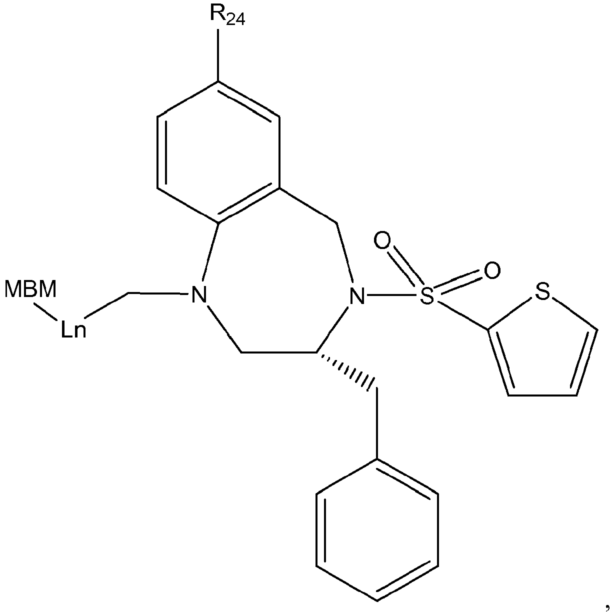 wherein R₂₄ is selected from the group consisting of H, alkyl, substituted alkyl, halogen, and cyano; and
   wherein:
   MBM is a metal binding moiety;
   Lₙ is a linker, wherein n is an integer from 0 to 1;
   under the proviso that the metal binding moiety is not a metal binding moiety selected from the group consisting of: 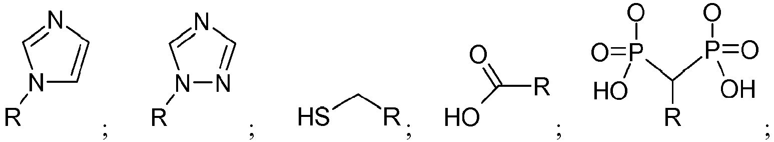 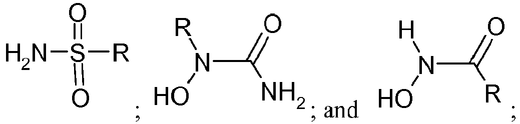 wherein:
      R is an attachment site through which the metal binding moiety can be attached to the targeting moiety, optionally through linker, Lₙ.

In addition to these targeting moieties, other known targeting moieties, identified by the screens outlined below or shown to bind to FT can be used. Thus, suitable targeting moieties include, but are not limited to, small organic molecules including known drugs and drug candidates, polysaccharides, fatty acids, vaccines, polypeptides, proteins (including peptides, as described herein), nucleic acids, carbohydrates, lipids, hormones including proteinaceous and steroid hormones, growth factors, receptor ligands, antigens, antibodies and enzymes, (as outlined below, collectively "candidate agents"), and the like.

FT activity can be measured using established methods. Lobell R.B. et al., Journal of Biomolecular Screening, 8:430-438 (2003). Manne V, et al., Proc. Nat. Acad. Sci., 87:7541-7545 (1990).

### 5. Neprilysin

Neprilysin (NEP), also known as neutral endopeptidase, CD10, and common acute lymphoblastic leukemia antigen (CALLA), is a zinc-dependent metalloenzyme that degrades a number of small secreted peptides. Synthesized as a membrane-bound protein, the NEP ectodomain is released into the extracellular domain after it has been transported from the Golgi apparatus to the cell surface. In neurons, NEP is regulated by the protein nicastrin, a component of the gamma secretase complex that performs a necessary step in processing amyloid precursor protein to amyloid beta. NEP is also associated with other biochemical processes, and is particularly highly expressed in kidney and lung tissues. NEP is highly active in the metabolism of signaling peptides such as enkephalins, substance P, endothelin, and atrial natriuretic factor. Associations have also been observed between NEP expression and various types of cancer. In cancer biomarker studies, the NEP gene is often referred to as CD10 or CALLA. In some types of cancer, such as metastatic carcinoma and some advanced melanomas, NEP is overexpressed. In other types, however, NEP is downregulated, and thus unable to modulate the pro-growth autocrine signaling of cancer cells via secreted peptides such as mammalian homologs related to bombesin. Due to the broad range of activity of NEP, inhibitors of the enzyme have potential for use in a variety of diseases such as Alzheimer's Disease, hypertension, congestive heart failure and cancer. Lopez-Otin, C., and Matrisian, L. M., Nat Rev Cancer, 7:800-8 (2007). Turner A. J. and Nalivaeva, M. M., Int. Rev. Neurobiol., 82:113-35 (2007).

A number of inhibitors of NEP, including ecadotril, candoxatril, omapatrilat, have been examined in clinical trials for the treatment of hypertension and congestive heart failure. Iyengar S. and Abraham W. T., Heart Fail. Clin., 1:95-102 (2005).

A series of thioester derivatives have been disclosed as potent inhibitors of NEP. See U.S. Patent Nos. 5,296,509 and 5,331,008, each of which is incorporated herein by reference in its entirety.

A series of cycloalkyl-substituted glutaramide analogues have been demonstrated to be potent inhibitors of NEP with potential use as antihypertensive agents. See U.S. Patent No. 4,975,444, which is incorporated herein by reference in its entirety.

A series of novel mercaptoacetylamide derivatives have been disclosed as potent inhibitors of NEP. See U.S. Patent Nos. 5,430,145 and 6,602,866, each of which is incorporated herein by reference in its entirety.

A series of bicyclic carboxylic acid derivatives have been demonstrated to be potent and selective inhibitors of NEP. See U.S. Patent No. 5,508,272, which is incorporated herein by reference in its entirety.

A series of mercaptoacetylamide derivatives have been disclosed as inhibitors of NEP. See U.S. Patent No. 6,602,866, which is incorporated herein by reference in its entirety.

A series of N-mercaptoacyl phenylalanine derivatives have been disclosed as potent inhibitors of NEP. See WO 2003/104200.

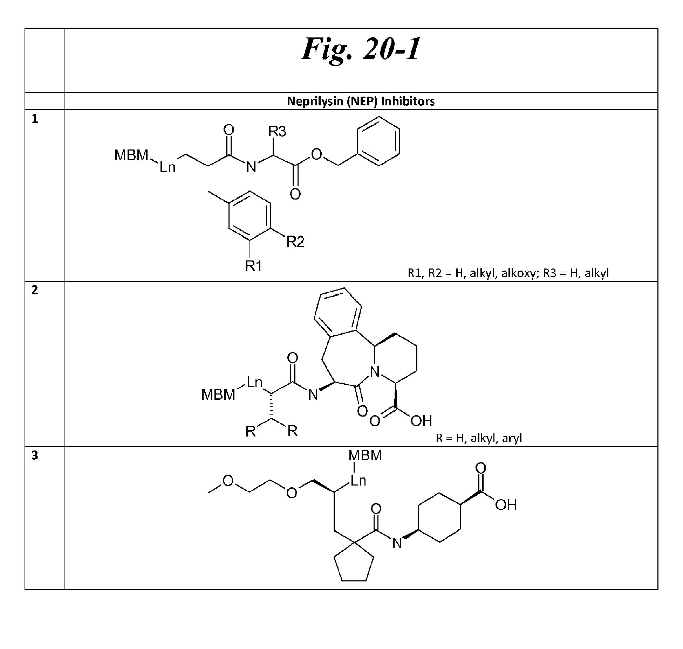

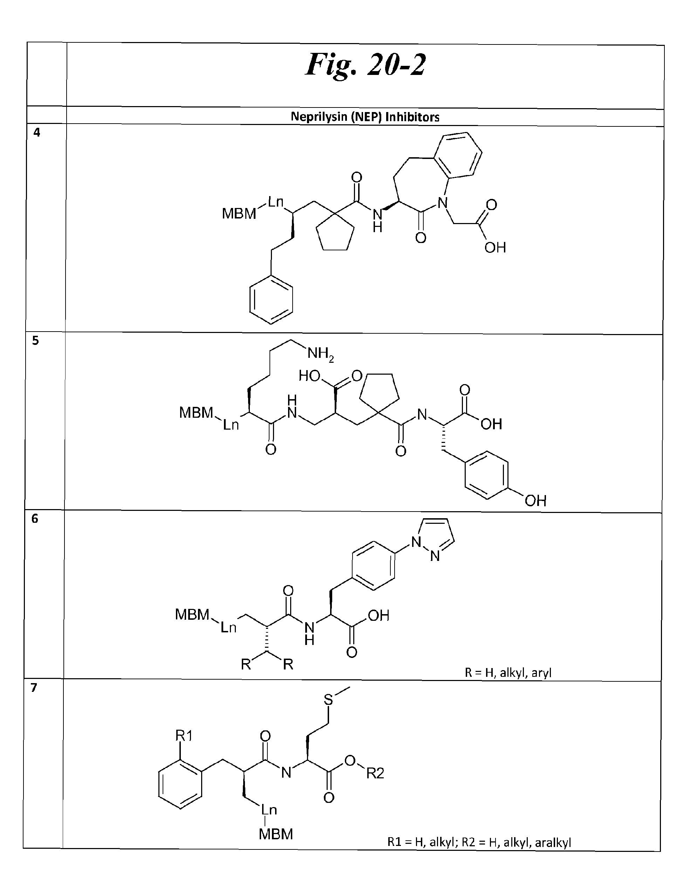

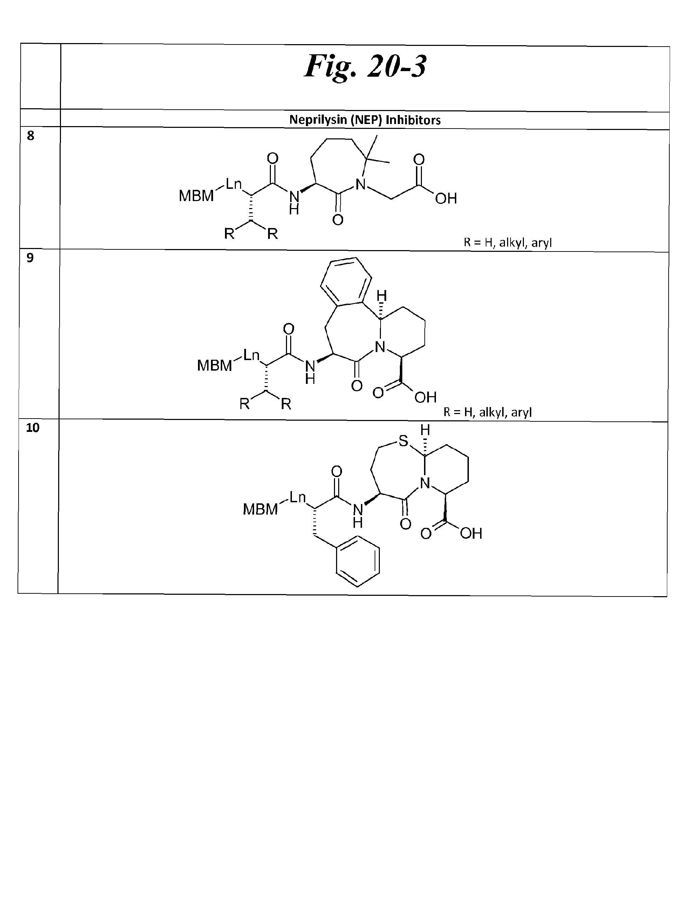

Figure 20 depicts a number of inhibitors of NEP, which are suitable for use as targeting moieties in the present invention. Figure 20 shows the structure of these known inhibitors along with possible sites of attachment of the linkers and metal binding moieties ("R"), as well as possible derivatives.

Accordingly, in some embodiments, the metalloenzyme inhibitor is a neprilysin (NEP) inhibitor comprising a targeting moiety, a metal binding moiety, and optionally a linker, and wherein the inhibitor has a formula selected from the group consisting of:
(a) 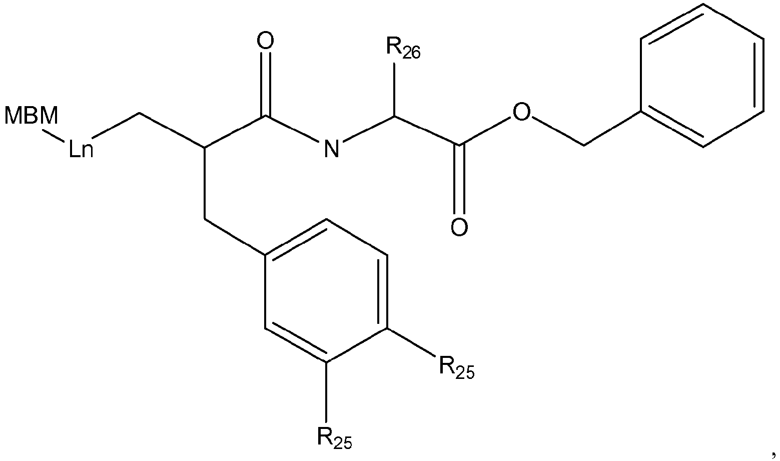 wherein:
   each R₂₅ is selected from the group consisting of alkyl, substituted alkyl, and alkoxyl; and
   R₂₆ is selected from the group consisting of H, alkyl, and substituted alkyl;
(b) 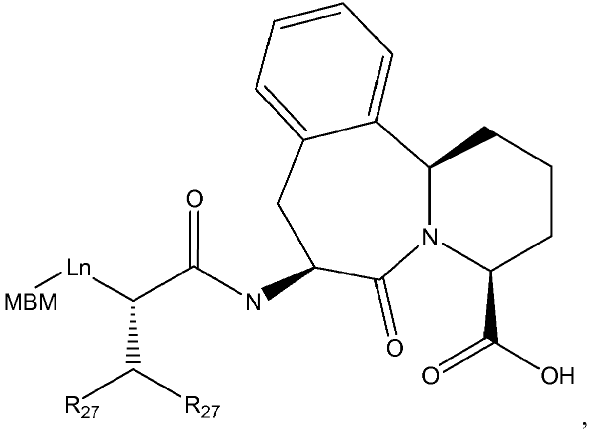 wherein each R₂₇ is independently selected from the group consisting of H, alkyl, substituted alkyl, aryl, and substituted aryl;
(c) 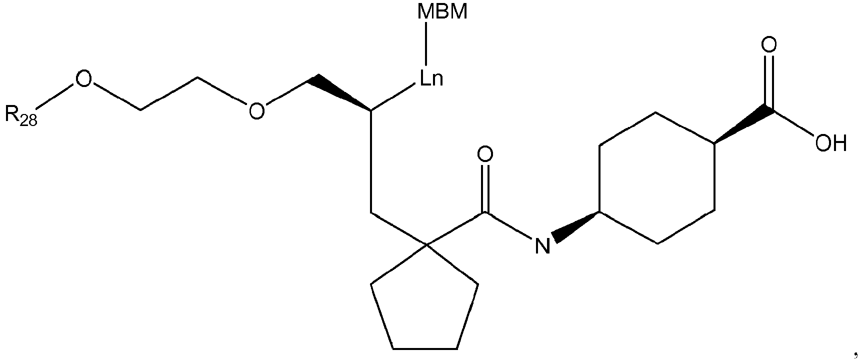 wherein R₂₈ is alkyl or substituted alkyl;
(d) 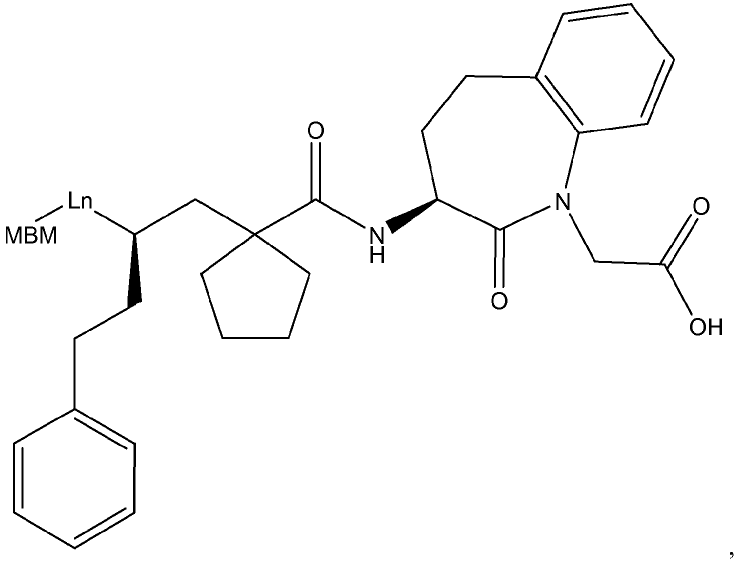
(e) 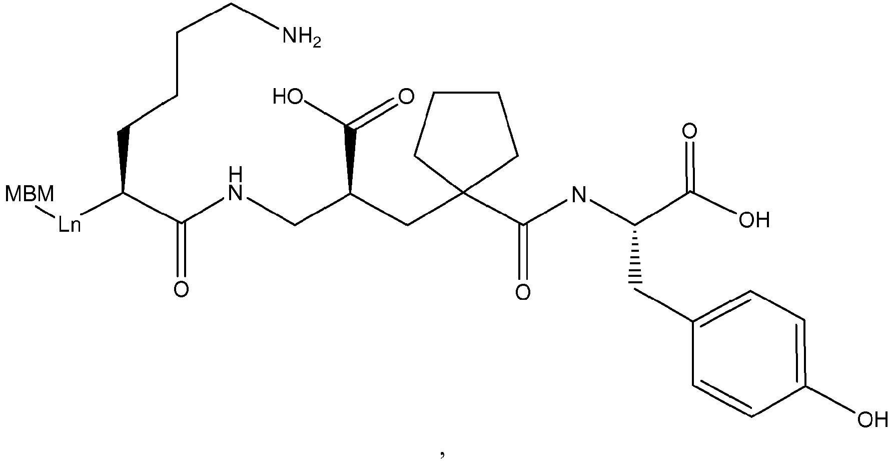
(f) 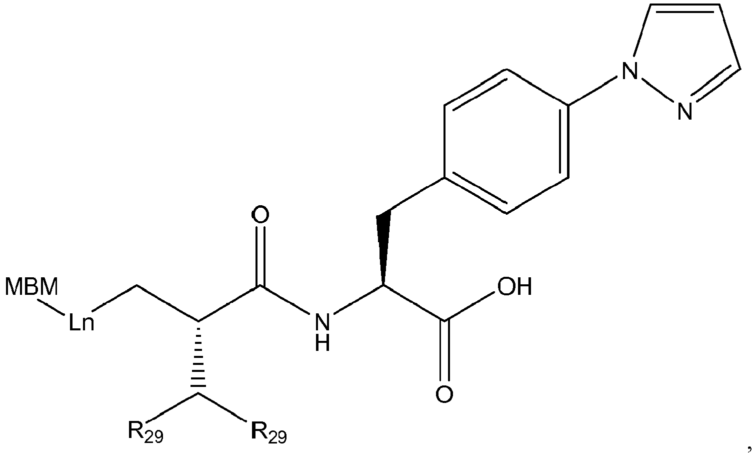 wherein each R₂₉ is selected from the group consisting of H, alkyl, substituted alkyl, aryl, and substituted aryl;
(g) 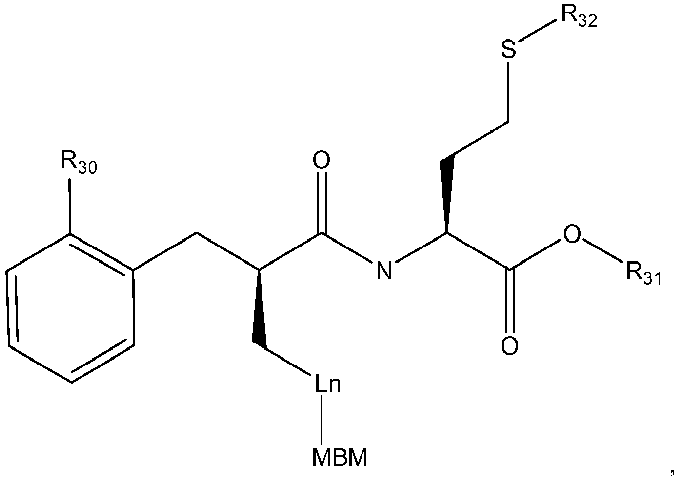 wherein:
   R₃₀ is selected from the group consisting of H, alkyl, and substituted alkyl;
   R₃₁ is selected from the group consisting of H, alkyl, substituted alkyl, and aralkyl; and
   R₃₂ is alkyl or substituted alkyl;
(h) 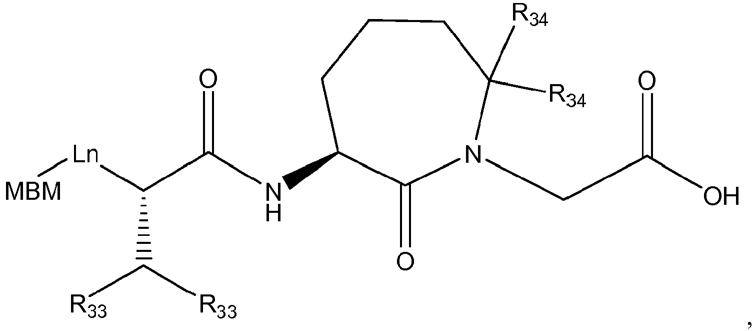 wherein:
   each R₃₃ is independently selected from the group consisting of H, alkyl, substituted alkyl, aryl, and substituted aryl; and
   each R₃₄ is independently alkyl or substituted alkyl;
(i) 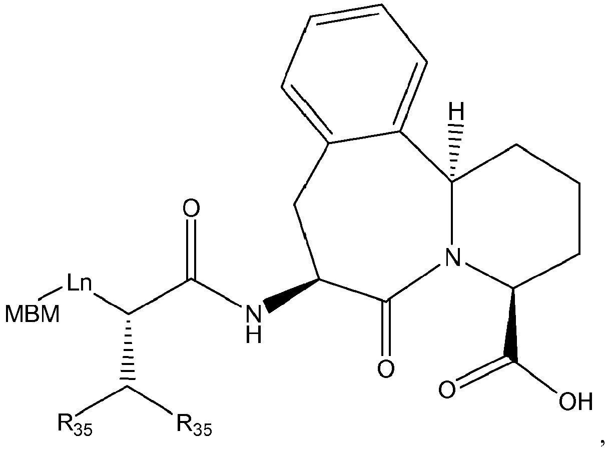 wherein each R₃₅ is independently selected from the group consisting ofH, alkyl, substituted alkyl, aryl, and substituted aryl; and
(j) 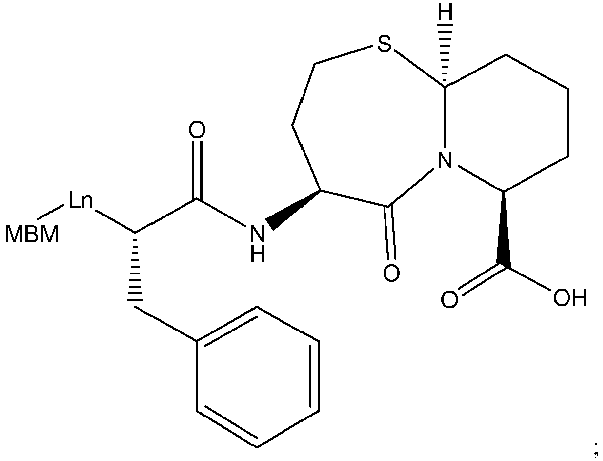 and wherein:
   MBM is a metal binding moiety;
   Lₙ is a linker, wherein n is an integer from 0 to 1;
   under the proviso that the metal binding moiety is not a metal binding moiety selected from the group consisting of: 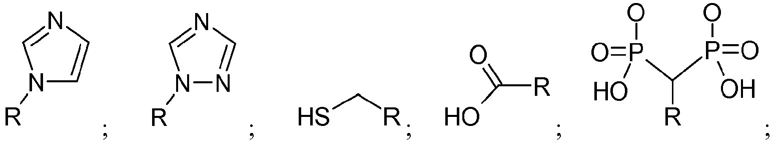 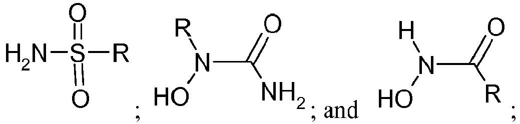 wherein:
      R is an attachment site through which the metal binding moiety can be attached to the targeting moiety, optionally through linker, Lₙ.

In addition to these targeting moieties, other known targeting moieties, identified by the screens outlined below or shown to bind to NEP can be used. Thus, suitable targeting moieties include, but are not limited to, small organic molecules including known drugs and drug candidates, polysaccharides, fatty acids, vaccines, polypeptides, proteins (including peptides, as described herein), nucleic acids, carbohydrates, lipids, hormones including proteinaceous and steroid hormones, growth factors, receptor ligands, antigens, antibodies and enzymes, (as outlined below, collectively "candidate agents"), and the like.

NEP activity can be measured using established methods. Indig F. E., et al., FEBS Lett., 255:237-240 (1989). Li C. and Hersh L. B., Neprilysin: assay methods, purification, and characterization in Methods in Enzymology (Barrett, A. J., ed.) Vol. 248, pp. 253-263, Academic Press, New York (1995).

### 6. Thromboxane Synthase

Thromboxane synthase (TS) is a heme-containing metalloenzyme. TS catalyzes the conversion of the prostaglandin endoperoxide into thromboxane A2, a potent vasoconstrictor and inducer of platelet aggregation. In concert with prostacyclin, thromboxane A2 plays a pivotal role in the maintenance of hemostasis. Due to the physiologic roles of thromboxane, inhibitors of TS have been studies for the treatment of a variety of diseases, such as stroke, transient ischemic attack, asthma, hypertension, peripheral vascular disease and inflammatory bowel disease. Dogne J. M. et al., Curr. Pharm. Des., 12:903-23 (2006).

Ozagrel sodium and ozagrel hydrochloride are potent TS inhibitors that are marketed for the treatment of cerebral ischemic symptoms resulting from cerebrovascular spasms after surgery for subarachnoid hemorrhage. See U.S. Patent No. 4,226,878, which is incorporated herein by reference in its entirety.

A series of 3-(1-imidazolylalkyl)indoles have been disclosed as potent inhibitors of TS for potential use as anti-platelet agents. See U.S. Patent No. 4,273,782, which is incorporated herein by reference in its entirety.

A series of N-(phenoxyalkyl)imidazoles have been demonstrated to be selective inhibitors of the TS with potential use in the treatment of stroke. See U.S. Patent No. 4,602,016, which is incorporated herein by reference in its entirety.

A series of imidazole derivatives have been disclosed as inhibitors of TS with potential use as anticoagulants. See U.S. Patent No. 6,258,834, which is incorporated herein by reference in its entirety.

A series of [[[(3-pyridinyl)methylene]amino]oxy]alkanoic acids and esters have been demonstrated to be selective and potent inhibitors of TS with potential use in inflammatory bowel disease. See U.S. Patent No. 4,746,671, which is incorporated herein by reference in its entirety.

A series of thianaphthene derivatives have been disclosed as potent inhibitors of TS with potential use in the treatment of inflammatory diseases such as asthma. See U.S. Patent Nos. 4,847,272 and 5,021,444, each of which is incorporated herein by reference in its entirety.

A series of N-imidazolyl derivatives of bicyclic compounds have been demonstrated to be potent inhibitors of TS for potential use in renal diseases. See U.S. Patent No. 4,882,347, which is incorporated herein by reference in its entirety.

A series of pyridyl derivatives have been disclosed as inhibitors of TS with potential use in peripheral vascular disease. See U.S. Patent No. 5,482,948, which is incorporated herein by reference in its entirety.

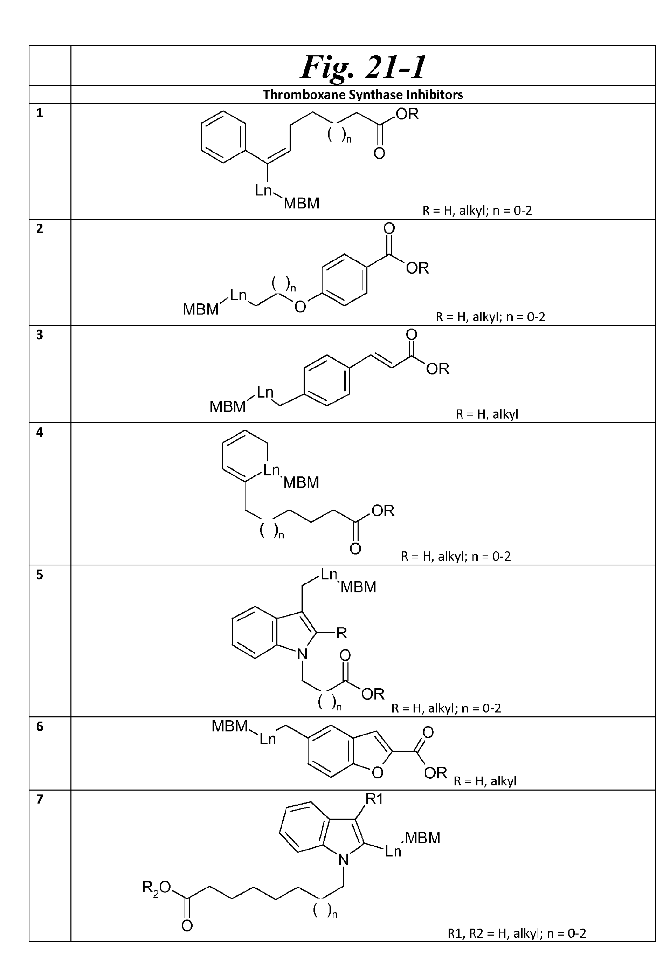

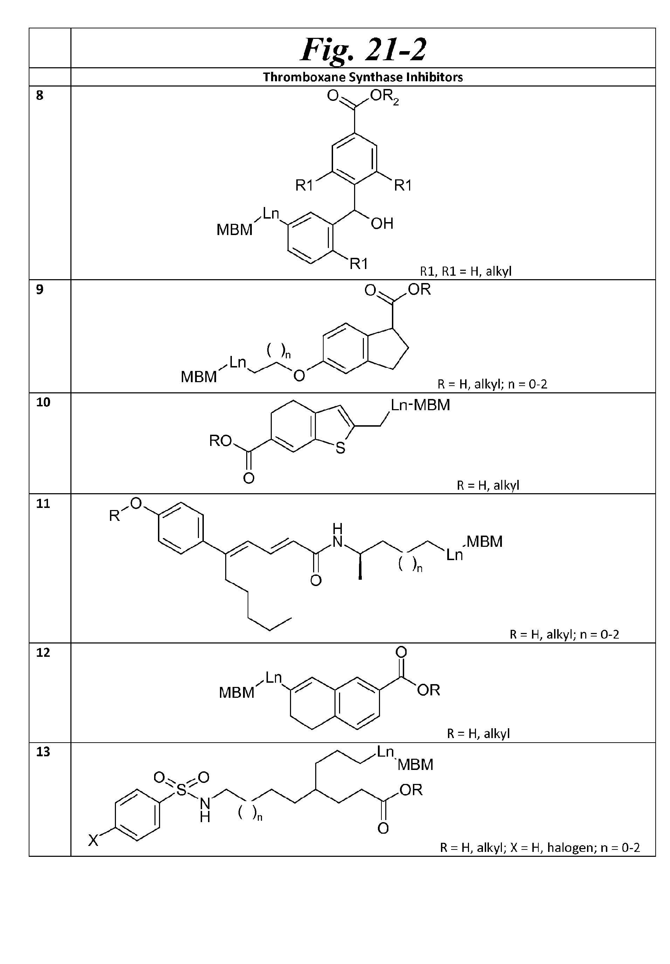

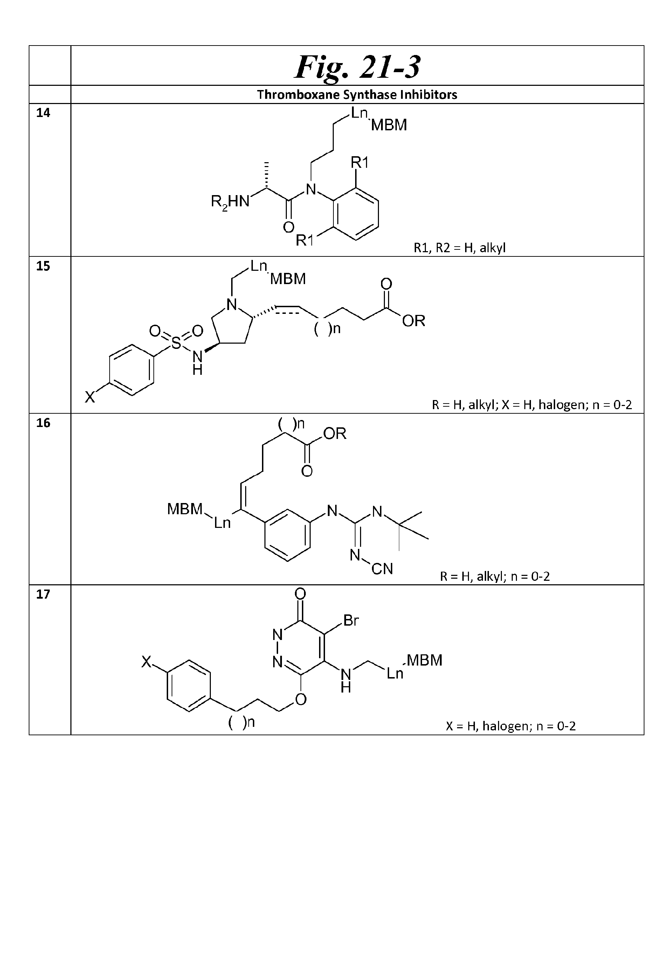

Figure 21 depicts a number of inhibitors of TS, which are suitable for use as targeting moieties in the present invention. Figure 21 shows the structure of these known inhibitors along with possible sites of attachment of the linkers and metal binding moieties ("R"), as well as possible derivatives.

Accordingly, in some embodiments, the metalloenzyme inhibitor is a thromboxane synthase inhibitor comprising a targeting moiety, a metal binding moiety, and optionally a linker, and wherein the inhibitor has a formula selected from the group consisting of:
(a) 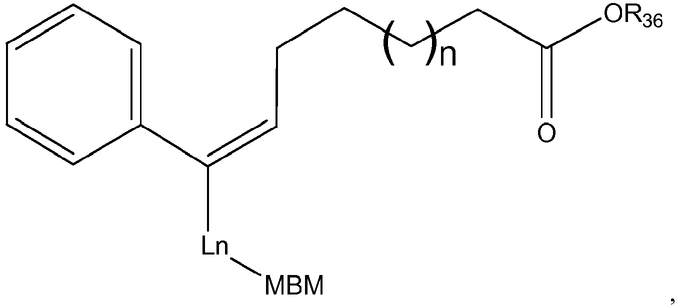 wherein:
   n is an integer from 0 to 2; and
   R₃₆ is selected from the group consisting of H, alkyl, and substituted alkyl;
(b) 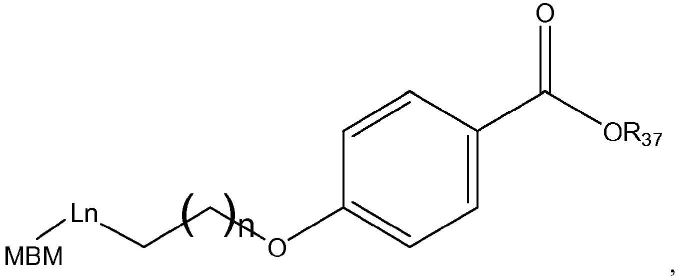 wherein:
   n is an integer from 0 to 2; and
   R₃₇ is selected from the group consisting of H, alkyl, and substiuted alkyl;
(c) 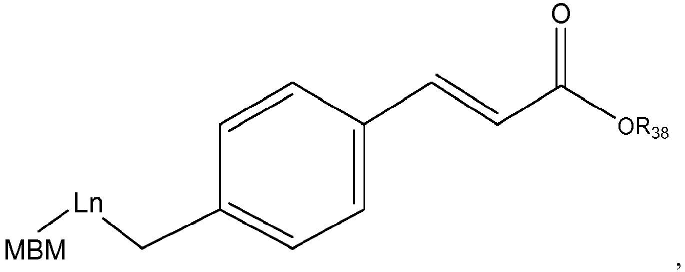 wherein R₃₈ is selected from the group consisting of H, alkyl, and substituted alkyl;
(d) 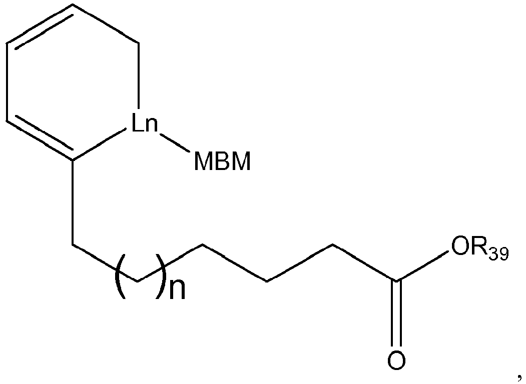 wherein:
   n is an integer from 0 to 2; and
   R₃₉ is selected from the group consisting of H, alkyl, and substituted alkyl;
(e) 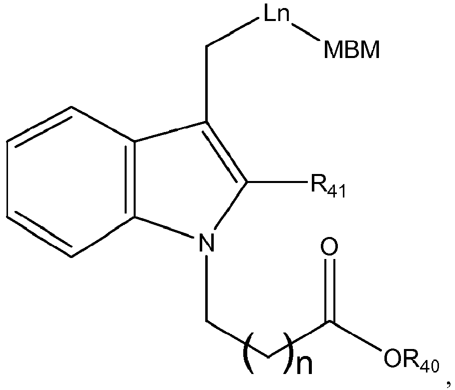 wherein:
   n is an integer from 0 to 2; and
   R₄₀ and R₄₁ are each independently selected from the group consisting of H, alkyl, and substituted alkyl;
(f) 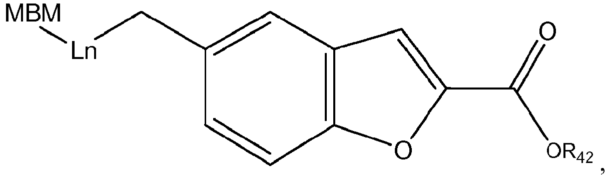 wherein R₄₂ is selected from the group consisting of H, alkyl, and substituted alkyl;
(g) 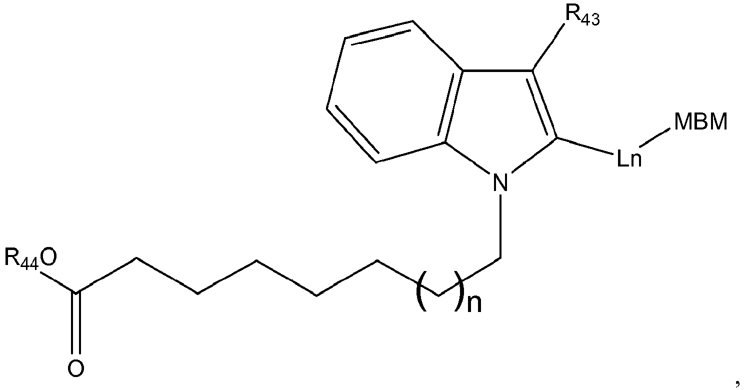 wherein:
   n is an integer from 0 to 2; and
   R₄₃ and R₄₄ are each independently selected from the group consisting of H, alkyl, and substituted alkyl;
(h) 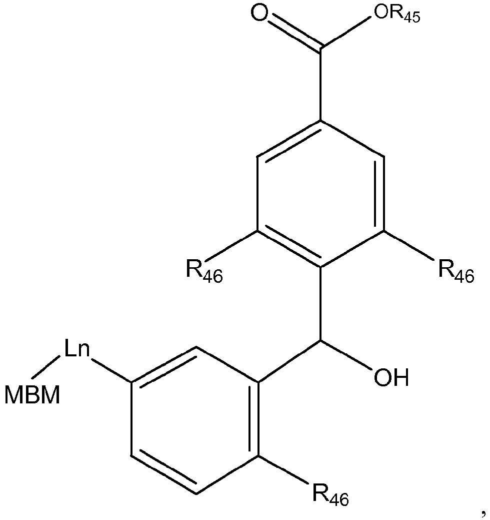 wherein R₄₅ and each R₄₆ are independently selected from the group consisting of H, alkyl, and substituted alkyl;
(i) 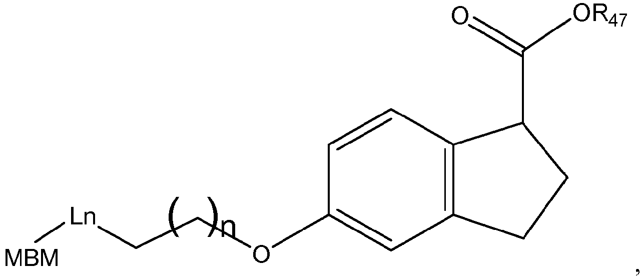 wherein:
   n is an integer from 0 to 2; and
   R₄₇ is selected from the group consisting of H, alkyl, and substituted alkyl;
(j) 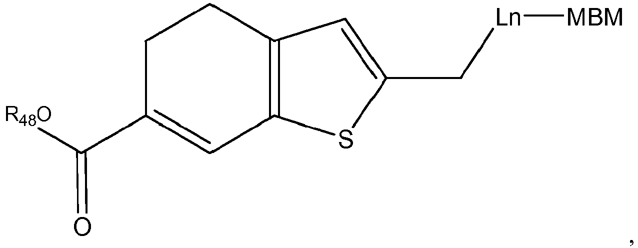 wherein R₄₈ is selected from the group consisting of H, alkyl, and substituted alkyl;
(k) 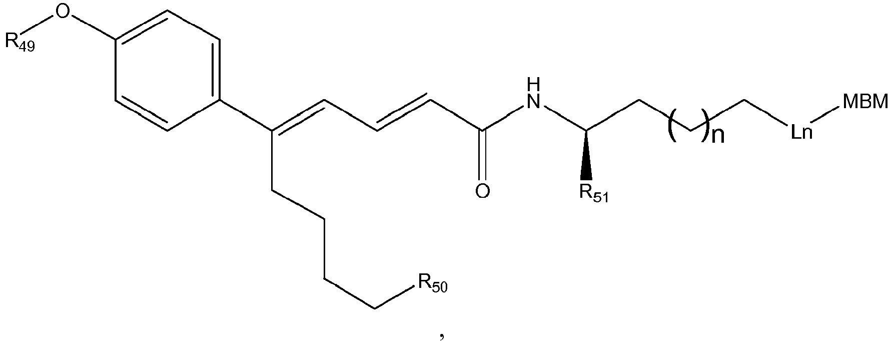 wherein:
   n is an integer from 0 to 2; and
   R₄₉ is selected from the group consisting of H, alkyl, and substituted alkyl; and
   R₅₀ and R₅₁ are each independently alkyl or substituted alkyl;
(l) 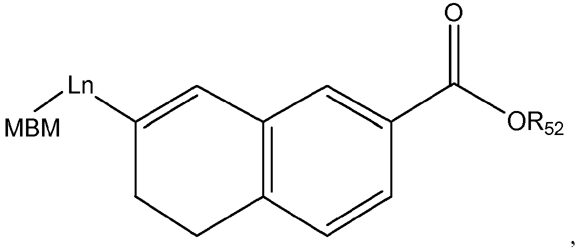 wherein R₅₂ is selected from the group consisting of H, alkyl, and substituted alkyl;
(m) 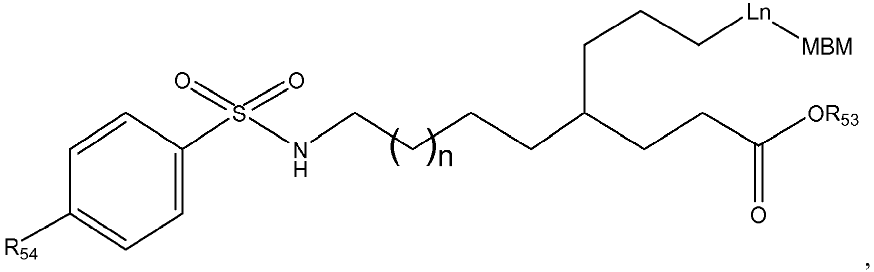 wherein:
   n is an integer from 0 to 2;
   R₅₃ is selected from the group consisting of H, alkyl, and substituted alkyl; and
   R₅₄ is H or halogen;
(n) 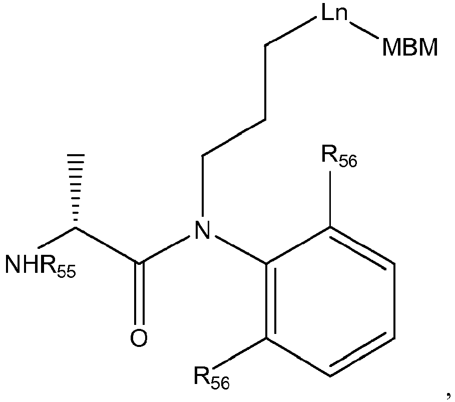 wherein:
   R₅₅ and each R₅₆ is independently selected from the group consisting of H, alkyl, and substituted alkyl;
(o) 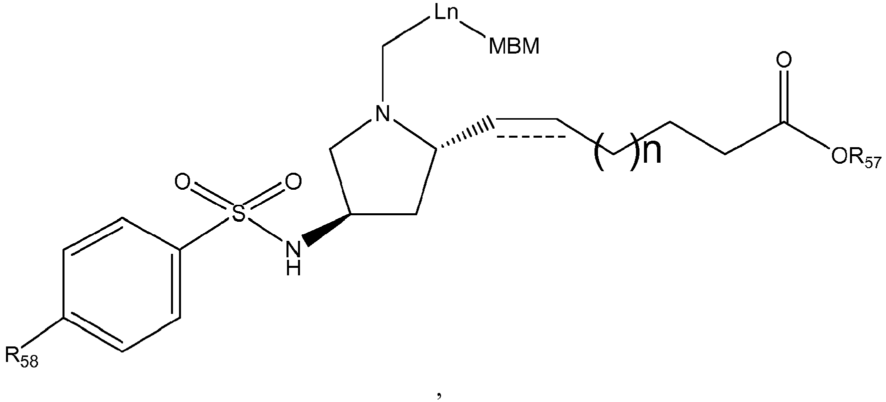 wherein:
   n is an integer from 0 to 2;
   R₅₇ is selected from the group consisting of H, alkyl, and substituted alkyl; and
   R₅₈ is H or halogen;
(p) 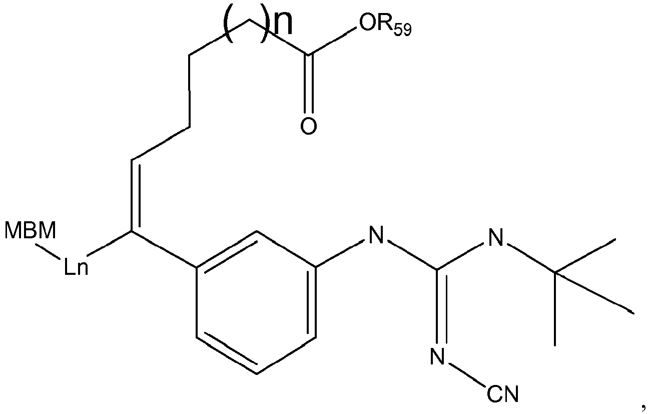 wherein:
   n is an integer from 0 to 2; and
   R₅₉ is selected from the group consisting of H, alkyl, and substituted alkyl;
(q) 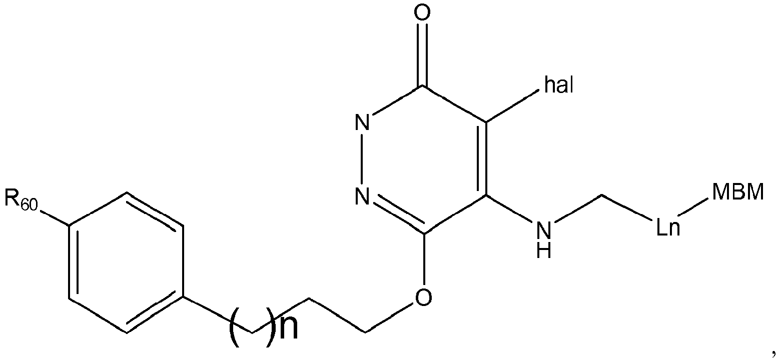 wherein:
   n is an integer from 0 to 2;
   hal is halogen; and
   R₆₀ is H or halogen; and
   wherein:
   MBM is a metal binding moiety;
   Lₙ is a linker, wherein n is an integer from 0 to 1;
   under the proviso that the metal binding moiety is not a metal binding moiety selected from the group consisting of: 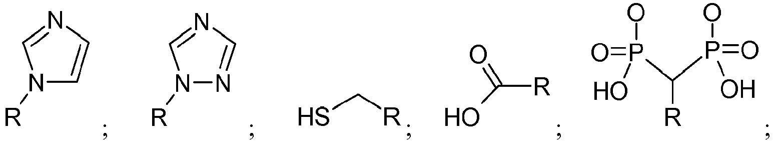 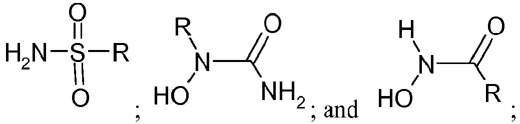 wherein:
      R is an attachment site through which the metal binding moiety can be attached to the targeting moiety, optionally through linker, Lₙ.

In addition to these targeting moieties, other known targeting moieties, identified by the screens outlined below or shown to bind to TS can be used. Thus, suitable targeting moieties include, but are not limited to, small organic molecules including known drugs and drug candidates, polysaccharides, fatty acids, vaccines, polypeptides, proteins (including peptides, as described herein), nucleic acids, carbohydrates, lipids, hormones including proteinaceous and steroid hormones, growth factors, receptor ligands, antigens, antibodies and enzymes, (as outlined below, collectively "candidate agents"), and the like.

TS activity can be measured using established methods. Dannhardt et al., Archiv der Pharmazie, 331:359 - 364 (1999). Hecker M. et al., Arch Biochem Biophys, 254:124-35 (1987).

### 7. Anthrax Lethal Factor

Anthrax lethal factor (ALF) is a zinc-containing metalloenzyme that is critical in the pathogenesis of anthrax. ALF is a highly specific protease that cleaves members of the mitogen-activated protein kinase kinase (MAPKK) family near the amino termini, leading to the inhibition of one or more signaling pathways. ALF comprises four domains: domain I binds the membrane-translocating component of anthrax toxin, the protective antigen; domains II, III and IV together create a long deep groove that holds the 16-residue N-terminal tail of MAPKK-2 before cleavage. Domain II resembles the ADP-ribosylating toxin from Bacillus cereus, but the active site has been mutated and recruited to augment substrate recognition. Domain III is inserted into domain II, and seems to have arisen from a repeated duplication of a structural element of domain II. Domain IV is related to the zinc metalloprotease family, and contains the catalytic centre; it also resembles domain I. Although antibiotics may be used to kill the anthrax bacteria, the level of toxin has often become so high in the bloodstream that removing the bacteria alone is not sufficient to prevent death. Therefore, the use of agents that specifically inhibit the activity of ALF are of potential benefit in the treatment of anthrax infection. Pannifer A. D. et al., Nature, 414:229-233 (2001).

A series of 4-oxo-2-thioxothiazolidin-3-yl derivatives have been demonstrated to be inhibitors of ALF. See WO 2000/010573.

A series of hydroxamate analogues have been disclosed as potent inhibitors of ALF with potential use in the treatment of anthrax infection. See U.S. Patent Application Publication No. 2005/148,629.

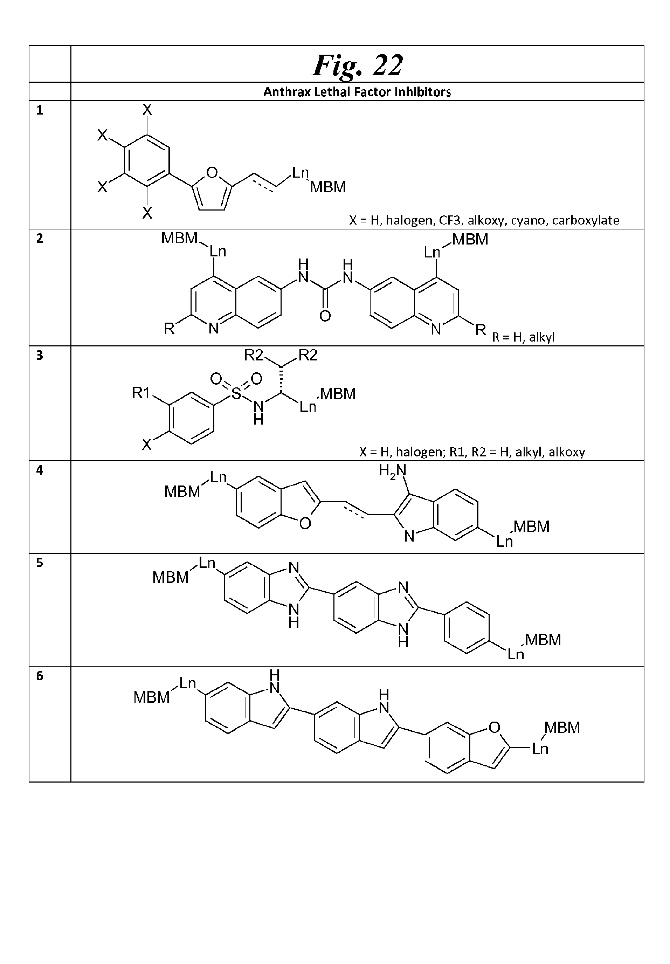

Figure 22 depicts a number of inhibitors of ALF, which are suitable for use as targeting moieties in the present invention. Figure 22 shows the structure of these known inhibitors along with possible sites of attachment of the linkers and metal binding moieties ("R"), as well as possible derivatives.

Accordingly, in some embodiments, the metalloenzyme inhibitor is an anthrax lethal factor inhibitor comprising a targeting moiety, a metal binding moiety, and optionally a linker, and wherein the inhibitor has a formula selected from the group consisting of:
(a) 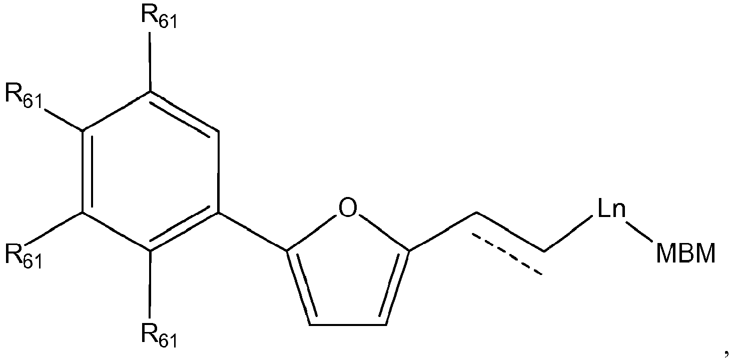 wherein each R₆₁ is independently selected from the group consisting of H, halogen, CF₃, alkoxy, cyano, and carboxylate;
(b) 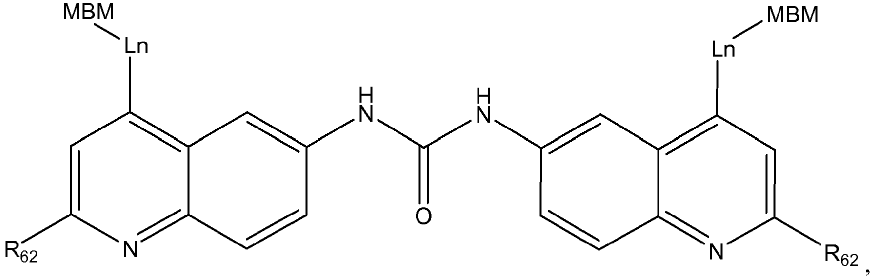 wherein each R₆₂ is independently selected from the group consisting of H, alkyl, and substituted alkyl;
(c) 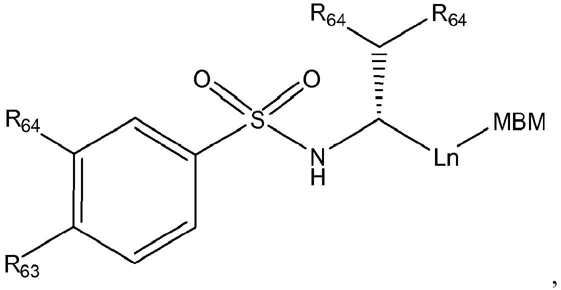 wherein:
   R₆₃ is H or halogen; and
   each R₆₄ is independently selected from the group consisting of H, alkyl, substituted alkyl, and alkoxyl;
(d) 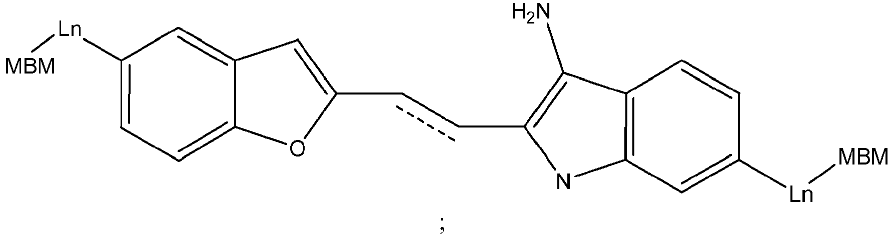
(e) 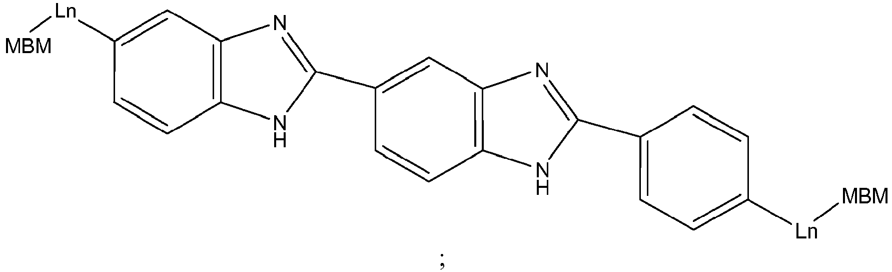
(f) 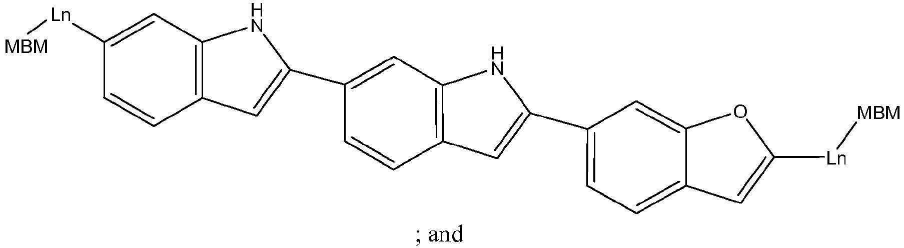 and
   wherein:
   MBM is a metal binding moiety;
   Lₙ is a linker, wherein n is an integer from 0 to 1;
   under the proviso that the metal binding moiety is not a metal binding moiety selected from the group consisting of: 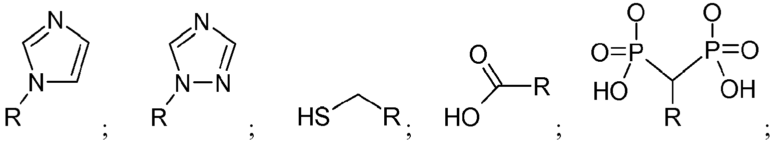 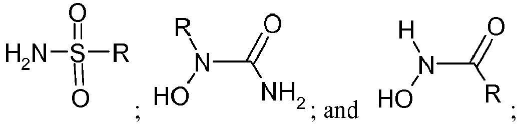 wherein:
      R is an attachment site through which the metal binding moiety can be attached to the targeting moiety, optionally through linker, Lₙ.

In addition to these targeting moieties, other known targeting moieties, identified by the screens outlined below or shown to bind to ALF can be used. Thus, suitable targeting moieties include, but are not limited to, small organic molecules including known drugs and drug candidates, polysaccharides, fatty acids, vaccines, polypeptides, proteins (including peptides, as described herein), nucleic acids, carbohydrates, lipids, hormones including proteinaceous and steroid hormones, growth factors, receptor ligands, antigens, antibodies and enzymes, (as outlined below, collectively "candidate agents"), and the like.

ALF activity can be measured using established methods. Tonello F. et al., Nature, 418:386 (2002). Johnson S. L. et al., Bioorganic Chemistry, 35:306-312 (2007).

### 8. Endothelin Converting Enzyme (ECE)

Endothelin converting enzyme (ECE) is a membrane-bound zinc metalloenzyme. Endothelin-1 (ET-1) is a potent peptidic vasoconstrictor. Similar to many other peptidic hormones, ET-1 is initially synthesized as a large precursor and subsequently cleaved by proteolytic enzymes to form the mature peptide. The final step of post-translational processing is catalyzed by ECE. Thus, inhibitors of ECE should suppress the biosynthesis of ET-1 and, therefore, would have beneficial effects on diseases where an overproduction of ET-1 may play a pathogenic role. As such, inhibitors of ECE have been examined for potential use in the treatment of hypertension, congestive heart failure, cerebrovascular disease, angina and diabetic nephropathy. Turner A. J. et al., Trends in Pharmacological Sciences, 19:483-486 (1998).

A series of phosphono/biaryl substituted amino acid derivatives have been disclosed as inhibitors of ECE with potential use in the treatment of hypertension. See U.S. Patent Nos. 5,273,990 and 5,250,522, each of which is incorporated herein by reference in its entirety.

A series of benzazepin-, benzoxazepin- and benzothiazepin-N-acetic acid-derivatives have been demonstrated to be potent inhibitors of ECE for potential use in congestive heart failure. See U.S. Patent No. 5,677,297, which is incorporated herein by reference in its entirety.

A series of thiol derivatives have been disclosed as inhibitors of ECE for use in the treatment of hypertension. See WO 1999/055726.

A series of heteroaryl-substituted thiol derivatives have been disclosed as inhibitors of ECE for potential use in cerebrovascular diseases. See U.S. Patent No. 6,426,354, which is incorporated herein by reference in its entirety.

A series of pyrrolidine derivatives have been demonstrated to be inhibitors of ECE with potential use in the treatment of congestive heart failure. See U.S. Patent Nos. 6,790,860 and 7,189,756, each of which is incorporated herein by reference in its entirety.

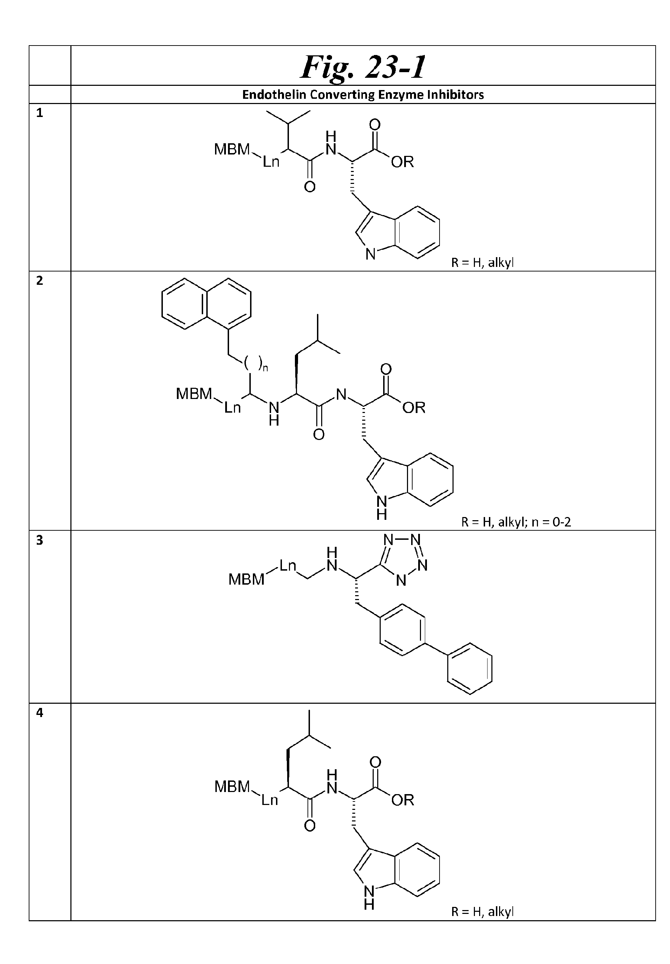

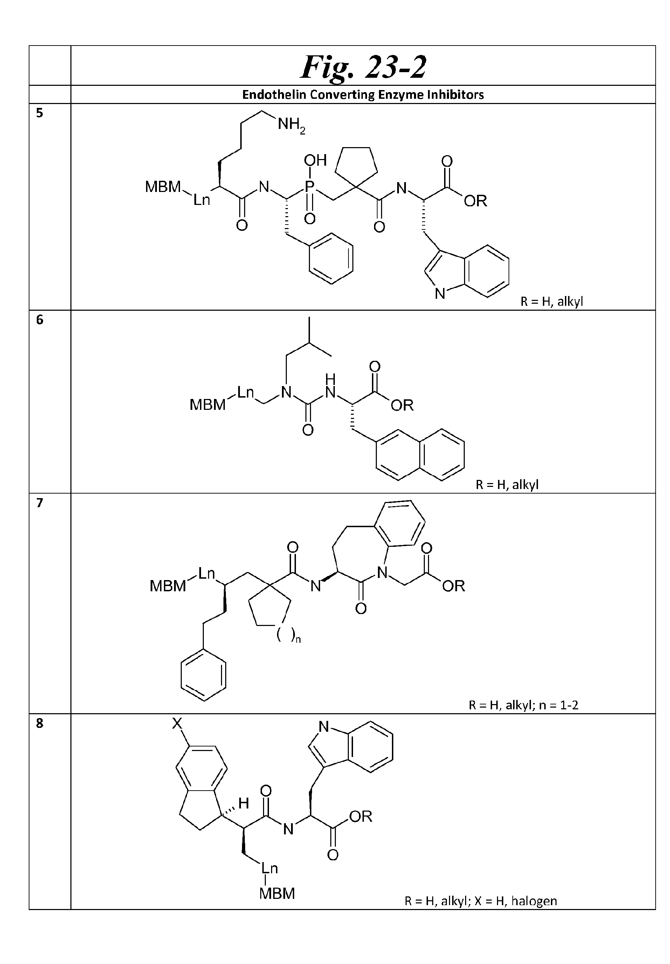

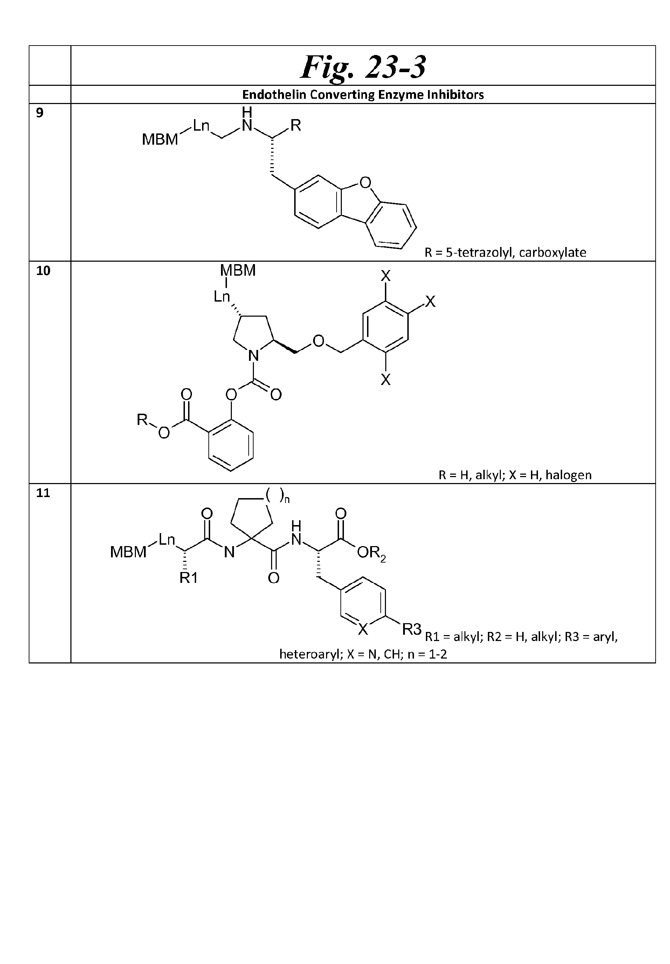

Figure 23 depicts a number of inhibitors of ECE, which are suitable for use as targeting moieties in the present invention. Figure 23 shows the structure of these known inhibitors along with possible sites of attachment of the linkers and metal binding moieties ("R"), as well as possible derivatives.

Accordingly, in some embodiments, the metalloenzyme inhibitor is an endothelin converting enzyme inhibitor comprising a targeting moiety, a metal binding moiety, and optionally a linker, and wherein the inhibitor has a formula selected from the group consisting of:
(a) 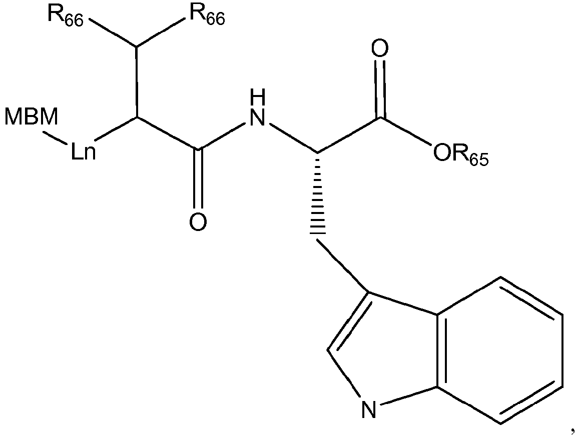 wherein:
   R₆₅ is selected from the group consisting of H, alkyl, and substituted alkyl; and
   each R₆₆ is independently alkyl or substituted alkyl;
(b) 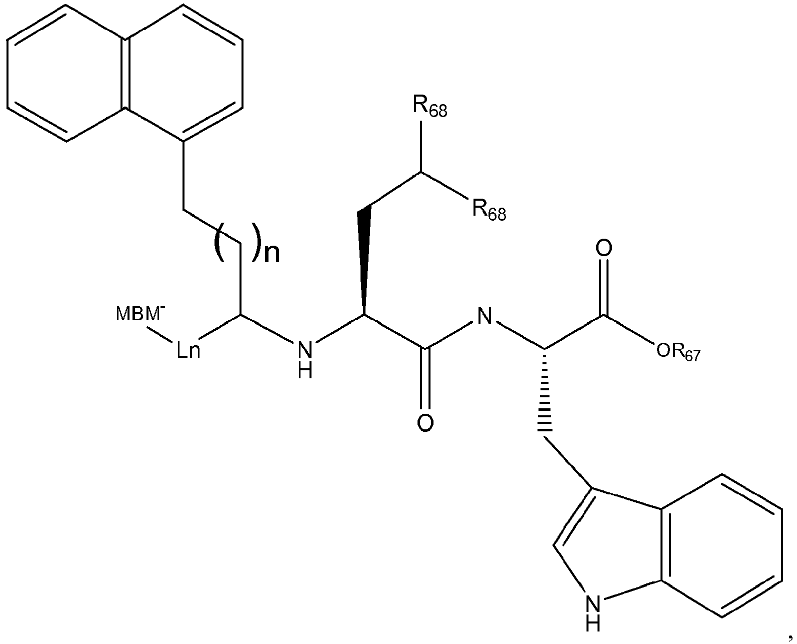
   n is an integer from 0 to 2; and
   R₆₇ is selected from the group consisting of H, alkyl, and substituted alkyl; and
   each R₆₈ is independently alkyl or substituted alkyl;
(c) 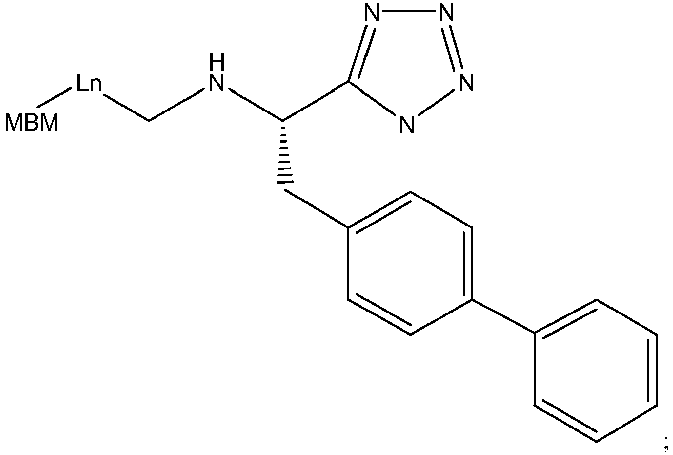
(d) 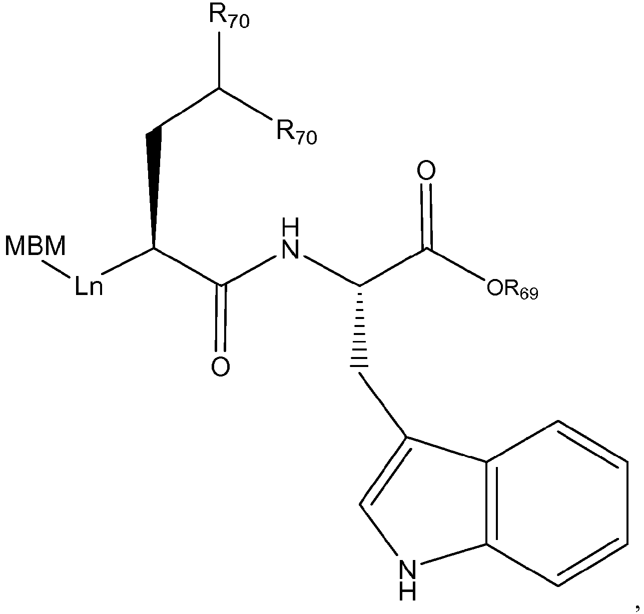 wherein:
   R₆₉ is selected from the group consisting of H, alkyl, and substituted alkyl; and
   cach R₇₀ is independently alkyl or substituted alkyl;
(e) 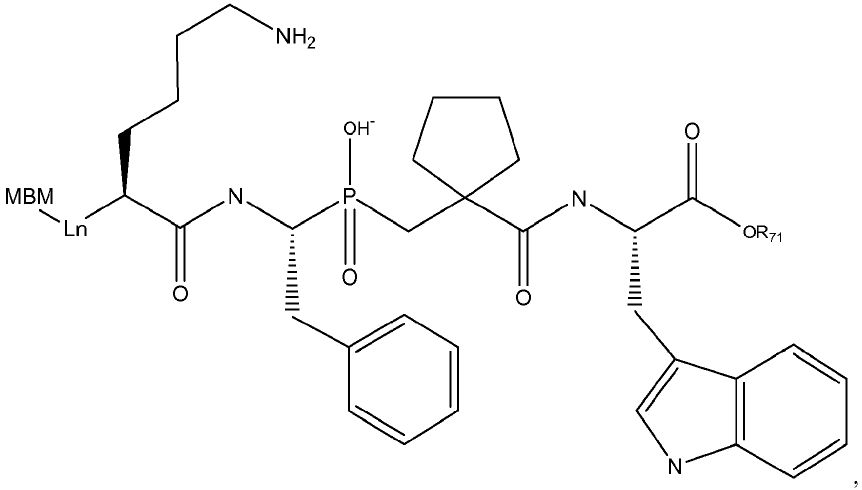 wherein R₇₁ is selected from the group consisting ofH, alkyl, and substituted alkyl;
(f) 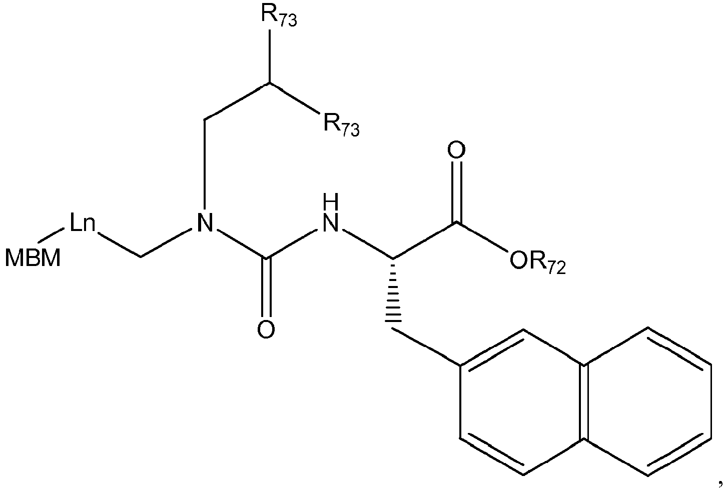 wherein:
   R₇₂ is selected from the group consisting of H, alkyl, and substituted alkyl; and
   each R₇₃ is independently alkyl or substituted alkyl;
(g) 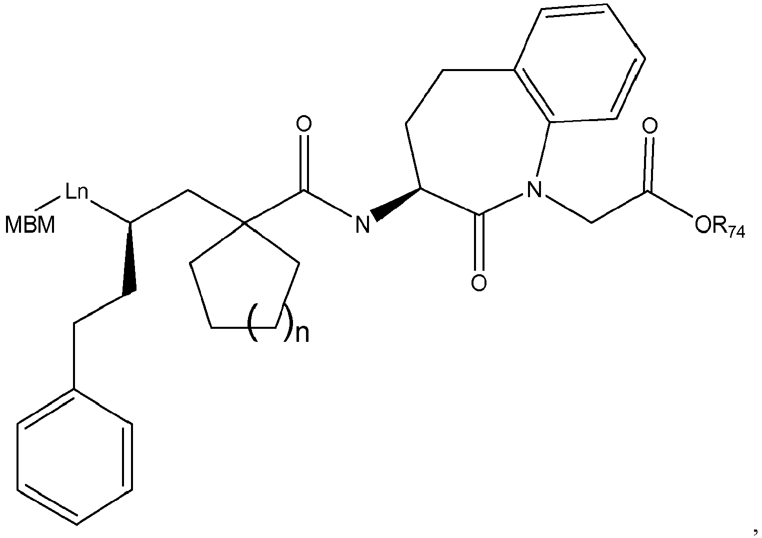
   n is an integer from 1 to 2; and
   R₇₄ is selected from the group consisting of H, alkyl, and substituted alkyl;
(h) 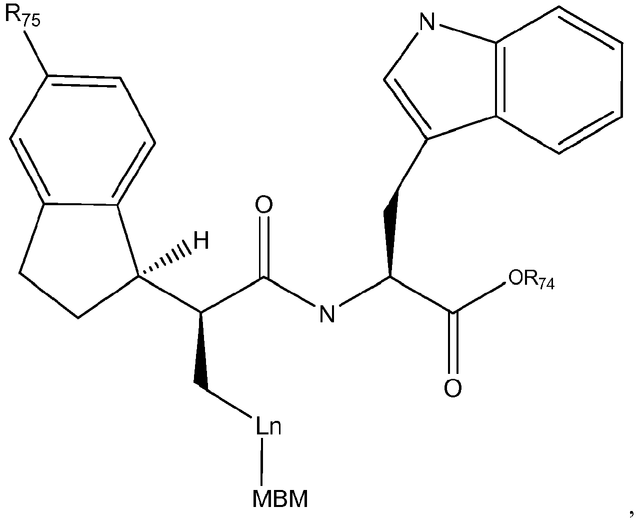 wherein:
   R₇₄ is selected from the group consisting of H, alkyl, and substituted alkyl;
   R₇₅ is H or halogen;
(i) 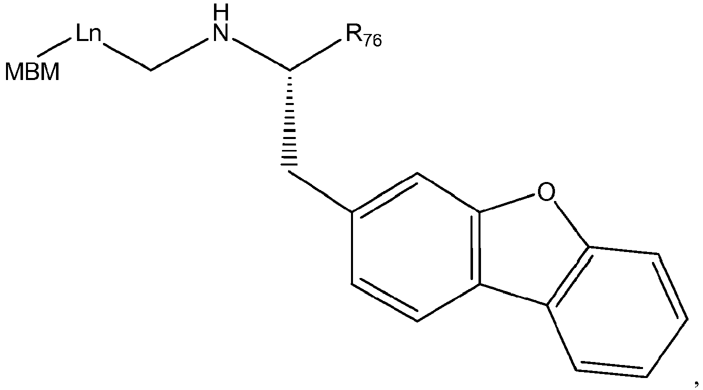 wherein R₇₆ is 5-tetrazolyl or carboxylate;
(j) 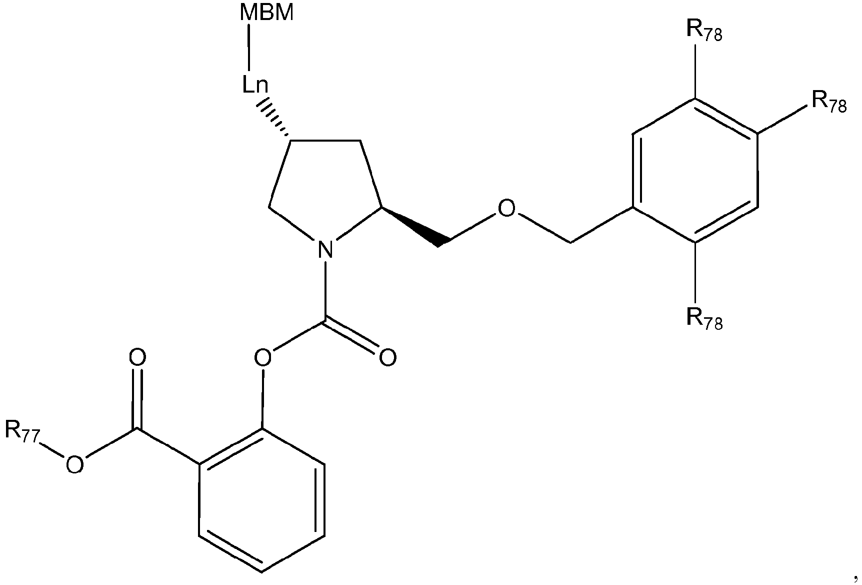 wherein:
   R₇₇ is selected from the group consisting of H, alkyl, and substituted alkyl; and
   each R₇₈ is independently H or halogen;
(k) 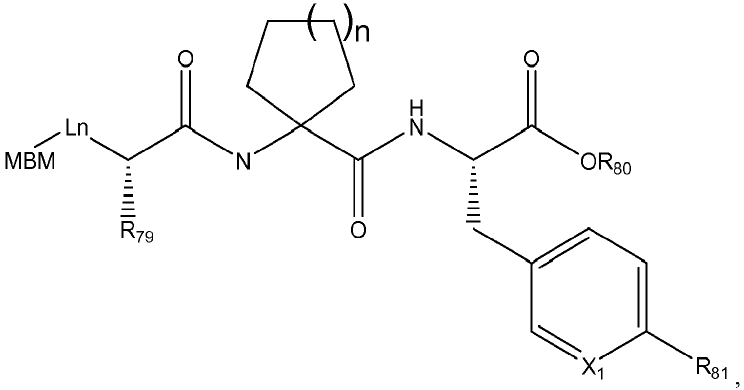 wherein:
   n is an integer from 1 to 2;
   X₁ is N or CH;
   R₇₉ is alkyl or substituted alkyl;
   R₈₀ is selected from the group consisting of H, alkyl, and substituted alkyl;
   R₈₁ is selected from the group consisting of aryl, substituted aryl, heteroaryl, and substituted heteroaryl; and
   wherein:
   MBM is a metal binding moiety;
   Lₙ is a linker, wherein n is an integer from 0 to 1;
   under the proviso that the metal binding moiety is not a metal binding moiety selected from the group consisting of: 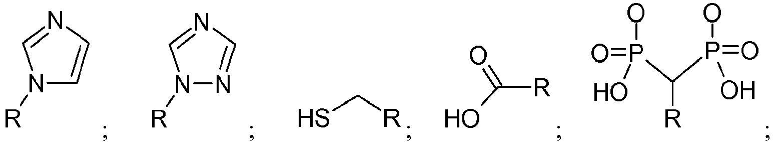 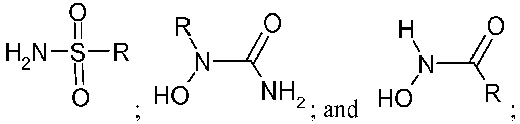 wherein:
      R is an attachment site through which the metal binding moiety can be attached to the targeting moiety, optionally through linker, Lₙ.

In addition to these targeting moieties, other known targeting moieties, identified by the screens outlined below or shown to bind to ECE can be used. Thus, suitable targeting moieties include, but are not limited to, small organic molecules including known drugs and drug candidates, polysaccharides, fatty acids, vaccines, polypeptides, proteins (including peptides, as described herein), nucleic acids, carbohydrates, lipids, hormones including proteinaceous and steroid hormones, growth factors, receptor ligands, antigens, antibodies and enzymes, (as outlined below, collectively "candidate agents"), and the like.

ECE activity can be measured using established methods. Parnot C. et al., Hypertension, 30:837-844 (1997); Ahn K. et al., Biochem Biophys Res Comm, 243:184-190 (1998).

### 9. Methionine aminopeptidase (MAP)

Methionine aminopeptidase (MAP) is a cobalt-containing, membrane-bound metalloenzyme. MAP is responsible for removing the amino-terminal methionine from nascent proteins during protein processing in eukaryotes. Two subfamilies of MAP enzymes are known to exist. While being evolutionary related, they only share a limited amount of sequence similarity mostly clustered around the residues shown to be involved in cobalt-binding. The first family consists of enzymes from prokaryotes as well as eukaryotic MAP-1, while the second group is made up of archaeal MAP and eukaryotic MAP-2. The second subfamily also includes proteins which do not seem to be MAP, but that are clearly evolutionary related such as mouse proliferation-associated protein 1 and fission yeast curved DNA-binding protein. Inhibitors of MAP have been shown to be potent inhibitors of angiogenesis. As such, inhibitors of MAP have potential for use in the treatment of cancer. Liu S. et al., Science, 282:1324 - 1327 (1998).

A series of hydrazide and alkoxyamide analogues have been disclosed as inhibitors of MAP with potential use in the treatment of cancer. See U.S. Patent No. 6,887,863 and U.S. Patent Application Publication No. 2004/077,031, each of which is incorporated herein by reference in its entirety.

A series of triazole derivatives have been demonstrated to be potent inhibitors of MAP with potential use as anti-neoplastic agents. See WO 2003/031434.

A series of sulfonamide derivatives have been disclosed as potent inhibitors of MAP with potential use in the treatment of cancer. See U.S. Patent Application Publication Nos. 2004/068012, 2004/157836, and 2004/167128, each of which is incorporated herein by reference in its entirety.

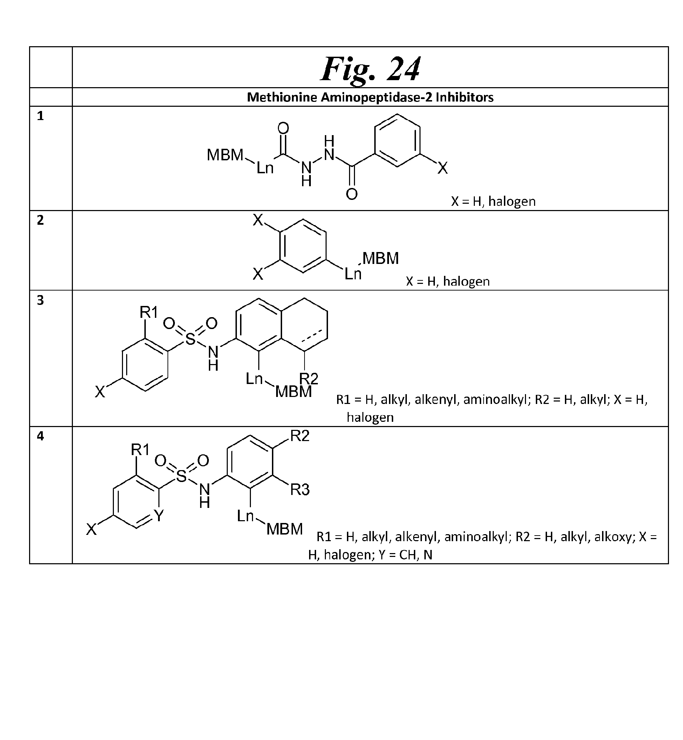

Figure 24 depicts a number of inhibitors of MAP, which are suitable for use as targeting moieties in the present invention. Figure 24 shows the structure of these known inhibitors along with possible sites of attachment of the linkers and metal binding moieties ("R"), as well as possible derivatives.

Accordingly, in some embodiments, the metalloenzyme inhibitor is a methionine aminopeptidase-2 inhibitor comprising a targeting moiety, a metal binding moiety, and optionally a linker, and wherein the inhibitor has a formula selected from the group consisting of:
(a) 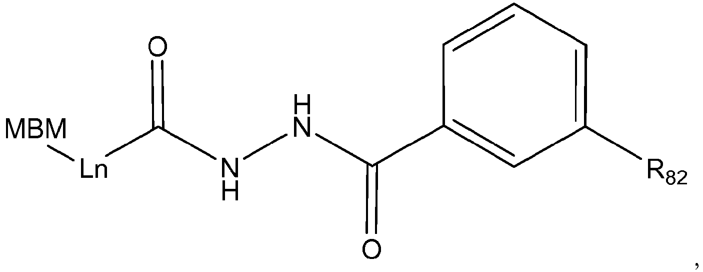 wherein R₈₂ is H or halogen;
(b) 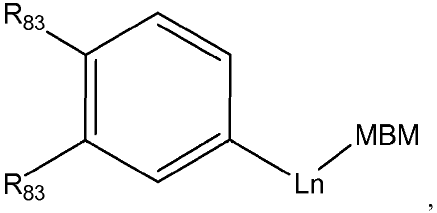 wherein each R₈₃ is independently H or halogen;
(c) 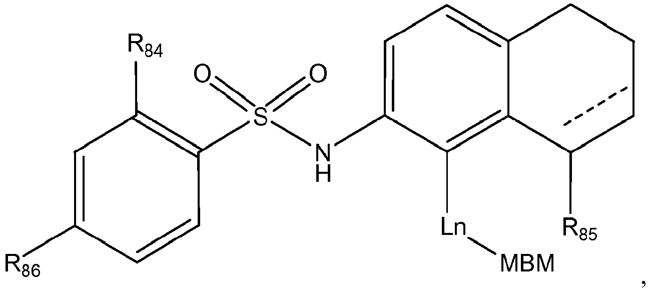 wherein:
   R₈₄ is selected from the group consisting of H, alkyl, substituted alkyl, alkenyl, and aminoalkyl;
   R₈₅ is selected from the group consiting of H, alkyl, and substituted alkyl; and
   R₈₆ is H or halogen;
(d) 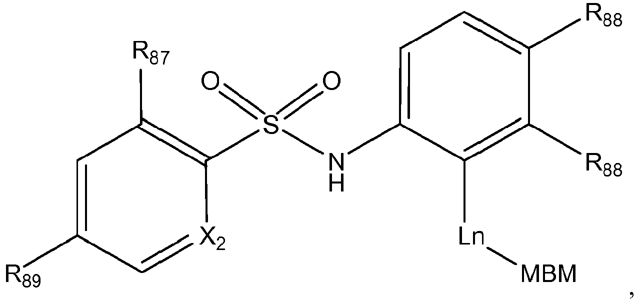 wherein:
   R₈₇ is selected from the group consisting of H, alkyl, substituted alkyl, alkenyl, and aminoalkyl;
   each R₈₈ is independently selected from the group consisting of H, alkyl, substituted alkyl, and alkoxyl;
   R₈₉ is H or halogen; and
   X₂ CH or N;
   wherein:
   MBM is a metal binding moiety;
   Lₙ is a linker, wherein n is an integer from 0 to 1;
   under the proviso that the metal binding moiety is not a metal binding moiety selected from the group consisting of: 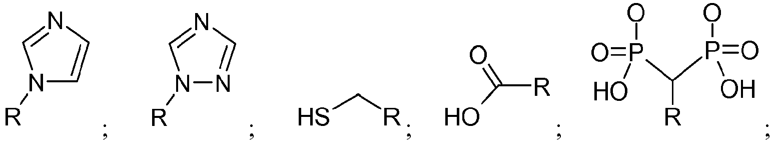 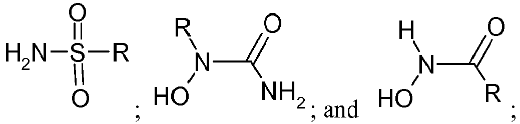 wherein:
      R is an attachment site through which the metal binding moiety can be attached to the targeting moiety, optionally through linker, Lₙ.

In addition to these targeting moieties, other known targeting moieties, identified by the screens outlined below or shown to bind to MAP can be used. Thus, suitable targeting moieties include, but are not limited to, small organic molecules including known drugs and drug candidates, polysaccharides, fatty acids, vaccines, polypeptides, proteins (including peptides, as described herein), nucleic acids, carbohydrates, lipids, hormones including proteinaceous and steroid hormones, growth factors, receptor ligands, antigens, antibodies and enzymes, (as outlined below, collectively "candidate agents"), and the like.

MAP activity can be measured using established methods. Larrabee J. A. et al., Anal Biochem, 269:194-198 (1999); Griffith E. C. et al., Proc Natl Acad Sci USA, 95:15183-15188(1998).

### 10. Peptide deformylase (PDF)

Peptide deformylase (PDF) is a highly conserved, iron-containing metalloenzyme. PDF is an essential enzyme in the bacterial life cycle. It catalyses the removal of the terminal formyl group from the N-terminal methionine residue of the nascent bacterial polypeptide chain. Thus, inhibition of PDF, by interfering with the bacterial protein maturation process, is providing a promising new and exciting target for antibiotic therapeutic intervention without interfering with eukaryotic metabolism. Antibiotics based on PDF inhibition have the potential to provide the much needed antibacterial activity against most of the major drug-resistant pathogens and could play a significant role in the treatment of community-acquired upper respiratory tract infections. Chen D., Yuan Z., Expert Opin Investig Drugs, 14:1107-16 (2005).

(-)- actinonin has been demonstrated to be a potent inhibitor of PDF. See U.S. Patent No. 6,660,741, which is incorporated herein by reference in its entirety.

A series of hydroxamate derivatives have been disclosed as potent inhibitors of PDF for potential use in infectious disease. See U.S. Patent Nos. 6,423,690, 6,787,522 and 7,148,198, each of which is incorporated herein by reference in its entirety.

A series of succinate derivatives have been disclosed as inhibitors of PDF. See U.S. Patent No. 6,797,820, which is incorporated herein by reference in its entirety.

A series of hydroxamic acid derivatives have been demonstrated to be potent inhibitors of PDF for use as antibacterial agents. See U.S. Patent No. 6,716,878, which is incorporated herein by reference in its entirety.

A series of urea-containing compounds have been disclosed as potent inhibitors of PDF. See U.S. Patent No. 6,852,752, which is incorporated herein by reference in its entirety.

A series of hydroxamate-containing compounds have been demonstrated to be potent inhibitors of PDF. See U.S. Patent No. 7,186,719, which is incorporated herein by reference in its entirety.

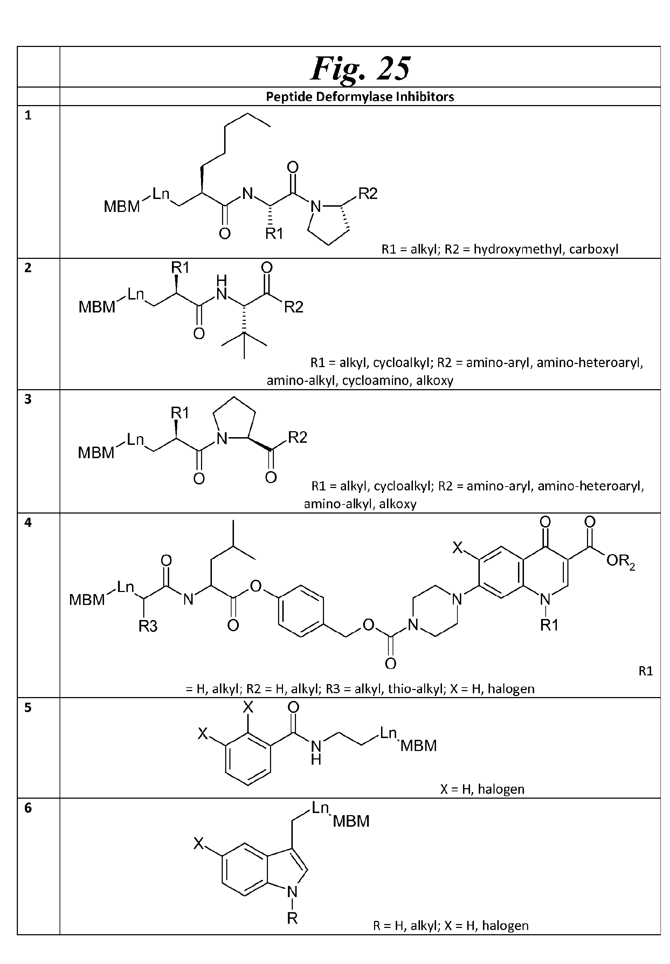

Figure 25 depicts a number of inhibitors of PDF, which are suitable for use as targeting moieties in the present invention. Figure 25 shows the structure of these known inhibitors along with possible sites of attachment of the linkers and metal binding moieties ("R"), as well as possible derivatives.

Accordingly, in some embodiments, the metalloenzyme inhibitor is a peptide deformylase inhibitor comprising a targeting moiety, a metal binding moiety, and optionally a linker, and wherein the inhibitor has a formula selected from the group consisting of:
(a) 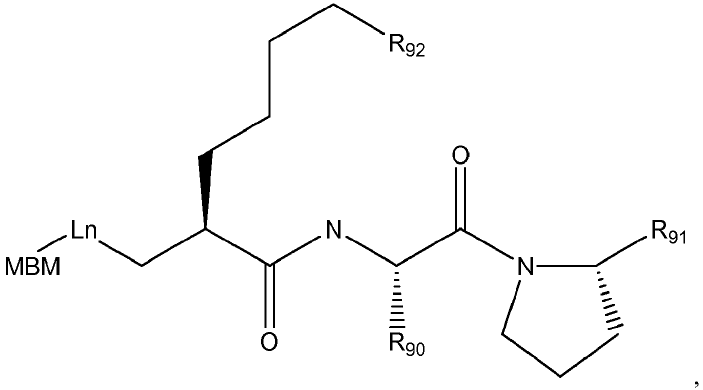
   R₉₀ and R₉₂ are each independently alkyl or substituted alkyl;
   R₉₁ is hydroxymethyl or carboxyl,
(b) 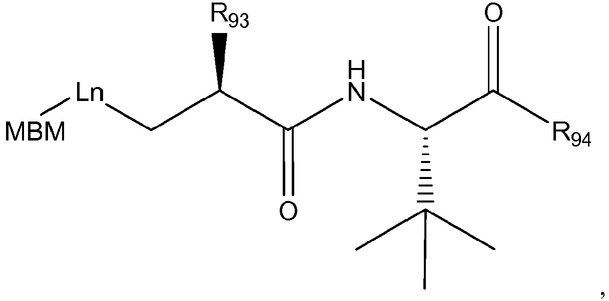 wherein:
   R₉₃ is selected from the group consisting of alkyl, substituted alkyl, cycloalkyl, substituted cycloalkyl, heterocycloalkyl, and substituted heterocycloalkyl; and
   R₉₄ is selected from the group consisting of amino-aryl, amino-heteroaryl, amino-alkyl, cycloamino, and alkoxyl;
(c) 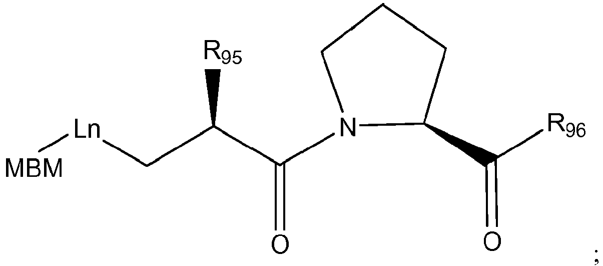 wherein:
   R₉₅ is selected from the group consisting of alkyl, substituted alkyl, cycloalkyl, substituted cycloalkyl, heterocycloalkyl, and substituted heterocycloalkyl; and
   R₉₆ is selected from the group consisting of amino-aryl, amino-heteroaryl, amino-alkyl, and alkoxyl;
(d) 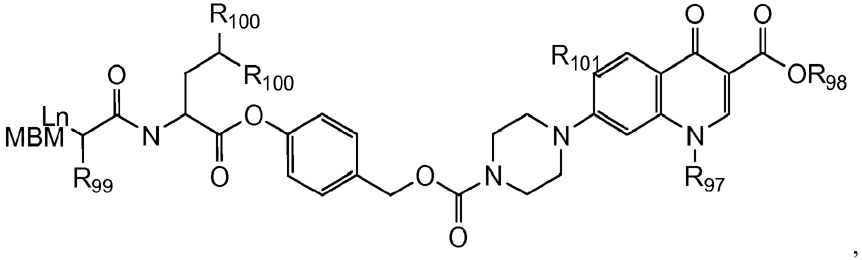 wherein:
   R₉₇ and R₉₈ are each independently selected from the group consisting of H, alkyl, and substituted alkyl;
   R₉₉ is selected from the group consisting of alkyl, substituted alkyl, and thio-alkyl;
   R₁₀₀ is alkyl or substituted alkyl; and
   R₁₀₁ is H or halogen;
(e) 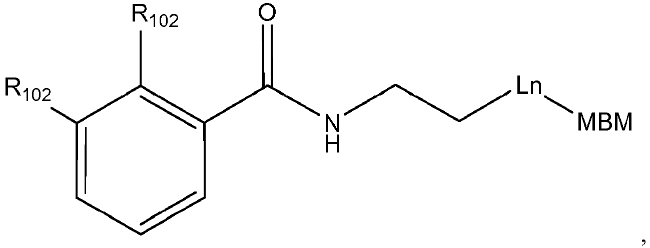 wherein each R₁₀₂ is independently H or halogen;
(f) 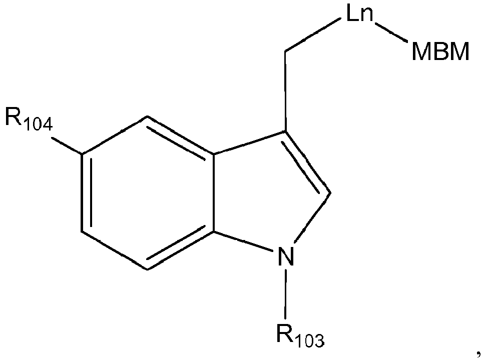
   wherein R₁₀₃ is selected from the group consisting of H, alkyl, and substituted alkyl; and
   R₁₀₄ is H or halogen;
   wherein:
   MBM is a metal binding moiety;
   Lₙ is a linker, wherein n is an integer from 0 to 1;
   under the proviso that the metal binding moiety is not a metal binding moiety selected from the group consisting of: 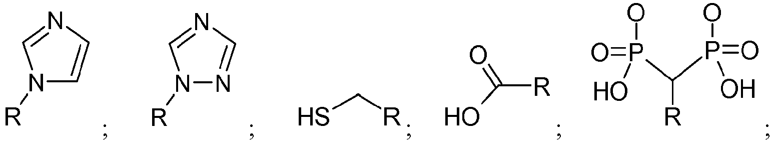 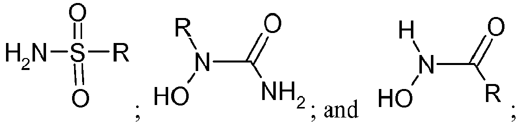 wherein:
      R is an attachment site through which the metal binding moiety can be attached to the targeting moiety, optionally through linker, Lₙ.

In addition to these targeting moieties, other known targeting moieties, identified by the screens outlined below or shown to bind to PDF can be used. Thus, suitable targeting moieties include, but are not limited to, small organic molecules including known drugs and drug candidates, polysaccharides, fatty acids, vaccines, polypeptides, proteins (including peptides, as described herein), nucleic acids, carbohydrates, lipids, hormones including proteinaceous and steroid hormones, growth factors, receptor ligands, antigens, antibodies and enzymes, (as outlined below, collectively "candidate agents"), and the like.

PDF activity can be measured using established methods. Wei Y., Pei D., Analytical Biochem, 250:29-34 (1997). Guo et al., Analytical Biochem, 273:298-304 (1999).

### 11. HIV integrase (HI)

HIV integrase (HI) is a magnesium-containing metalloenzyme. HI is one of the three key enzymes of the pol gene of HIV. HIV integrase is involved in the integration of HIV DNA into host chromosomal DNA. There is no functional equivalent of this enzyme in human cells. Integration of HIV DNA into the host cell genome occurs by a carefully defined sequence of DNA tailoring (3'-processing) and coupling (joining or integration) reactions. Integration of the viral DNA is essential for the subsequent transcription of the viral genome which leads to production of new viral genomic RNA and viral proteins needed for the production of the next round of infectious virus. Given the importance of HI in the life cycle of the HIV virus, inhibitors of HI have been a target for anti-HIV therapies. Nair V., Curr Pharm Des, 9:2553-65 (2003).

A series of 4-oxoquinoline compounds have been disclosed as inhibitors of HI. See U.S. Patent No. 7,176,220 and U.S. Patent Application Publication Nos. 2005/239,819 and 2006/217,413, each which is incorporated herein by reference in its entirety.

A series of N-substituted hydroxypyrimidinone carboxamide derivatives have been shown to be potent inhibitors of HI. See U.S. Patent No. 7,169,780 and U.S. Patent Application Publication No. 2005/025,774, each of which is incorporated herein by reference in its entirety.

A series of indole derivatives have been disclosed as inhibitors of HI. See U.S. Patent Nos. 6,333,323, 6,506,787 and 6,716,605, each of which is incorporated herein by reference in its entirety.

A series ofN- and S-containing dioxo-butryic acid derivatives have been demonstrated to be potent inhibitors of HI. See U.S. Patent Nos. 6,262,055 and 6,306,891, each of which is incorporated herein by reference in its entirety.

A series of bicyclic heterocycles have been disclosed as inhibitors of HI. See WO 2007/064316.

A series of naphthyridin-6-one derivatives have been demonstrated to be inhibitors of HI. See U.S. Patent Application Publication No. 2007/099,915, which is incorporated herein by reference in its entirety.

A series of pyridine-5-carboxamide compounds have been disclosed as inhibitors of HI. See U.S. Patent Application Publication No. 2007/039802, which is incorporated herein by reference in its entirety.

A series of methylphosphonic acid derivatives have been disclosed as inhibitors of HI. See U.S. Patent Application Publication No. 2007/019,101, which is incorporated herein by reference in its entirety.

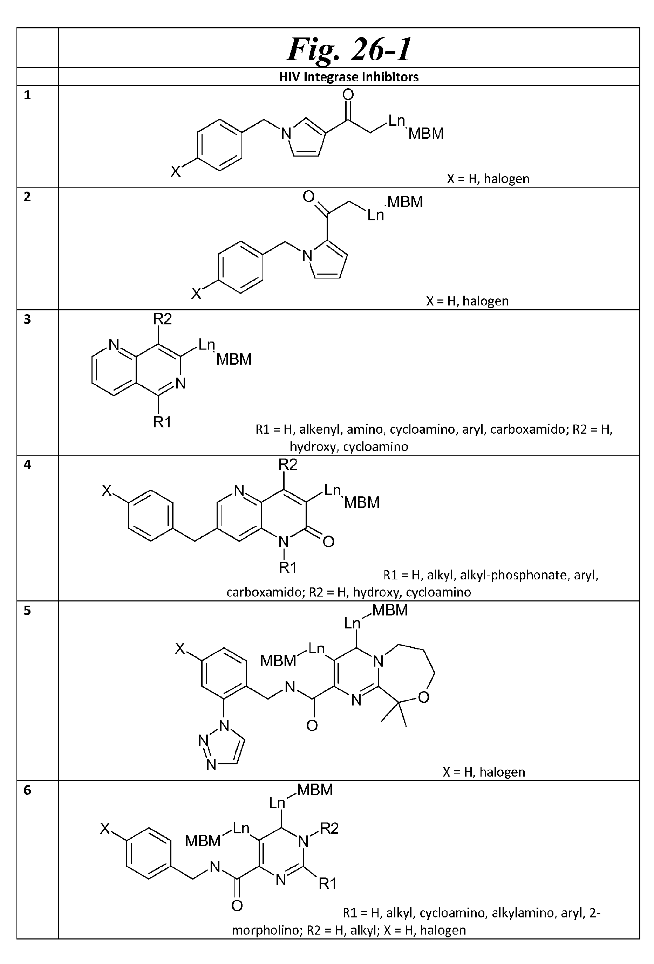

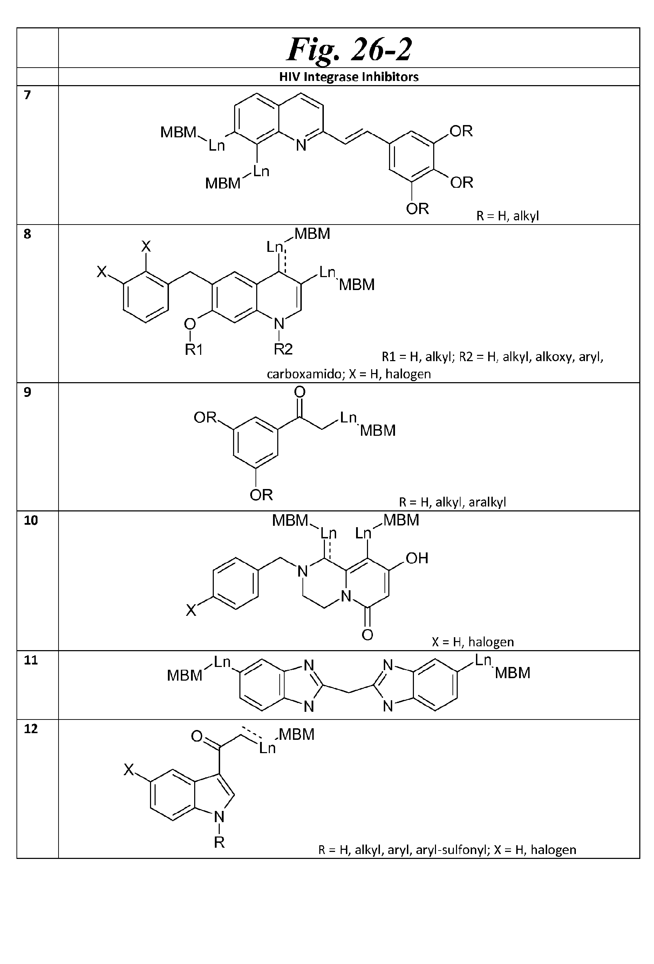

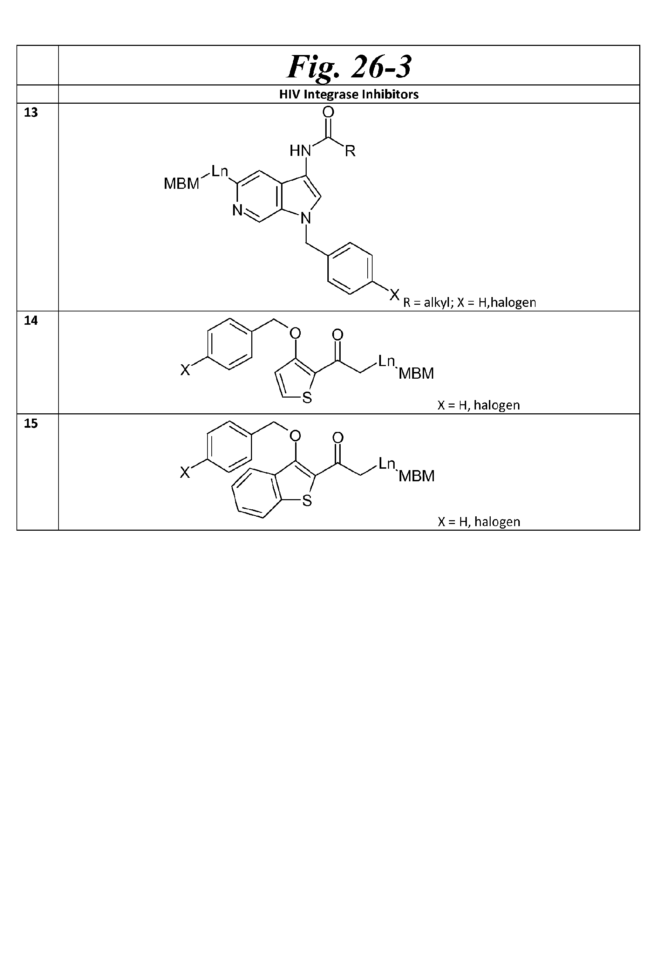

Figure 26 depicts a number of inhibitors of HI, which are suitable for use as targeting moieties in the present invention. Figure 26 shows the structure of these known inhibitors along with possible sites of attachment of the linkers and metal binding moieties ("R"), as well as possible derivatives.

Accordingly, in some embodiments, the metalloenzyme inhibitor is an HIV integrase inhibitor comprising a targeting moiety, a metal binding moiety, and optionally a linker, and wherein the inhibitor has a formula selected from the group consisting of:
(a) 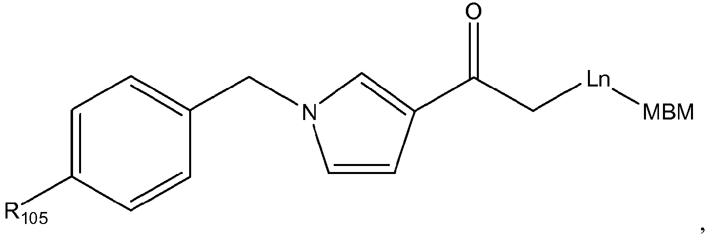 wherein R₁₀₅ is H or halogen;
(b) 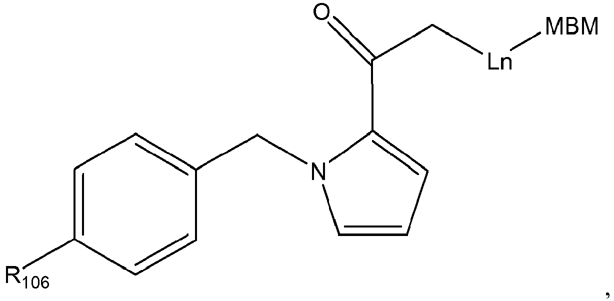 wherein R₁₀₆ is H or halogen;
(c) 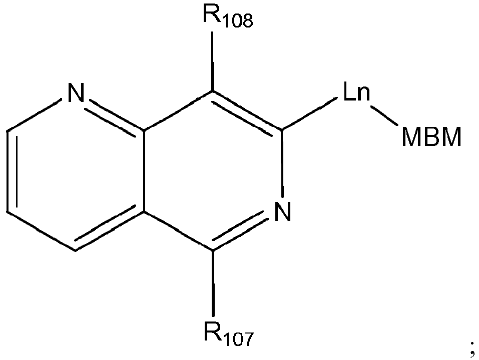 wherein:
   R₁₀₇ is selected from the group consisting of H, alkenyl, amino, cycloamino, aryl, substituted aryl, carboxamido;
   R₁₀₈ is selected from the group consisting of H, hydroxyl, and cycloamino ;
(d) 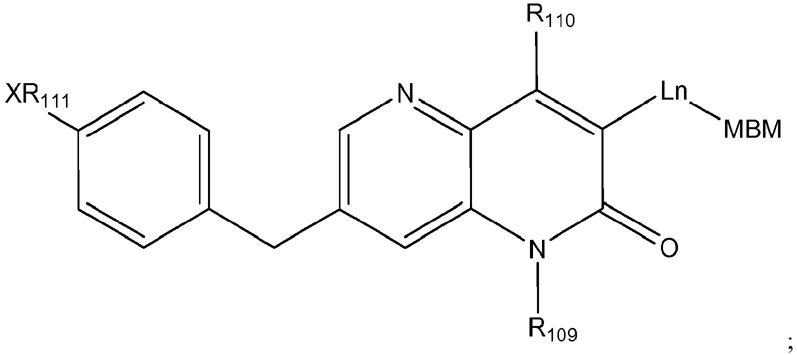 wherein:
   R₁₀₉ is selected from the group consisting of H, alkyl, alkyl-phosphonate, aryl, substituted carboxamido;
   R₁₁₀ is selected from the group consisting of H, hydroxyl, and cycloamino; and
   R₁₁₁ is H or halogen;
(e) 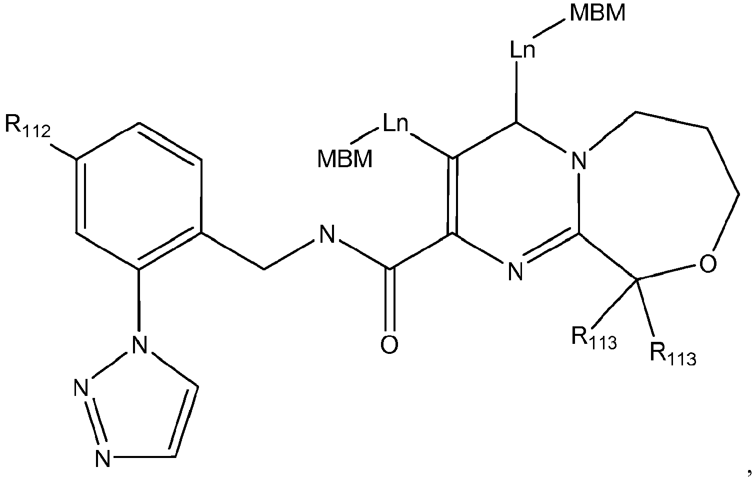 wherein:
   R₁₁₂ is H or halogen; and
   each R₁₁₃ is independently alkyl or substituted alkyl;
(f) 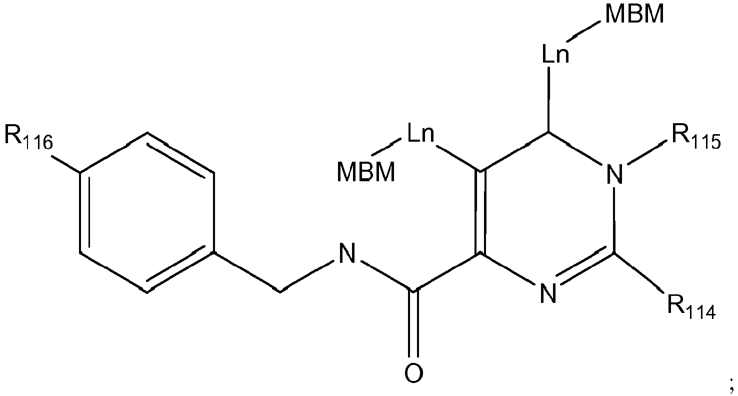 wherein:
   R₁₁₄ is selected from the group consisting of H, alkyl, substituted cycloamino, alkylamino, aryl, substituted aryl, and 2-morpholino;
   R₁₁₅ is selected from the group consisting of H, alkyl, and substituted alkyl; and
   R₁₁₆ is H or halogen;
(g) 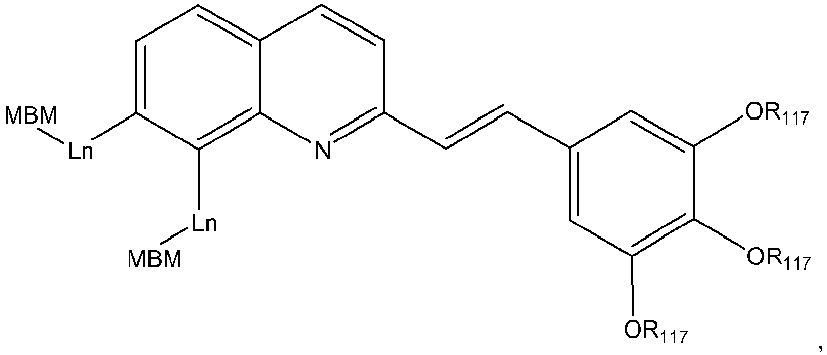 wherein each R₁₁₇ is independently selected from the group consisting of H, alkyl, and substituted alkyl;
(h) 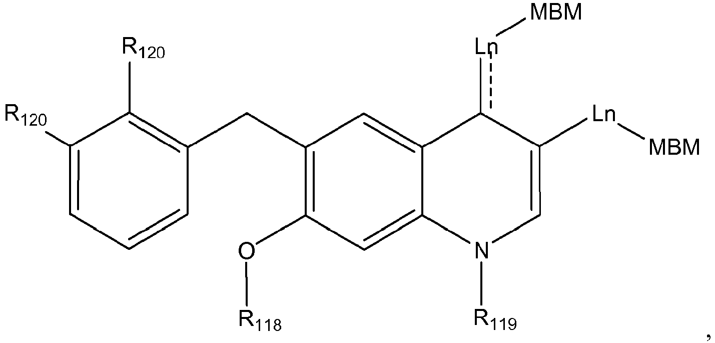
   R₁₁₈ is selected from the group consisting of H, alkyl, and substituted alkyl;
   R₁₁₉ is selected from the group consisting of H, alkyl, alkoxyl, aryl, substituted aryl, and carboxamido; and
   each R₁₂₀ is independently H or halogen;
(i) 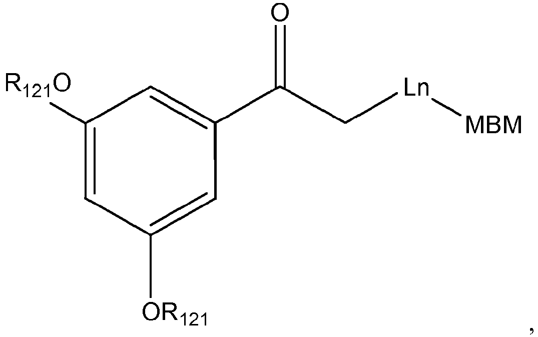 wherein each R₁₂₁ is independently selected from the group consisting of H, alkyl, substituted, and aralkyl;
(j) 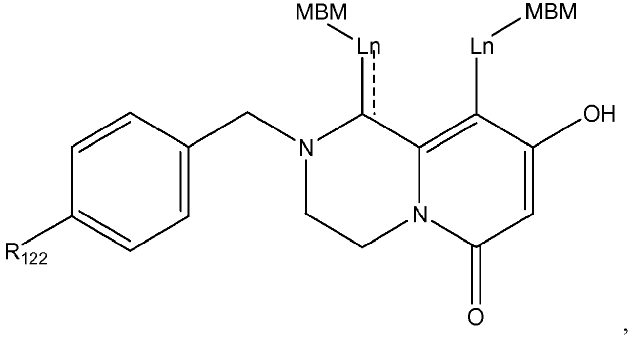 wherein R₁₂₂ is H or halogen;
(k) 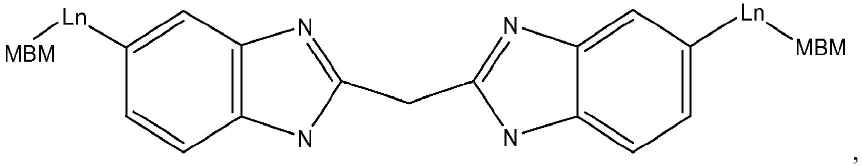
(l) 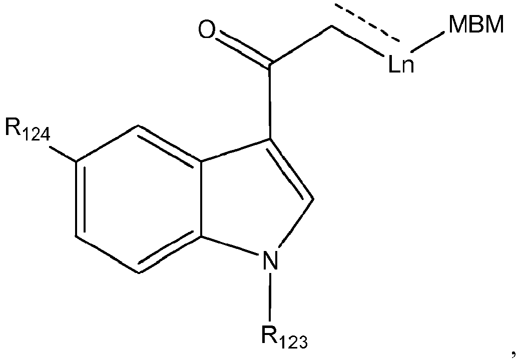
   R₁₂₃ is selected from the group consisting of H, alkyl, substituted alkyl, aryl, substituted aryl, and aryl-sulfonyl;
   R₁₂₄ is H or halogen;
(m) 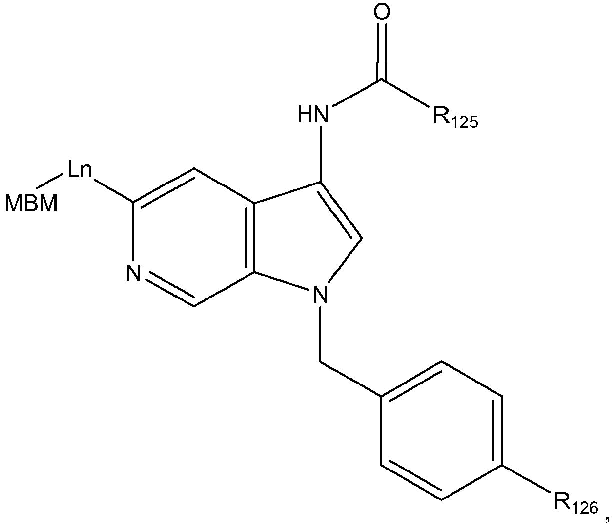 wherein:
   R₁₂₅ is alkyl or substituted alkyl; and
   R₁₂₆ is H or halogen;
(n) 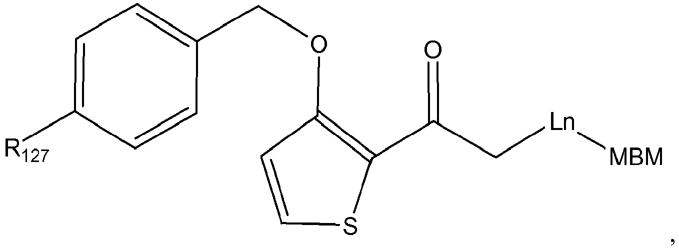 wherein R₁₂₇ is H or halogen;
(o) 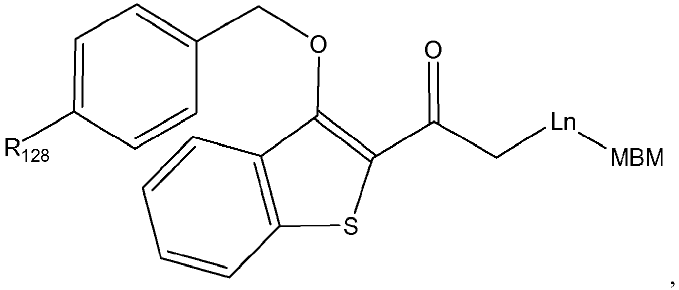 R₁₂₈ is H or halogen; and
   wherein:
   MBM is a metal binding moiety;
   Lₙ is a linker, wherein n is an integer from 0 to 1;
   under the proviso that the metal binding moiety is not a metal binding moiety selected from the group consisting of: 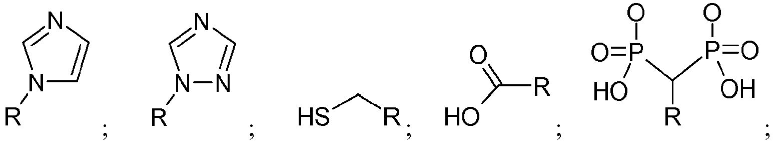 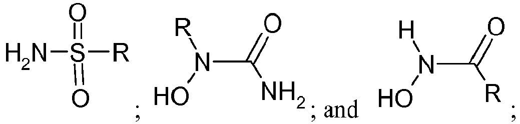 wherein:
      R is an attachment site through which the metal binding moiety can be attached to the targeting moiety, optionally through linker, Lₙ.

In addition to these targeting moieties, other known targeting moieties, identified by the screens outlined below or shown to bind to HI can be used. Thus, suitable targeting moieties include, but are not limited to, small organic molecules including known drugs and drug candidates, polysaccharides, fatty acids, vaccines, polypeptides, proteins (including peptides, as described herein), nucleic acids, carbohydrates, lipids, hormones including proteinaceous and steroid hormones, growth factors, receptor ligands, antigens, antibodies and enzymes, (as outlined below, collectively "candidate agents"), and the like.

HI activity can be measured using established methods. John S. et al., J Biomol Screen, 10:606-614 (2005); Fesen M. R. et al., Proc Nat Acad Sci, 90:2399-2403 (1993).

### 12. TNF-alpha converting enzyme (TACE)

TNF-alpha converting enzyme (TACE) is a membrane-bound, zinc metalloenzyme also known as ADAM 17. TACE is involved in the processing of tumor necrosis factor alpha (TNF-alpha) at the surface of the cell, and from within the intracellular membranes of the trans-Golgi network. This process, which is also known as 'shedding', involves the cleavage and release of a soluble ectodomain from membrane bound pro-proteins (such as pro-TNF-alpha). TACE was the first 'sheddase' to be identified, and is also understood to play a role in the release of a diverse variety of membrane-anchored cytokines, cell adhesion molecules, receptors, ligands and enzymes. Since TNF-alpha is a potent and pivotal mediator in the inflammatory process, the inhibition of TACE may represent another means by which the effects of TNF-alpha can be modulated. Inhibitors of TACE have potential for use in a variety of inflammatory diseases such as rheumatoid arthritis, inflammatory bowel disease and psoriasis. Black R. et al., Nature, 385:729-33 (1997).

A series of macrocyclic compounds have been disclosed as inhibitors of TACE for potential use in rheumatoid arthritis. See WO 1997/018207 and WO 1998/051665.

A series of reverse hydroxamate derivatives have been demonstrated to be potent inhibitors of TAGE with potential use in rheumatoid arthritis. See WO 1998/038179.

A series of (4-Arylsulfonylamino)-tetrahydropyran-4-carboxylic acid hydroxamides have been disclosed as inhibitors of TACE with potential use in the treatment of arthritis. See WO 1999/052889.

A series of acetylenic ortho-sulfonamido and phosphinic acid amido bicyclic heteroaryl hydroxamic acids have been demonstrated to be potent inhibitors of TACE. See WO 2000/044745.

A series of acetylenic alpha-amino acid-based sulfonamide hydroxamic acid derivatives have been disclosed as inhibitors of TACE with potential use in rheumatoid arthritis and cancer. See U.S. Patent No. 6,225,311, which is incorporated herein by reference in its entirety.

A series of hydroxamate derivatives have been demonstrated to be inhibitors of TACE with potential use in psoriasis and inflammatory bowel disease. See WO 2003/082287.

A series of hydroxamic acid derivatives have been disclosed as inhibitors of TACE with potential use in arthritis. See U.S. Patent No. 6,500,983, which is incorporated herein by reference in its entirety.

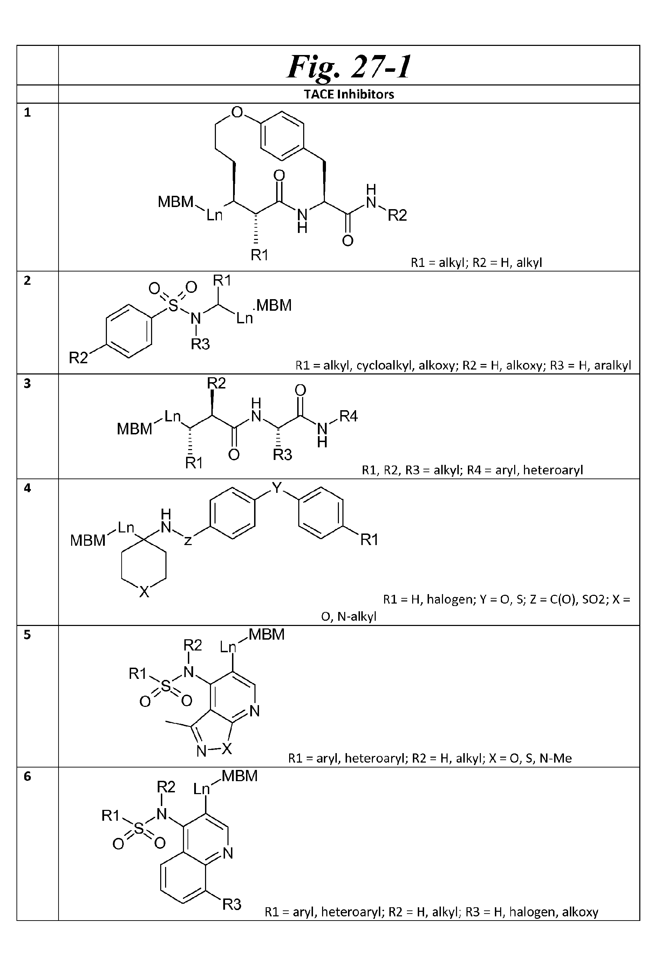

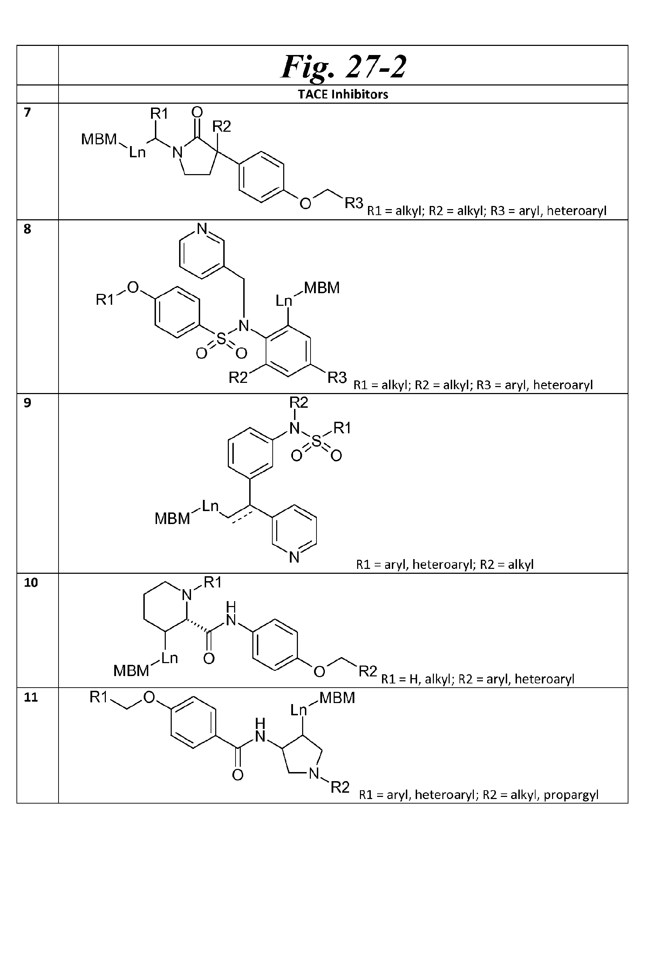

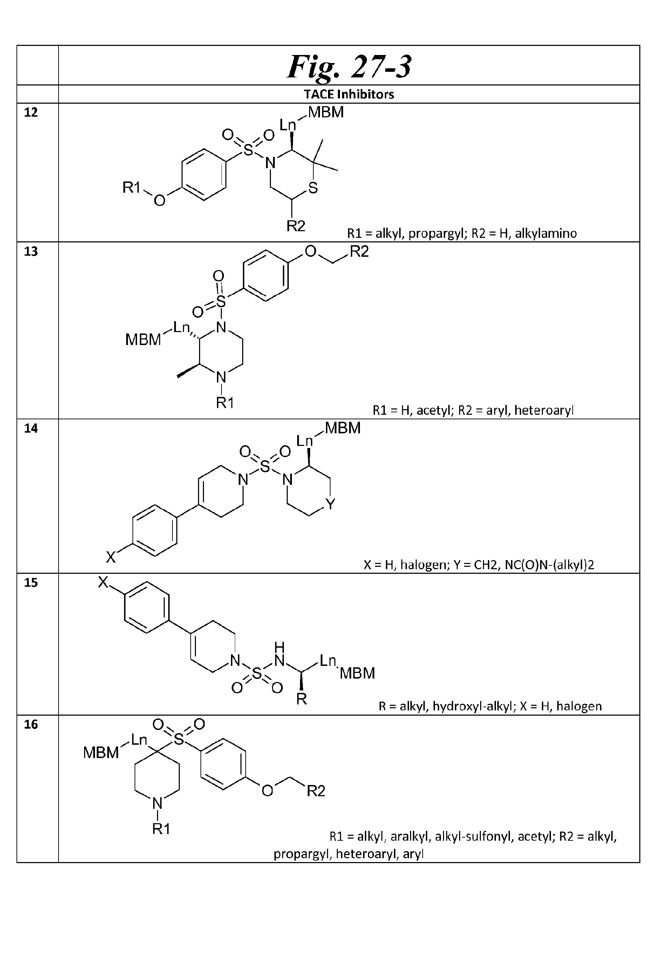

Figure 27 depicts a number of inhibitors of TACE, which are suitable for use as targeting moieties in the present invention. Figure 27 shows the structure of these known inhibitors along with possible sites of attachment of the linkers and metal binding moieties ("R"), as well as possible derivatives.

Accordingly, in some embodiments, the metalloenzyme inhibitor is a TNF-alpha converting enzyme (TACE) inhibitor comprising a targeting moiety, a metal binding moiety, and optionally a linker, and wherein the inhibitor has a formula selected from the group consisting of:
(a) 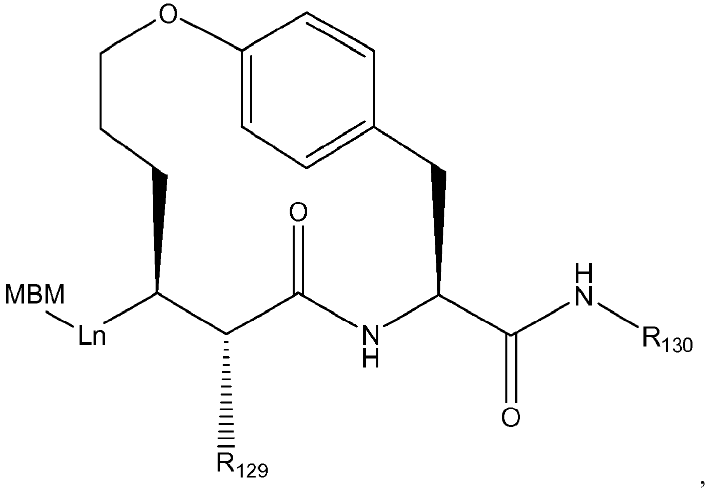 wherein:
   R₁₂₉ is alkyl or substituted alkyl; and
   R₁₃₀ is selected from the group consisting of H, alkyl, and substituted alkyl;
(b) 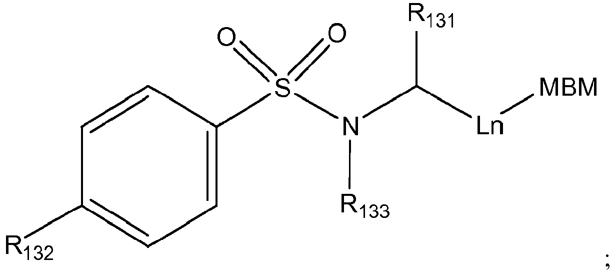 wherein:
   R₁₃₁ is selected from the group consisting of alkyl, substituted alkyl, cycloalkyl, substituted cycloalkyl, heterocycloalkyl, substituted heterocycloalkyl, and alkoxy;
   R₁₃₂ = H, alkoxy; and
   R₁₃₃ = H, aralkyl;
(c) 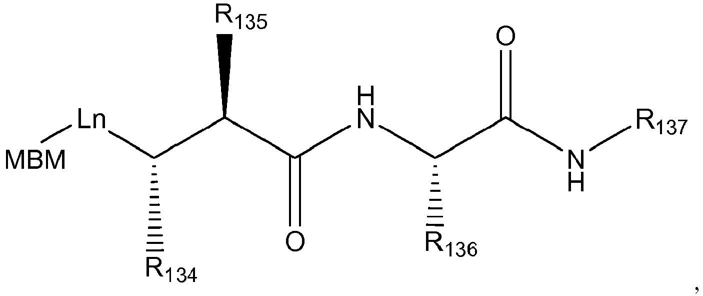 wherein:
   R₁₃₄, R₁₃₅, and R₁₃₆ are each alkyl or substituted alkyl;
   R₁₃₇ is selected from the group consisting of aryl, substituted aryl, heteroaryl, and substituted heteroaryl;
(d) 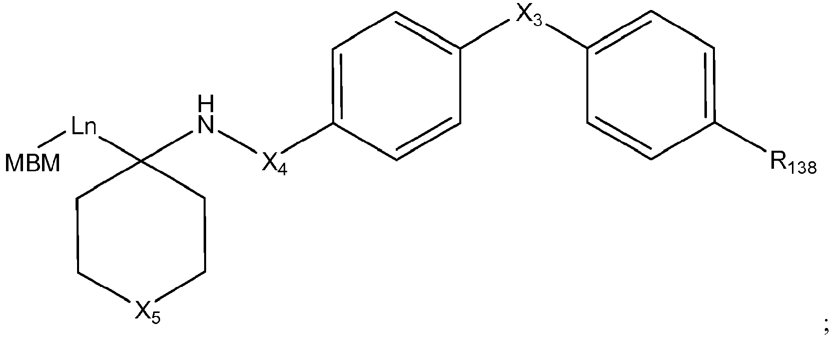 wherein:
   R₁₃₈ is H or halogen;
   X₃ is O or S;
   X₄ is C(=O) or S(=O)₂; and
   X₅ is O or N-alkyl;
(e) 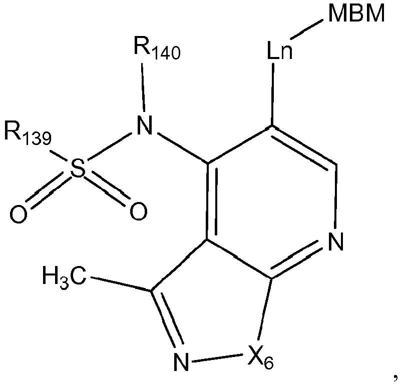 wherein:
   R₁₃₉ is selected from the group consisting of aryl, substituted aryl, heteroaryl, and substituted heteroaryl;
   R₁₄₀ is selected from the group consisting of H, alkyl, and substituted alkyl;
   X₆ is selected from the group consisting of O, S, and N-R₁₄₁, wherein R₁₄₁ is alkyl or substituted alkyl;
(f) 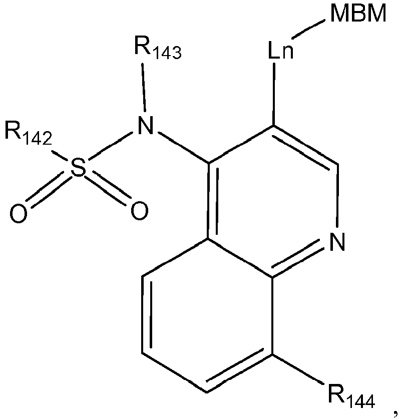 wherein:
   R₁₄₂ is selected from the group consisting of aryl, substituted aryl, heteroaryl, and substituted heteroaryl;
   R₁₄₃ is selected from the group consisting of H, alkyl, and substituted alkyl; and
   R₁₄₄ is selected from the group consisting of H, halogen, and alkoxyl;
(g) 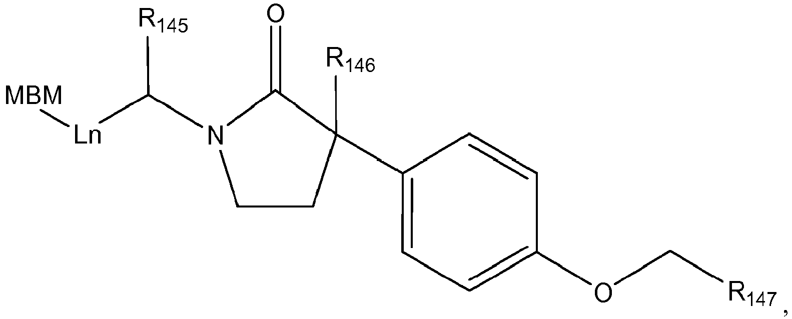 wherein:
   R₁₄₅ and R₁₄₆ are each independently alkyl or substituted alkyl;
   R₁₄₇ is selected from the group consisting of aryl, substituted aryl, heteroaryl, and substituted heteroaryl;
(h) 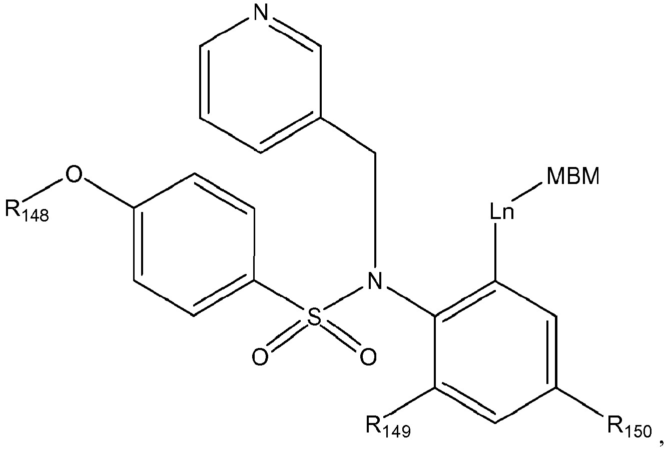 wherein:
   R₁₄₈ and R₁₄₉ are each independently alkyl or substituted alkyl;
   R₁₅₀ is selected from the group consisting of aryl, substituted aryl, heteroaryl, and substituted heteroaryl;
(i) 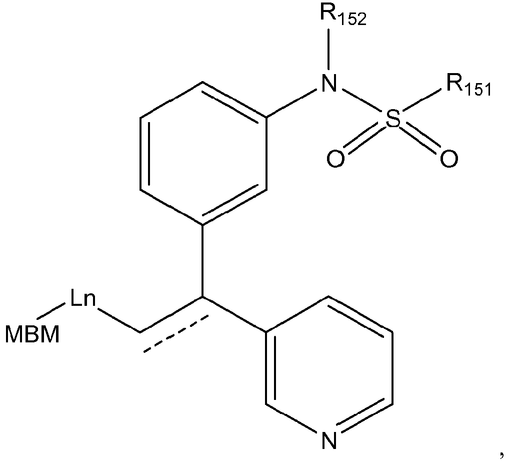 wherein:
   R₁₅₁ is selected from the group consisting of aryl, substituted aryl, heteroaryl, and substituted heteroaryl; and
   R₁₅₂ is alkyl or substituted alkyl;
(j) 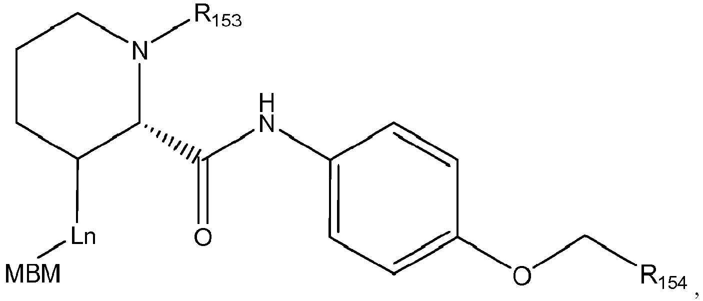 wherein:
   R₁₅₃ is selected from the group consisting of H, alkyl, and substituted alkyl; and
   R₁₅₄ is selected from the group consisting of aryl, substituted aryl, heteroaryl, and substituted heteroaryl;
(k) 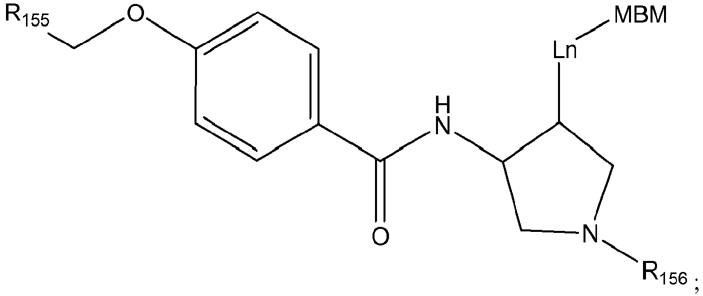 wherein:
   R₁₅₅ is selected from the group consisting of aryl, substituted aryl, heteroaryl, and substituted heteroaryl; and
   R₁₅₆ is selected from the group consisting of alkyl, substituted alkyl, and propargyl;
(l) 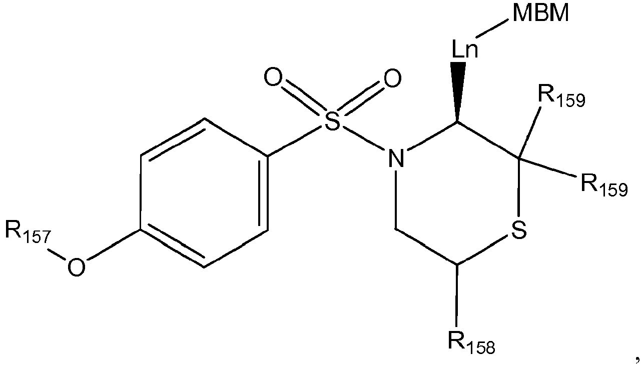 wherein:
   R₁₅₇ is selected from the group consisting of alkyl, substituted alkyl, and propargyl;
   R₁₅₈ is H or alkylamino; and
   each R₁₅₉ is independently alkyl or substituted alkyl;
(m) 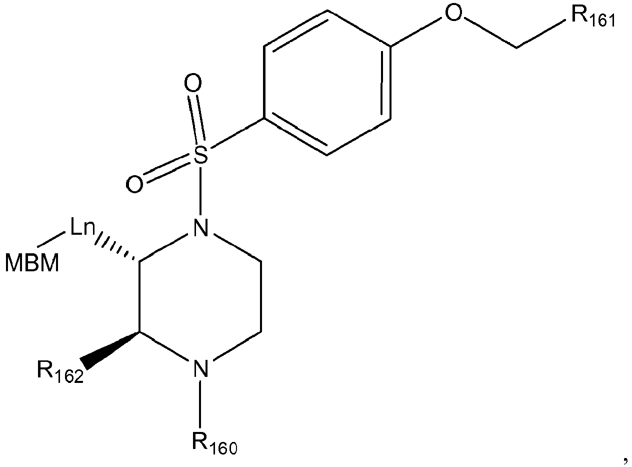 wherein:
   R₁₆₀ is H or acetyl;
   R₁₆₁ is selected from the group consisting of aryl, substituted aryl, heteroaryl, and substituted heteroaryl; and
   R₁₆₂ is alkyl or substituted alkyl;
(n) 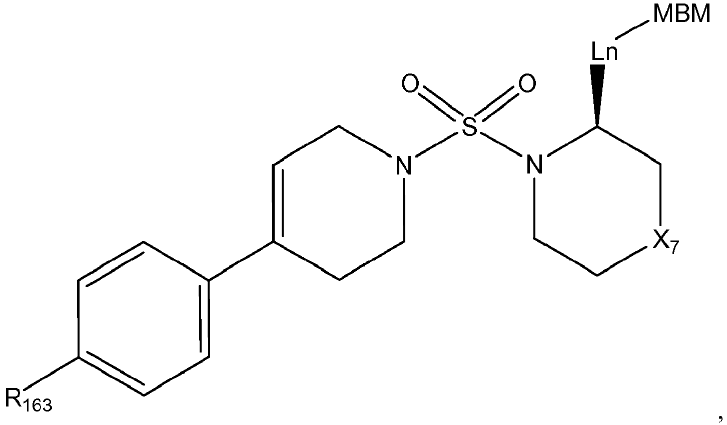
   R₁₆₃ is H or halogen;
   X₇ is CH₂ or NC(O)N-(alkyl)₂;
(o) 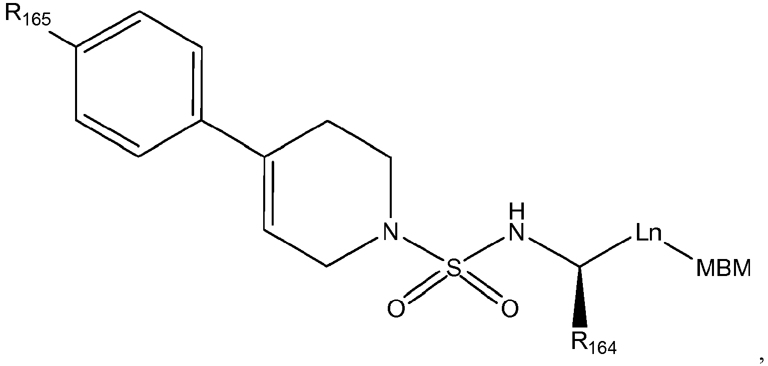 wherein:
   R₁₆₄ is selected from the group consisting of alkyl, substituted alkyl, and hydroxyl-alkyl; and
   R₁₆₅ is H or halogen;
(p) 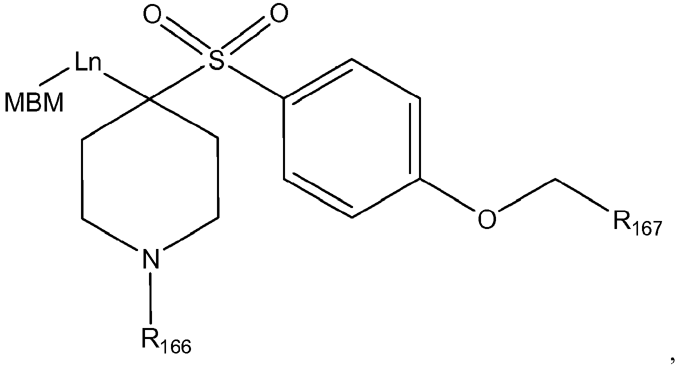 wherein:
   R₁₆₆ is selected from the group consisting of alkyl, substituted alkyl, aralkyl, alkyl-sulfonyl, and acetyl;
   R₁₆₇ is selected from the group consisting of alkyl, substituted alkyl, propargyl, heteroaryl, substituted heteroaryl, aryl, and substituted aryl;
   wherein:
   MBM is a metal binding moiety;
   Lₙ is a linker, wherein n is an integer from 0 to 1;
   under the proviso that the metal binding moiety is not a metal binding moiety selected from the group consisting of: 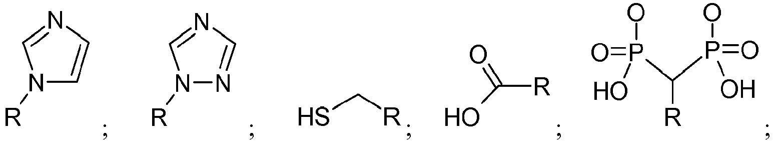 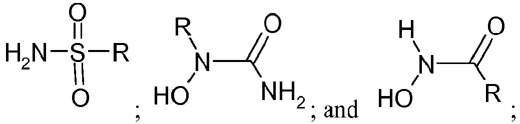 wherein:
      R is an attachment site through which the metal binding moiety can be attached to the targeting moiety, optionally through linker, Lₙ.

In addition to these targeting moieties, other known targeting moieties, identified by the screens outlined below or shown to bind to TACE can be used. Thus, suitable targeting moieties include, but are not limited to, small organic molecules including known drugs and drug candidates, polysaccharides, fatty acids, vaccines, polypeptides, proteins (including peptides, as described herein), nucleic acids, carbohydrates, lipids, hormones including proteinaceous and steroid hormones, growth factors, receptor ligands, antigens, antibodies and enzymes, (as outlined below, collectively "candidate agents"), and the like.

TACE activity can be measured using established methods. Kirkegaard T. et al., Clin Exp Immunol, 135:146-154 (2004). Neumann, U. et al., Anal Biochem, 328:166-172 (2004).

### 13. UDP-(3-O-acyl)-N-acetylglucosamine deacetylase (LpxC)

UDP-(3-O-acyl)-N-acetylglucosamine deacetylase (LpxC) is a bacterial zinc-containing metalloenzyme. The enzyme LpxC catalyzes the deacetylation of UDP-3-O-(R-3-hydroxymyristoyl) GlcNAc, the committed step in the biosynthesis of Lipid A. The cell wall of gram-negative bacteria is surrounded by an outer membrane that is comprised of charged lipopolysaccharide (LPS) molecules. The highly charged outer membrane serves to prevent entry of hydrophobic molecules into the bacteria. Lipid A is the hydrophobic anchor of LPS and is essential for bacterial survival. As a consequence, the enzymes in the Lipid A biosynthetic pathway are targets for the development of antimicrobial agents. Inhibitors of the bacterial enzyme LpxC have been demonstrated to have antimicrobial activity, validating LpxC as a useful drug target. Jackman J. E. et al., J Bio Chem, 275:11002-9 (2000).

A series of N-hydroxyamide derivatives have been disclosed as inhibitors of LpxC for use as antibacterial agents. See WO 2004/007444.

A series of hydroxamate derivatives have been demonstrated to be inhibitors of LpxC for use in antibacterial therapy. See WO 2004/062601.

A series of hydroxamic acid derivatives have been disclosed as inhibitors of LpxC for use as antibacterial agents See WO 2007/069020.

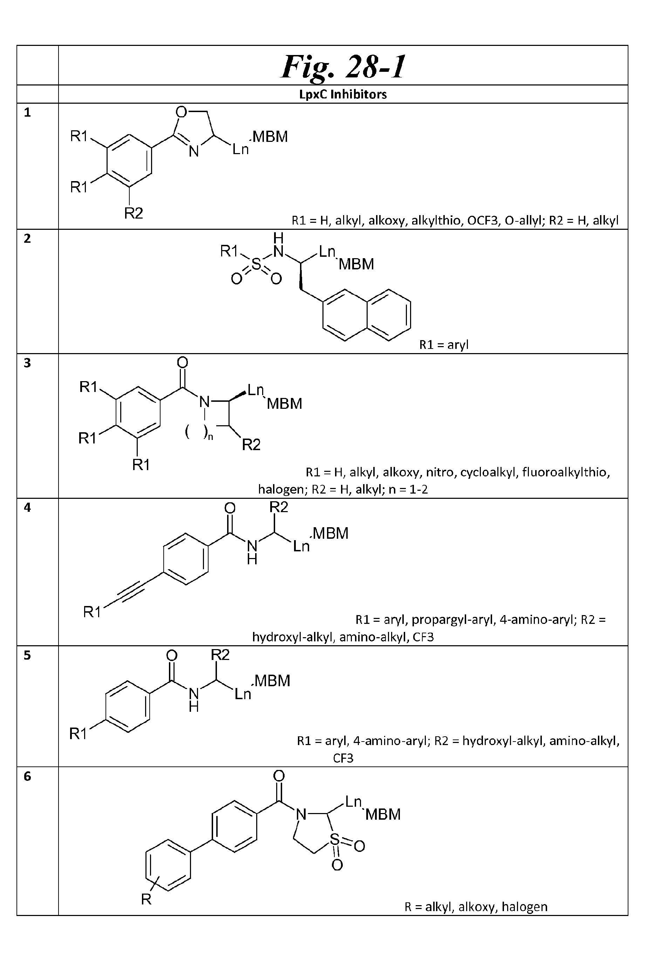

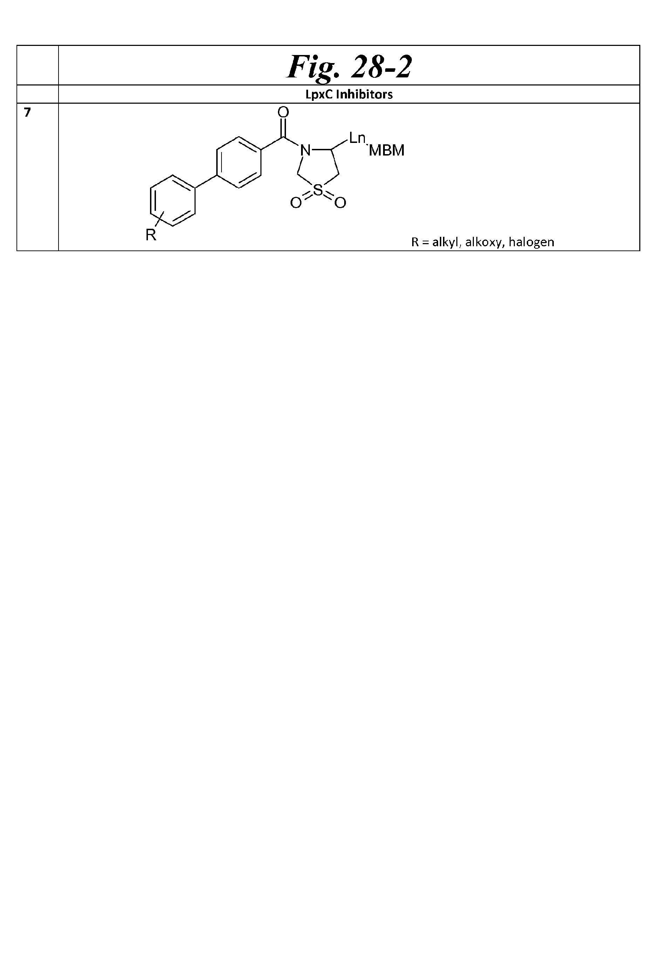

Figure 28 depicts a number of inhibitors of LpxC, which are suitable for use as targeting moieties in the present invention. Figure 28 shows the structure of these known inhibitors along with possible sites of attachment of the linkers and metal binding moieties ("R"), as well as possible derivatives.

Accordingly, in some embodiments, the metalloenzyme inhibitor is a UDP-(3-O-acyl)-N-acetylglucosamine deacetylase (LpxC) inhibitor comprising a targeting moiety, a metal binding moiety, and optionally a linker, and wherein the inhibitor has a formula selected from the group consisting of:
(a) 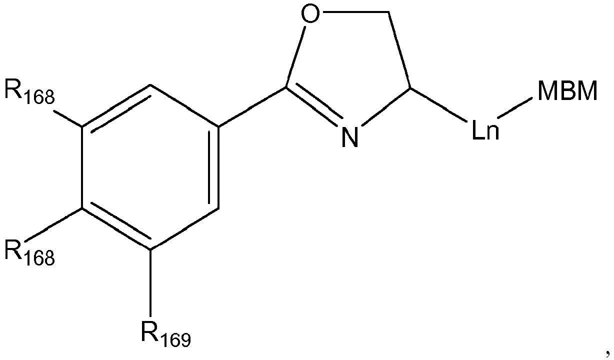 wherein:
   each R₁₆₈ is independently selected from the group consisting of H, alkyl, substituted alkyl, alkoxyl, alkylthio, OCF₃, and O-allyl;
   R₁₆₉ is selected from the group consisting of H, alkyl, and substituted alkyl;
(b) 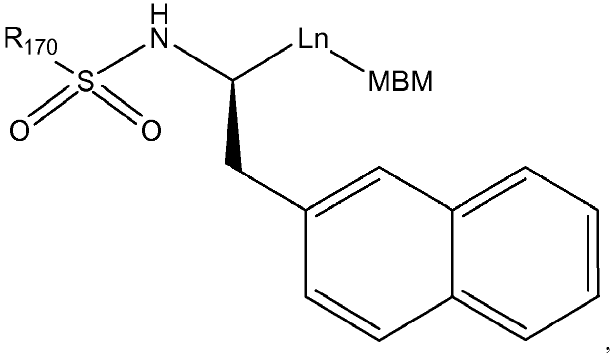 wherein R₁₇₀ is aryl or substituted aryl;
(c) 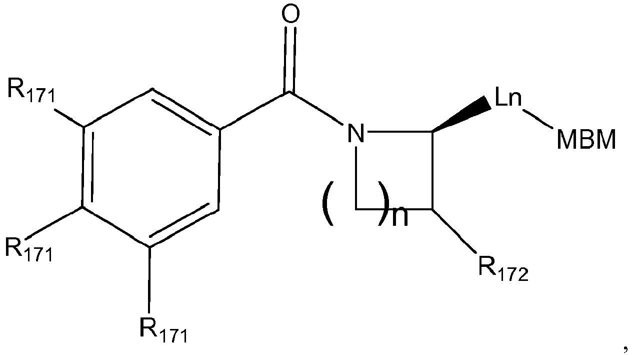 wherein:
   n is an integer from 1 to 2;
   each R₁₇₁ is independently selected from the group consisting of H, alkyl, substituted alkyl, alkoxyl, nitro, cycloalkyl, substituted cycloalkyl, heterocycloalkyl, substituted heterocycloalkyl, fluoroalkylthio, and halogen;
   R₁₇₂ is selected from the group consisting of H, alkyl, and substituted alkyl;
(d) 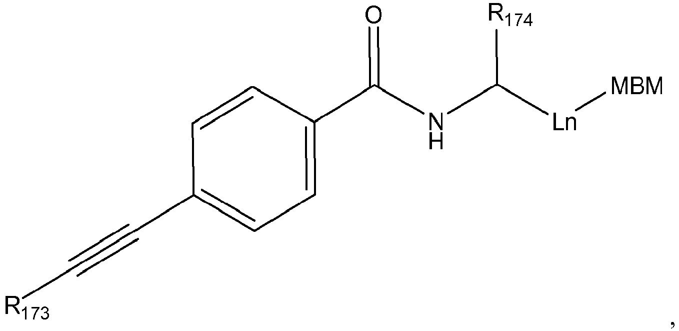 wherein:
   R₁₇₃ is selected from the group consisting of aryl, substituted aryl, propargyl-aryl, and 4-amino-aryl; and
   R₁₇₄ is selected from the group consisting of hydroxyl-alkyl, amino-alkyl, and CF₃;
(e) 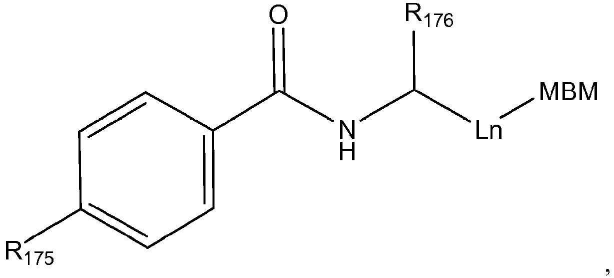 wherein:
   R₁₇₅ is selected from the group consisting of aryl, substituted aryl, and 4-amino-aryl; and
   R₁₇₆ is selected from the group consisting of hydroxyl-alkyl, amino-alkyl, and CF₃;
(f) 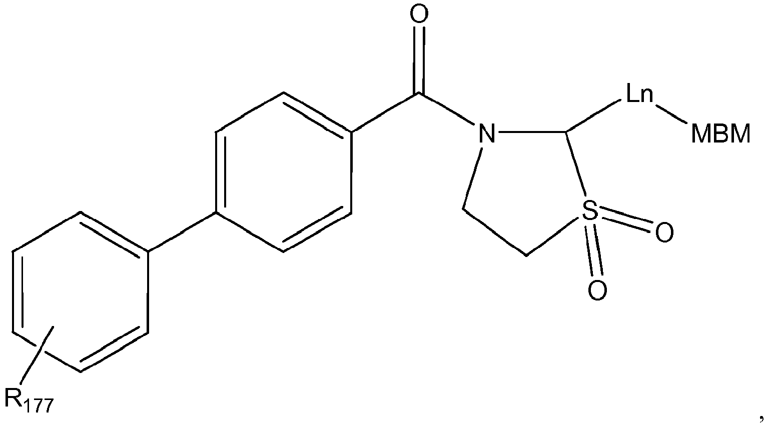 wherein R₁₇₇ is selected from the group consisting of alkyl, substituted alkyl, alkoxyl, and halogen;
(g) 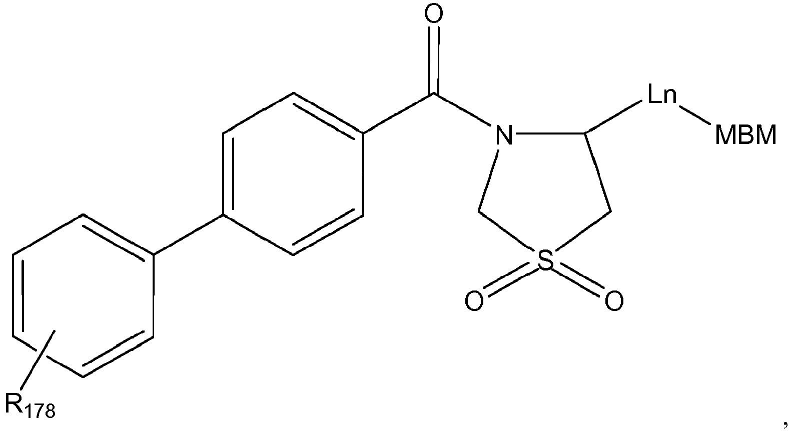 wherein R₁₇₈ is selected from the group consisting of alkyl, substituted alkyl, alkoxyl, and halogen;
   wherein:
   MBM is a metal binding moiety;
   Lₙ is a linker, wherein n is an integer from 0 to 1;
   under the proviso that the metal binding moiety is not a metal binding moiety selected from the group consisting of: 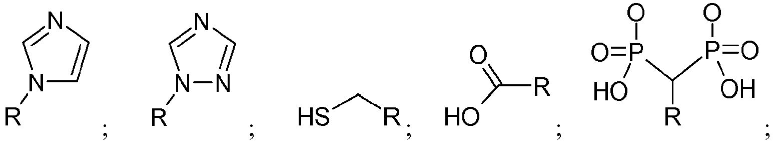 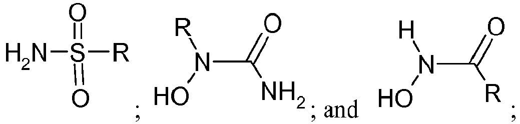 wherein:
      R is an attachment site through which the metal binding moiety can be attached to the targeting moiety, optionally through linker, Lₙ.

In addition to these targeting moieties, other known targeting moieties, identified by the screens outlined below or shown to bind to LpxC can be used. Thus, suitable targeting moieties include, but are not limited to, small organic molecules including known drugs and drug candidates, polysaccharides, fatty acids, vaccines, polypeptides, proteins (including peptides, as described herein), nucleic acids, carbohydrates, lipids, hormones including proteinaceous and steroid hormones, growth factors, receptor ligands, antigens, antibodies and enzymes, (as outlined below, collectively "candidate agents"), and the like.

LpxC activity can be measured using established methods. Wang W. et al., Anal Biochem, 290:338-346 (2001); Clements J. M. et al., Antimicrobial Agents and Chemotherapy, 46:1793-1799 (2002).

### 14. Histone Deacetylases (HDAC)

The histone deacetylases (HDAC) are a family of closely-related, zinc-containing metalloenzymes. Histone acetylation plays an important role in regulation of gene expression as hyperacetylated chromatin is transcriptionally active and hypoacetylated chromatin is transcriptionally silent. HDAC removes acetyl groups from ε-N-acetyl lysine amino acids on histones and the action is opposite to that of histone acetyltransferase. Deacetylation causes the histones to wrap more tightly around the DNA, thus interfering with the transcription of genes by blocking access by transcription factors. The overall result of histone deacetylation is a global reduction in gene expression. Due to the effects of HDAC on gene expression, inhibitors of HDAC are being studied for the treatment for cancer and neurodegenerative diseases. Monneret C., Anticancer Drugs, 18:363-70 (2007).

Suberanilohydroxamic acid and derivatives have been disclosed as potent inhibitors of HDAC. See U.S. Patent Nos. 5,369,108 and 6,087,367, each of which is incorporated herein by reference in its entirety.

A series of carbamic acid compounds containing a sulfonamide linkage have been demonstrated to be potent inhibitors of HDAC. See U.S. Patent No. 7,183,298, which is incorporated herein by reference in its entirety.

A series of sulfonyl-derivatives have been disclosed as inhibitors of HDAC. See U.S. Patent No. 7,205,304, which is incorporated herein by reference in its entirety.

A series of hydroxamate-containing compounds have been demonstrated to be potent inhibitors of HDAC. See U.S. Patent Application Publication No. 2005/187,261, which is incorporated herein by reference in its entirety.

A series of propenohydroxamic acid derivatives have been disclosed as inhibitors of HDAC. See W02002/022577.

A series of acetylene derivatives have been demonstrated to be potent inhibitors of HDAC. See U.S. Patent Application Publication No. 2005/131,018, which is incorporated herein by reference in its entirety.

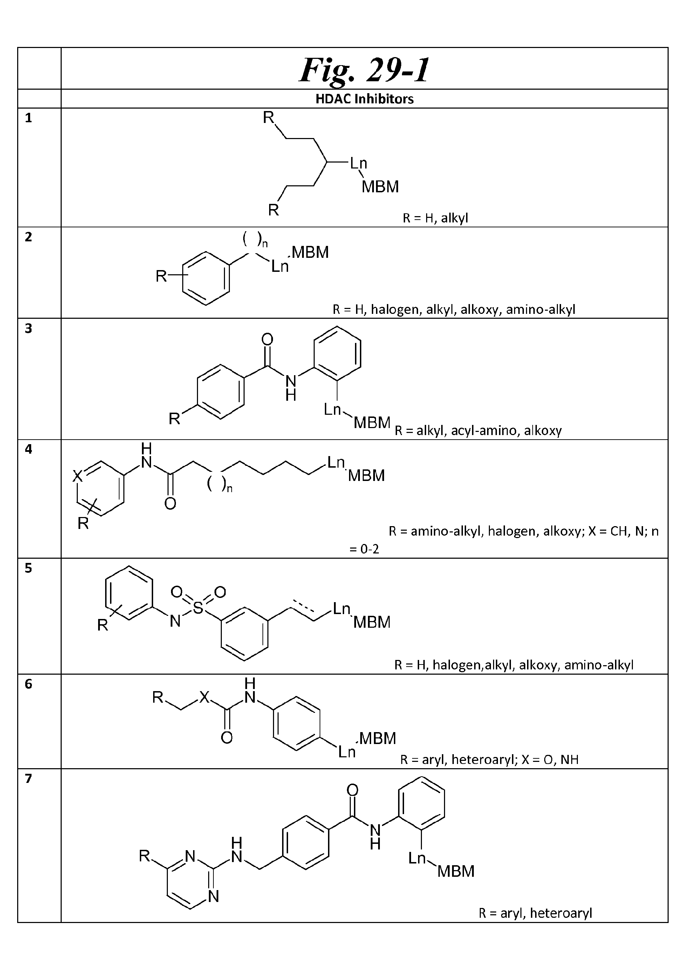

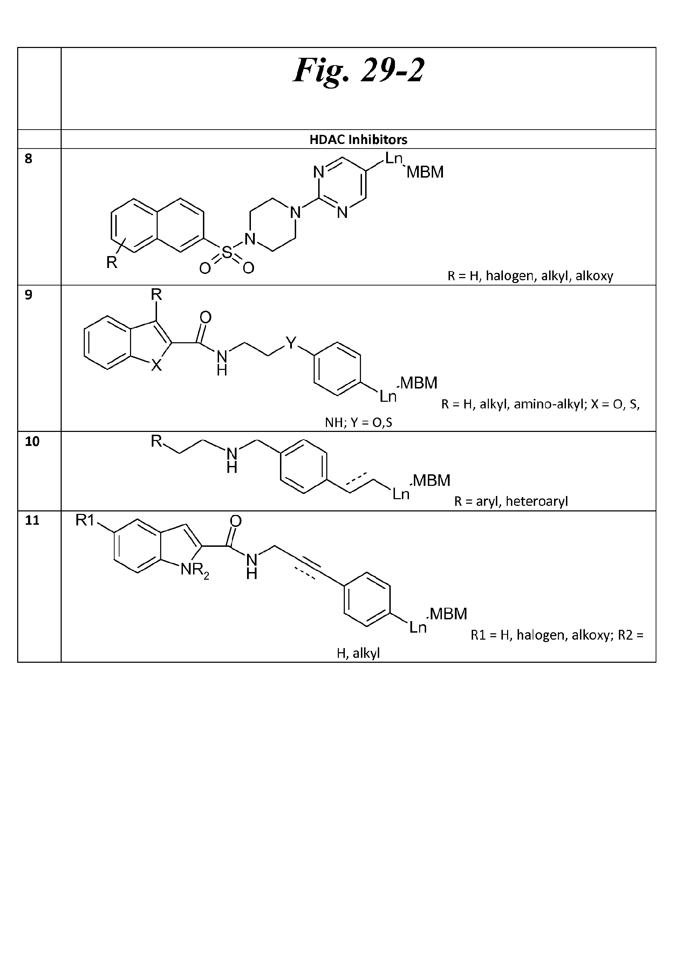

Figure 29 depicts a number of inhibitors of HDAC, which are suitable for use as targeting moieties in the present invention. Figure 29 shows the structure of these known inhibitors along with possible sites of attachment of the linkers and metal binding moieties ("R"), as well as possible derivatives.

Accordingly, in some embodiments, the metalloenzyme inhibitor is an histone deacetylase (HDAC) inhibitor comprising a targeting moiety, a metal binding moiety, and optionally a linker, and wherein the inhibitor has a formula selected from the group consisting of:
(a) 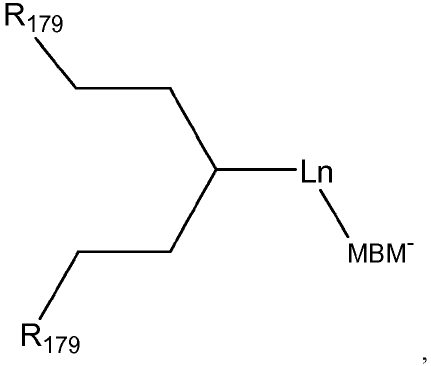 wherein each R₁₇₉ is independently selected from the group consisting of H, alkyl, and substituted alkyl;
(b) 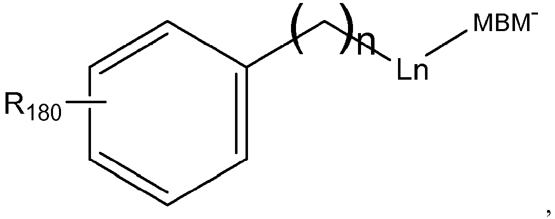 wherein:
   n is an integer from 1 to 2;
   R₁₈₀ is selected from the group consisting of H, halogen, alkyl, substituted alkyl, alkoxyl, amino-alkyl;
(c) 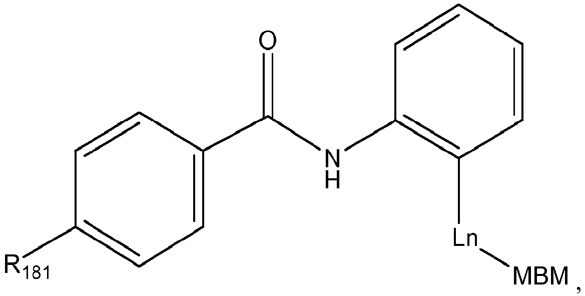 wherein R₁₈₁ is selected from the group consisting of alkyl, substituted alkyl, acyl-amino, and alkoxyl;
(d) 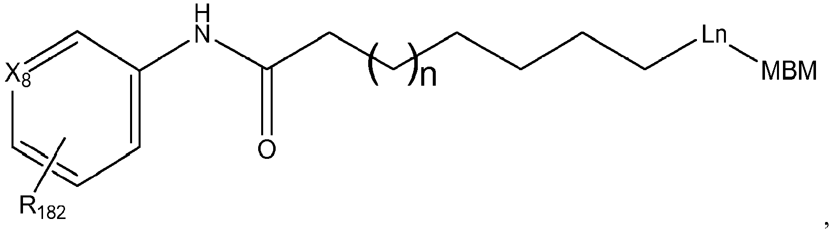 wherein:
   n is an integer from 0 to 2;
   R₁₈₂ is selected from the group consisting of amino-alkyl, halogen, and alkoxyl;
   X₈ is CH or N;
(e) 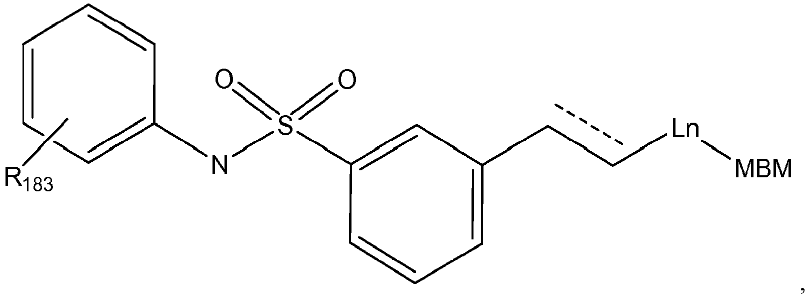 wherein:
   R₁₈₃ is selected from the group consisting ofH, halogen, alkyl, substituted alkyl, alkoxyl, and amino-alkyl;
(f) 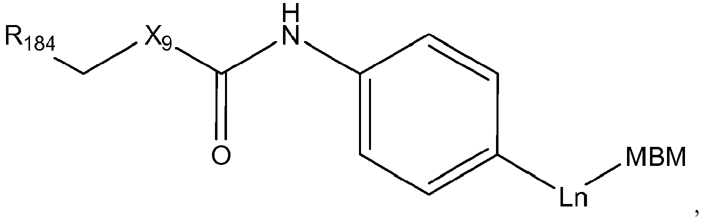 wherein:
   R₁₈₄ is selected from the group consisting of aryl, substituted aryl, heteroaryl, and substituted heteroaryl; and
   X₉ is O or NH;
(g) 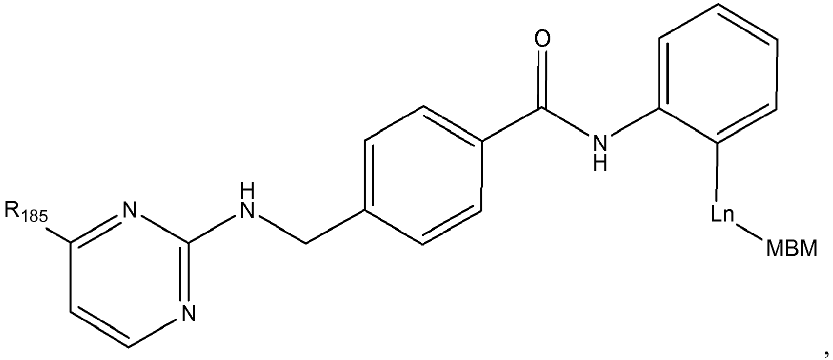 wherein R₁₈₅ is selected from the group consisting of aryl, substituted aryl, heteroaryl, and substituted heteroaryl;
(h) 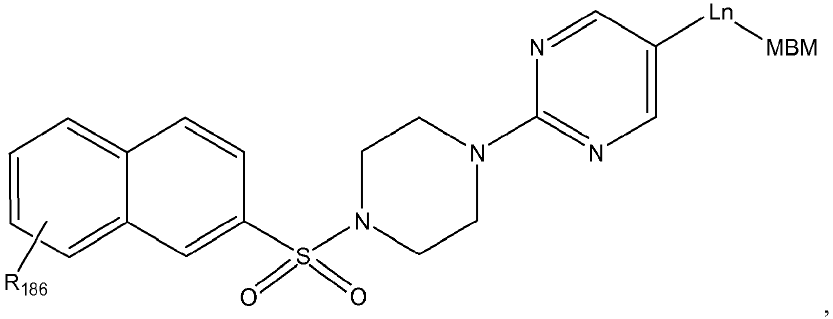 wherein R₁₈₆ is selected from the group consisting of H, halogen, alkyl, substituted alkyl, and alkoxyl;
(i) 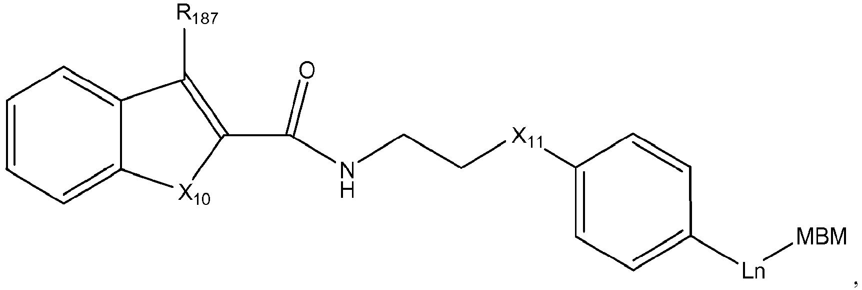 wherein:
   R₁₈₇ is selected from the group consisting of H, alkyl, substituted alkyl, and amino-alkyl;
   X₁₀ is selected from the group consisting of O, S and NH; and
   X₁₁ is O or S;
(j) 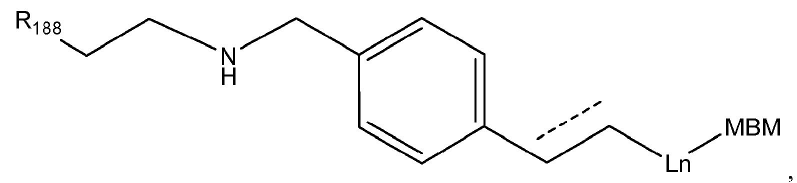 wherein R₁₈₈ is selected from the group consisting of aryl, substituted aryl, heteroaryl, and substituted heteroaryl;
(k) 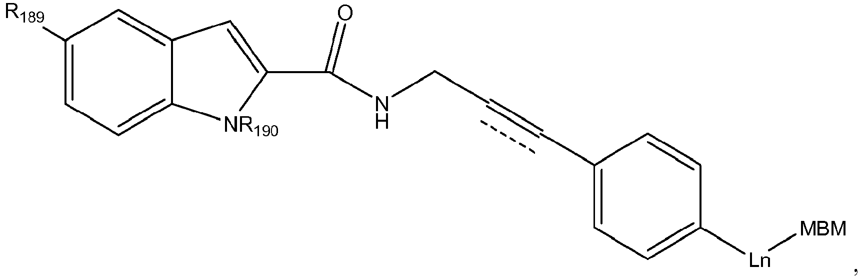 wherein:
   R₁₈₉ is selected from the group consisting of H, halogen, and alkoxyl; and
   R₁₉₀ is selected from the group consisting of H, alkyl, and substituted alkyl;
   wherein:
   MBM is a metal binding moiety;
   Lₙ is a linker, wherein n is an integer from 0 to 1;
   under the proviso that the metal binding moiety is not a metal binding moiety selected from the group consisting of: 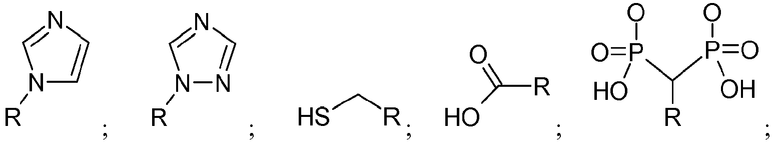 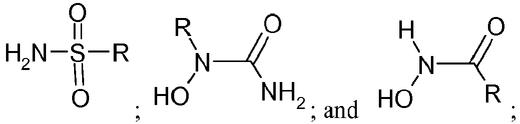 wherein:
      R is an attachment site through which the metal binding moiety can be attached to the targeting moiety, optionally through linker, Lₙ.

In addition to these targeting moieties, other known targeting moieties, identified by the screens outlined below or shown to bind to HDAC can be used. Thus, suitable targeting moieties include, but are not limited to, small organic molecules including known drugs and drug candidates, polysaccharides, fatty acids, vaccines, polypeptides, proteins (including peptides, as described herein), nucleic acids, carbohydrates, lipids, hormones including proteinaceous and steroid hormones, growth factors, receptor ligands, antigens, antibodies and enzymes, (as outlined below, collectively "candidate agents"), and the like.

HDAC activity can be measured using established methods. Wegener D. et al., Chem Biol, 10:61-8 (2003); Ciossek T. et al., Anal Biochem, 372:72-81 (2007).

### 15. Matrix Metalloproteinases (MMP)

The matrix metalloproteinases (MMP) are a closely-related family of zinc-dependent metalloproteases which are capable of degrading a large number of extracellular matrix proteins. The MMPs are initially synthesized as inactive zymogens with a pro-peptide domain that must be removed before the enzyme is active. The pro-peptide domain is part of the "cysteine switch." This contains a conserved cysteine residue which interacts with the zinc in the active site and prevents binding and cleavage of the substrate keeping the enzyme in an inactive form. The MMPs play an important role in tissue remodeling associated with various physiological and pathological processes such as morphogenesis, angiogenesis, tissue repair, cirrhosis, arthritis and metastasis. MMP-2 and MMP-9 are thought to be important in metastasis. MMP-1 is thought to be important in rheumatoid and osteo-arthritis. Inhibitors of MMP have been studies for a broad range of therapeutic uses including cancer, arthritis, diabetic retinopathy, inflammatory bowel disease and myocardial infarction. Sang Q. A. et al., Curr Top Med Chem, 6:289-316 (2006).

A series of hydroxamate derivatives have been disclosed as inhibitors of MMP. See U.S. Patent No. 5,240,958 and 5,310,763, which is incorporated herein by reference in its entirety.

A series of arylsulfonamido-substituted hydroxamic acid derivatives have been demonstrated to be potent inhibitors of MMP. See U.S. Patent Nos. 5,455,258, 5,506,242, and 5,552,419, each of which is incorporated herein by reference in its entirety.

An additional series of arylsulfonamido-substituted hydroxamic acids have been disclosed as inhibitors of MMP. See U.S. Patent No. 5,753,653, which is incorporated herein by reference in its entirety.

A series of hydroxamic acid derivatives with tricyclic substitution have been demonstrated to be potent inhibitors of MMP. See U.S. Patent No. 5,710,167, which is incorporated herein by reference in its entirety.

A series of thio-substituted peptide derivatives have been disclosed as inhibitors of MMP. See U.S. Patent No. 6,818,622, which is incorporated herein by reference in its entirety.

A series of aminobutanoic acid derivatives have been demonstrated to be potent inhibitors of MMP. See U.S. Patent No. 6,420,427, which is incorporated herein by reference in its entirety.

A series of (4-Arylsulfonylamino)-tetrahydropyran-4-carboxylic acid hydroxamide derivatives have been disclosed as inhibitors of MMP. See WO 1999/052889.

A series of reverse hydroxamate compounds have been demonstrated to be potent inhibitors of MMP. See WO 2000/044739.

A series of acetylenic alpha-amino acid-based sulfonamide hydroxamic acid derivatives have been disclosed as inhibitors of MMP. See U.S. Patent No. 6,225,311, which is incorporated herein by reference in its entirety.

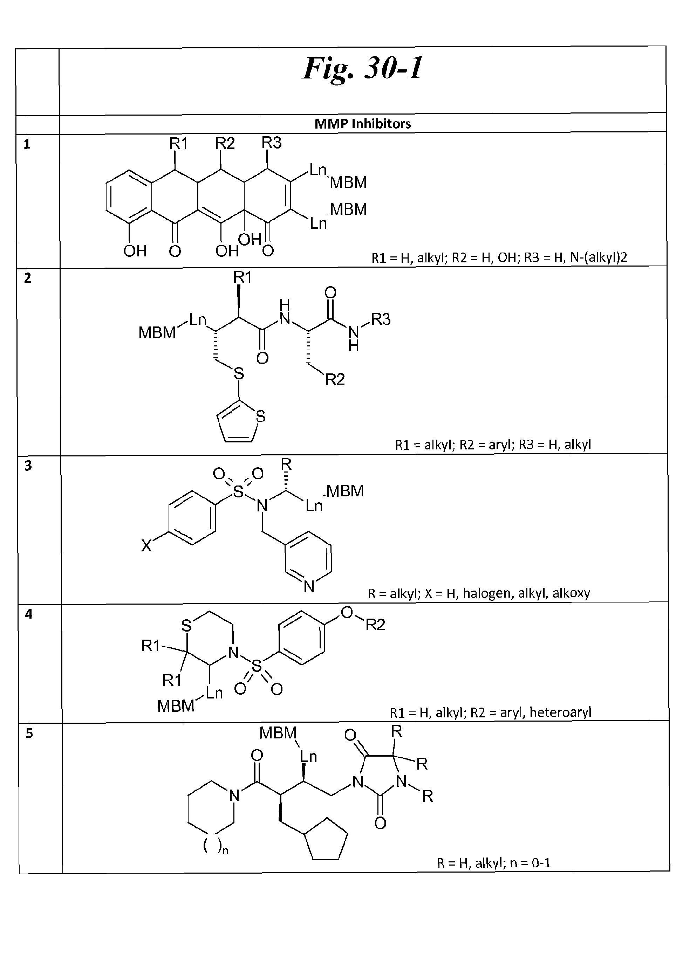

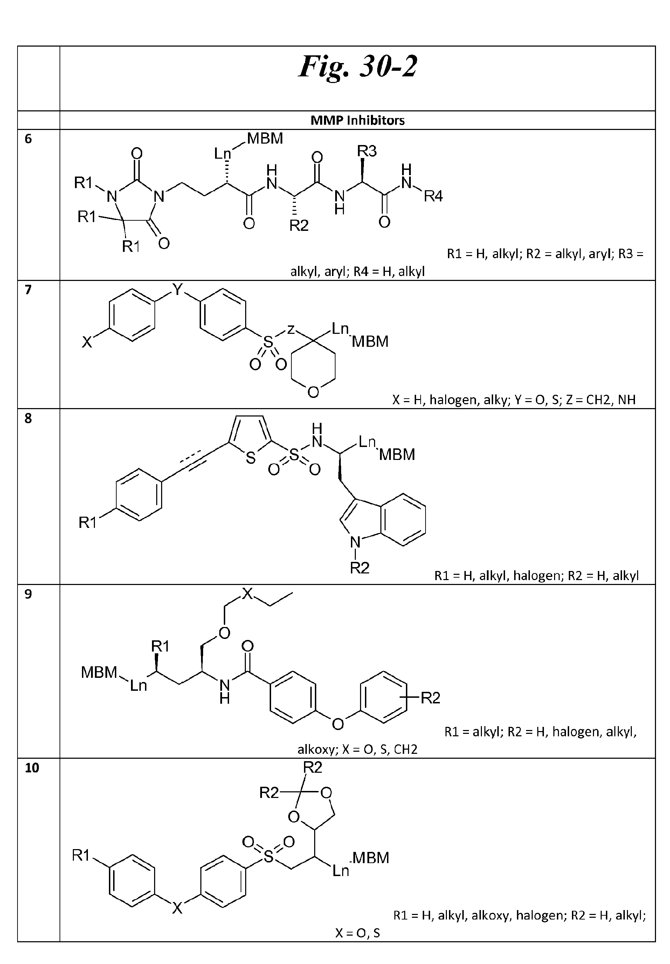

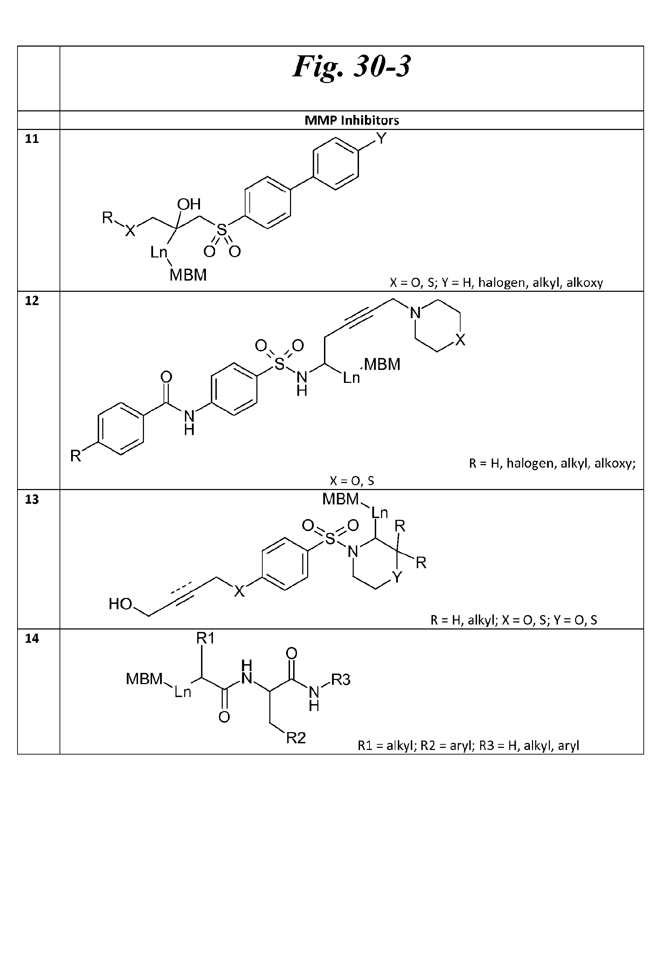

Figure 30 depicts a number of inhibitors of MMP, which are suitable for use as targeting moieties in the present invention. Figure 30 shows the structure of these known inhibitors along with possible sites of attachment of the linkers and metal binding moieties ("R"), as well as possible derivatives.

Accordingly, in some embodiments, the metalloenzyme inhibitor is a matrix metalloproteinase (MMP) inhibitor comprising a targeting moiety, a metal binding moiety, and optionally a linker, and wherein the inhibitor has a formula selected from the group consisting of:
(a) 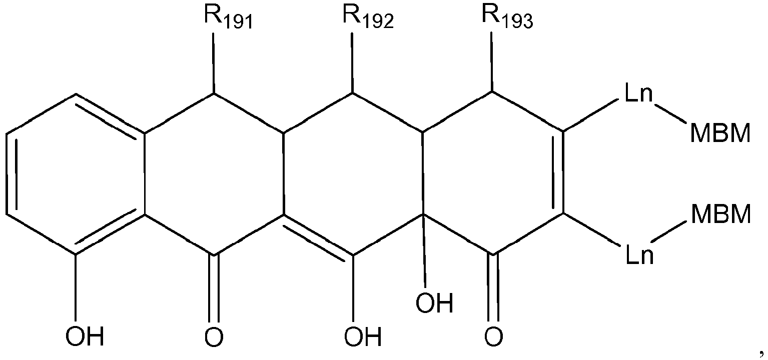 wherein:
   R₁₉₁ is selected from the group consisting of H, alkyl, and substituted alkyl;
   R₁₉₂ is H or OH;and
   R₁₉₃ is H or N-(alkyl)₂;
(b) 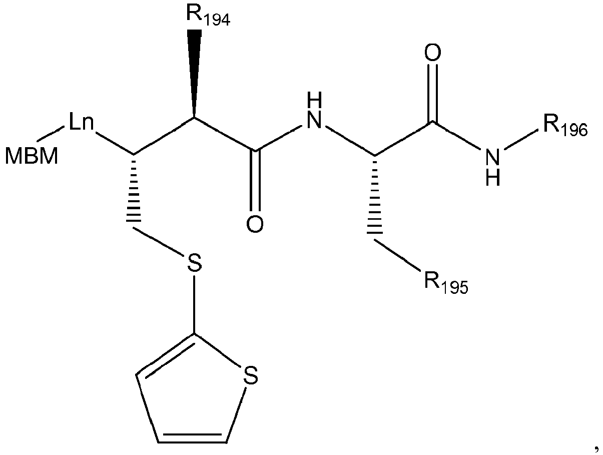 wherein:
   R₁₉₄ is alkyl or substituted alkyl;
   R₁₉₅ is aryl or substituted aryl; and
   R₁₉₆ is selected from the group consisting of H, alkyl, and substituted alkyl;
(c) 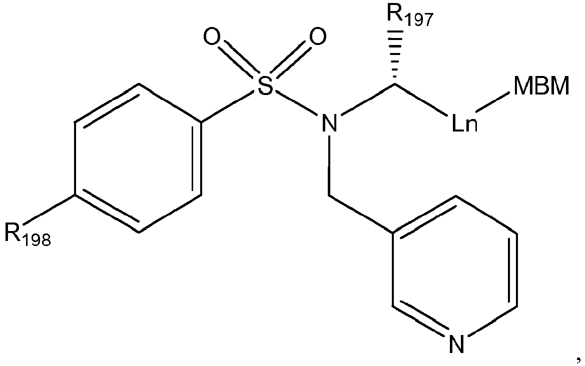 wherein:
   R₁₉₇ is alkyl or substituted alkyl; and
   R₁₉₈ is selected from the group consisting of H, halogen, alkyl, substituted alkyl, and alkoxyl;
(d) 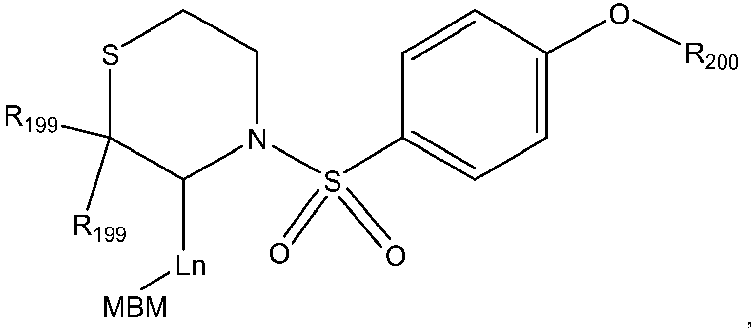 wherein:
   each R₁₉₉ is independently selected from the group consisting of H, alkyl, and substituted alkyl; and
   R₂₀₀ is selected from the group consisting of aryl, substituted aryl, heteroaryl, and substituted heteroaryl;
(e) 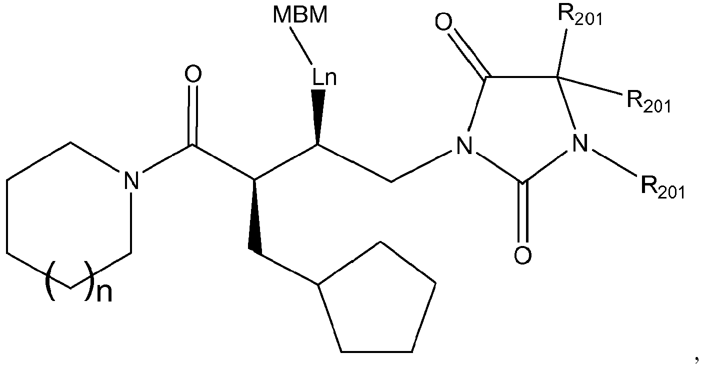 wherein:
   n is an integer from 0 to 1;
   each R₂₀₁ is independently selected from the group consisting of H, alkyl, and substituted alkyl;
(f) 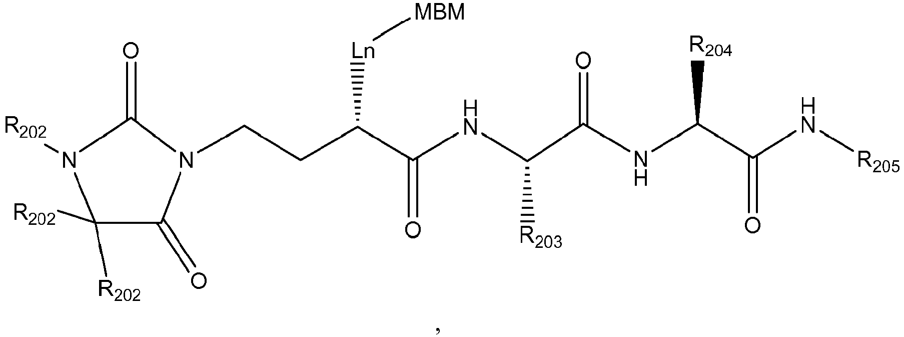 wherein:
   each R₂₀₂ is independently selected from the group consisting of H, alkyl, and substituted alkyl;
   R₂₀₃ and R₂₀₄ are each independently selected from the group consisting of alkyl, substituted alkyl, aryl, and substituted aryl; and
   R₂₀₅ is selected from the group consisting of H, alkyl, and substituted alkyl;
(g) 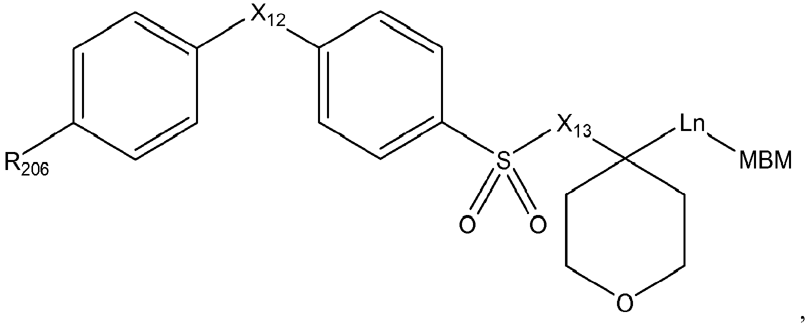 wherein:
   R₂₀₆ is selected from the group consisting of H, halogen, alkyl, and substituted alkyl;
   X₁₂ is O or S; and
   X₁₃ is CH₂ or NH;
(h) 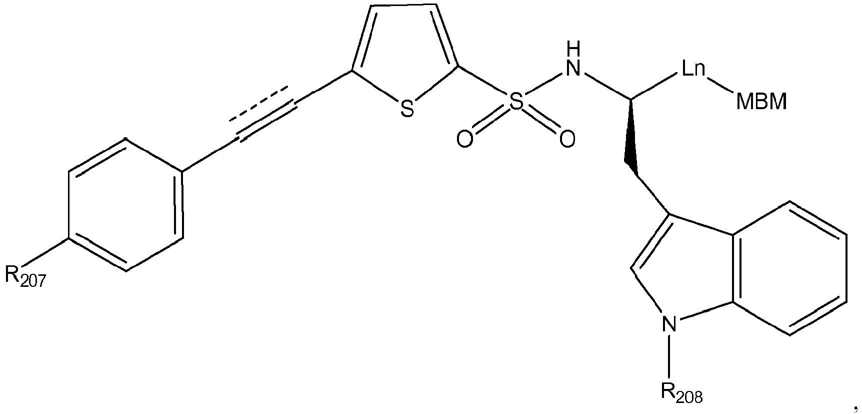 wherein:
   R₂₀₇ is selected from the group consisting of H, alkyl, substituted alkyl, and halogen; and
   R₂₀₈ is selected from the group consisting of H, alkyl, and substituted alkyl;
(i) 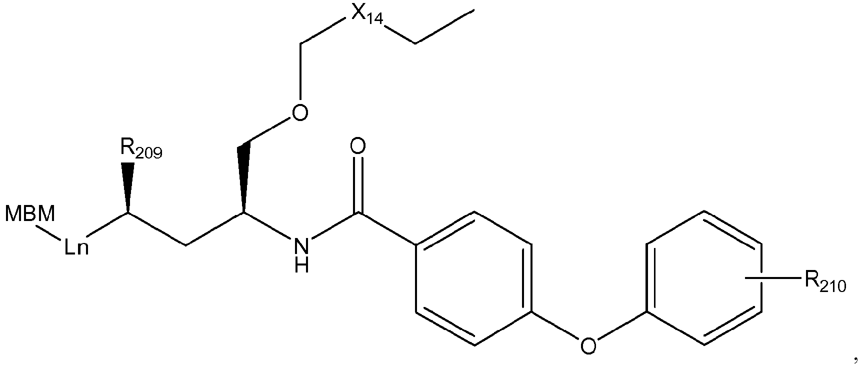 wherein:
   R₂₀₉ is alkyl or substituted alkyl;
   R₂₁₀ is selected from the group consisting of H, halogen, alkyl, substituted alkyl, and alkoxyl; and
   X₁₄ is selected from the group consisting of O, S, and CH₂;
(j) 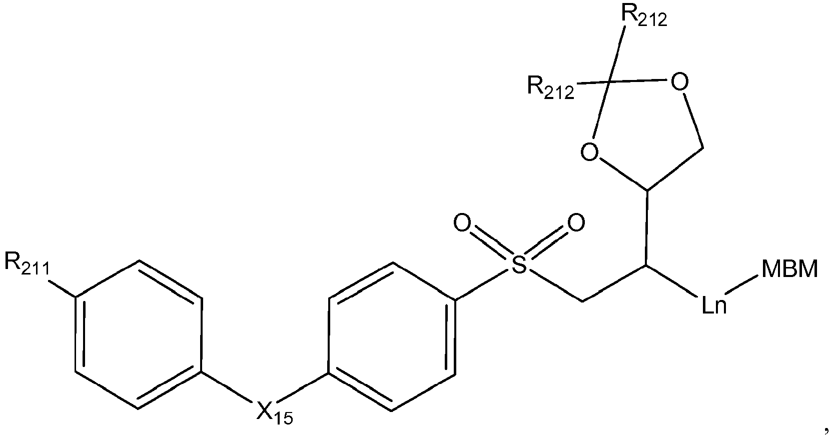 wherein:
   R₂₁₁ is selected from the group consisting of H, alkyl, substituted alkyl, alkoxyl, and halogen;
   each R₂₁₂ is independently selected from the group consisting of H, alkyl, and substituted alkyl; and
   X₁₅ is O or S;
(k) 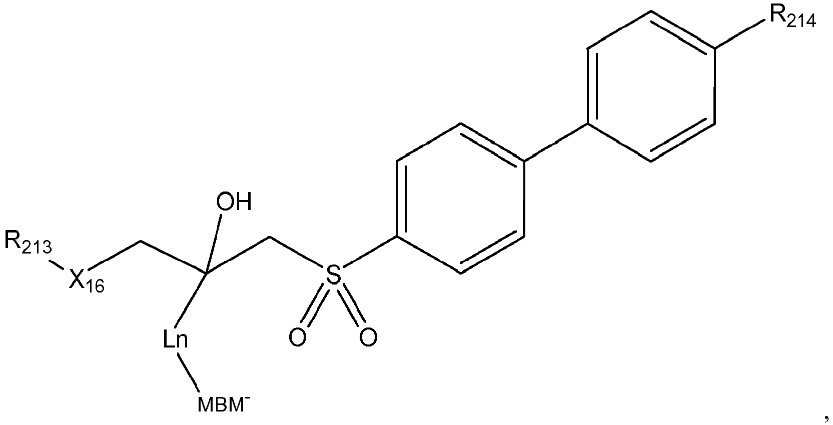 wherein:
   R₂₁₃ is alkyl or substituted alkyl;
   R₂₁₄ is selected from the group consisting of H, alkyl, substituted alkyl, halogen, and alkoxyl
   X₁₆ is O or S;
(l) 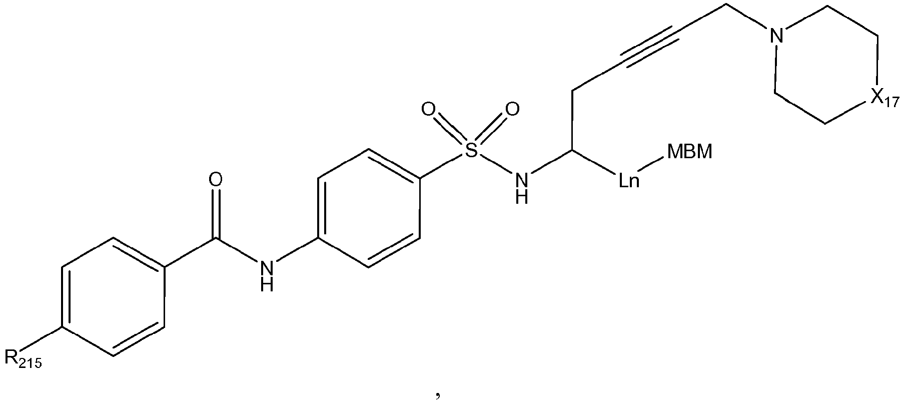 wherein:
   R₂₁₅ is selected from the group consisting of H, halogen, alkyl, substituted alkyl, and alkoxyl;
   X₁₇ is O or S;
(m) 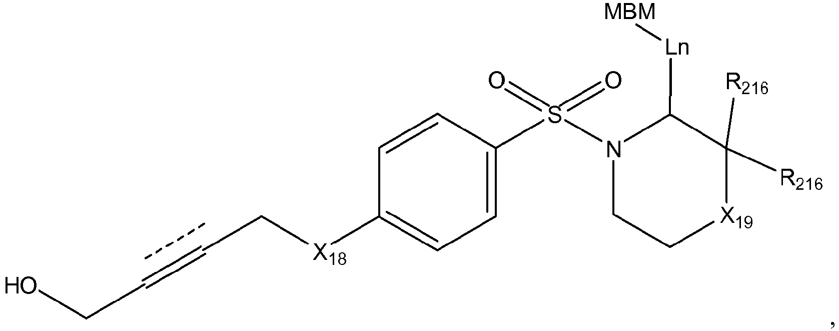 wherein:
   each R₂₁₆ is independently selected from the group consisting of H, alkyl, and substituted alkyl;
   X₁₈ and X₁₉ are each independently O or S;
(n) 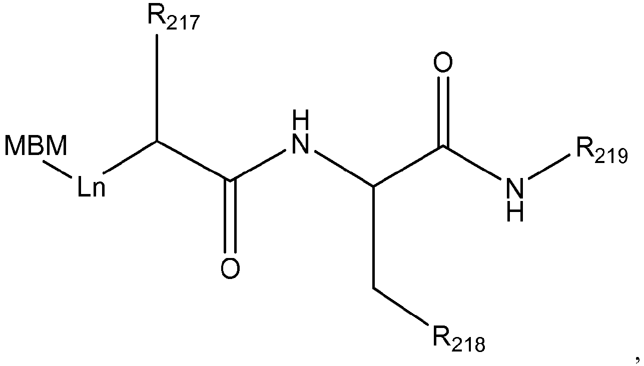 wherein:
   R₂₁₇ is alkyl or substituted alkyl;
   R₂₁₈ is aryl or substituted aryl; and
   R₂₁₉ is selected from the group consisting of H, alkyl, substituted alkyl, aryl, and substituted aryl; and
   wherein:
   MBM is a metal binding moiety;
   Lₙ is a linker, wherein n is an integer from 0 to 1;
   under the proviso that the metal binding moiety is not a metal binding moiety selected from the group consisting of: 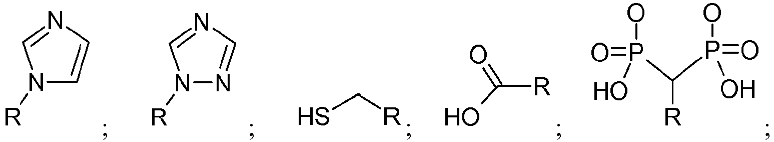 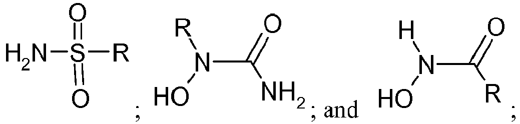 wherein:
      R is an attachment site through which the metal binding moiety can be attached to the targeting moiety, optionally through linker, Lₙ.

In addition to these targeting moieties, other known targeting moieties, identified by the screens outlined below or shown to bind to MMP can be used. Thus, suitable targeting moieties include, but are not limited to, small organic molecules including known drugs and drug candidates, polysaccharides, fatty acids, vaccines, polypeptides, proteins (including peptides, as described herein), nucleic acids, carbohydrates, lipids, hormones including proteinaceous and steroid hormones, growth factors, receptor ligands, antigens, antibodies and enzymes, (as outlined below, collectively "candidate agents"), and the like.

MMP activity can be measured using established methods. Weingarten H. and Feder J., Anal Biochem, 147:437-445 (1985); Knight C. G. et al., FEBS Lett, 296:263-269 (1992).

### C. Lingers

The inhibitors of the presently disclosed subject matter also optionally include a linker. That is, in some instances, the targeting moiety is linked directly to the metal binding moieties. Optionally, linkers comprising at least one atom can be used. By "linker" herein is meant at least one atom that provides a covalent linkage between the metal binding moiety and the targeting moiety. In some cases, there can be a single linker used, for example when the inhibitor has the general formula MBM-linker-TM or TM-linker-MBM. Alternatively, several linkers could be used; for example, in the case where more than one metal binding moieties or targeting moiety is used: MBM1-linker-MBM2-linker-TM, and the like.

Preferred linkers include, but are not limited to, alkyl or aryl groups, including substituted alkyl and heteroalkyl and aryl and heteroaryl groups, as outlined herein. Short straight alkyl chains are useful in many embodiments. The selection of the linker is generally done using well known molecular modeling techniques. In addition, the length of this linker can be important in order to achieve optimal results. In general, this can be modeled using the crystal structure of the metalloenzyme.

In some cases, the metal binding moieties and targeting moieties are covalently attached using well known chemistries. In many cases, both the metal binding moieties and the targeting moiety contains a chemical functional group that is used to add the components of the presently disclosed subject matter together, as is outlined herein. Thus, in general, the components of the presently disclosed subject matter are attached through the use of functional groups on each that can then be used for attachment. Preferred functional groups for attachment are amino groups, carboxy groups, oxo groups and thiol groups. These functional groups can then be attached, either directly or indirectly through the use of a linker. Linkers are well known in the art; for example, homo-or hetero-bifunctional linkers as are well known (see 1994 Pierce Chemical Company catalog, technical section on cross-linkers, pages 155-200, incorporated herein by reference). Alternatively, the whole molecule is synthesized in steps, rather than by joining two pieces.

### D. Presently Disclosed Inhibitors

As described herein, the presently disclosed inhibitors comprise one or more targeting moieties and one or more metal binding moieties. As will be appreciated by those in the art, specific presently disclosed inhibitors comprise any of the targeting moieties outlined herein joined with an optional linker to any of the metal binding moieties outlined herein, such as those of the figures. Thus, Figure 1A structures can be joined with Figure 16 (1) structures, and the like. In addition, any of the targeting moieties can be joined with classes and/or subclasses of metal binding moieties, to form inhibitors to be tested for specific enzymatic properties such as Ki.

Thus, for example, any independently selected metal binding moiety, or class or subclass of metal binding moiety listed in Figures can be added to any independently selected targeting moiety. For example, 5-membered aromatic rings with heteroatoms can be added to any independently selected carbonic anhydrase inhibitor depicted in Figure 16. Any and all combinations and subcombinations of any size are contemplated.

### III. Screening for Metalloenzyme Inhibitors

Screens can be designed to find targeting moieties that can bind to metalloenzyme proteins, and then these targeting moieties can be linked to the metal binding moieties to form metalloenzyme candidate inhibitors and then used in assays that evaluate the ability of the candidate inhibitors to modulate metalloenzyme bioactivity. Alternatively, targeting moieties can be linked with the metal binding moiety to first screen for binding activity to metalloenzymes and then screen inhibiting activity, or in opposite order. Thus, as will be appreciated by those in the art, there are a number of different assays which can be run; binding assays and activity assays.

### A. Target moiety screening

In a preferred embodiment, the methods comprise combining metalloenzyme proteins and a candidate targeting moiety, and determining the binding of the targeting moiety to the metalloenzyme proteins. In general, as described herein, the assays are done by contacting a metalloenzyme protein with one or more targeting moieties to be tested.

Targeting moieties encompass numerous chemical classes. In one embodiment, the target moiety is an organic molecule, preferably small organic compounds having a molecular weight of more than 100 and less than about 2,500 daltons. Particularly preferred are small organic compounds having a molecular weight of more than 100 and less than about 2,000 daltons, more preferably less than about 1500 daltons, more preferably less than about 1000 daltons, more preferably less than 500 daltons. Targeting moieties comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Targeting moieties are also found among biomolecules including peptides, saccharides, fatty acids, steroids, purines, pyrimidines, derivatives, structural analogs or combinations thereof.

Targeting moieties are obtained from a wide variety of sources including libraries of synthetic or natural compounds. For example, numerous means are available for random and directed synthesis of a wide variety of organic compounds and biomolecules, including expression of randomized oligonucleotides. Alternatively, libraries of natural compounds in the form of bacterial, fungal, plant and animal extracts are available or readily produced. Additionally, natural or synthetically produced libraries and compounds are readily modified through conventional chemical, physical and biochemical means. Known pharmacological agents can be subjected to directed or random chemical modifications, such as acylation, alkylation, esterification, amidification to produce structural analogs.

In a preferred embodiment, the targeting moieties are organic chemical moieties, a wide variety of which are available in the literature.

In a preferred embodiment, the targeting moieties are obtained from combinatorial chemical libraries, a wide variety of which are available in the literature. By "combinatorial chemical library" herein is meant a collection of diverse chemical compounds generated in a defined or random manner, generally, but not always, by chemical synthesis. Millions of chemical compounds can be synthesized through combinatorial mixing.

In some embodiments, a library of different targeting moieties is used. Preferably, the library should provide a sufficiently structurally diverse population of randomized agents to effect a probabilistically sufficient range of diversity to allow binding to a particular target.

Once expressed and purified, if necessary, the metalloenzyme proteins are used in screening assays for the identification of metalloenzyme candidate inhibitors comprising metal binding moieties and targeting moieties that bind to the metalloenzyme proteins and inhibit metalloenzyme activity.

In a preferred embodiment, the targeting moieties are screened first by using candidate agents as outlined herein for their desired properties and then linked to the metal binding moiety to form metalloenzyme candidate inhibitors for further screening using the method provided in the presently disclosed subject matter.

In another embodiment, the targeting moiety is not pre-screened. The targeting moieties are linked to the metal binding moiety, and then are used for screening using the method provided in the presently disclosed subject matter.

The targeting moieties are contacted with the metalloenzyme protein under reaction conditions that favor agent-target interactions. Generally, this will be physiological conditions. Incubations can be performed at any temperature which facilitates optimal activity, typically between 4 °C and 40 °C. Incubation periods are selected for optimum activity, but also can be optimized to facilitate rapid high through put screening. Typically between 0.1 and 1 hour will be sufficient. Excess reagent is generally removed or washed away, in the case of solid phase assays. Assay formats are discussed below.

A variety of other reagents can be included in the assays. These include reagents like salts, neutral proteins, e.g., albumin, detergents, and the like which can be used to facilitate optimal metalloenzyme protein-targeting moiety binding and/or reduce non-specific or background interactions. Also reagents that otherwise improve the efficiency of the assay, such as protease inhibitors, nuclease inhibitors, anti-microbial agents, and the like, can be used. The mixture of components can be added in any order that provides for the requisite binding.

In one embodiment, solution phase binding assays are done. Generally in this embodiment, fluorescence resonance energy transfer (FRET) assays are done, by labeling both the targeting moieties and metalloenzyme proteins with different fluorophores with overlapping spectra. As energy transfer is distance dependent, in the absence of binding the excitation at one wavelength does not produce an emission spectra. Only if the two labels are close, e.g., when binding has occurred, will excitation at one wavelength result in the desired emission spectra of the second label.

In some embodiments, solid phase (heterogeneous) assays are done. In this case, binding assays are done wherein either the metalloenzyme protein or the targeting moiety is non-diffusably bound to an insoluble solid support, and detection is done by adding the other component which is labeled, as described below.

The insoluble supports can be made of any composition to which the compositions can be bound, is readily separated from soluble material, and is otherwise compatible with the overall method of screening. The surface of such supports can be solid or porous and of any convenient shape. Examples of suitable supports include microtiter plates, arrays, membranes and beads, and include, but are not limited to, glass and modified or functionalized glass, plastics (including acrylics, polystyrene and copolymers of styrene and other materials, polypropylene, polyethylene, polybutylene, polyurethanes, Teflon, and the like.), polysaccharides, nylon or nitrocellulose, resins, silica or silica based materials including silicon and modified silicon, carbon, metals, inorganic glasses, plastics, ceramics, and a variety of other polymers. In a some embodiments, the solid supports allow optical detection and do not themselves appreciably fluoresce. In addition, as is known the art, the solid support can be coated with any number of materials, including polymers, such as dextrans, acrylamides, gelatins, agarose, and the like. Exemplary solid supports include silicon, glass, polystyrene and other plastics and acrylics. Microtiter plates and arrays are especially convenient because a large number of assays can be carried out simultaneously, using small amounts of reagents and samples. The particular manner of binding of the composition is not crucial so long as it is compatible with the reagents and overall methods of the presently disclosed subject matter, maintains the activity of the composition and is nondiffusable.

In a preferred embodiment, the metalloenzyme protein is bound to the support, and a library of targeting moieties is added to the assay. Alternatively, the targeting moiety is bound to the support and the metalloenzyme protein is added. Attachment to the solid support is accomplished using well known methods, and will depend on the composition of the two materials to be attached. In general, for covalent attachment, attachment linkers are utilized through the use of functional groups on each component that can then be used for attachment. Preferred functional groups for attachment are amino groups, carboxy groups, oxo groups, hydroxyl groups and thiol groups. These functional groups can then be attached, either directly or indirectly through the use of a linker. Linkers are well known in the art; for example, homo-or hetero-bifunctional linkers as are well known (see 1994 Pierce Chemical Company catalog, technical section on cross-linkers, pages 155-200, incorporated herein by reference). In some embodiments, absorption or ionic interactions are utilized. In some cases, small molecule candidate agents are synthesized directly on microspheres, for example, which can then be used in the assays of the presently disclosed subject matter.

Following binding of the protein or targeting moiety, excess unbound material is removed by washing. The surface can then be blocked through incubation with bovine serum albumin (BSA), casein or other innocuous protein or other moiety.

In the binding assays, either the metalloenzyme protein, the targeting moiety (or, in some cases, the metal binding moiety, or substrate of metalloenzymes, described below) is labeled. By "labeled" herein is meant that the compound is either directly or indirectly labeled with a label which provides a detectable signal, e.g., radioisotope, fluorescers, enzyme, antibodies, particles such as magnetic particles, chemiluminescers, or specific binding molecules, and the like. Specific binding molecules include pairs, such as biotin and streptavidin, digoxin and antidigoxin and the like. For the specific binding members, the complementary member would normally be labeled with a molecule which provides for detection, in accordance with known procedures, as outlined above. The label can directly or indirectly provide a detectable signal.

Specific labels include optical dyes, including, but not limited to, chromophores, phosphors and fluorophores, with the latter being specific in many instances. Fluorophores can be either "small molecule" fluores, or proteinaceous fluores as described above. The labeled metal donor (e.g., the metal binding component) can be a chemical probe (such as Zinquin or Zinbo5) which undergoes a spectroscopic change when it releases the metal ion as described herein.

By "fluorescent label" is meant any molecule that can be detected via its inherent fluorescent properties. Suitable fluorescent labels include, but are not limited to, fluorescein, rhodamine, tetramethylrhodamine, eosin, erythrosin, coumarin, methyl-coumarins, pyrene, Malacite green, stilbene, Lucifer Yellow, Cascade BlueJ, Texas Red, IAEDANS, EDANS, BODIPY FL, LC Red 640, Cy 5, Cy 5.5, LC Red 705, Oregon green, the Alexa-Fluor dyes (Alexa Fluor 350, Alexa Fluor 430, Alexa Fluor 488, Alexa Fluor 546, Alexa Fluor 568, Alexa Fluor 594, Alexa Fluor 633, Alexa Fluor 660, Alexa Fluor 680), Cascade Blue, Cascade Yellow and R-phycoerythrin (PE) (Molecular Probes, Eugene, OR), FITC, Rhodamine, and Texas Red (Pierce, Rockford, IL), Cy5, Cy5.5, Cy7 (Amersham Life Science, Pittsburgh, PA). Suitable optical dyes, including fluorophores, are described in *Molecular Probes Handbook* by Richard P. Haugland, hereby expressly incorporated by reference.

In one embodiment, the metalloenzyme protein is attached to the support, adding labeled targeting moiety, washing off excess reagent, and determining whether the label is present on the solid support. Various blocking and washing steps can be utilized as is known in the art.

In one embodiment, the targeting moieties are immobilized to the support, and a labeled metalloenzyme protein is added to determine binding.

Activity assays are done as are known in the art.

In one embodiment, any of the assays outlined herein can utilize robotic systems for high throughput screening. Many systems are generally directed to the use of 96 (or more) well microtiter plates, but as will be appreciated by those in the art, any number of different plates or configurations can be used. In addition, any or all of the steps outlined herein can be automated; thus, for example, the systems can be completely or partially automated.

As will be appreciated by those in the art, there are a wide variety of components which can be used, including, but not limited to, one or more robotic arms; plate handlers for the positioning of microplates; automated lid handlers to remove and replace lids for wells on non-cross contamination plates; tip assemblies for sample distribution with disposable tips; washable tip assemblies for sample distribution; 96 well loading blocks; cooled reagent racks; microtiter plate pipette positions (optionally cooled); stacking towers for plates and tips; and computer systems.

Fully robotic or microfluidic systems include automated liquid-, particle-, cell-and organism-handling including high throughput pipetting to perform all steps of screening applications. This includes liquid, particle, cell, and organism manipulations such as aspiration, dispensing, mixing, diluting, washing, accurate volumetric transfers; retrieving, and discarding of pipette tips; and repetitive pipetting of identical volumes for multiple deliveries from a single sample aspiration. These manipulations are cross-contamination-free liquid, particle, cell, and organism transfers. This instrument performs automated replication of microplate samples to filters, membranes, and/or daughter plates, high-density transfers, full-plate serial dilutions, and high capacity operation.

In a preferred embodiment, chemically derivatized particles, plates, tubes, magnetic particle, or other solid phase matrix with specificity to the assay components are used. The binding surfaces of microplates, tubes or any solid phase matrices include non-polar surfaces, highly polar surfaces, modified dextran coating to promote covalent binding, antibody coating, affinity media to bind fusion proteins or peptides, surface-fixed proteins such as recombinant protein A or G, nucleotide resins or coatings, and other affinity matrix are useful in this presently disclosed subject matter.

In a preferred embodiment, platforms for multi-well plates, multi-tubes, minitubes, deep-well plates, microfuge tubes, cryovials, square well plates, filters, chips, optic fibers, beads, and other solid-phase matrices or platform with various volumes are accommodated on an upgradable modular platform for additional capacity. This modular platform includes a variable speed orbital shaker, electroporator, and multi-position work decks for source samples, sample and reagent dilution, assay plates, sample and reagent reservoirs, pipette tips, and an active wash station.

In a preferred embodiment, thermocycler and thermoregulating systems are used for stabilizing the temperature of the heat exchangers such as controlled blocks or platforms to provide accurate temperature control of incubating samples from 4°C to 100 °C.

In some preferred embodiments, the instrumentation will include a detector, which can be a wide variety of different detectors, depending on the labels and assay. In a preferred embodiment, useful detectors include a microscope(s) with multiple channels of fluorescence; plate readers to provide fluorescent, ultraviolet and visible spectrophotometric detection with single and dual wavelength endpoint and kinetics capability, fluorescence resonance energy transfer (FRET), SPR systems, luminescence, quenching, two-photon excitation, and intensity redistribution; CCD cameras to capture and transform data and images into quantifiable formats; and a computer workstation. These will enable the monitoring of the size, growth and phenotypic expression of specific markers on cells, tissues, and organisms; target validation; lead optimization; data analysis, mining, organization, and integration of the high-throughput screens with the public and proprietary databases.

These instruments can fit in a sterile laminar flow or fume hood, or are enclosed, self-contained systems as needed. Flow cytometry or capillary electrophoresis formats can be used for individual capture of magnetic and other beads, particles, cells, and organisms.

The flexible hardware and software allow instrument adaptability for multiple applications. The software program modules allow creation, modification, and running of methods. The system diagnostic modules allow instrument alignment, correct connections, and motor operations. The customized tools, labware, and liquid, particle, cell and organism transfer patterns allow different applications to be performed. The database allows method and parameter storage. Robotic and computer interfaces allow communication between instruments.

In a preferred embodiment, the robotic workstation includes one or more heating or cooling components. Depending on the reactions and reagents, either cooling or heating can be required, which can be done using any number of known heating and cooling systems, including Peltier systems.

In a preferred embodiment, the robotic apparatus includes a central processing unit that communicates with a memory and a set of input/output devices (e.g., keyboard, mouse, monitor, printer, and the like.) through a bus. The general interaction between a central processing unit, a memory, input/output devices, and a bus is known in the art. Thus, a variety of different procedures, depending on the experiments to be run, are stored in the CPU memory.

Accordingly, in some embodiments, the presently disclosed subject matter provides a method of screening for inhibitors of a metalloenzyme, the method comprising: (a) providing a candidate inhibitor comprising: (i) a targeting moiety; (ii) a metal binding moiety; and (iii) optionally a linker; (b) contacting the inhibitor candidate with a metalloenzyme; and (c) determining the activity of the metalloenzyme.

In some embodiments, the metal binding moiety is a metal binding moiety of iron. In some embodiments, the metal binding moiety is selected from the group consisting of the metal binding moieties presented in Figures 1-15.

In some embodiments, the determining can be accomplished by measuring a substrate of a metalloenzyme. In some embodiments of the presently disclosed screening method, the determining is done by measuring a product resulting from the hydrolysis of the substrate by at least one step. In other embodiments, the determining is done by measuring a product resulting from the demethylation of the substrate by more than one step.

### IV. Pharmaceutical Compositions and Methods of Treatment

As previously discussed, the presently disclosed inhibitors inhibit the activity of metalloenzyme. As a consequence of these activities, the active compounds of the presently disclosed subject matter can be used in a variety of *in vitro, in vivo* and *ex vivo* contexts to inhibit activity, particularly in cases where metalloenzyme activity is implicated in disease states.

Accordingly, in some embodiments, the presently disclosed subject matter provides a method of inhibiting a metalloenzyme comprising contacting the metalloenzyme with a presently disclosed inhibitor. In some embodiments, the metalloenzyme is selected from the group consisting of carbonic anhydrase, catechol O-methyl transferase (COMT), farnesyl diphosphate synthase, farnesyl transferase, neprilysin (NEP), thromboxane synthase, anthrax lethal factor, endothelin converting enzyme, methionine aminopeptidase-2, peptide deformylase, HIV integrase, TNF-alpha converting enzyme (TACE), UDP-(3-O-acyl)-N-acetylglucosamine deacetylase (LpxC), histone deacetylase (HDAC), and matrix metalloproteinase (MMP).

Further, in some embodiments, the presently disclosed subject matter provides a method of treating a metalloenzyme related disorder comprising administering a composition of the presently disclosed inhibitors or a prodrug or salt thereof to a patient in need thereof. In some embodiments, the disorder is selected from disorders associated with carbonic anhydrase, catechol O-methyl transferase (COMT), farnesyl diphosphate synthase, farnesyl transferase, neprilysin (NEP), thromboxane synthase, anthrax lethal factor, endothelin converting enzyme, methionine aminopeptidase-2, peptide deformylase, HIV integrase, TNF-alpha converting enzyme (TACE), UDP-(3-O-acyl)-N-acetylglucosamine deacetylase (LpxC), histone deacetylase (HDAC), and matrix metalloproteinase (MMP).

When used to treat or prevent such diseases, the active compounds can be administered singly, as mixtures of one or more active compounds or in mixture or combination with other agents useful for treating such diseases and/or the symptoms associated with such diseases. The active compounds also can be administered in a mixture or in combination with agents useful to treat other disorders. The active compounds can be administered per se in the form of prodrugs or as pharmaceutical compositions, comprising an active compound or prodrug.

Pharmaceutical compositions comprising the active compounds of the presently disclosed subject matter (or prodrugs thereof) can be manufactured by means of conventional mixing, dissolving, granulating, dragee-making levigating, emulsifying, encapsulating, entrapping or lyophilization processes. The compositions can be formulated in conventional manner using one or more physiologically acceptable carriers, diluents, excipients or auxiliaries which facilitate processing of the active compounds into preparations which can be used pharmaceutically.

The active compound or prodrug can be formulated in the pharmaceutical compositions per se, or in the form of a hydrate, solvate, N-oxide or pharmaceutically acceptable salt, as previously described. Typically, such salts are more soluble in aqueous solutions than the corresponding free acids and bases, but salts having lower solubility than the corresponding free acids and bases also can be formed.

Pharmaceutical compositions of the presently disclosed subject matter can take a form suitable for virtually any mode of administration, including, for example, topical, ocular, oral, baccal, systemic, nasal, injection, transdermal, rectal, vaginal, and the like, or a form suitable for administration by inhalation or insufflation.

For topical administration, the active compound(s) or prodrug(s) can be formulated as solutions, gels, ointments, creams, suspensions, and the like, as are well-known in the art.

Systemic formulations include those designed for administration by injection, e.g., subcutaneous, intravenous, intramuscular, intrathecal or intraperitoneal injection, as well as those designed for transdermal, transmucosal oral or pulmonary administration.

Useful injectable preparations include sterile suspensions, solutions or emulsions of the active compound(s) in aqueous or oily vehicles. The compositions also can contain formulating agents, such as suspending, stabilizing and/or dispersing agent. The formulations for injection can be presented in unit dosage form, e.g., in ampules or in multidose containers, and can contain added preservatives.

Alternatively, the injectable formulation can be provided in powder form for reconstitution with a suitable vehicle, including but not limited to sterile pyrogen free water, buffer, dextrose solution, and the like, before use. To this end, the active compound(s) can be dried by any art-known technique, such as lyophilization, and reconstituted prior to use.

For transmucosal administration, penetrants appropriate to the barrier to be permeated are used in the formulation. Such penetrants are known in the art.

For oral administration, the pharmaceutical compositions can take the form of, for example, lozenges, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulfate). The tablets can be coated by methods well known in the art with, for example, sugars or enteric coatings.

Liquid preparations for oral administration can take the form of, for example, elixirs, solutions, syrups or suspensions, or they can be presented as a dry product for constitution with water or other suitable vehicle before use. Such liquid preparations can be prepared by conventional means with pharmaceutically acceptable additives such as suspending agents (e.g., sorbitol syrup, cellulose derivatives or hydrogenated edible fats); emulsifying agents (e.g., lecithin or acacia); non aqueous vehicles (e.g., almond oil, oily esters, ethyl alcohol or fractionated vegetable oils); and preservatives (e.g., methyl or propyl p hydroxybenzoates or sorbic acid). The preparations also can contain buffer salts, preservatives, flavoring, coloring and sweetening agents as appropriate.

Preparations for oral administration can be suitably formulated to give controlled release of the active compound or prodrug, as is well known.

For buccal administration, the compositions can take the form of tablets or lozenges formulated in conventional manner.

For rectal and vaginal routes of administration, the active compound(s) can be formulated as solutions (for retention enemas) suppositories or ointments containing conventional suppository bases such as cocoa butter or other glycerides.

For nasal administration or administration by inhalation or insufflation, the active compound(s) or prodrug(s) can be conveniently delivered in the form of an aerosol spray from pressurized packs or a nebulizer with the use of a suitable propellant, e.g., dichlorodifluoromethane, trichlorofluoromethane, dichlorotetrafluoroethane, fluorocarbons, carbon dioxide or other suitable gas. In the case of a pressurized aerosol, the dosage unit can be determined by providing a valve to deliver a metered amount. Capsules and cartridges for use in an inhaler or insufflator (for example capsules and cartridges comprised of gelatin) can be formulated containing a powder mix of the compound and a suitable powder base such as lactose or starch.

A specific example of an aqueous suspension formulation suitable for nasal administration using commercially-available nasal spray devices includes the following ingredients: active compound or prodrug (0.5-20 mg/ml); benzalkonium chloride (0.1-0.2 mg/mL); polysorbate 80 (TWEEN® 80; 0.5-5 mg/ml); carboxymethylcellulose sodium or microcrystalline cellulose (1-15 mg/ml); phenylethanol (1-4 mg/ml); and dextrose (20-50 mg/ml). The pH of the final suspension can be adjusted to range from about pH5 to pH7, with a pH of about pH 5.5 being typical.

For ocular administration, the active compound(s) or prodrug(s) can be formulated as a solution, emulsion, suspension, and the like, suitable for administration to the eye. A variety of vehicles suitable for administering compounds to the eye are known in the art. Specific non-limiting examples are described in U.S. Patent No. 6,261,547; U.S. Patent No. 6,197,934; U.S. Patent No. 6,056,950; U.S. Patent No. 5,800,807; U.S. Patent No. 5,776,445; U.S. Patent No. 5,698,219; U.S. Patent No. 5,521,222; U.S. Patent No. 5,403,841; U.S. Patent No. 5,077,033; U.S. Patent No. 4,882,150; and U.S. Patent No. 4,738,851, each of which is incorporated herein by reference in its entirety.

For prolonged delivery, the active compound(s) or prodrug(s) can be formulated as a depot preparation for administration by implantation or intramuscular injection. The active ingredient can be formulated with suitable polymeric or hydrophobic materials (e.g., as an emulsion in an acceptable oil) or ion exchange resins, or as sparingly soluble derivatives, e.g., as a sparingly soluble salt. Alternatively, transdermal delivery systems manufactured as an adhesive disc or patch which slowly releases the active compound(s) for percutaneous absorption can be used. To this end, permeation enhancers can be used to facilitate transdermal penetration of the active compound(s). Suitable transdermal patches are described in for example, U.S. Patent No. 5,407,713; U.S. Patent No. 5,352,456; U.S. Patent No. 5,332,213; U.S. Patent No. 5,336,168; U.S. Patent No. 5,290,561; U.S. Patent No. 5,254,346; U.S. Patent No. 5,164,189; U.S. Patent No. 5,163,899; U.S. Patent No. 5,088,977; U.S. Patent No. 5,087,240; U.S. Patent No. 5,008,110; and U.S. Patent No. 4,921,475, each of which is incorporated herein by reference in its entirety.

Alternatively, other pharmaceutical delivery systems can be employed. Liposomes and emulsions are well-known examples of delivery vehicles that can be used to deliver active compound(s) or prodrug(s). Certain organic solvents such as dimethylsulfoxide (DMSO) also can be employed, although usually at the cost of greater toxicity.

The pharmaceutical compositions can, if desired, be presented in a pack or dispenser device which can contain one or more unit dosage forms containing the active compound(s). The pack can, for example, comprise metal or plastic foil, such as a blister pack. The pack or dispenser device can be accompanied by instructions for administration.

The active compound(s) or prodrug(s) of the presently disclosed subject matter, or compositions thereof, will generally be used in an amount effective to achieve the intended result, for example in an amount effective to treat or prevent the particular disease being treated. The compound(s) can be administered therapeutically to achieve therapeutic benefit or prophylactically to achieve prophylactic benefit. By therapeutic benefit is meant eradication or amelioration of the underlying disorder being treated and/or eradication or amelioration of one or more of the symptoms associated with the underlying disorder such that the patient reports an improvement in feeling or condition, notwithstanding that the patient can still be afflicted with the underlying disorder. For example, administration of a compound to a patient suffering from an allergy provides therapeutic benefit not only when the underlying allergic response is eradicated or ameliorated, but also when the patient reports a decrease in the severity or duration of the symptoms associated with the allergy following exposure to the allergen. As another example, therapeutic benefit in the context of asthma includes an improvement in respiration following the onset of an asthmatic attack, or a reduction in the frequency or severity of asthmatic episodes. Therapeutic benefit also includes halting or slowing the progression of the disease, regardless of whether improvement is realized.

For prophylactic administration, the compound can be administered to a patient at risk of developing one of the previously described diseases. Alternatively, prophylactic administration can be applied to avoid the onset of symptoms in a patient diagnosed with the underlying disorder.

The amount of compound administered will depend upon a variety of factors, including, for example, the particular indication being treated, the mode of administration, whether the desired benefit is prophylactic or therapeutic, the severity of the indication being treated and the age and weight of the patient, the bioavailability of the particular active compound, and the like. Determination of an effective dosage is well within the capabilities of those skilled in the art.

Effective dosages can be estimated initially from *in vitro* assays. For example, an initial dosage for use in animals can be formulated to achieve a circulating blood or serum concentration of active compound that is at or above an IC50 of the particular compound as measured in as *in vitro* assay, such as the *in vitro* CHMC or BMMC and other *in vitro* assays known in the art. Calculating dosages to achieve such circulating blood or serum concentrations taking into account the bioavailability of the particular compound is well within the capabilities of skilled artisans. For guidance, see Fingl & Woodbury, "General Principles," In: Goodman and Gilman's The Pharmaceutical Basis of Therapeutics, Chapter 1, pp. 1-46, latest edition, Pagamonon Press, and the references cited therein.

Initial dosages also can be estimated from *in vivo* data, such as animal models. Animal models useful for testing the efficacy of compounds to treat or prevent the various diseases described above are well-known in the art.

Dosage amounts will typically be in the range of from about 0.0001 or 0.001 or 0.01 mg/kg/day to about 100 mg/kg/day, but can be higher or lower, depending upon, among other factors, the activity of the compound, its bioavailability, the mode of administration and various factors discussed above. Dosage amount and interval can be adjusted individually to provide plasma levels of the compound(s) which are sufficient to maintain therapeutic or prophylactic effect. In cases of local administration or selective uptake, such as local topical administration, the effective local concentration of active compound(s) can not be related to plasma concentration. Skilled artisans will be able to optimize effective local dosages without undue experimentation.

The compound(s) can be administered once per day, a few or several times per day, or even multiple times per day, depending upon, among other things, the indication being treated and the judgment of the prescribing physician.

Preferably, the compound(s) will provide therapeutic or prophylactic benefit without causing substantial toxicity. Toxicity of the compound(s) can be determined using standard pharmaceutical procedures. The dose ratio between toxic and therapeutic (or prophylactic) effect is the therapeutic index. Compounds(s) that exhibit high therapeutic indices are preferred.

All publications, patent applications, patents, and other references are herein incorporated by reference to the same extent as if each individual publication, patent application, patent, and other reference was specifically and individually indicated to be incorporated by reference. It will be understood that, although a number of patent applications, patents, and other references are referred to herein, such reference does not constitute an admission that any of these documents forms part of the common general knowledge in the art.

Although the foregoing subject matter has been described in some detail by way of illustration and example for purposes of clarity of understanding, it will be understood by those skilled in the art that certain changes and modifications can be practiced within the scope of the appended claims.

Embodiments of the invention are defined in the following numbered paragraphs:
1. A metalloenzyme inhibitor, wherein the metalloenzyme inhibitor is a carbonic anhydrase inhibitor comprising a targeting moiety, a metal binding moiety, and optionally a linker, and wherein the inhibitor has a formula selected from the group consisting of:
   (a) 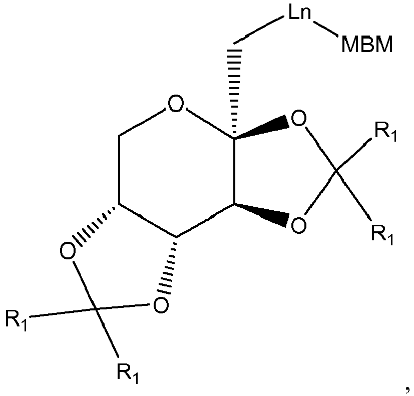 wherein each R₁ is independently alkyl or substituted alkyl;
   (b) 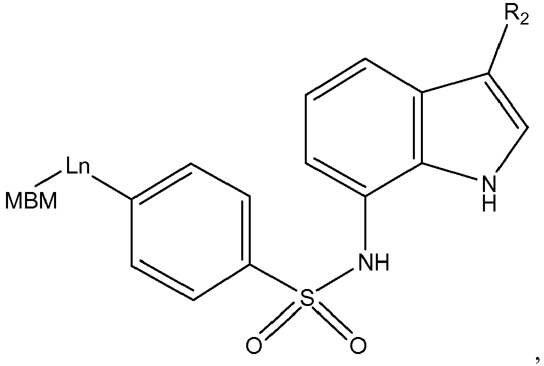 wherein R₂ is H or halogen;
   (c) 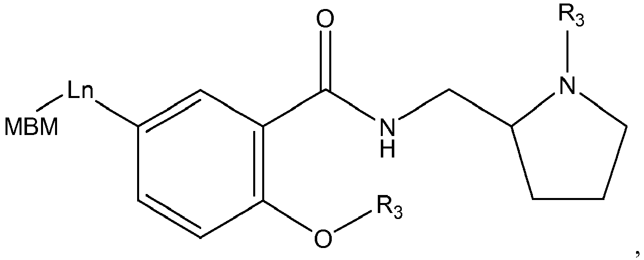 wherein each R₃ is independently alkyl or substituted alkyl;
   (d) 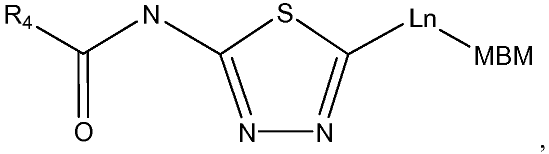 wherein R₄ is alkyl or substituted alkyl;
   (e) 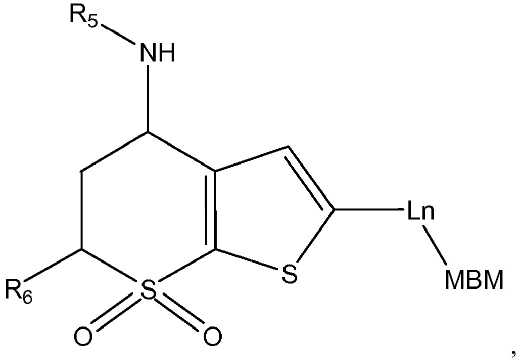 wherein:
      R₅ is alkyl or substituted alkyl; and
      R₆ is selected from the group consisting of H, alkyl, and substituted alkyl;
   (f) 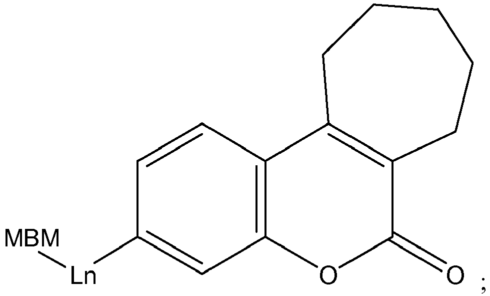
   (g) 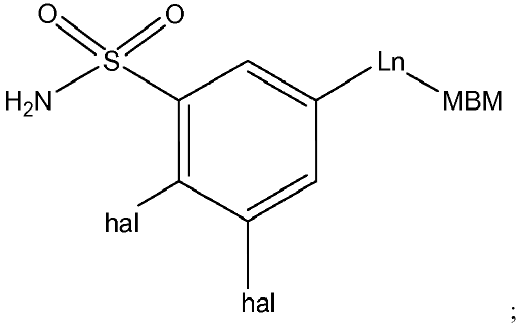 and
   (h) 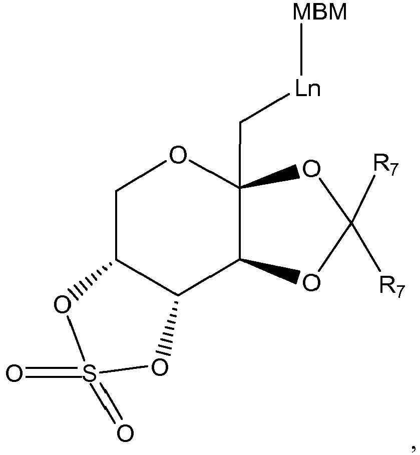 wherein each R₇ is independently alkyl or substituted alkyl; and
      wherein:
      MBM is a metal binding moiety;
      Lₙ is a linker, wherein n is an integer from 0 to 1;
      hal is halogen;
      under the proviso that the metal binding moiety is not a metal binding moiety selected from the group consisting of: 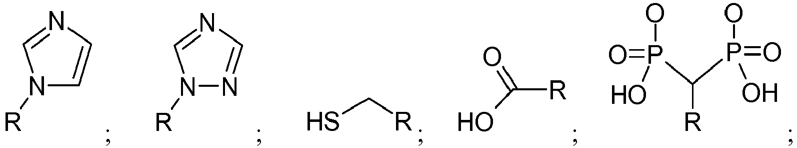 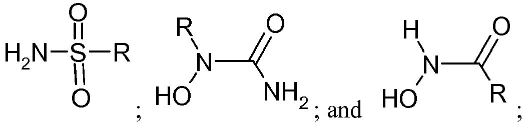 wherein:
         R is an attachment site through which the metal binding moiety can be attached to the targeting moiety, optionally through linker, Lₙ; and
         pharmaceutically acceptable salts thereof.
2. The carbonic anhydrase inhibitor of para 1, wherein the inhibitor is selected from the group of carbonic anhydrase inhibitors presented in Figure 16.
3. A metalloenzyme inhibitor, wherein the metalloenzyme inhibitor is a catechol *O*-methyl transferase inhibitor comprising a targeting moiety, a metal binding moiety, and optionally a linker, and wherein the inhibitor has a formula selected from the group consisting of:
   (a) 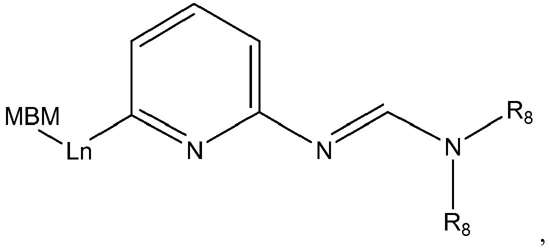 wherein each R₈ is independently selected from the group consisting of H, alkyl, and substituted alkyl;
   (b) 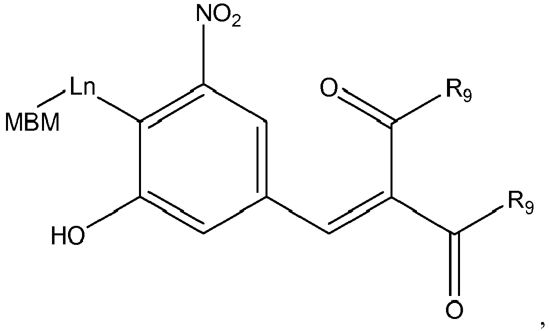 wherein each R₉ is independently alkyl or substituted alkyl;
   (c) 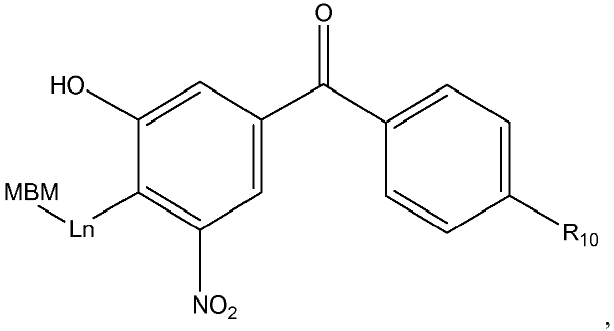 wherein R₁₀ is selected from the group consisting of H, alkyl, and substituted alkyl;
   (d) 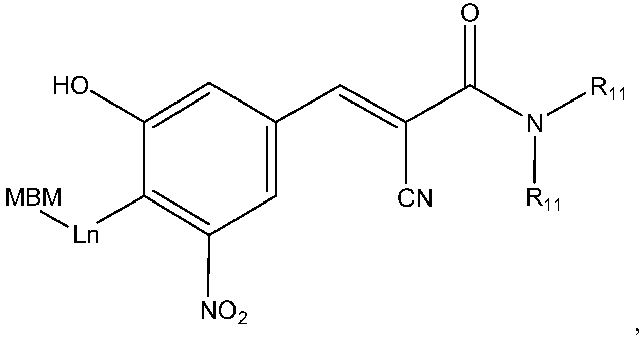 wherein each R₁₁ is independently selected from the group consisting of alkyl and substituted alkyl; and
   (e) 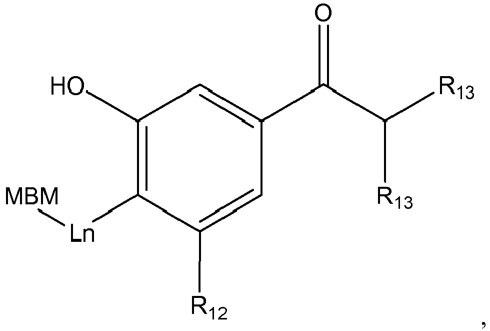 wherein:
      R₁₂ is H or NO₂; and
      R₁₃ is selected from the group consisting of H, alkyl, substituted alkyl, aryl, and substituted aryl; and
      wherein:
      MBM is a metal binding moiety;
      Lₙ is a linker, wherein n is an integer from 0 to 1;
      under the proviso that the metal binding moiety is not a metal binding moiety selected from the group consisting of: 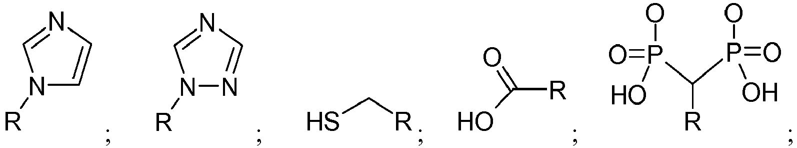 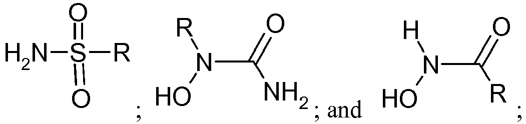 wherein:
         R is an attachment site through which the metal binding moiety can be attached to the targeting moiety, optionally through linker, Lₙ.
4. The catechol *O*-methyl transferase inhibitor of para 3, wherein the inhibitor is selected from the group of 5-lipoxygenase inhibitors presented in Figure 17.
5. A metalloenzyme inhibitor, wherein the metalloenzyme inhibitor is a farnesyl diphosphate synthase inhibitor comprising a targeting moiety, a metal binding moiety, and optionally a linker, and wherein the inhibitor has a formula selected from the group consisting of:
   (a) 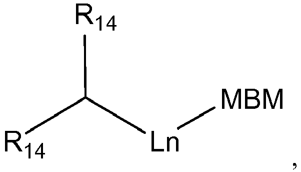 wherein each R₁₄ is independently selected from the group consiting of H, alkyl, substituted alkyl, aralkyl, halogen, 1-imidazolyl-methyl, and alkyl-amino;
   (b) 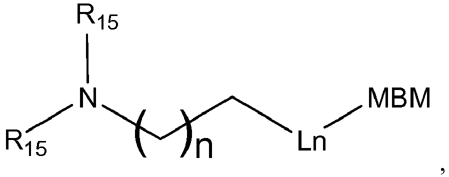 wherein:
      n is an integer from 0 to 3; and
      each R₁₅ is selected from the group consisting of H, alkyl, substituted alkyl, cycloalkyl, substituted cycloalkyl, heterocycloalkyl, and substituted heterocycloalkyl;
   (c) 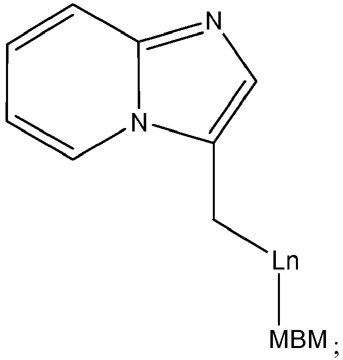
   (d) 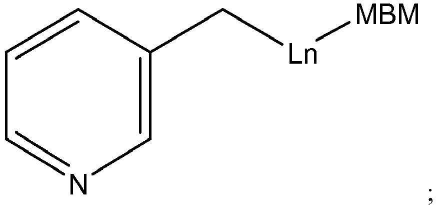 and
   (e) 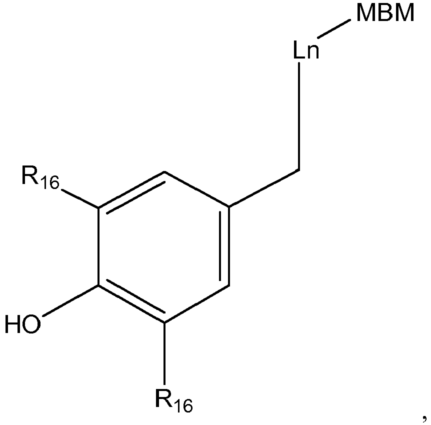 wherein each R₁₆ is independently selected from the group consisting of H, alkyl, and substituted alkyl; and
      wherein:
      MBM is a metal binding moiety;
      Lₙ is a linker, wherein n is an integer from 0 to 1;
      under the proviso that the metal binding moiety is not a metal binding moiety selected from the group consisting of: 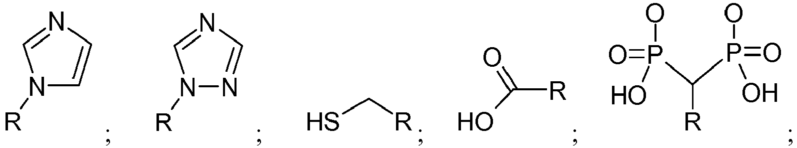 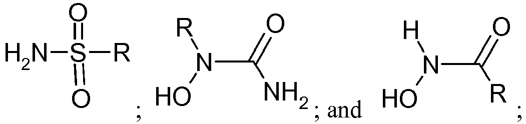 wherein:
         R is an attachment site through which the metal binding moiety can be attached to the targeting moiety, optionally through linker, Lₙ.
6. The farnesyl diphosphate synthase inhibitor of para 5, wherein the inhibitor is selected from the group of farnesyl diphosphate synthase inhibitors presented in Figure 18.
7. A metalloenzyme inhibitor, wherein the metalloenzyme inhibitor is a farnesyl transferase inhibitor comprising a targeting moiety, a metal binding moiety, and optionally a linker, and wherein the inhibitor has a formula selected from the group consisting of:
   (a) 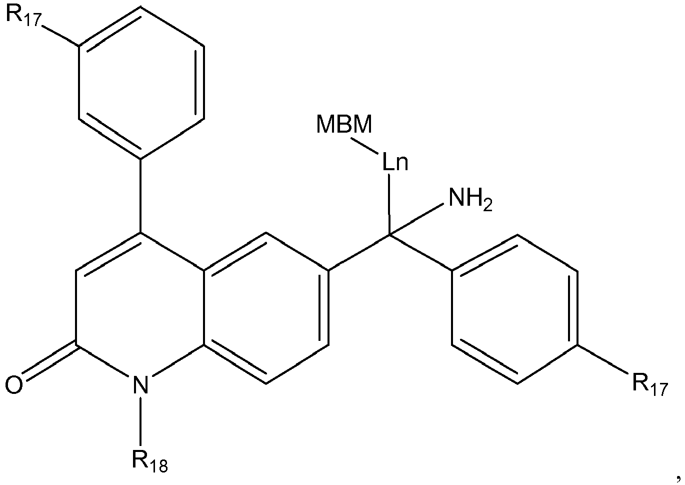 wherein:
      each R₁₇ is independently H or halogen; and
      R₁₈ is alkyl or substituted alkyl;
   (b) 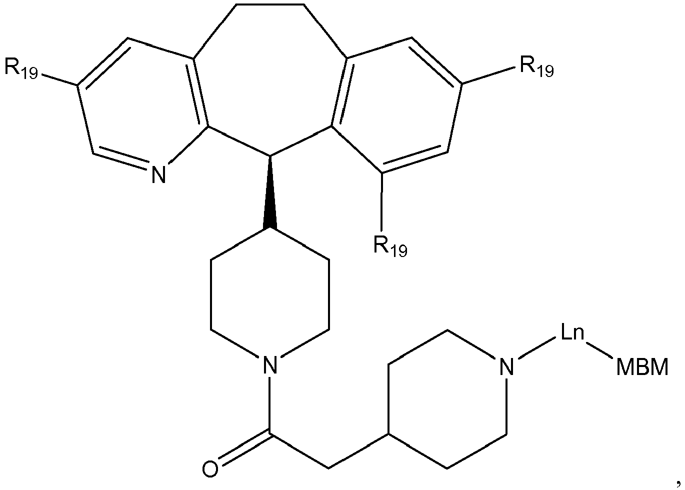 wherein each R₁₉ is independently H or halogen;
   (c) 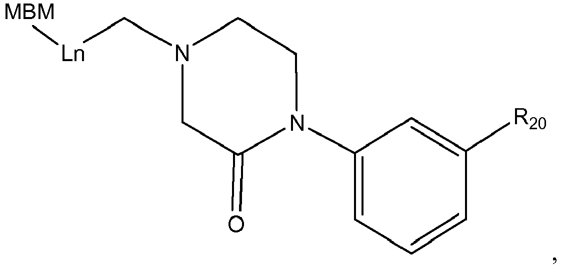 wherein R₂₀ H or halogen;
   (d) 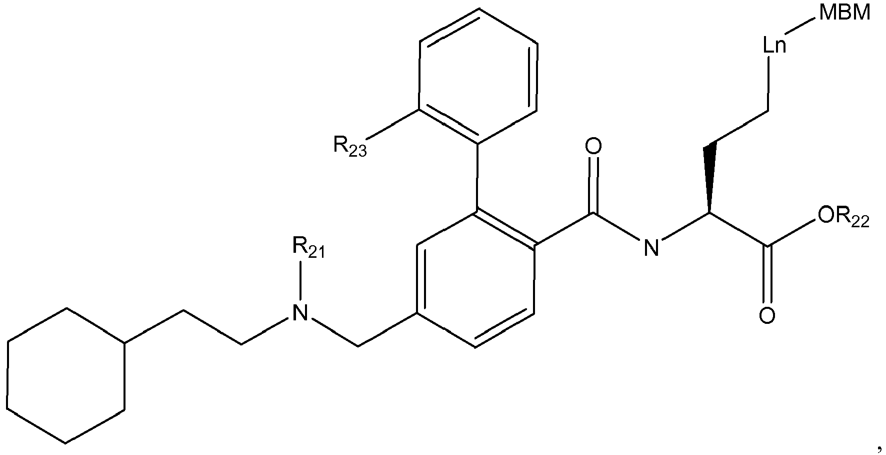 wherein:
      R₂₁ is selected from the group consisting of alkyl, substituted alkyl, cycloalkyl, substituted cycloalkyl, heterocycloalkyl, and substituted heterocycloalkyl;
      R₂₂ is selected from the group consisting of H, alkyl, and substituted alkyl; and
      R₂₃ is alkyl or substituted alkyl;
   (e) 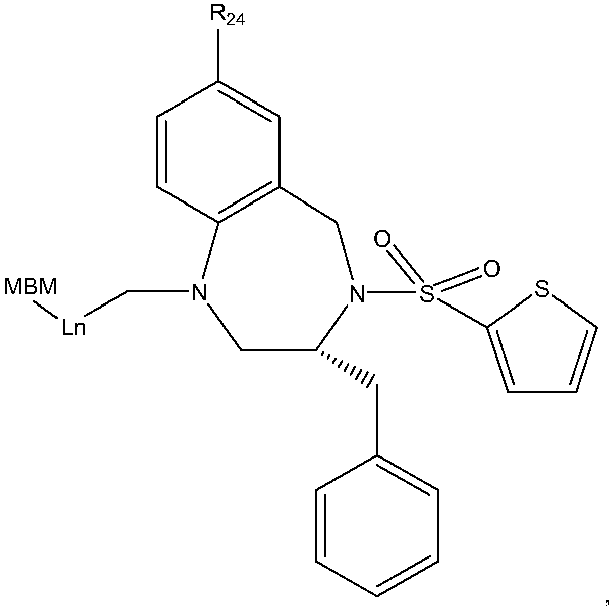 wherein R₂₄ is selected from the group consisting of H, alkyl, substituted alkyl, halogen, and cyano; and
      wherein:
      MBM is a metal binding moiety;
      Lₙ is a linker, wherein n is an integer from 0 to 1;
      under the proviso that the metal binding moiety is not a metal binding moiety selected from the group consisting of: 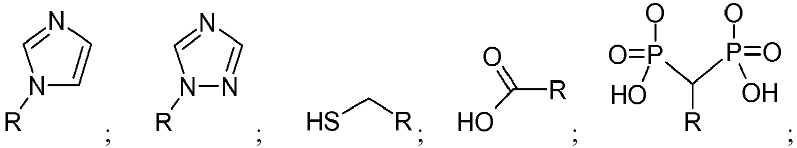 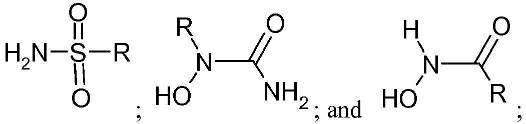 wherein:
         R is an attachment site through which the metal binding moiety can be attached to the targeting moiety, optionally through linker, Lₙ.
8. The farnesyl transferase inhibitor of para 7, wherein the inhibitor is selected from the group of farnesyl transferase inhibitors presented in Figure 19.
9. A metalloenzyme inhibitor, wherein the metalloenzyme inhibitor is a neprilysin (NEP) inhibitor comprising a targeting moiety, a metal binding moiety, and optionally a linker, and wherein the inhibitor has a formula selected from the group consisting of:
   (a) 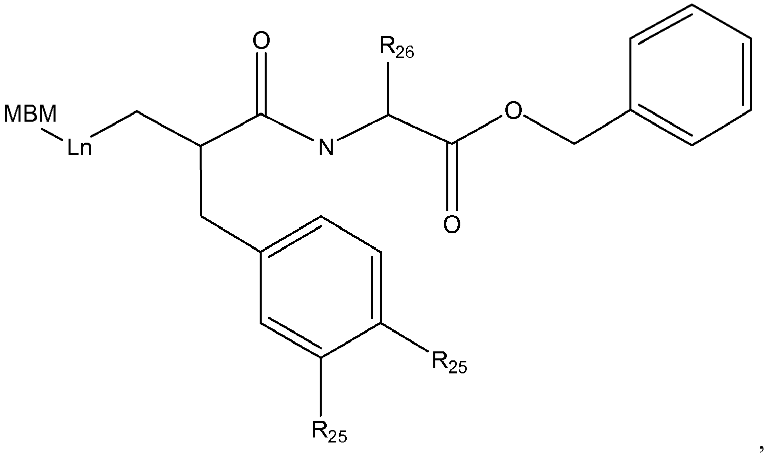 wherein:
      each R₂₅ is selected from the group consisting of alkyl, substituted alkyl, and alkoxyl; and
      R₂₆ is selected from the group consisting of H, alkyl, and substituted alkyl;
   (b) 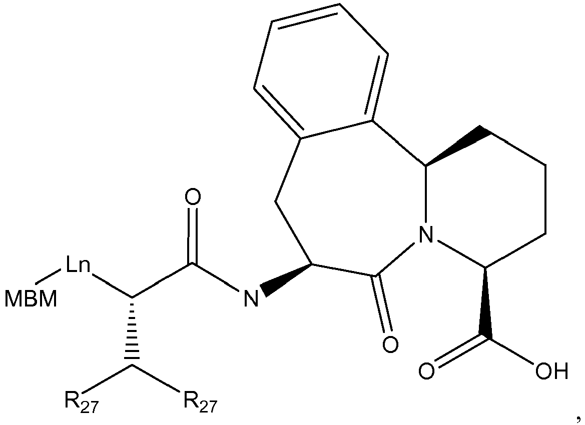 wherein each R₂₇ is independently selected from the group consisting of H, alkyl, substituted alkyl, aryl, and substituted aryl;
   (c) 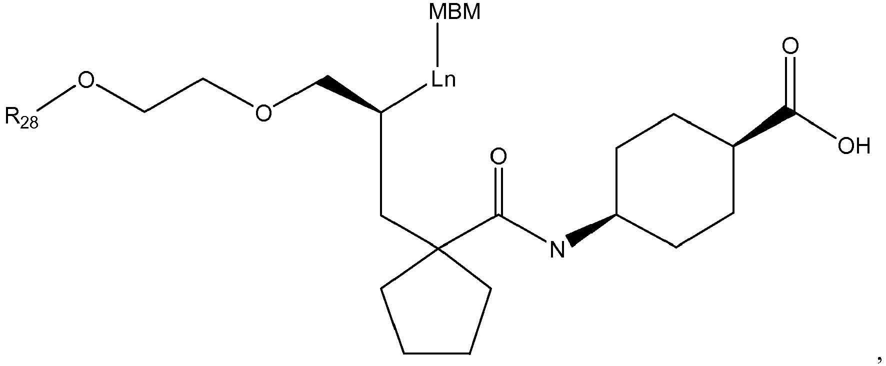 wherein R₂₈ is alkyl or substituted alkyl;
   (d) 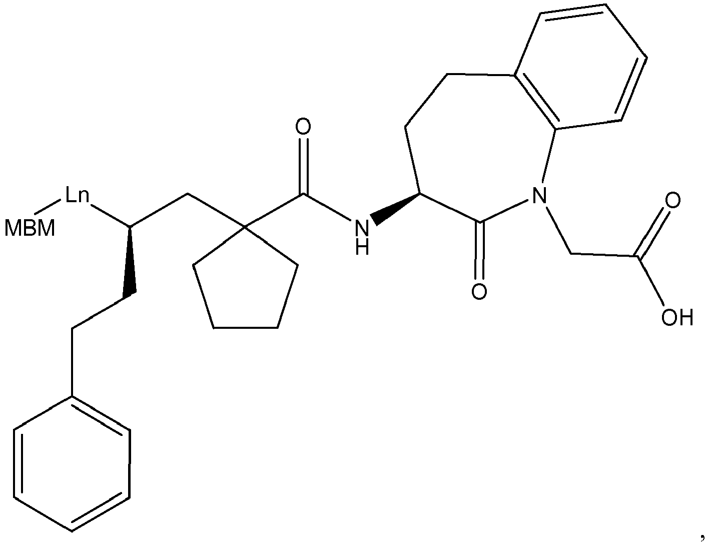
   (e) 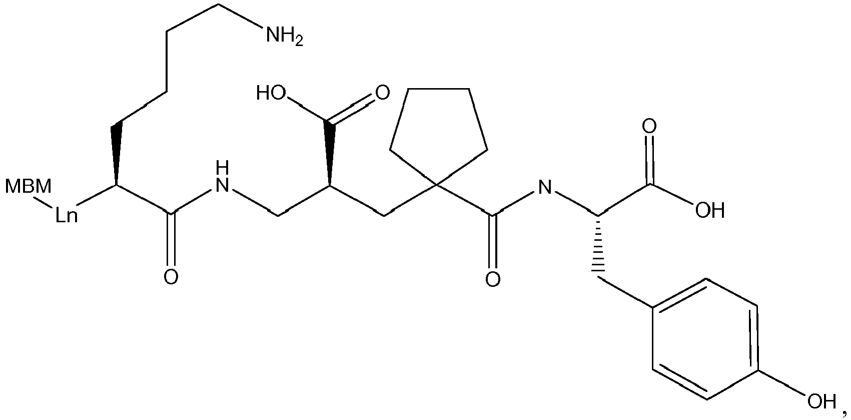
   (f) 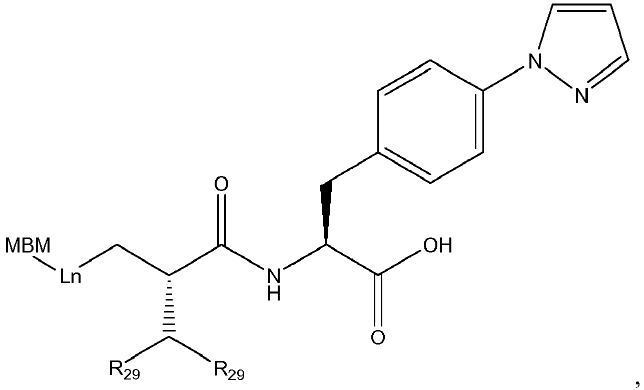 wherein each R₂₉ is selected from the group consisting of H, alkyl, substituted alkyl, aryl, and substituted aryl;
   (g) 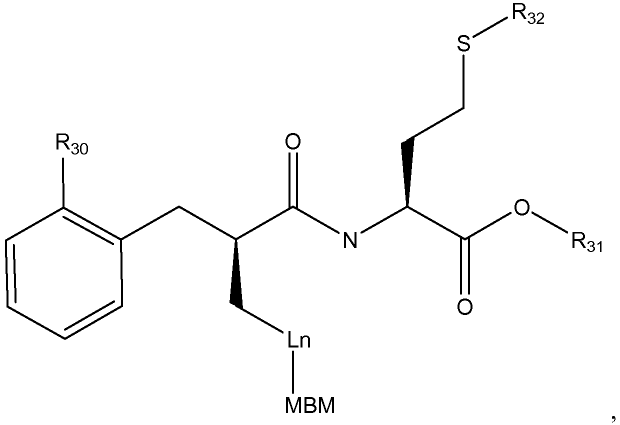 wherein:
      R₃₀ is selected from the group consisting of H, alkyl, and substituted alkyl;
      R₃₁ is selected from the group consisting of H, alkyl, substituted alkyl, and aralkyl; and
      R₃₂ is alkyl or substituted alkyl;
   (h) 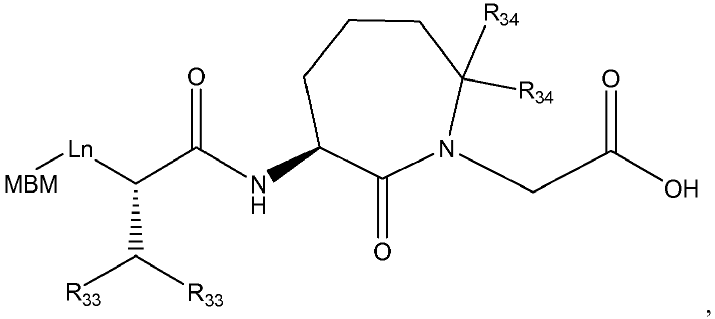 wherein:
      each R₃₃ is independently selected from the group consisting of H, alkyl, substituted alkyl, aryl, and substituted aryl; and
      each R₃₄ is independently alkyl or substituted alkyl;
   (i) 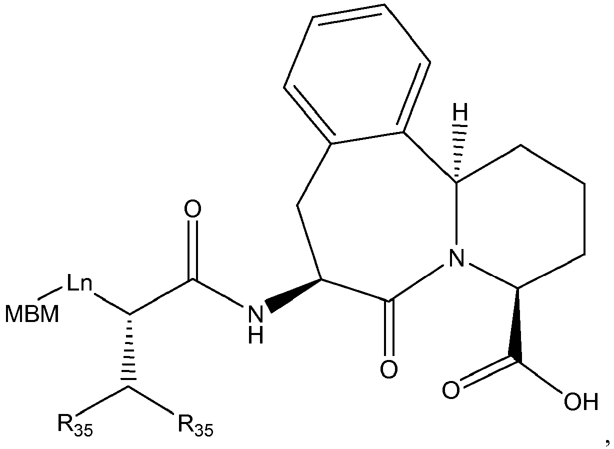 wherein each R₃₅ is independently selected from the group consisting of H, alkyl, substituted alkyl, aryl, and substituted aryl; and
   (j) 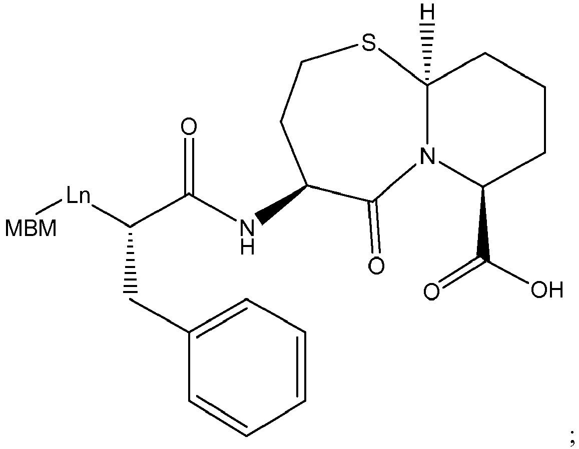 and wherein:
      MBM is a metal binding moiety;
      Lₙ is a linker, wherein n is an integer from 0 to 1;
      under the proviso that the metal binding moiety is not a metal binding moiety selected from the group consisting of: 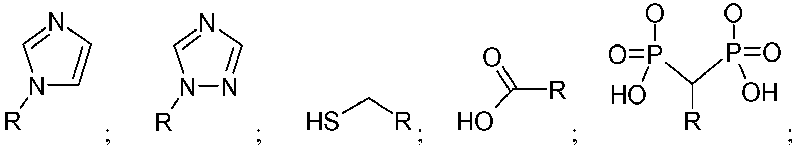 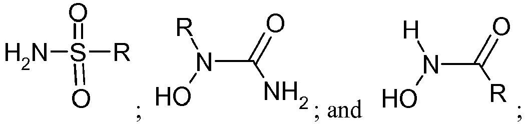 wherein:
         R is an attachment site through which the metal binding moiety can be attached to the targeting moiety, optionally through linker, Lₙ.
10. The neprilysin inhibitor of para 9, wherein the inhibitor is selected from the group of neprilysin inhibitors presented in Figure 20.
11. A metalloenzyme inhibitor, wherein the metalloenzyme inhibitor is a thromboxane synthase inhibitor comprising a targeting moiety, a metal binding moiety, and optionally a linker, and wherein the inhibitor has a formula selected from the group consisting of:
   (a) 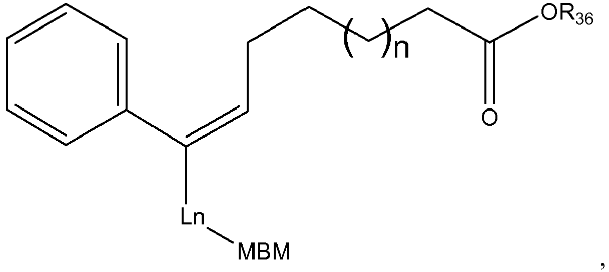 wherein:
      n is an integer from 0 to 2; and
      R₃₆ is selected from the group consisting of H, alkyl, and substituted alkyl;
   (b) 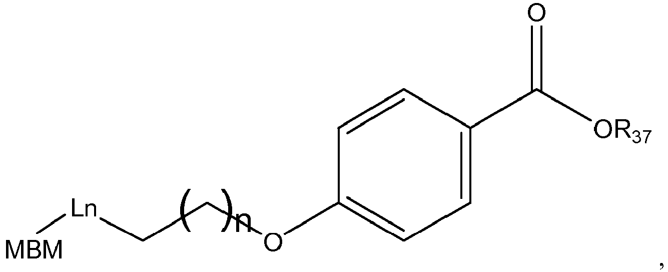 wherein:
      n is an integer from 0 to 2; and
      R₃₇ is selected from the group consisting of H, alkyl, and substiuted alkyl;
   (c) 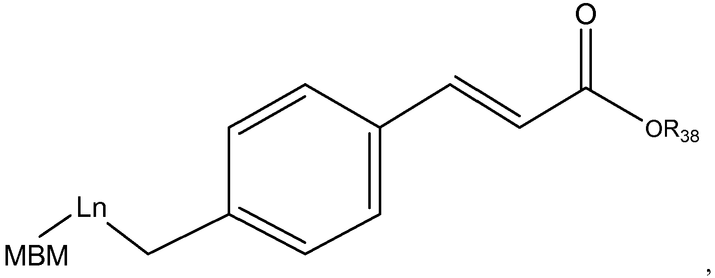 wherein R₃₈ is selected from the group consisting of H, alkyl, and substituted alkyl;
   (d) 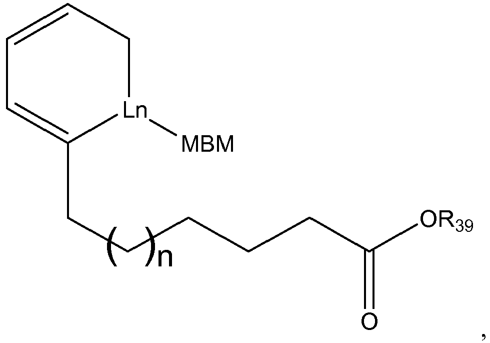 wherein:
      n is an integer from 0 to 2; and
      R₃₉ is selected from the group consisting of H, alkyl, and substituted alkyl;
   (e) 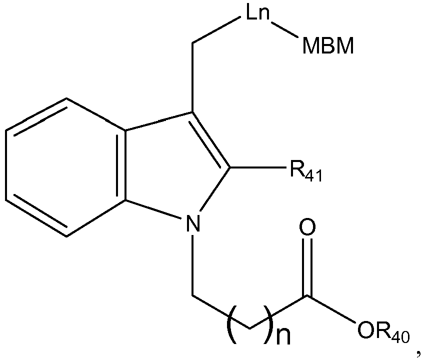 wherein:
      n is an integer from 0 to 2; and
      R₄₀ and R₄₁ are each independently selected from the group consisting of H, alkyl, and substituted alkyl;
   (f) 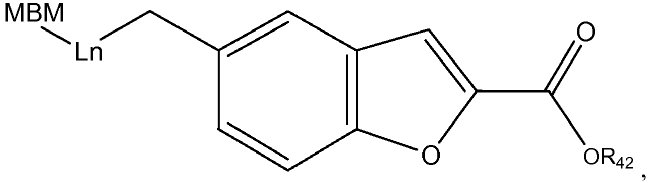 wherein R₄₂ is selected from the group consisting of H, alkyl, and substituted alkyl;
   (g) 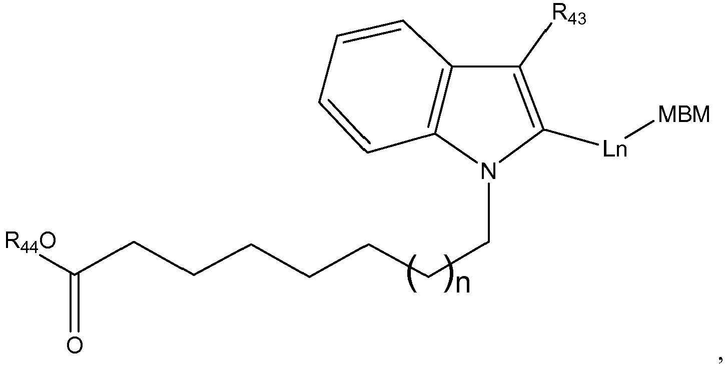 wherein:
      n is an integer from 0 to 2; and
      R₄₃ and R₄₄ are each independently selected from the group consisting of H, alkyl, and substituted alkyl;
   (h) 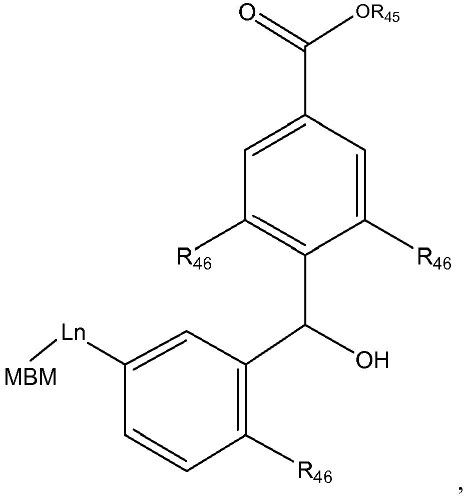 wherein R₄₅ and each R₄₆ are independently selected from the group consisting of H, alkyl, and substituted alkyl;
   (i) 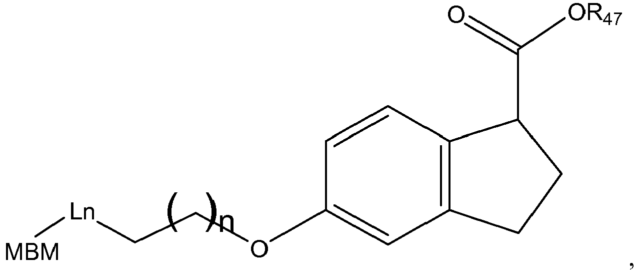 wherein:
      n is an integer from 0 to 2; and
      R₄₇ is selected from the group consisting of H, alkyl, and substituted alkyl;
   (j) 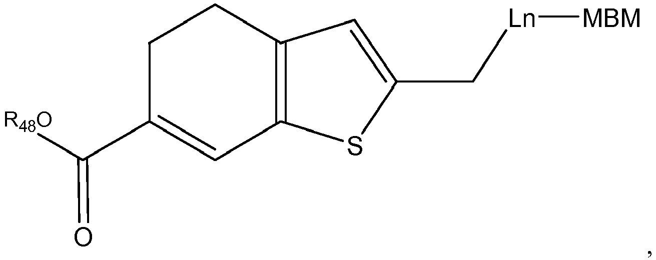 wherein R₄₈ is selected from the group consisting of H, alkyl, and substituted alkyl;
   (k) 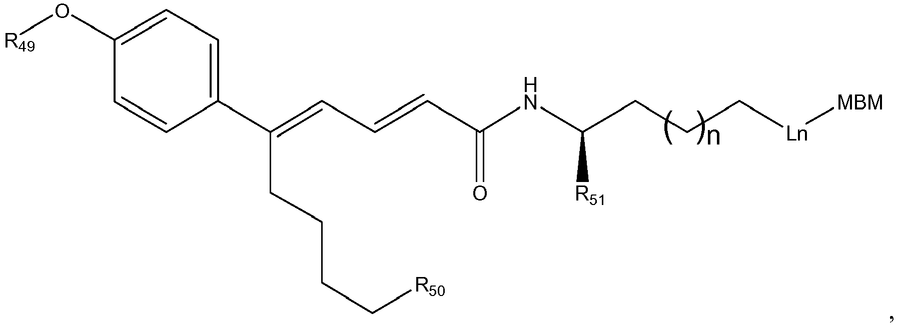 wherein:
      n is an integer from 0 to 2; and
      R₄₉ is selected from the group consisting of H, alkyl, and substituted alkyl; and
      R₅₀ and R₅₁ are each independently alkyl or substituted alkyl;
   (l) 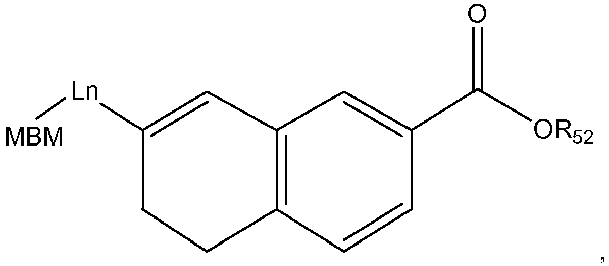 wherein R₅₂ is selected from the group consisting of H, alkyl, and substituted alkyl;
   (m) 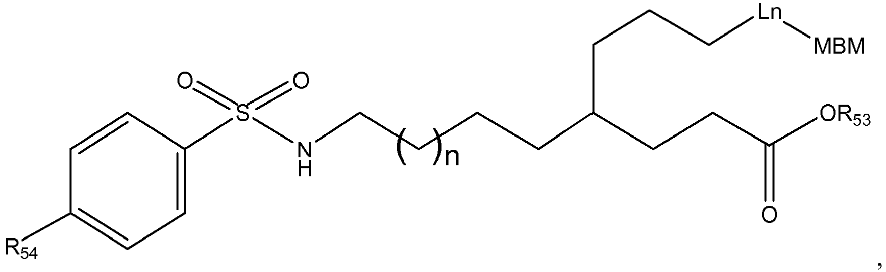 wherein:
      n is an integer from 0 to 2;
      R₅₃ is selected from the group consisting of H, alkyl, and substituted alkyl; and
      R₅₄ is H or halogen;
   (n) 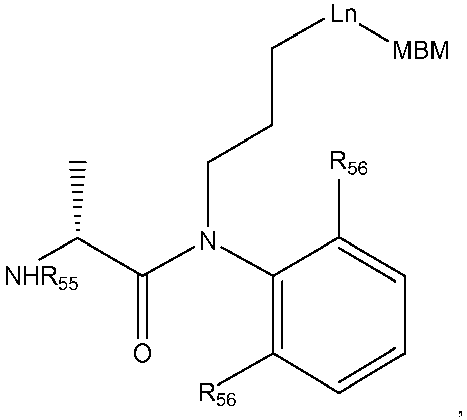 wherein:
      R₅₅ and each R₅₆ is independently selected from the group consisting of H, alkyl, and substituted alkyl;
   (o) 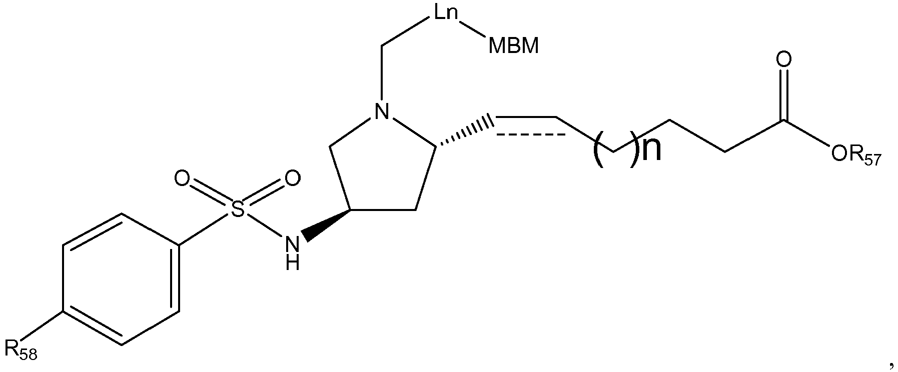 wherein:
      n is an integer from 0 to 2;
      R₅₇ is selected from the group consisting of H, alkyl, and substituted alkyl; and
      R₅₈ is H or halogen;
   (p) 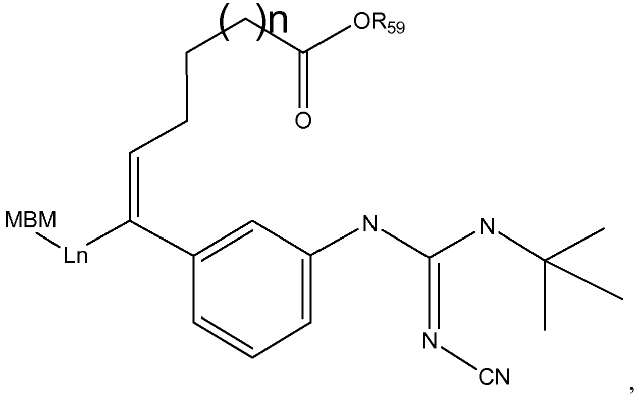 wherein:
      n is an integer from 0 to 2; and
      R₅₉ is selected from the group consisting of H, alkyl, and substituted alkyl;
   (q) 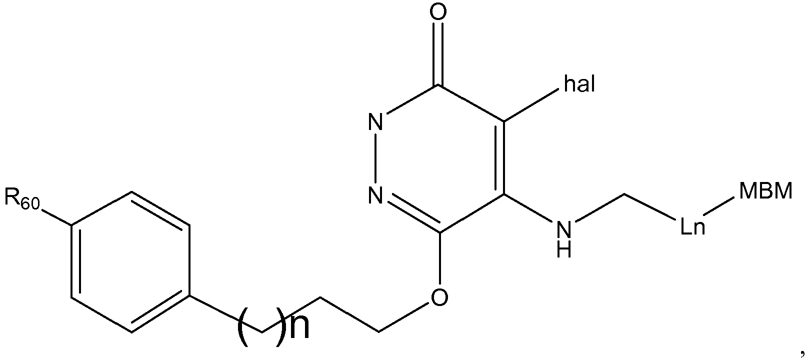 wherein:
      n is an integer from 0 to 2;
      hal is halogen; and
      R₆₀ is H or halogen; and
      wherein:
      MBM is a metal binding moiety;
      Lₙ is a linker, wherein n is an integer from 0 to 1;
      under the proviso that the metal binding moiety is not a metal binding moiety selected from the group consisting of: 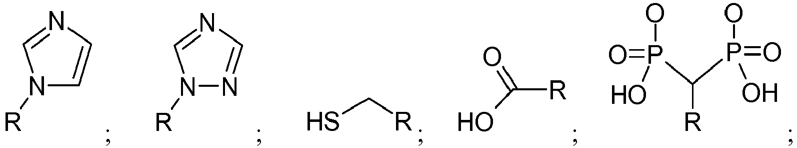 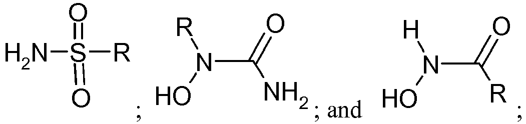 wherein:
         R is an attachment site through which the metal binding moiety can be attached to the targeting moiety, optionally through linker, Lₙ.
12. The thromboxane synthase inhibitor of para 11, wherein the inhibitor is selected from the group of thromboxane synthase inhibitors presented in Figure 21.
13. A metalloenzyme inhibitor, wherein the metalloenzyme inhibitor is an anthrax lethal factor inhibitor comprising a targeting moiety, a metal binding moiety, and optionally a linker, and wherein the inhibitor has a formula selected from the group consisting of:
   (a) 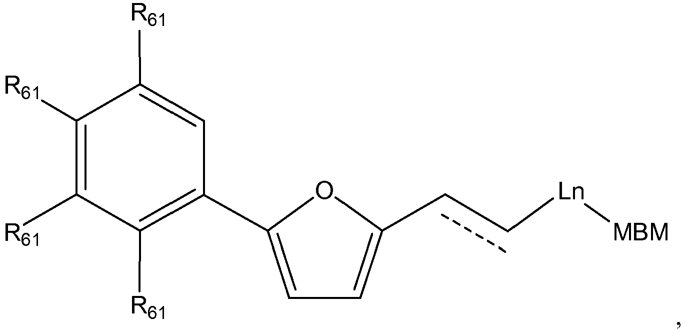 wherein each R₆₁ is independently selected from the group consisting of H, halogen, CF₃, alkoxy, cyano, and carboxylate;
   (b) 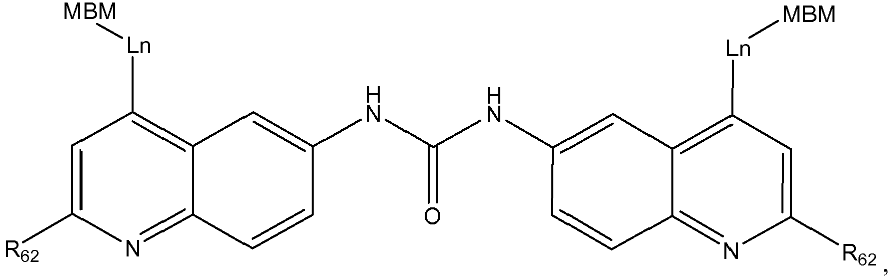 wherein each R₆₂ is independently selected from the group consisting of H, alkyl, and substituted alkyl;
   (c) 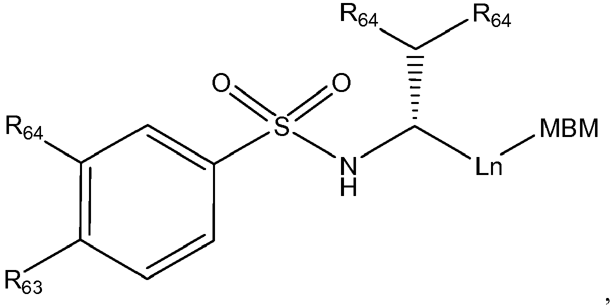 wherein:
      R₆₃ is H or halogen; and
      each R₆₄ is independently selected from the group consisting of H, alkyl, substituted alkyl, and alkoxyl;
   (d) 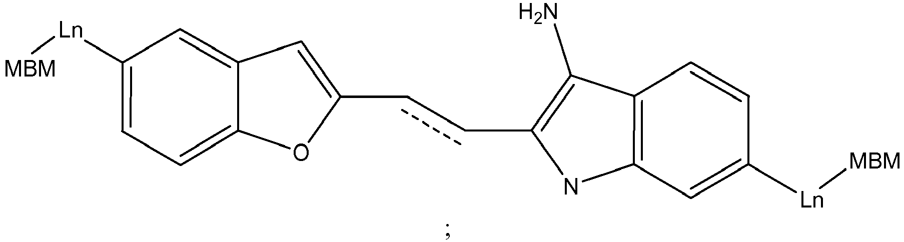
   (e) 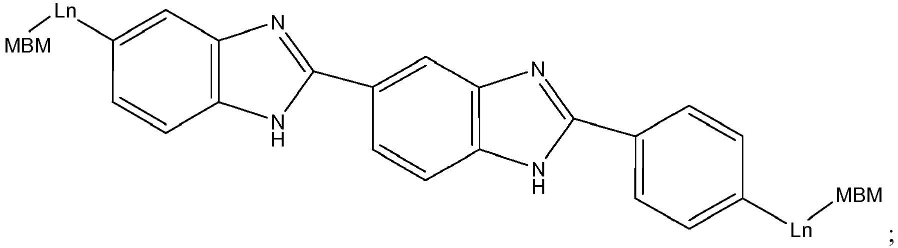
   (f) 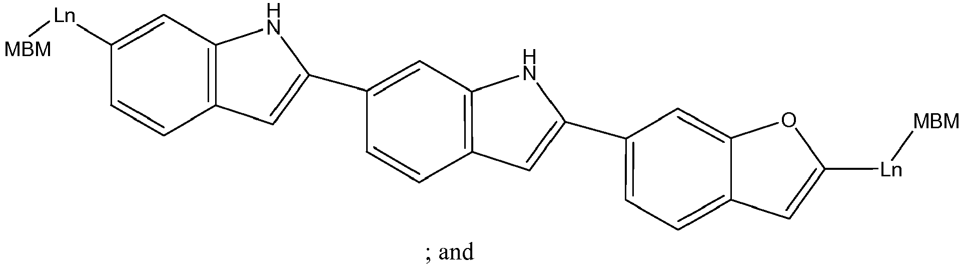 and
      wherein:
      MBM is a metal binding moiety;
      Lₙ is a linker, wherein n is an integer from 0 to 1;
      under the proviso that the metal binding moiety is not a metal binding moiety selected from the group consisting of: 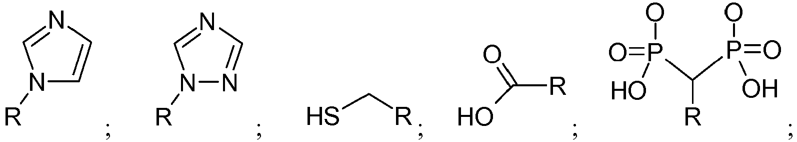 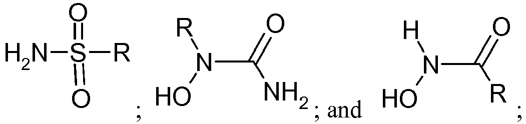 wherein:
         R is an attachment site through which the metal binding moiety can be attached to the targeting moiety, optionally through linker, Lₙ.
14. The anthrax lethal factor inhibitor of para 13, wherein the inhibitor is selected from the group of anthrax lethal factor inhibitors presented in Figure 22.
15. A metalloenzyme inhibitor, wherein the metalloenzyme inhibitor is an endothelin converting enzyme inhibitor comprising a targeting moiety, a metal binding moiety, and optionally a linker, and wherein the inhibitor has a formula selected from the group consisting of:
   (a) 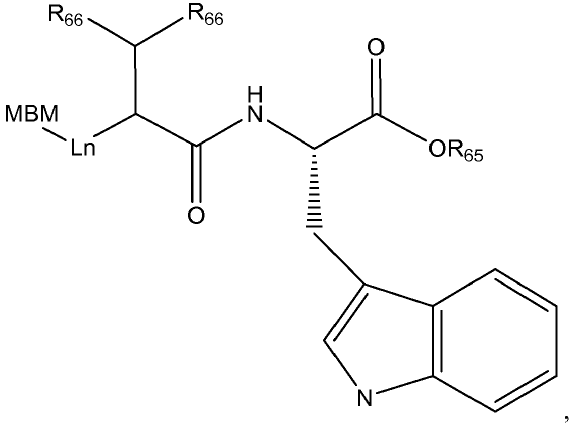 wherein:
      R₆₅ is selected from the group consisting of H, alkyl, and substituted alkyl; and
      each R₆₆ is independently alkyl or substituted alkyl;
   (b) 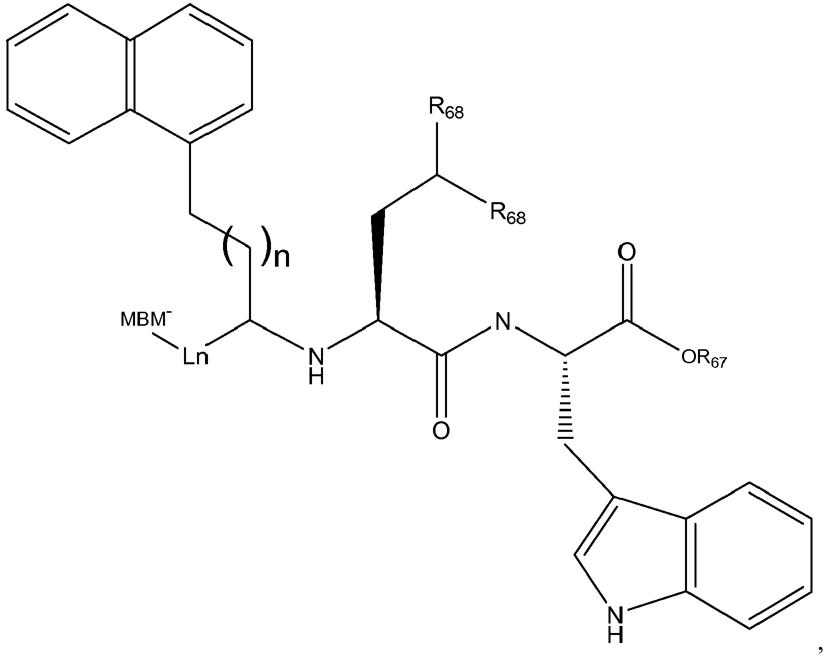
      n is an integer from 0 to 2; and
      R₆₇ is selected from the group consisting of H, alkyl, and substituted alkyl; and
      each R₆₈ is independently alkyl or substituted alkyl;
   (c) 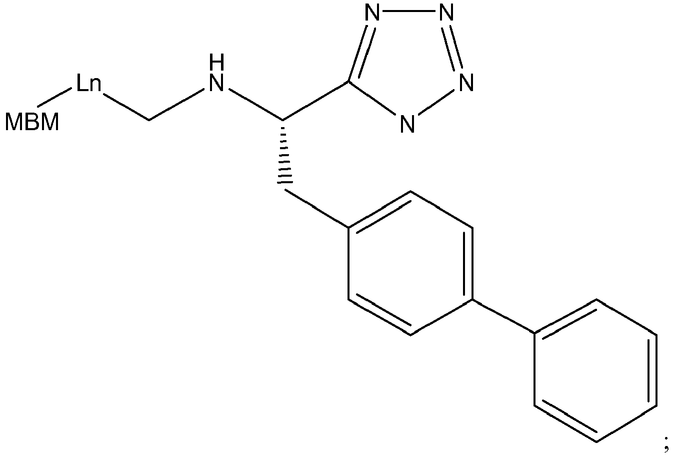
   (d) 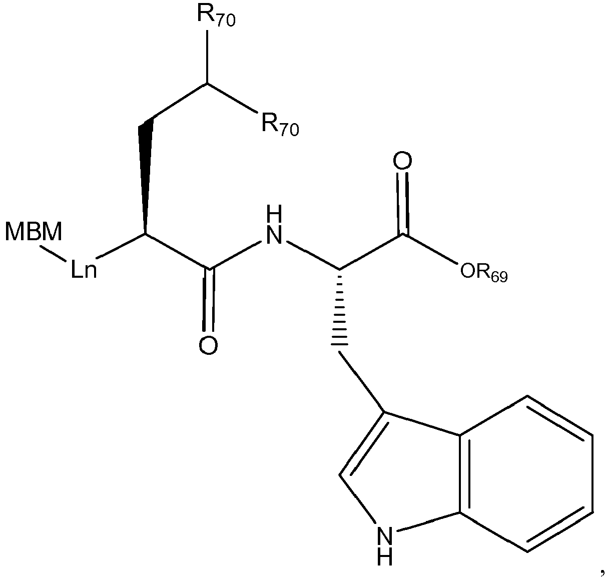 wherein:
      R₆₉ is selected from the group consisting of H, alkyl, and substituted alkyl; and
      each R₇₀ is independently alkyl or substituted alkyl;
   (e) 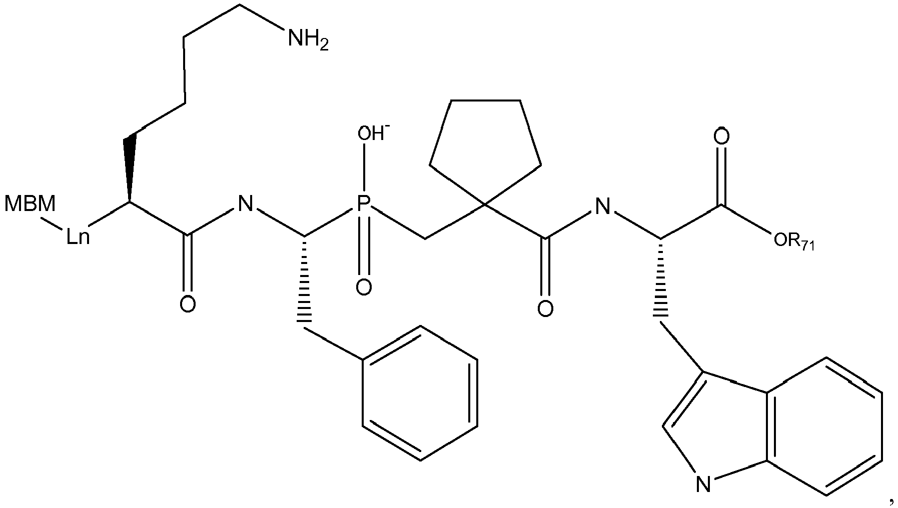 wherein R₇₁ is selected from the group consisting ofH, alkyl, and substituted alkyl;
   (f) 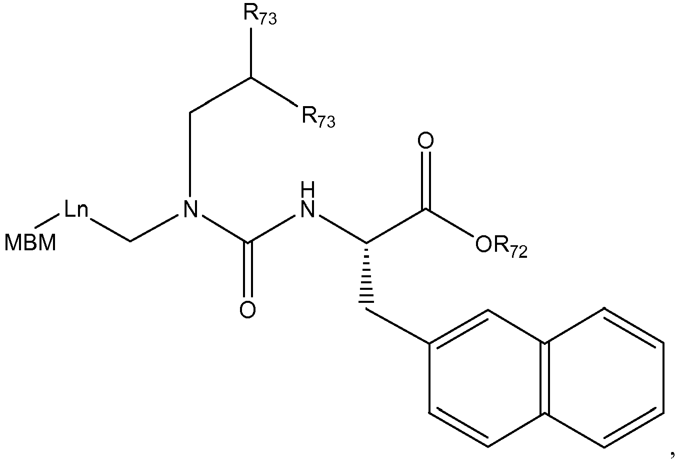 wherein:
      R₇₂ is selected from the group consisting of H, alkyl, and substituted alkyl; and
      each R₇₃ is independently alkyl or substituted alkyl;
   (g) 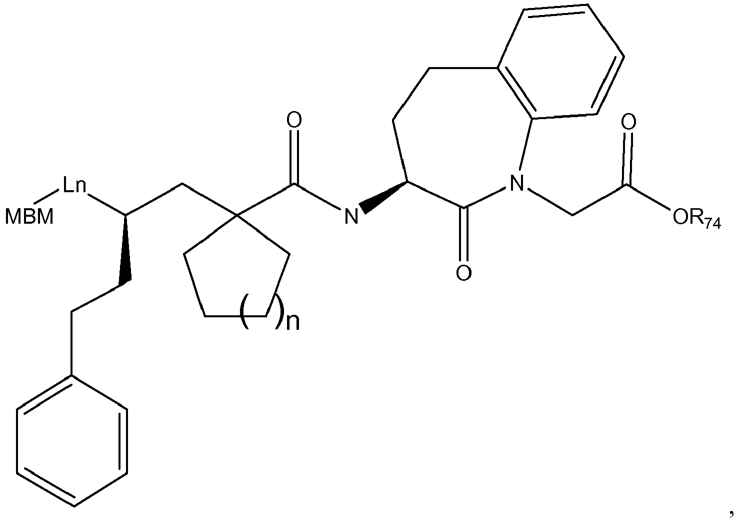
      n is an integer from 1 to 2; and
      R₇₄ is selected from the group consisting of H, alkyl, and substituted alkyl;
   (h) 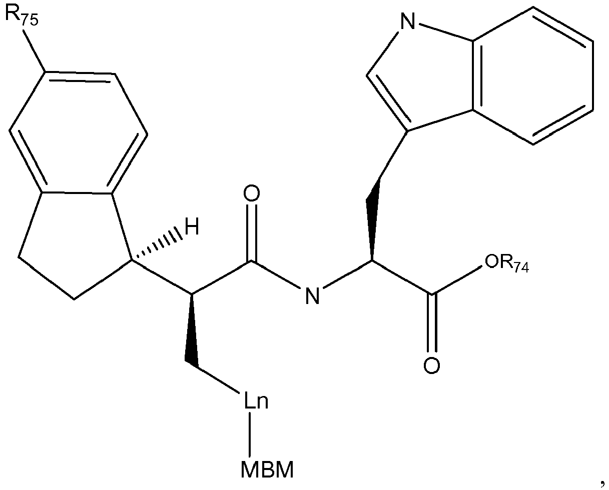 wherein:
      R₇₄ is selected from the group consisting of H, alkyl, and substituted alkyl;
      R₇₅ is H or halogen;
   (i) 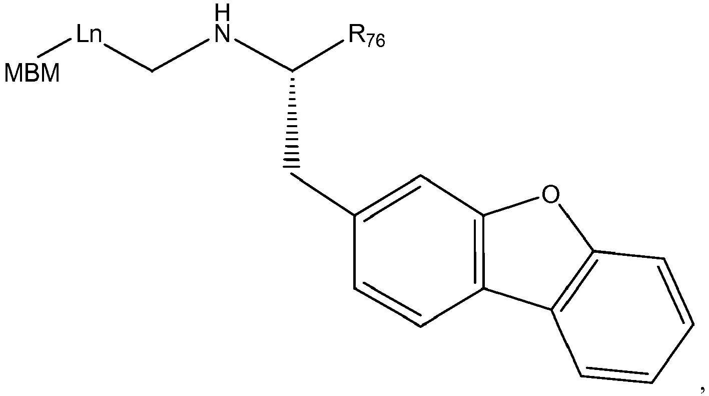 wherein R₇₆ is 5-tetrazolyl or carboxylate;
   (j) 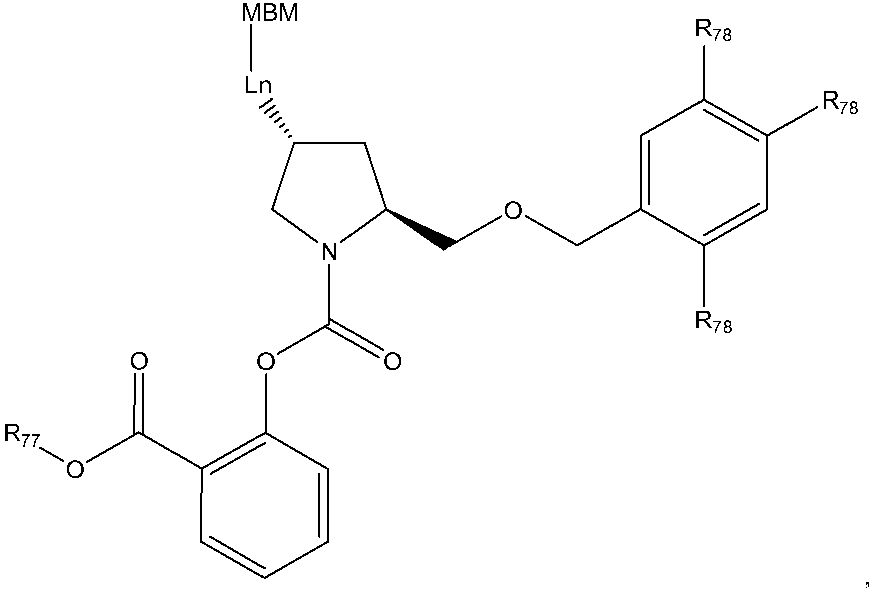 wherein:
      R₇₇ is selected from the group consisting of H, alkyl, and substituted alkyl; and
      each R₇₈ is independently H or halogen;
   (k) 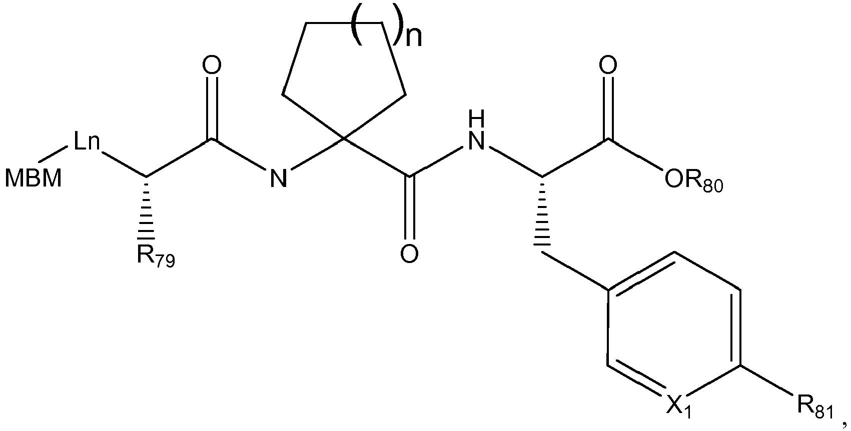 wherein:
      n is an integer from 1 to 2;
      X₁ is N or CH;
      R₇₉ is alkyl or substituted alkyl;
      R₈₀ is selected from the group consisting of H, alkyl, and substituted alkyl;
      R₈₁ is selected from the group consisting of aryl, substituted aryl, heteroaryl, and substituted heteroaryl; and
      wherein:
      MBM is a metal binding moiety;
      Lₙ is a linker, wherein n is an integer from 0 to 1;
      under the proviso that the metal binding moiety is not a metal binding moiety selected from the group consisting of: 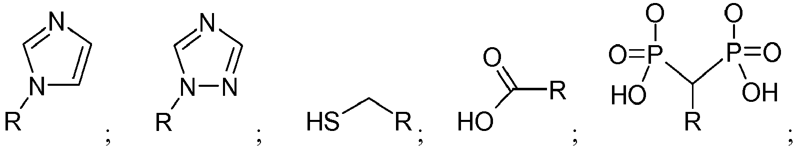 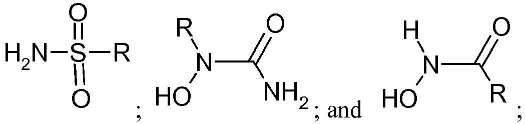 wherein:
         R is an attachment site through which the metal binding moiety can be attached to the targeting moiety, optionally through linker, Lₙ.
16. The endothelin converting enzyme inhibitor of para 15, wherein the inhibitor is selected from the group of endothelin converting enzyme inhibitors presented in Figure 23.
17. A metalloenzyme inhibitor, wherein the metalloenzyme inhibitor is a methionine aminopeptidase-2 inhibitor comprising a targeting moiety, a metal binding moiety, and optionally a linker, and wherein the inhibitor has a formula selected from the group consisting of:
   (a) 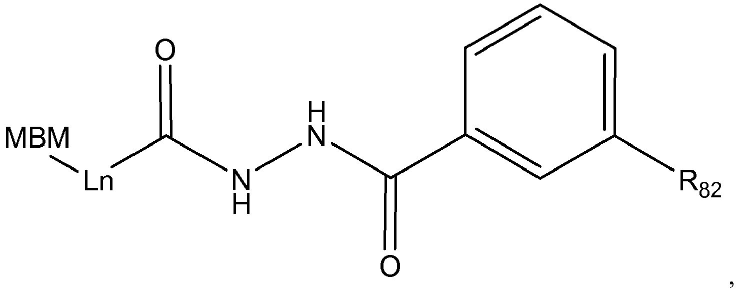 wherein R₈₂ is H or halogen;
   (b) 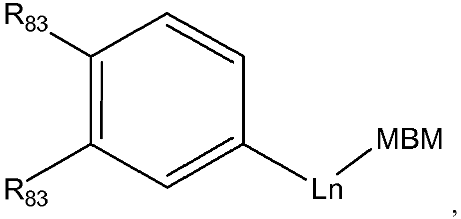 wherein each R₈₃ is independently H or halogen;
   (c) 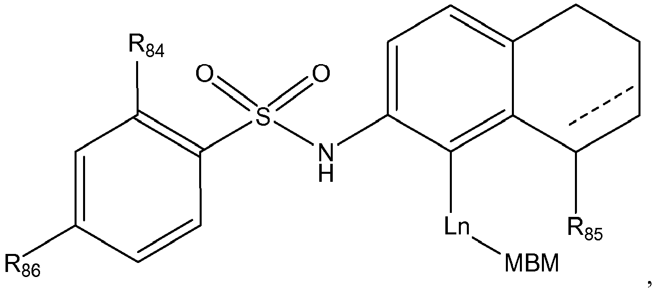 wherein:
      R₈₄ is selected from the group consisting of H, alkyl, substituted alkyl, alkenyl, and aminoalkyl;
      R₈₅ is selected from the group consiting of H, alkyl, and substituted alkyl; and
      R₈₆ is H or halogen;
   (d) 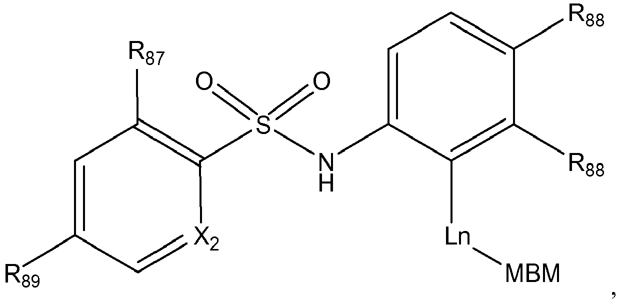 wherein:
      R₈₇ is selected from the group consisting of H, alkyl, substituted alkyl, alkenyl, and aminoalkyl;
      each R₈₈ is independently selected from the group consisting of H, alkyl, substituted alkyl, and alkoxyl;
      R₈₉ is H or halogen; and
      X₂ CH or N;
      wherein:
      MBM is a metal binding moiety;
      Lₙ is a linker, wherein n is an integer from 0 to 1;
      under the proviso that the metal binding moiety is not a metal binding moiety selected from the group consisting of: 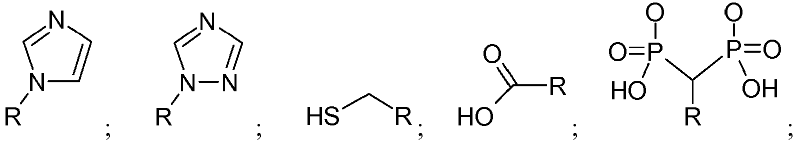 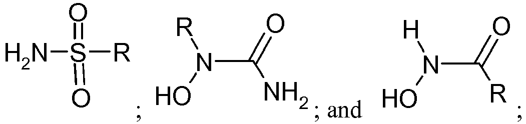 wherein:
         R is an attachment site through which the metal binding moiety can be attached to the targeting moiety, optionally through linker, Lₙ.
18. The methionine aminopeptidase-2 inhibitor of para 17, wherein the inhibitor is selected from the group of methionine aminopeptidase-2 inhibitors presented in Figure 24.
19. A metalloenzyme inhibitor, wherein the metalloenzyme inhibitor is a peptide deformylase inhibitor comprising a targeting moiety, a metal binding moiety, and optionally a linker, and wherein the inhibitor has a formula selected from the group consisting of:
   (a) 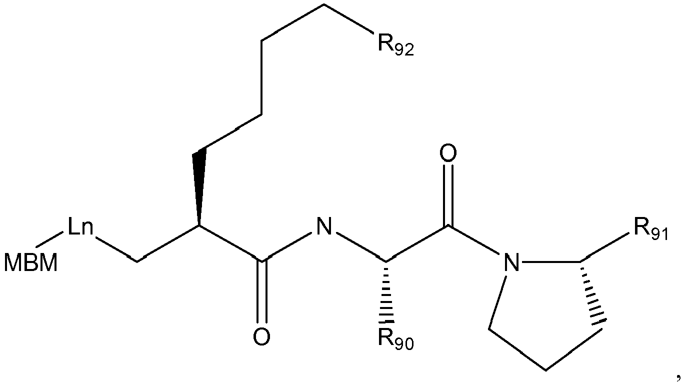
      R₉₀ and R₉₂ are each independently alkyl or substituted alkyl;
      R₉₁ is hydroxymethyl or carboxyl,
   (b) 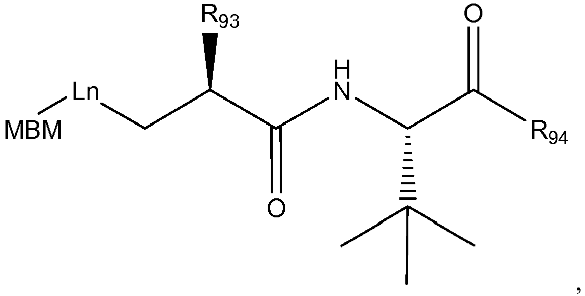 wherein:
      R₉₃ is selected from the group consisting of alkyl, substituted alkyl, cycloalkyl, substituted cycloalkyl, heterocycloalkyl, and substituted heterocycloalkyl; and
      R₉₄ is selected from the group consisting of amino-aryl, amino-heteroaryl, amino-alkyl, cycloamino, and alkoxyl;
   (c) 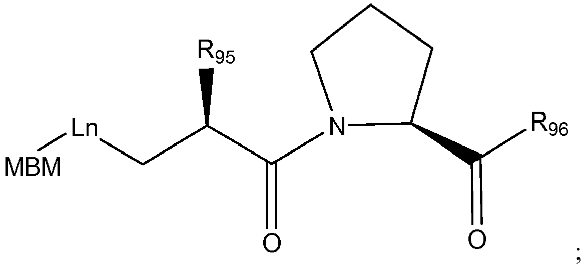 wherein:
      R₉₅ is selected from the group consisting of alkyl, substituted alkyl, cycloalkyl, substituted cycloalkyl, heterocycloalkyl, and substituted heterocycloalkyl; and
      R₉₆ is selected from the group consisting of amino-aryl, amino-heteroaryl, amino-alkyl, and alkoxyl;
   (d) 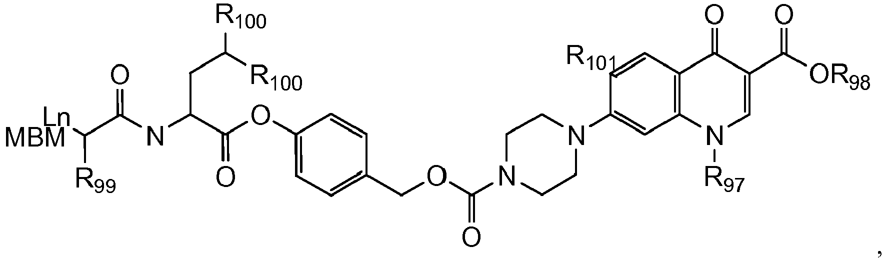 wherein:
      R₉₇ and R₉₈ are each independently selected from the group consisting of H, alkyl, and substituted alkyl;
      R₉₉ is selected from the group consisting of alkyl, substituted alkyl, and thio-alkyl;
      R₁₀₀ is alkyl or substituted alkyl; and
      R₁₀₁ is H or halogen;
   (e) 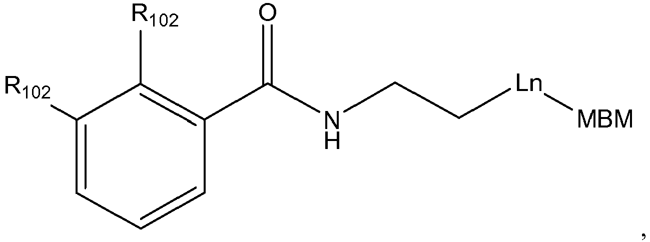 wherein each R₁₀₂ is independently H or halogen;
   (f) 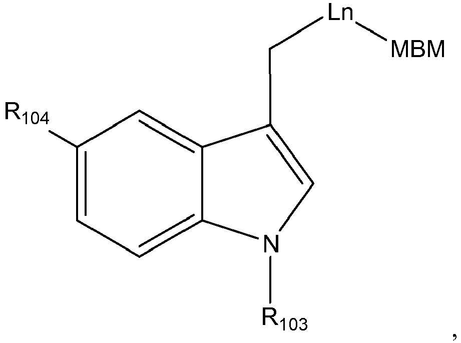
      wherein R₁₀₃ is selected from the group consisting of H, alkyl, and substituted alkyl; and
      R₁₀₄ is H or halogen;
      wherein:
      MBM is a metal binding moiety;
      Lₙ is a linker, wherein n is an integer from 0 to 1;
      under the proviso that the metal binding moiety is not a metal binding moiety selected from the group consisting of: 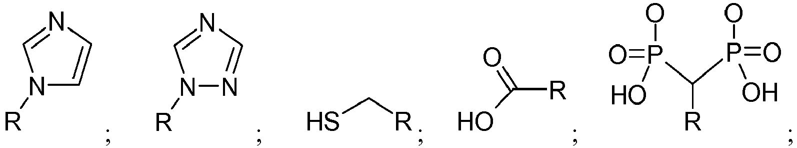 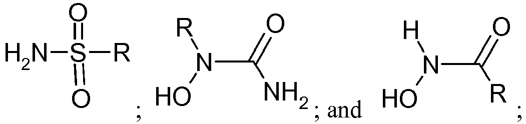 wherein:
         R is an attachment site through which the metal binding moiety can be attached to the targeting moiety, optionally through linker, Lₙ.
20. The peptide deformylase inhibitor of para 19, wherein the inhibitor is selected from the group of peptide deformylase inhibitors presented in Figure 25.
21. A metalloenzyme inhibitor, wherein the metalloenzyme inhibitor is an HIV integrase inhibitor comprising a targeting moiety, a metal binding moiety, and optionally a linker, and wherein the inhibitor has a formula selected from the group consisting of:
   (a) 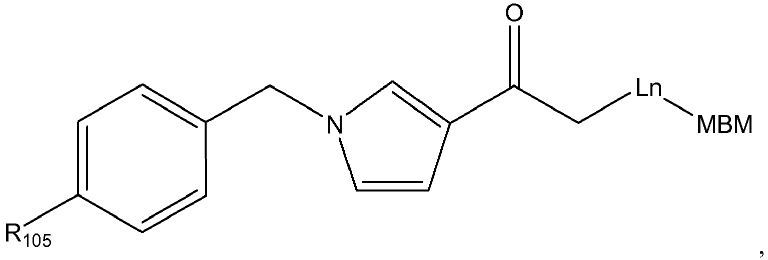 wherein R₁₀₅ is H or halogen;
   (b) 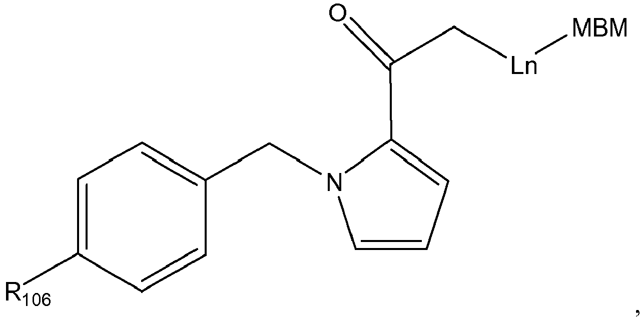 wherein R₁₀₆ is H or halogen;
   (c) 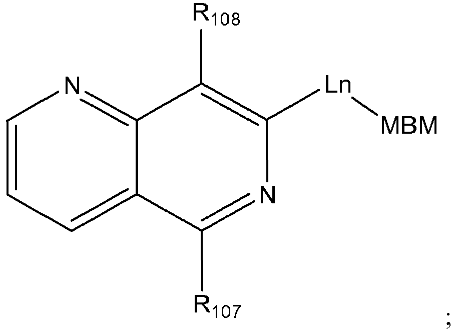 wherein:
      R₁₀₇ is selected from the group consisting of H, alkenyl, amino, cycloamino, aryl, substituted aryl, carboxamido;
      R₁₀₈ is selected from the group consisting of H, hydroxyl, and cycloamino ;
   (d) 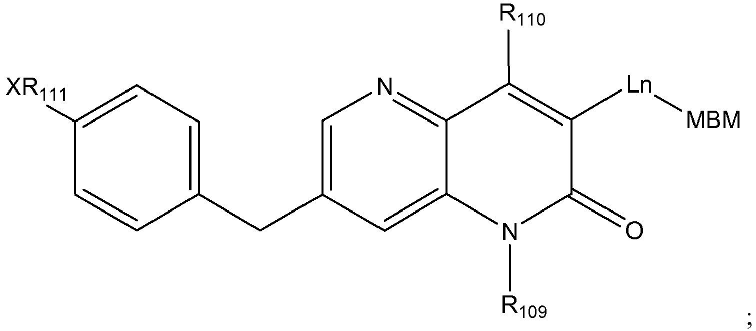 wherein:
      R₁₀₉ is selected from the group consisting of H, alkyl, alkyl-phosphonate, aryl, substituted carboxamido;
      R₁₁₀ is selected from the group consisting of H, hydroxyl, and cycloamino; and
      R₁₁₁ is H or halogen;
   (e) 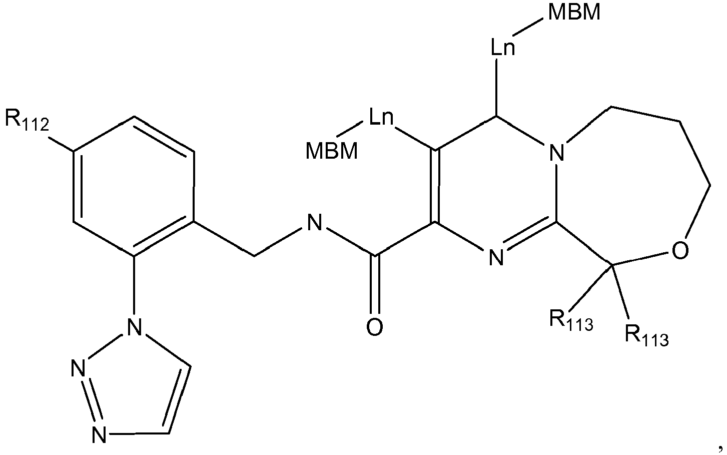 wherein:
      R₁₁₂ is H or halogen; and
      each R₁₁₃ is independently alkyl or substituted alkyl;
   (f) 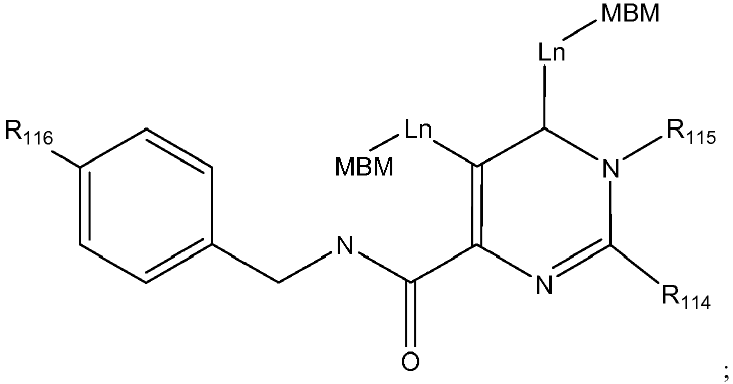 wherein:
      R₁₁₄ is selected from the group consisting of H, alkyl, substituted cycloamino, alkylamino, aryl, substituted aryl, and 2-morpholino;
      R₁₁₅ is selected from the group consisting of H, alkyl, and substituted alkyl; and
      R₁₁₆ is H or halogen;
   (g) 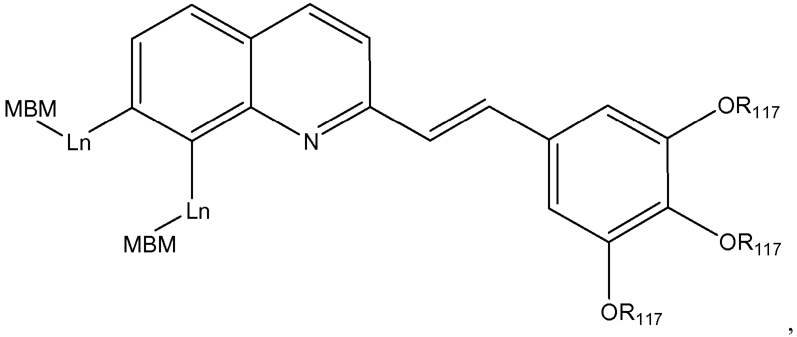 wherein each R₁₁₇ is independently selected from the group consisting of H, alkyl, and substituted alkyl;
   (h) 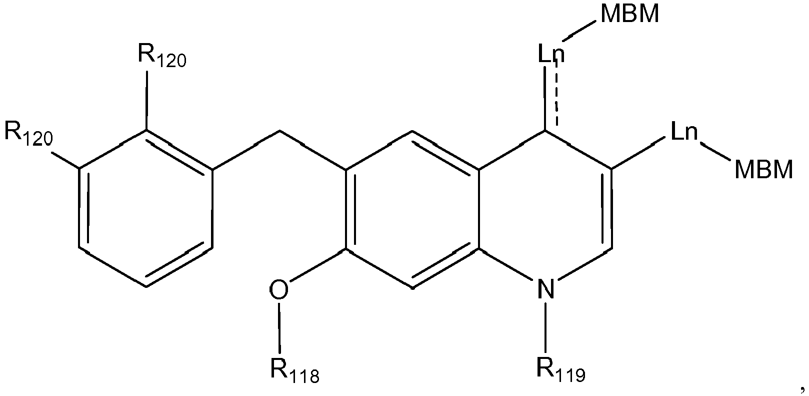
      R₁₁₈ is selected from the group consisting of H, alkyl, and substituted alkyl;
      R₁₁₉ is selected from the group consisting of H, alkyl, alkoxyl, aryl, substituted aryl, and carboxamido; and
      each R₁₂₀ is independently H or halogen;
   (i) 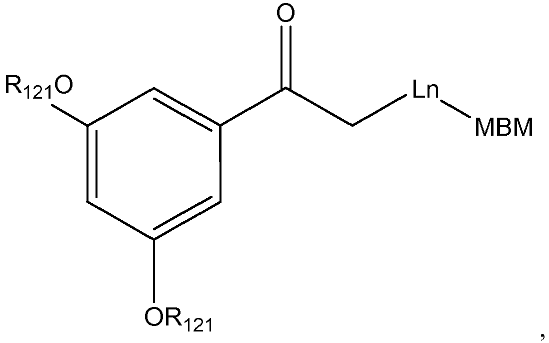 wherein each R₁₂₁ is independently selected from the group consisting of H, alkyl, substituted, and aralkyl;
   (j) 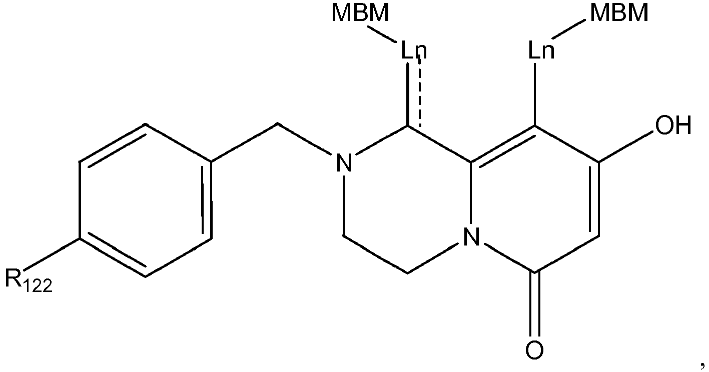 wherein R₁₂₂ is H or halogen;
   (k) 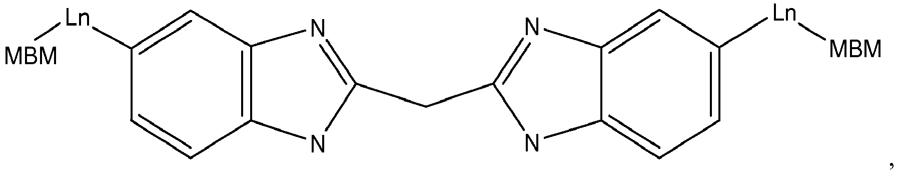
   (l) 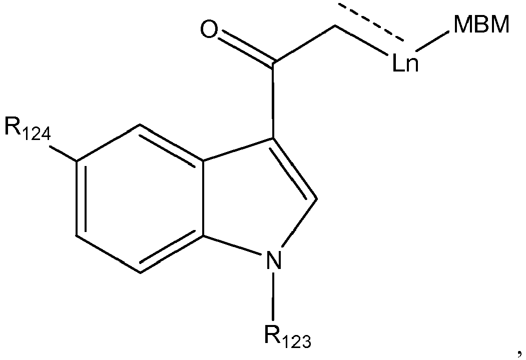
      R₁₂₃ is selected from the group consisting of H, alkyl, substituted alkyl, aryl, substituted aryl, and aryl-sulfonyl;
      R₁₂₄ is H or halogen;
   (m) 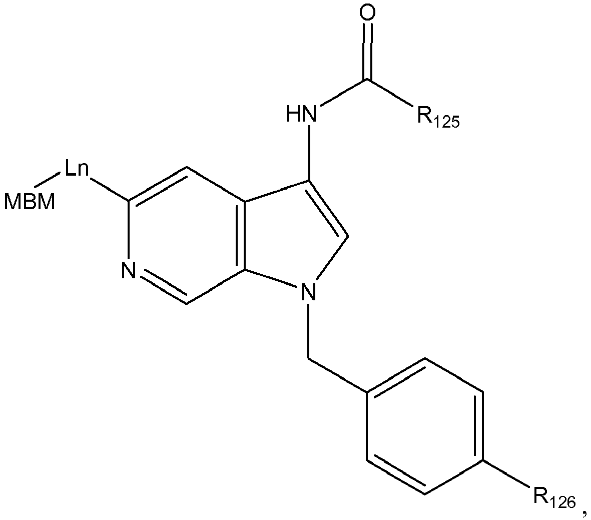 wherein:
      R₁₂₅ is alkyl or substituted alkyl; and
      R₁₂₆ is H or halogen;
   (n) 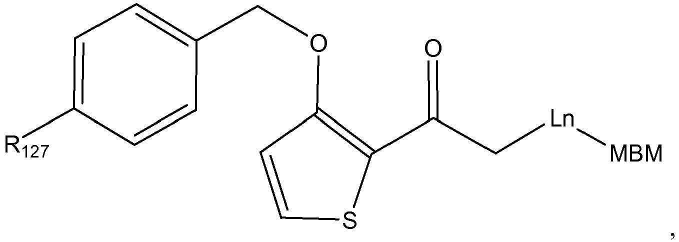 wherein R₁₂₇ is H or halogen;
   (o) 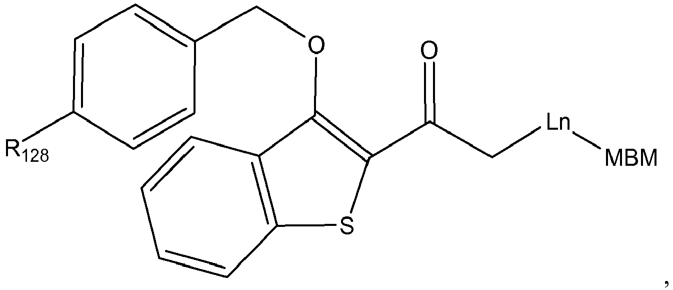 R₁₂₈ is H or halogen; and
      wherein:
      MBM is a metal binding moiety;
      Lₙ is a linker, wherein n is an integer from 0 to 1;
      under the proviso that the metal binding moiety is not a metal binding moiety selected from the group consisting of: 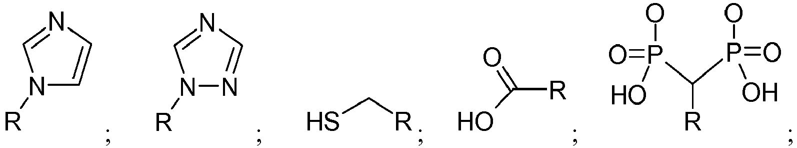 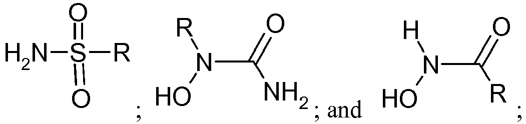 wherein:
         R is an attachment site through which the metal binding moiety can be attached to the targeting moiety, optionally through linker, Lₙ.
22. The HIV integrase inhibitor of para 21, wherein the inhibitor is selected from the group of HIV integrase inhibitors presented in Figure 26.
23. A metalloenzyme inhibitor, wherein the metalloenzyme inhibitor is a TNF-alpha converting enzyme (TACE) inhibitor comprising a targeting moiety, a metal binding moiety, and optionally a linker, and wherein the inhibitor has a formula selected from the group consisting of:
   (a) 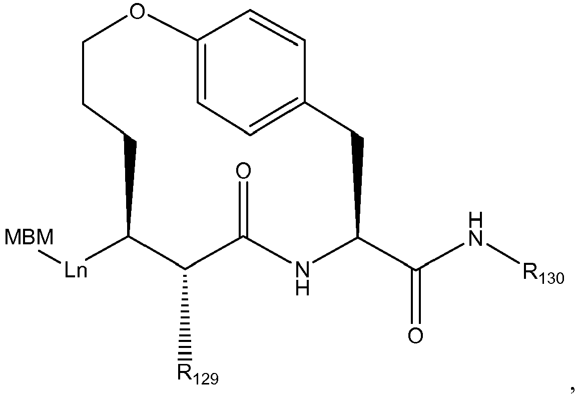 wherein:
      R₁₂₉ is alkyl or substituted alkyl; and
      R₁₃₀ is selected from the group consisting of H, alkyl, and substituted alkyl;
   (b) 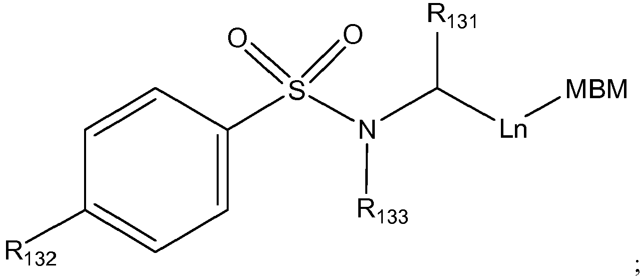 wherein:
      R₁₃₁ is selected from the group consisting of alkyl, substituted alkyl, cycloalkyl, substituted cycloalkyl, heterocycloalkyl, substituted heterocycloalkyl, and alkoxy;
      R₁₃₂ = H, alkoxy; and
      R₁₃₃ = H, aralkyl;
   (c) 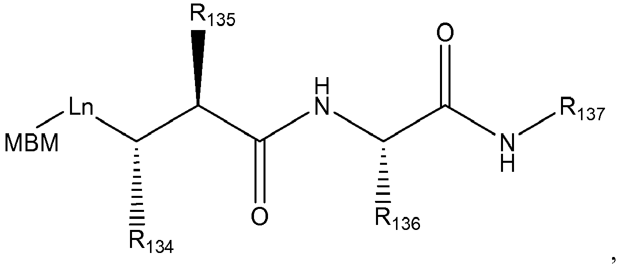 wherein:
      R₁₃₄, R₁₃₅, and R₁₃₆ are each alkyl or substituted alkyl;
      R₁₃₇ is selected from the group consisting of aryl, substituted aryl, heteroaryl, and substituted heteroaryl;
   (d) 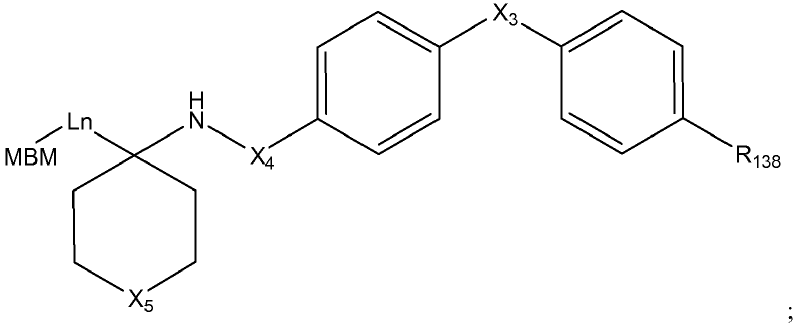 wherein:
      R₁₃₈ is H or halogen;
      X₃ is O or S;
      X₄ is C(=O) or S(=O)₂; and
      X₅ is O or N-alkyl;
   (e) 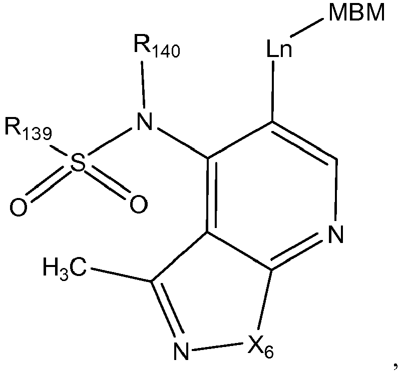 wherein:
      R₁₃₉ is selected from the group consisting of aryl, substituted aryl, heteroaryl, and substituted heteroaryl;
      R₁₄₀ is selected from the group consisting of H, alkyl, and substituted alkyl;
      X₆ is selected from the group consisting of O, S, and N-R₁₄₁, wherein R₁₄₁ is alkyl or substituted alkyl;
   (f) 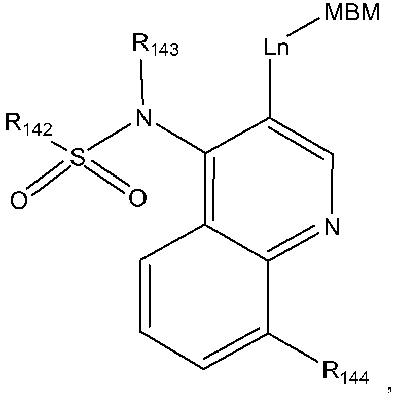 wherein:
      R₁₄₂ is selected from the group consisting of aryl, substituted aryl, heteroaryl, and substituted heteroaryl;
      R₁₄₃ is selected from the group consisting ofH, alkyl, and substituted alkyl; and
      R₁₄₄ is selected from the group consisting of H, halogen, and alkoxyl;
   (g) 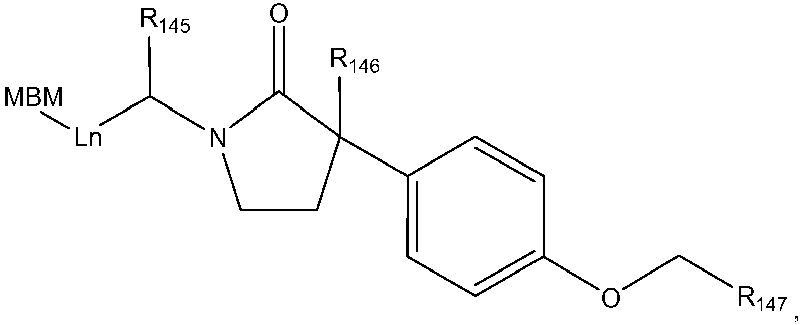 wherein:
      R₁₄₅ and R₁₄₆ are each independently alkyl or substituted alkyl;
      R₁₄₇ is selected from the group consisting of aryl, substituted aryl, heteroaryl, and substituted heteroaryl;
   (h) 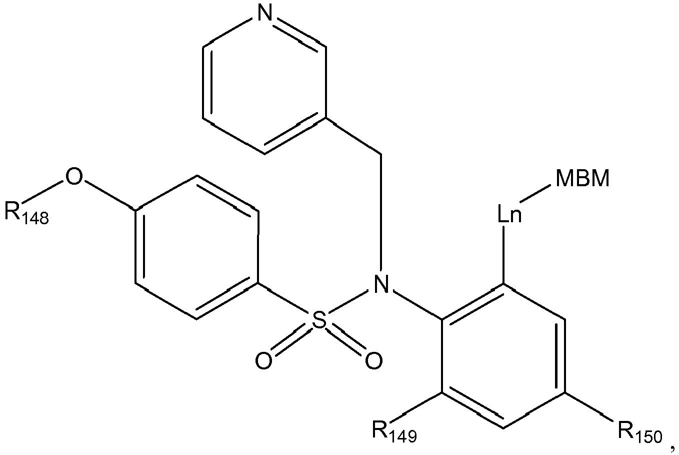 wherein:
      R₁₄₈ and R₁₄₉ are each independently alkyl or substituted alkyl;
      R₁₅₀ is selected from the group consisting of aryl, substituted aryl, heteroaryl, and substituted heteroaryl;
   (i) 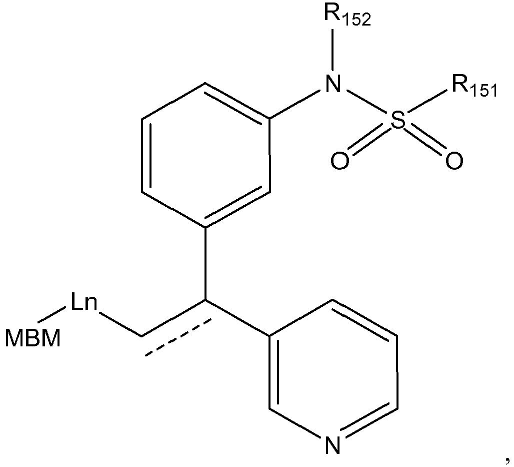 wherein:
      R₁₅₁ is selected from the group consisting of aryl, substituted aryl, heteroaryl, and substituted heteroaryl; and
      R₁₅₂ is alkyl or substituted alkyl;
   (j) 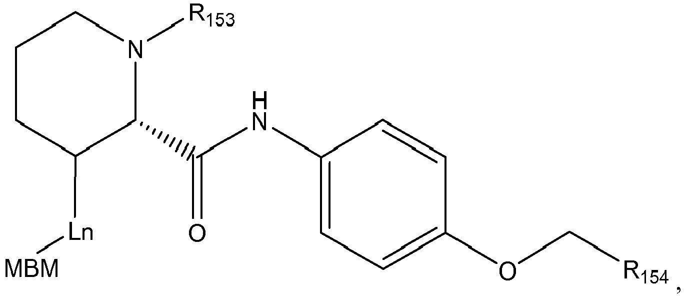 wherein:
      R₁₅₃ is selected from the group consisting of H, alkyl, and substituted alkyl; and
      R₁₅₄ is selected from the group consisting of aryl, substituted aryl, heteroaryl, and substituted heteroaryl;
   (k) 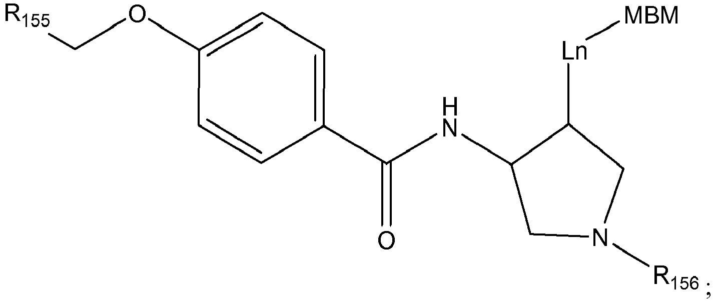 wherein:
      R₁₅₅ is selected from the group consisting of aryl, substituted aryl, heteroaryl, and substituted heteroaryl; and
      R₁₅₆ is selected from the group consisting of alkyl, substituted alkyl, and propargyl;
   (l) 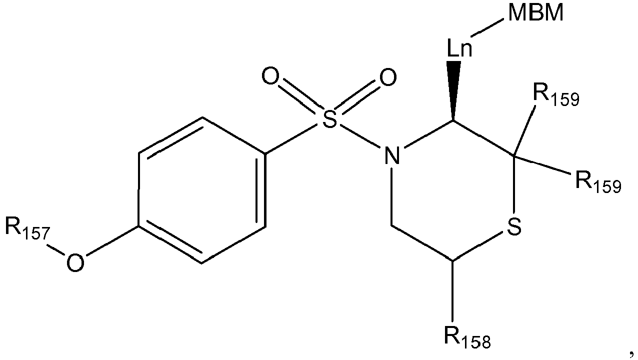 wherein:
      R₁₅₇ is selected from the group consisting of alkyl, substituted alkyl, and propargyl;
      R₁₅₈ is H or alkylamino; and
      each R₁₅₉ is independently alkyl or substituted alkyl;
   (m) 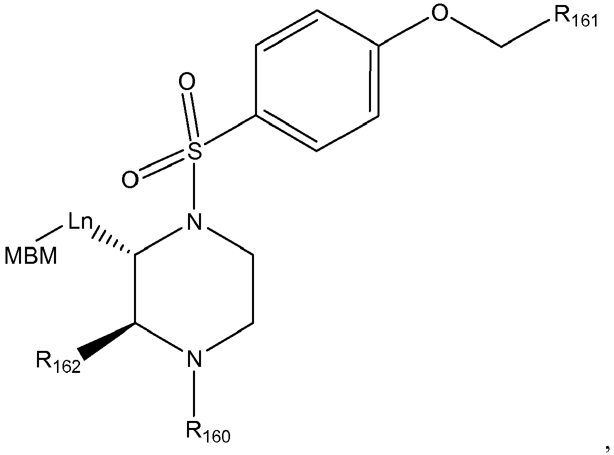 wherein:
      R₁₆₀ is H or acetyl;
      R₁₆₁ is selected from the group consisting of aryl, substituted aryl, heteroaryl, and substituted heteroaryl; and
      R₁₆₂ is alkyl or substituted alkyl;
   (n) 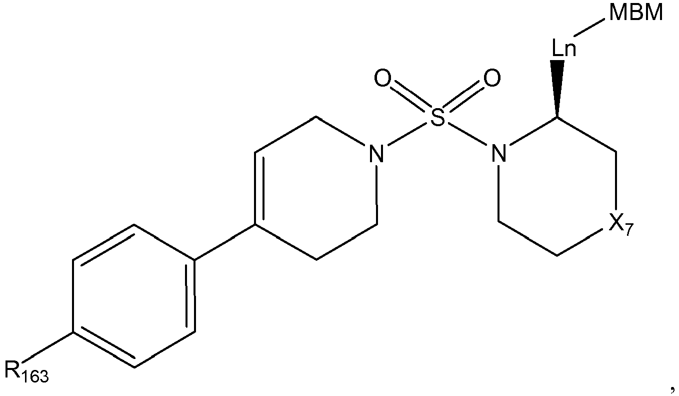
      R₁₆₃ is H or halogen;
      X₇ is CH₂ or NC(O)N-(alkyl)₂;
   (o) 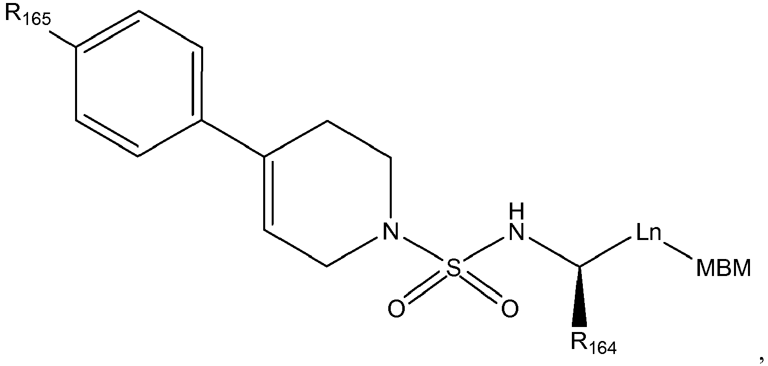 wherein:
      R₁₆₄ is selected from the group consisting of alkyl, substituted alkyl, and hydroxyl-alkyl; and
      R₁₆₅ is H or halogen;
   (p) 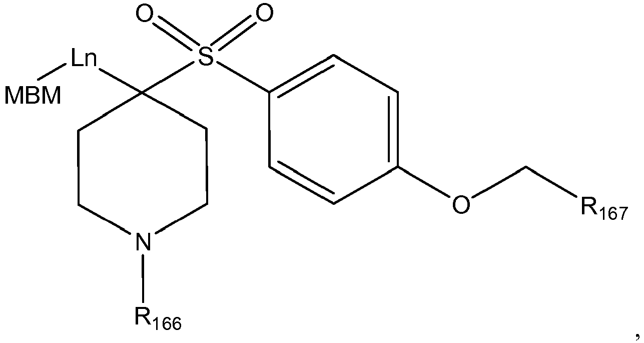 wherein:
      R₁₆₆ is selected from the group consisting of alkyl, substituted alkyl, aralkyl, alkyl-sulfonyl, and acetyl;
      R₁₆₇ is selected from the group consisting of alkyl, substituted alkyl, propargyl, heteroaryl, substituted heteroaryl, aryl, and substituted aryl;
      wherein:
      MBM is a metal binding moiety;
      Lₙ is a linker, wherein n is an integer from 0 to 1;
      under the proviso that the metal binding moiety is not a metal binding moiety selected from the group consisting of: 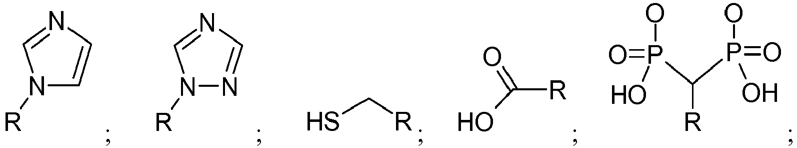 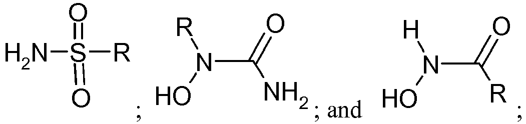 wherein:
         R is an attachment site through which the metal binding moiety can be attached to the targeting moiety, optionally through linker, Lₙ.
24. The TNF-alpha converting enzyme (TACE) inhibitor of para 23, wherein the inhibitor is selected from the group of TNF-alpha converting enzyme (TACE) inhibitors presented in Figure 27.
25. A metalloenzyme inhibitor, wherein the metalloenzyme inhibitor is a UDP-(3-O-acyl)-N-acetylglucosamine deacetylase (LpxC) inhibitor comprising a targeting moiety, a metal binding moiety, and optionally a linker, and wherein the inhibitor has a formula selected from the group consisting of:
   (a) 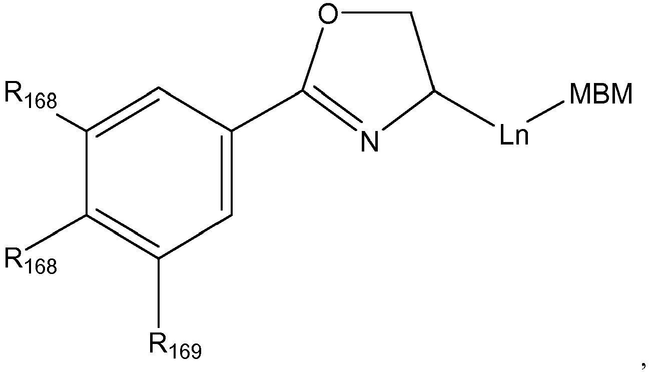 wherein:
      each R₁₆₈ is independently selected from the group consisting of H, alkyl, substituted alkyl, alkoxyl, alkylthio, OCF₃, and O-allyl;
      R₁₆₉ is selected from the group consisting of H, alkyl, and substituted alkyl;
   (b) 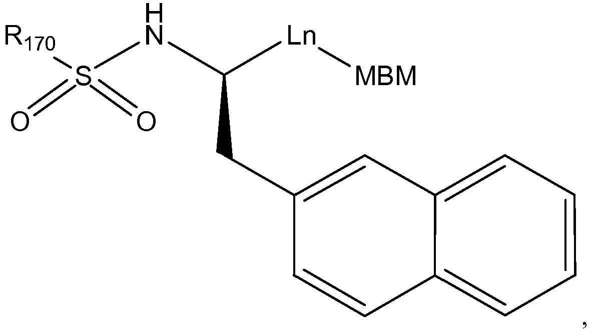 wherein R₁₇₀ is aryl or substituted aryl;
   (c) 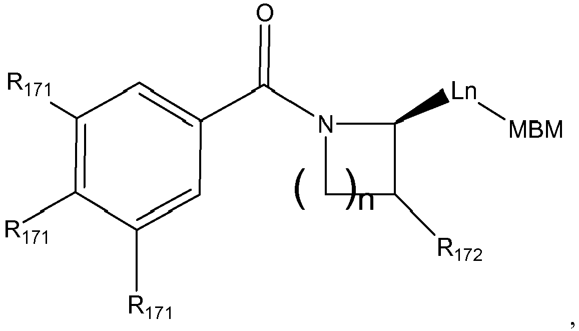 wherein:
      n is an integer from 1 to 2;
      each R₁₇₁ is independently selected from the group consisting of H, alkyl, substituted alkyl, alkoxyl, nitro, cycloalkyl, substituted cycloalkyl, heterocycloalkyl, substituted heterocycloalkyl, fluoroalkylthio, and halogen;
      R₁₇₂ is selected from the group consisting of H, alkyl, and substituted alkyl;
   (d) 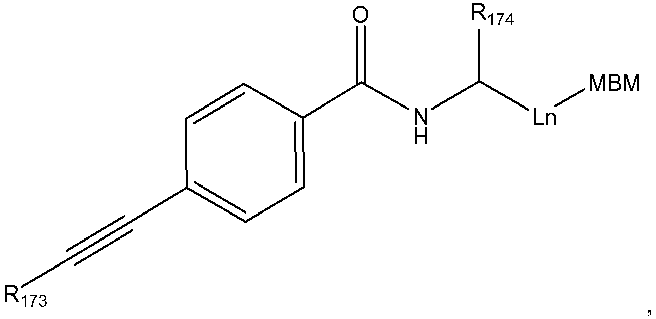 wherein:
      R₁₇₃ is selected from the group consisting of aryl, substituted aryl, propargyl-aryl, and 4-amino-aryl; and
      R₁₇₄ is selected from the group consisting of hydroxyl-alkyl, amino-alkyl, and CF₃;
   (e) 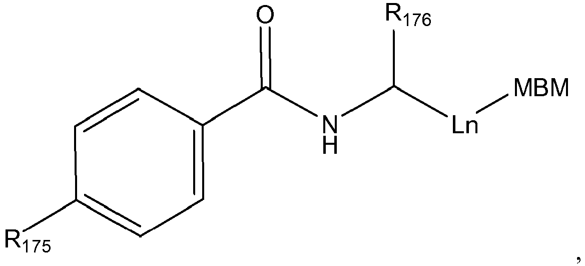 wherein:
      R₁₇₅ is selected from the group consisting of aryl, substituted aryl, and 4-amino-aryl; and
      R₁₇₆ is selected from the group consisting of hydroxyl-alkyl, amino-alkyl, and CF₃;
   (f) 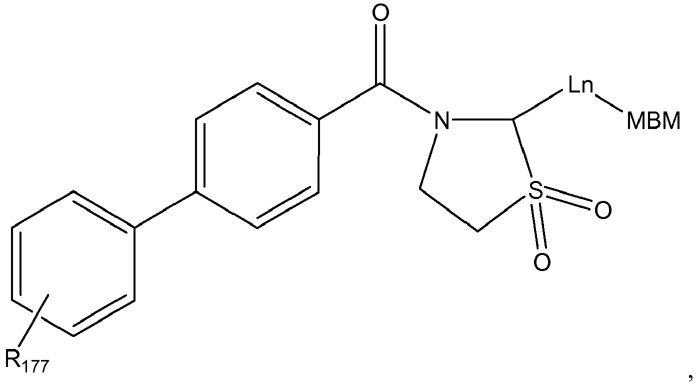 wherein R₁₇₇ is selected from the group consisting of alkyl, substituted alkyl, alkoxyl, and halogen;
   (g) 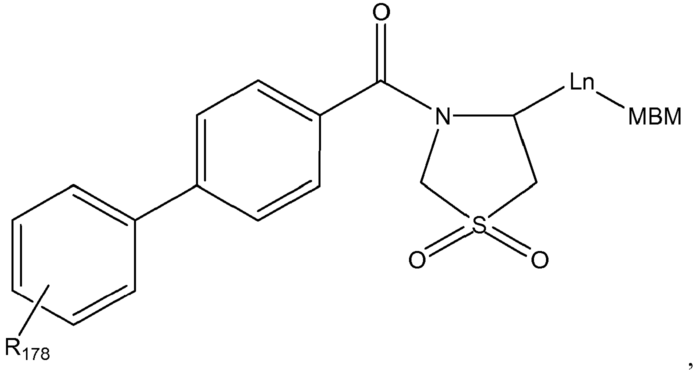 wherein R₁₇₈ is selected from the group consisting of alkyl, substituted alkyl, alkoxyl, and halogen;
      wherein:
      MBM is a metal binding moiety;
      Lₙ is a linker, wherein n is an integer from 0 to 1;
      under the proviso that the metal binding moiety is not a metal binding moiety selected from the group consisting of: 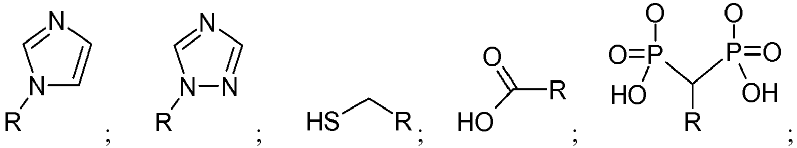 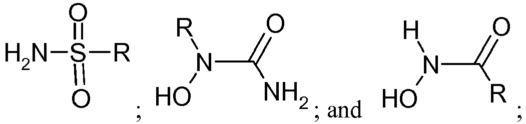 wherein:
         R is an attachment site through which the metal binding moiety can be attached to the targeting moiety, optionally through linker, Lₙ.
26. The UDP-(3-O-acyl)-N-acetylglucosamine deacetylase (LpxC) inhibitor of para 25, wherein the inhibitor is selected from the group of UDP-(3-O-acyl)-N-acetylglucosamine deacetylase (LpxC) inhibitors presented in Figure 28.
27. A metalloenzyme inhibitor, wherein the metalloenzyme inhibitor is an histone deacetylase (HDAC) inhibitor comprising a targeting moiety, a metal binding moiety, and optionally a linker, and wherein the inhibitor has a formula selected from the group consisting of:
   (a) 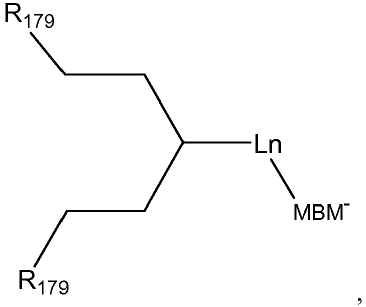 wherein each R₁₇₉ is independently selected from the group consisting of H, alkyl, and substituted alkyl;
   (b) 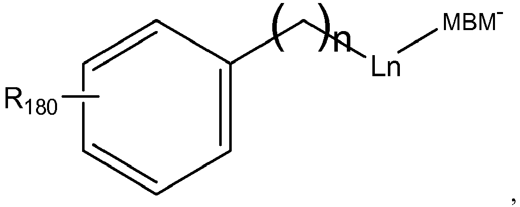 wherein:
      n is an integer from 1 to 2;
      R₁₈₀ is selected from the group consisting of H, halogen, alkyl, substituted alkyl, alkoxyl, amino-alkyl;
   (c) 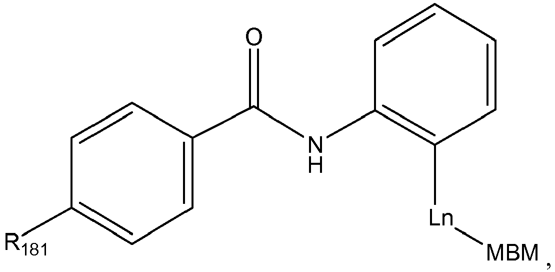 wherein R₁₈₁ is selected from the group consisting of alkyl, substituted alkyl, acyl-amino, and alkoxyl;
   (d) 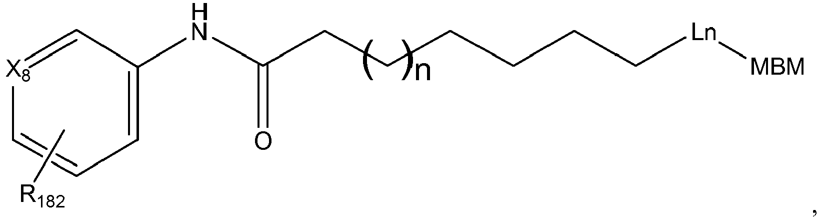 wherein:
      n is an integer from 0 to 2;
      R₁₈₂ is selected from the group consisting of amino-alkyl, halogen, and alkoxyl;
      X₈ is CH or N;
   (e) 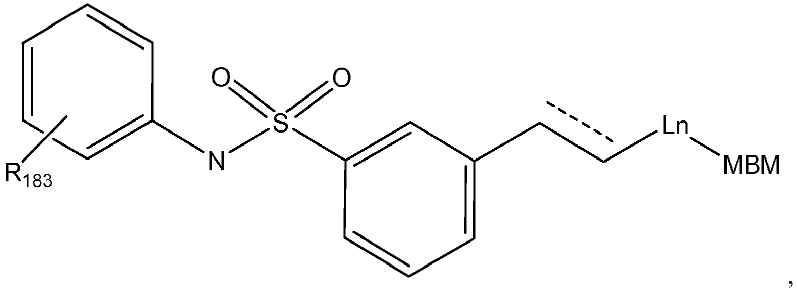 wherein:
      R₁₈₃ is selected from the group consisting of H, halogen, alkyl, substituted alkyl, alkoxyl, and amino-alkyl;
   (f) 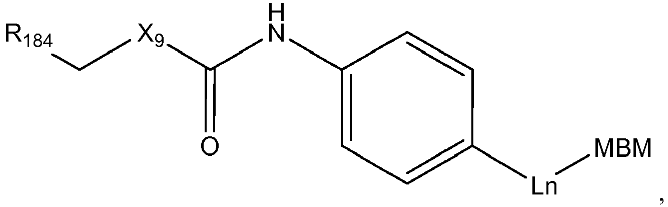 wherein:
      R₁₈₄ is selected from the group consisting of aryl, substituted aryl, heteroaryl, and substituted heteroaryl; and
      X₉ is O or NH;
   (g) 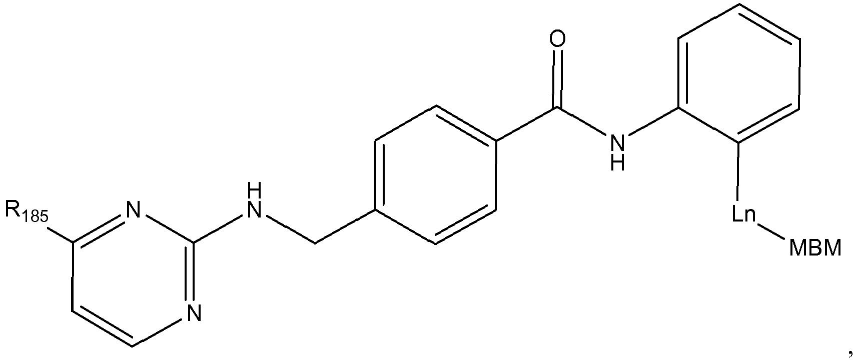 wherein R₁₈₅ is selected from the group consisting of aryl, substituted aryl, heteroaryl, and substituted heteroaryl;
   (h) 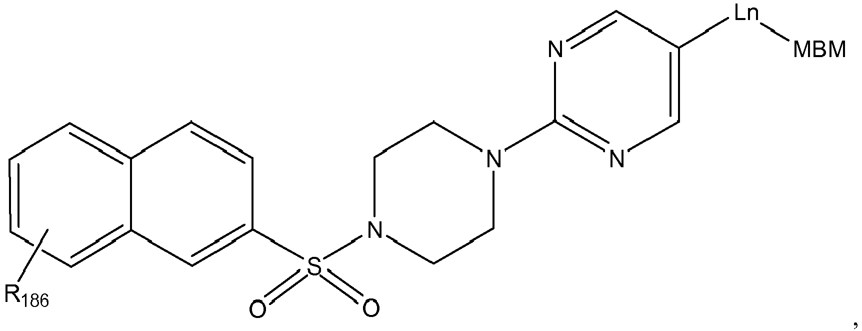 wherein R₁₈₆ is selected from the group consisting of H, halogen, alkyl, substituted alkyl, and alkoxyl;
   (i) 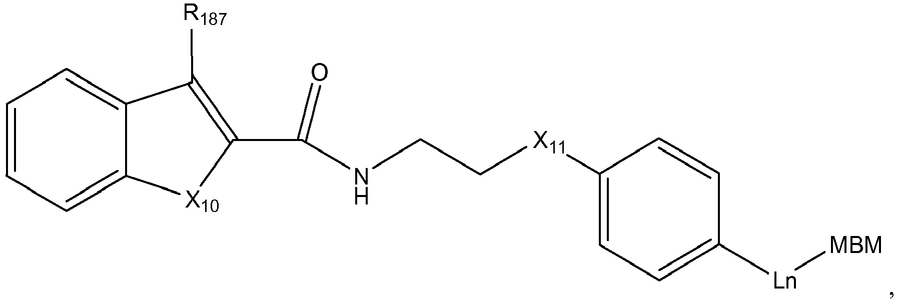 wherein:
      R₁₈₇ is selected from the group consisting of H, alkyl, substituted alkyl, and amino-alkyl;
      X₁₀ is selected from the group consisting of O, S, and NH; and
      X₁₁ is O or S;
   (j) 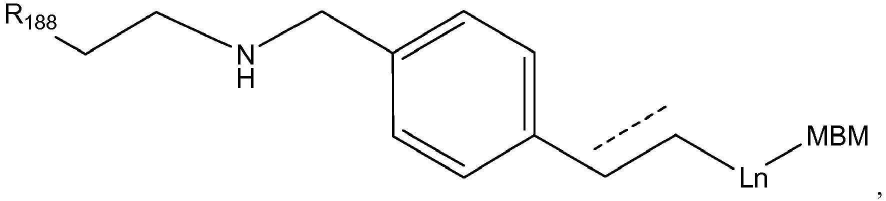 wherein R₁₈₈ is selected from the group consisting of aryl, substituted aryl, heteroaryl, and substituted heteroaryl;
   (k) 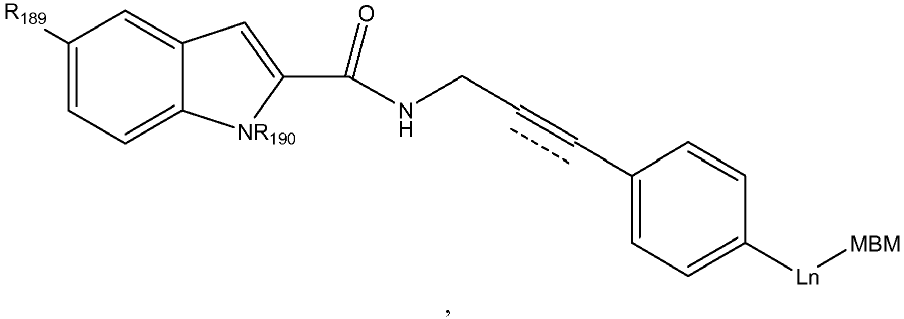 wherein:
      R₁₈₉ is selected from the group consisting of H, halogen, and alkoxyl; and
      R₁₉₀ is selected from the group consisting of H, alkyl, and substituted alkyl;
      wherein:
      MBM is a metal binding moiety;
      Lₙ is a linker, wherein n is an integer from 0 to 1;
      under the proviso that the metal binding moiety is not a metal binding moiety selected from the group consisting of: 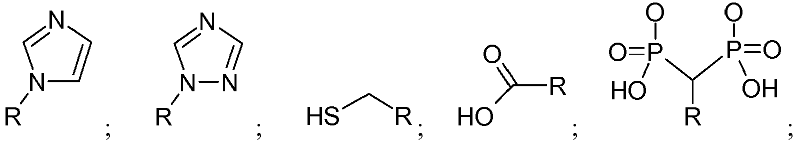 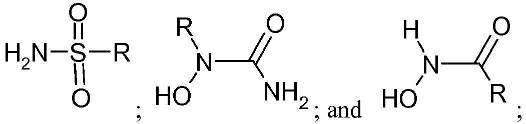 wherein:
         R is an attachment site through which the metal binding moiety can be attached to the targeting moiety, optionally through linker, Lₙ.
28. The histone deacetylase (HDAC) inhibitor of para 27, wherein the inhibitor is selected from the group of histone deacetylase (HDAC) inhibitors presented in Figure 29.
29. A metalloenzyme inhibitor, wherein the metalloenzyme inhibitor is a matrix metalloproteinase (MMP) inhibitor comprising a targeting moiety, a metal binding moiety, and optionally a linker, and wherein the inhibitor has a formula selected from the group consisting of:
   (a) 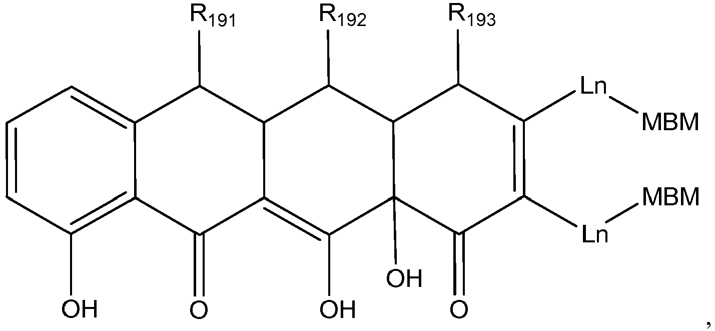 wherein:
      R₁₉₁ is selected from the group consisting of H, alkyl, and substituted alkyl;
      R₁₉₂ is H or OH;and
      R₁₉₃ is H or N-(alkyl)₂;
   (b) 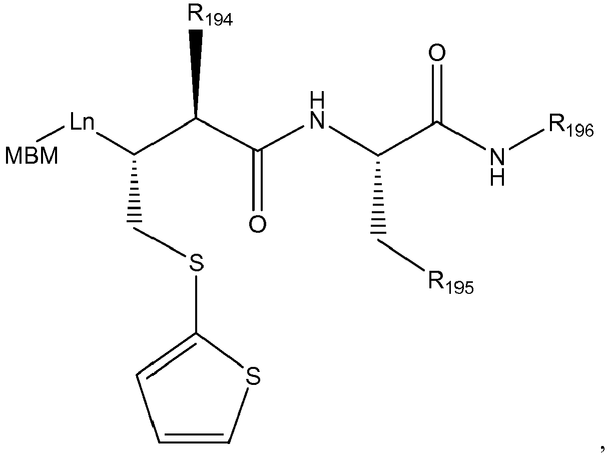 wherein:
      R₁₉₄ is alkyl or substituted alkyl;
      R₁₉₅ is aryl or substituted aryl; and
      R₁₉₆ is selected from the group consisting of H, alkyl, and substituted alkyl;
   (c) 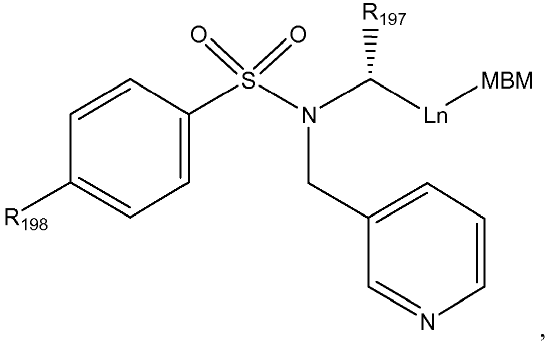 wherein:
      R₁₉₇ is alkyl or substituted alkyl; and
      R₁₉₈ is selected from the group consisting of H, halogen, alkyl, substituted alkyl, and alkoxyl;
   (d) 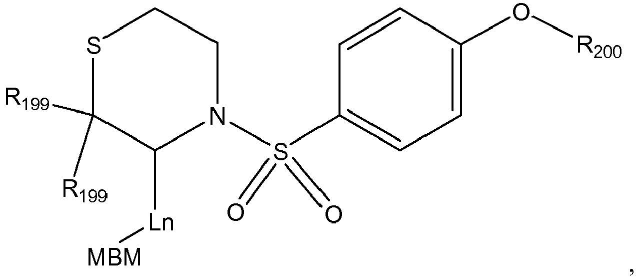 wherein:
      each R₁₉₉ is independently selected from the group consisting of H, alkyl, and substituted alkyl; and
      R₂₀₀ is selected from the group consisting of aryl, substituted aryl, heteroaryl, and substituted heteroaryl;
   (e) 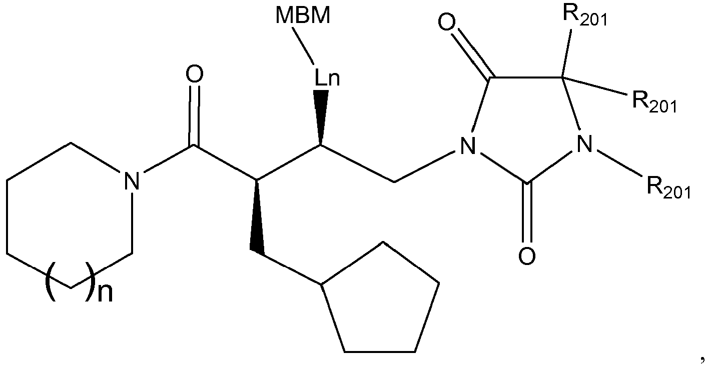 wherein:
      n is an integer from 0 to 1;
      each R₂₀₁ is independently selected from the group consisting of H, alkyl, and substituted alkyl;
   (f) 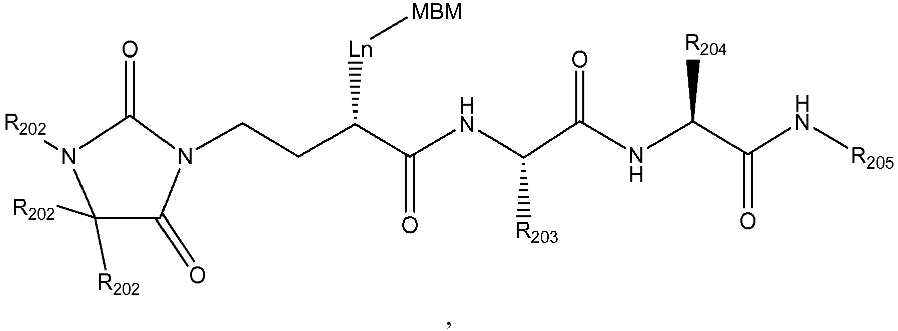 wherein:
      each R₂₀₂ is independently selected from the group consisting of H, alkyl, and substituted alkyl;
      R₂₀₃ and R₂₀₄ are each independently selected from the group consisting of alkyl, substituted alkyl, aryl, and substituted aryl; and
      R₂₀₅ is selected from the group consisting of H, alkyl, and substituted alkyl;
   (g) 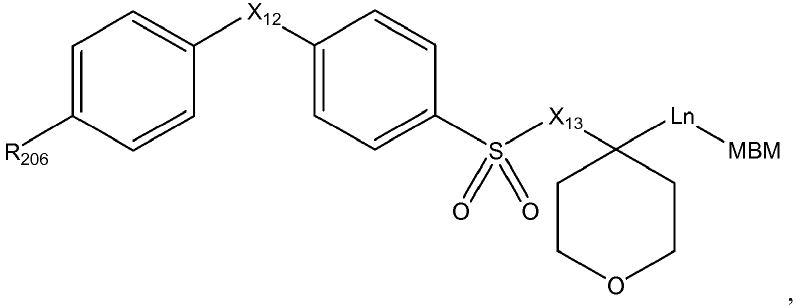 wherein:
      R₂₀₆ is selected from the group consisting of H, halogen, alkyl, and substituted alkyl;
      X₁₂ is O or S; and
      X₁₃ is CH₂ or NH;
   (h) 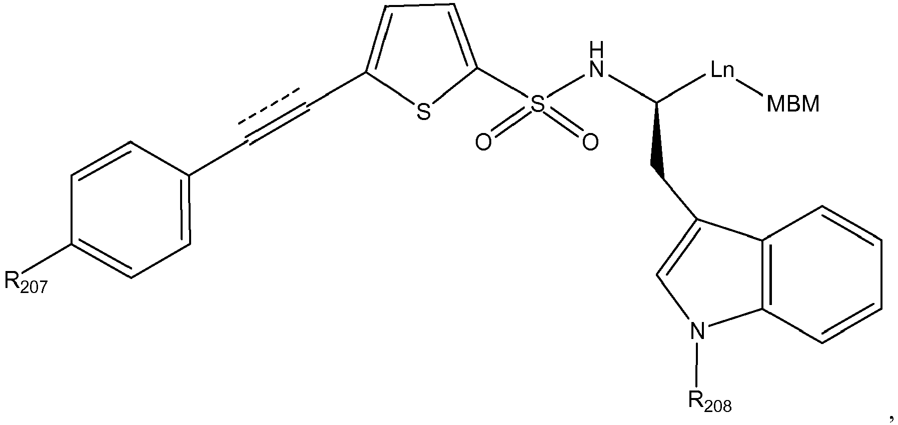 wherein:
      R₂₀₇ is selected from the group consisting of H, alkyl, substituted alkyl, and halogen; and
      R₂₀₈ is selected from the group consisting of H, alkyl, and substituted alkyl;
   (i) 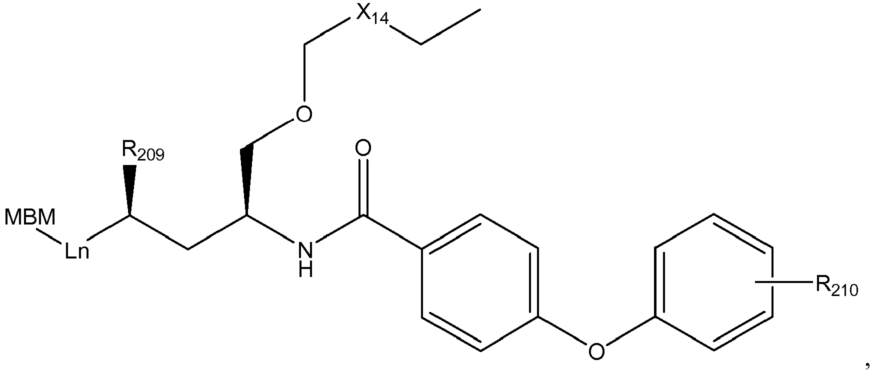 wherein:
      R₂₀₉ is alkyl or substituted alkyl;
      R₂₁₀ is selected from the group consisting of H, halogen, alkyl, substituted alkyl, and alkoxyl; and
      X₁₄ is selected from the group consisting of O, S, and CH₂;
   (j) 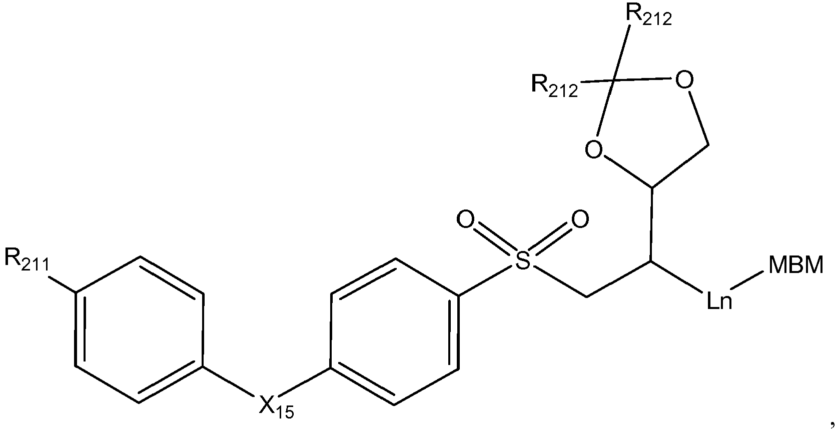 wherein:
      R₂₁₁ is selected from the group consisting of H, alkyl, substituted alkyl, alkoxyl, and halogen;
      each R₂₁₂ is independently selected from the group consisting of H, alkyl, and substituted alkyl; and
      X₁₅ is O or S;
   (k) 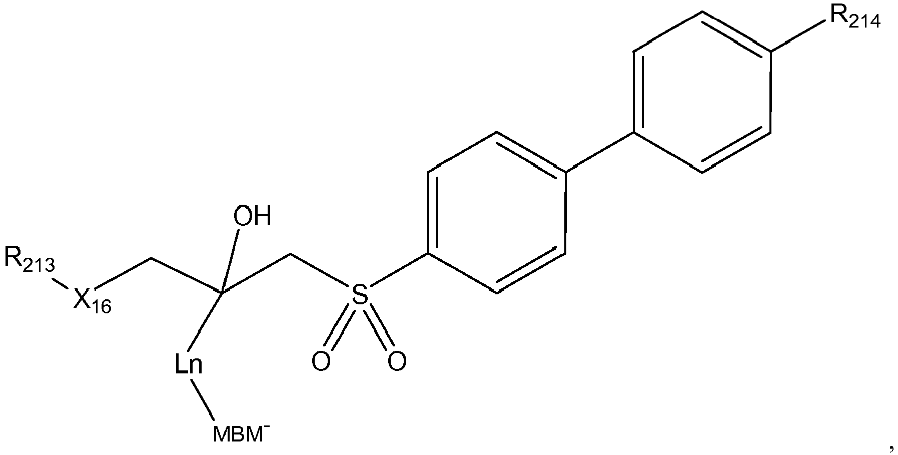 wherein:
      R₂₁₃ is alkyl or substituted alkyl;
      R₂₁₄ is selected from the group consisting of H, alkyl, substituted alkyl, halogen, and alkoxyl
      X₁₆ is O or S;
   (l) 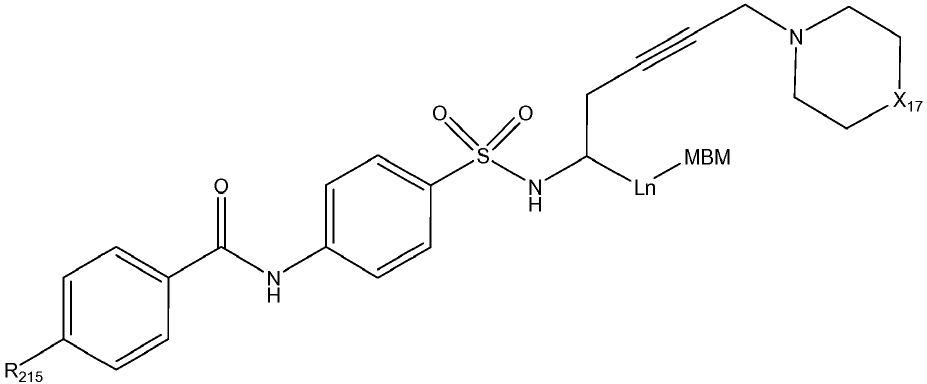 wherein:
      R₂₁₅ is selected from the group consisting of H, halogen, alkyl, substituted alkyl, and alkoxyl;
      X₁₇ is O or S;
   (m) 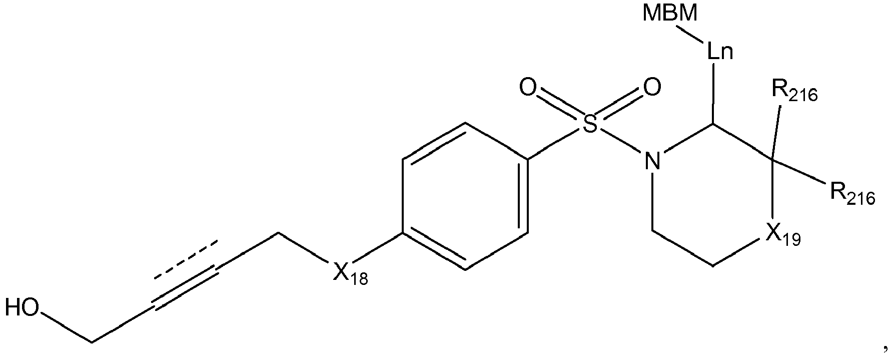 wherein:
      each R₂₁₆ is independently selected from the group consisting of H, alkyl, and substituted alkyl;
      X₁₈ and X₁₉ are each independently O or S;
   (n) 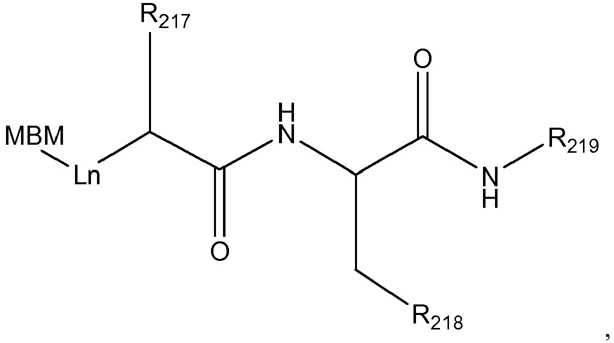 wherein:
      R₂₁₇ is alkyl or substituted alkyl;
      R₂₁₈ is aryl or substituted aryl; and
      R₂₁₉ is selected from the group consisting of H, alkyl, substituted alkyl, aryl, and substituted aryl; and
      wherein:
      MBM is a metal binding moiety;
      Lₙ is a linker, wherein n is an integer from 0 to 1;
      under the proviso that the metal binding moiety is not a metal binding moiety selected from the group consisting of: 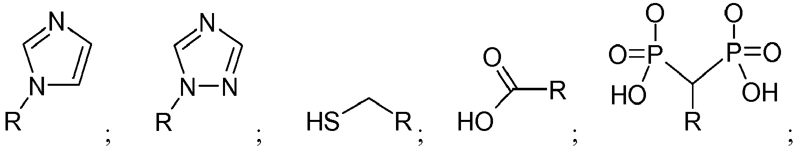 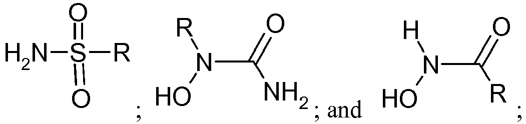 wherein:
         R is an attachment site through which the metal binding moiety can be attached to the targeting moiety, optionally through linker, Lₙ.
30. The matrix metalloproteinase (MMP) inhibitor of para 29, wherein the inhibitor is selected from the group of matrix metalloproteinase (MMP) inhibitors presented in Figure 30.
31. The metalloenzyme inhibitor of any of paras 1-30, wherein the metal binding moiety is selected from the group consisting of a sulfonyl moiety, a carbonyl moiety, a boronic acid or boronic ester moiety, a sulfur-containing moiety, a nitrogen-containing moiety, a phosphorous-containing moiety, a 5-membered heteroaromatic ring having one heteroatom, a 5-membered aromatic ring having two heteroatoms, a 5-membered heteroaromatic ring having three heteroatoms, a 5-membered heteroaromatic ring having four or five heteroatoms, a 5-membered saturated or partially unsaturated heteroalkyl ring having one heteroatom, a 5-membered saturated or partially unsaturated heteroalkyl ring having two heteroatoms, a six-membered aromatic ring, a 6-membered heteroaromatic ring having one heteroatom, a 6-membered aromatic ring having two heteroatoms, a 6-membered heteroaromatic ring having three or four heteroatoms, a 6-membered unsaturated or partially saturated heteroalkyl ring having one heteroatom, and a 6-membered unsaturated or partially saturated heteroalkyl ring having two heteroatoms; under the proviso that the metal binding moiety is not a metal binding moiety selected from the group consisting of: 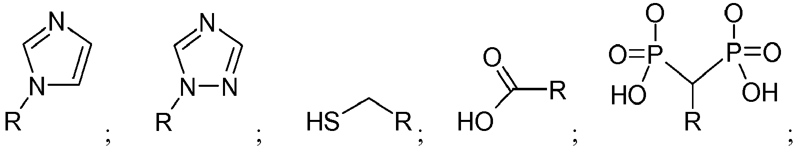 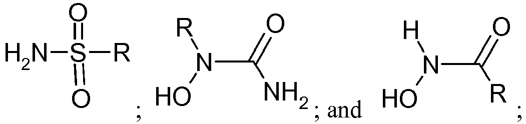 wherein:
   R is an attachment site through which the metal binding moiety can be attached to the targeting moiety, optionally through linker, Lₙ.
32. The metalloenzyme inhibitor of para 31, wherein the sulfonyl moiety has the following general formula: 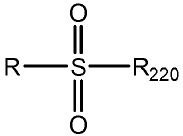 wherein:
   R is an attachment site through which the metal binding moiety can be attached to a targeting moiety, optionally through a linker, Lₙ, wherein n can be 0 or 1; and
   R₂₂₀ is selected from the group consisting of -OH, -SH, -NHNH₂, -NHOH, - NHOCH₃, -NHN(CH₃)₂, -NHNHC(=O)CH₃, -NHNHC(=O)NH₂, -NHNHC(=S)NH₂, - NHC≡N, -CH₂C≡N, -NHC(=O)CH₃, -NHC(=O)NH₂, -NHC(=S)NH₂, - NHC(=NH)NH₂, -CH₂C(=S)NH₂, -CH₂C(=O)NH₂, -CH₂P(=O)(OH)₂, -NHCH₂C≡N, - NHCH₂C(=O)-NH₂, -NHCH₂C(=NOH)-NH₂, -NHOCH₂C(=O)OH, 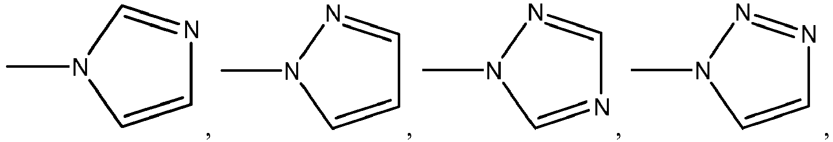 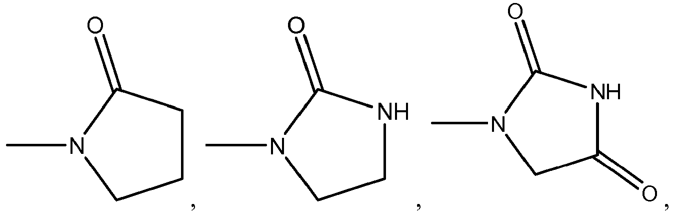 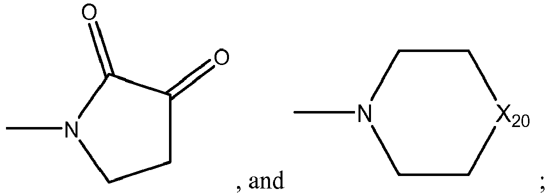 wherein X₂₀ is NH, O, or S.
33. The metalloenzyme inhibitor of para 31, wherein the carbonyl moiety has the following general formula: 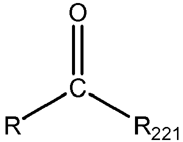 wherein:
   R is an attachment site through which the metal binding moiety can be attached to a targeting moiety, optionally through a linker, Lₙ, wherein n can be 0 or 1; and
   R₂₂₁ is selected from the group consisting of -NH₂, -SH, -NHNH₂, -N(OH)NH₂, -NHOH, -NCH₃OH, -NHOCH₃, -NCH₃OCH₃, -NHNHCH₃, -NHNHOH, - NHNHC(=O)CH₃, -NHNHC(=O)NH₂, -NHNHC(=S)NH₂, -NHC≡N, -NHC(=NH)NH₂, -CH₂C(=O)NH₂, -CH₂P(=O)(OH)₂, -NHCH₂C≡N, -NHCH₂C(=O)-NH₂, - NHC(=NOH)-NH₂, -OCH₂C(=O)-NH₂, -OCH₂C(=O)-OH, 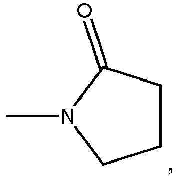 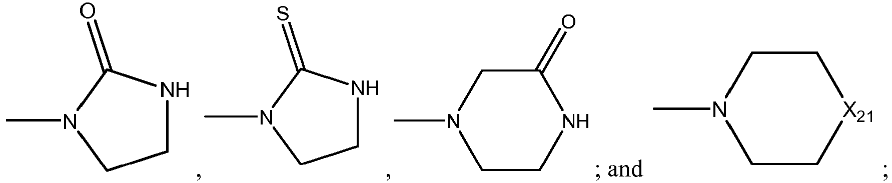 wherein X₂₁ is NH, S, or O; or R₂₂₁ is selected from the group consisting of: 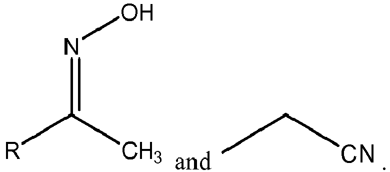
34. The metalloenzyme inhibitor of para 31, wherein the boronic acid or boronic ester moiety is selected from the group consisting of: 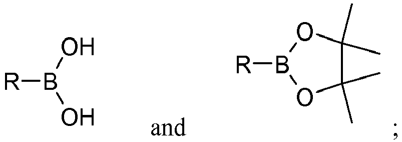 wherein:
   R is an attachment site through which the metal binding moiety can be attached to a targeting moiety, optionally through a linker, Lₙ, wherein n can be 0 or 1.
35. The metalloenzyme inhibitor of para 31, wherein the sulfur-containing moiety is selected from the group consisting of: R-SH, R-C(=S)-NH₂, 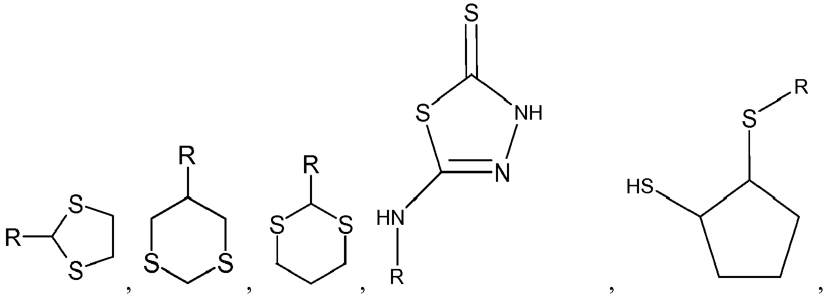 and 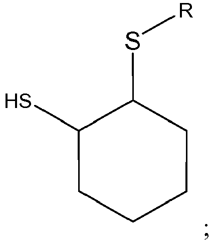 wherein:
   R is an attachment site through which the metal binding moiety can be attached to a targeting moiety, optionally through a linker, Lₙ, wherein n can be 0 or 1.
36. The metalloenzyme inhibitor of para 31, wherein the nitrogen-containing moiety has the following formula: 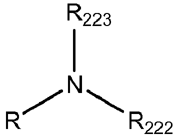 wherein:
   R is an attachment site through which the metal binding moiety can be attached to a targeting moiety, optionally through a linker, Lₙ, wherein n can be 0 or 1; and
   R₂₂₂ is selected from the group consisting of: -C(=O)-CH₃, -C(=O)-OCH₃, - C(=O)-NH₂, -C(=NH)-NH₂, -C(=O)-NHOH, -C(=NOH)-NH₂, -C(=O)-S-R₂₂₄, wherein R₂₂₄ is H or alkyl, -C(=S)-NH-R₂₂₅, wherein R₂₂₅ is H or alkyl, and;
   R₂₂₃ is selected from the group consisting of: -H, -OH, and -OCH₃;
      provided that when R₂₂₃ is OH, R₂₂₂ is not -C(=O)-NH₂ or -C(=O)-CH₃; or
      R₂₂₃ and R₂₂₂ together combine to form: 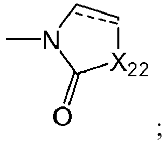
      wherein:
         X₂₂ is S or O; and
         a dashed line indicates that a bond can be present or absent.
37. The metalloenzyme inhibitor of para 31, wherein the phosphorous-containing moiety has the following formula:

   R-R₂₂₆-P(=X₂₃)(OH)(R₂₂₇)

   wherein:
   R is an attachment site through which the metal binding moiety can be attached to a targeting moiety, optionally through a linker, Lₙ, wherein n can be 0 or 1; and
   X₂₃ is O or S;
   R₂₂₆ is selected from the group consisting of -CH₂-, -O-, and -NH-; and
   R₂₂₇ is selected from the group consisting of -OH and -OCH₃.
38. The metalloenzyme inhibitor of para 31, wherein the 5-membered heteroaromatic ring having one heteroatom has the following formula: 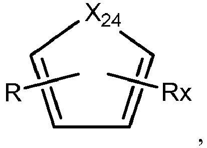 wherein:
   X₂₄ is selected from the group consisting of NH, O, and S.
   R is an attachment site through which the metal binding moiety can be attached to a targeting moiety, optionally through a linker, Lₙ, wherein n can be 0 or 1; and
   Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol.
39. The metalloenzyme inhibitor of para 31, wherein the 5-membered aromatic ring having two heteroatoms is selected from the group consisting of: 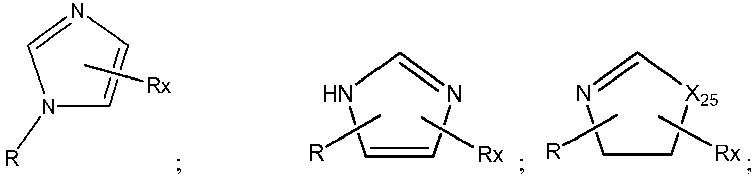 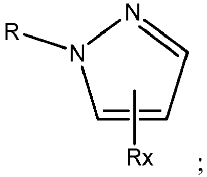 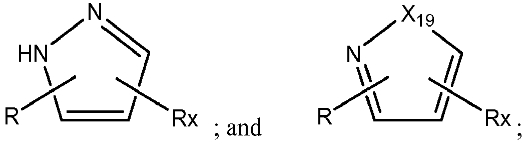 wherein:
   X₂₅ is O or S; and
   R is an attachment site through which the metal binding moiety can be attached to a targeting moiety, optionally through a linker, Lₙ, wherein n can be 0 or 1; and
   Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol;
   under the proviso that the metal binding moiety is not: 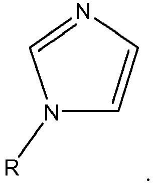
40. The metalloenzyme inhibitor of para 31, wherein the 5-membered heteroaromatic ring having three heteroatoms is selected from the group consisting of: 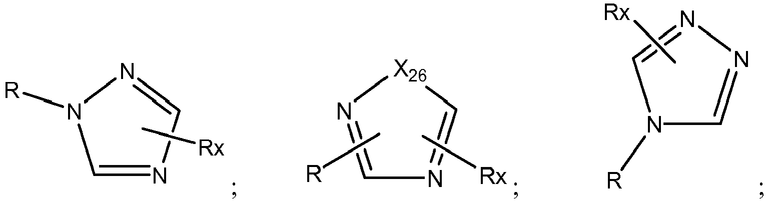 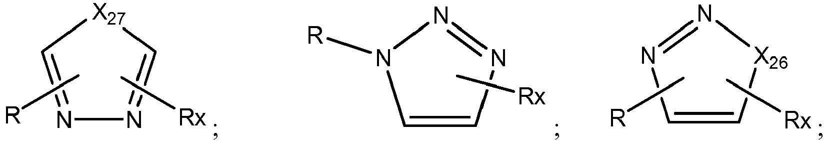 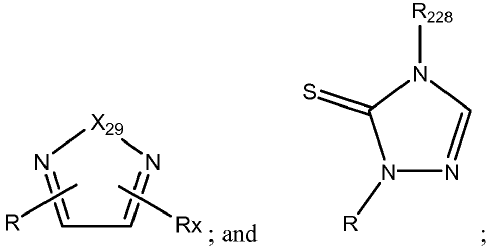 wherein:
   X₂₆ and X₂₈ are each independently selected from the group consisting of NH, O, and S;
   X₂₇ and X₂₉ are each independently O or S;
   R is an attachment site through which the metal binding moiety can be attached to a targeting moiety, optionally through a linker, Lₙ, wherein n can be 0 or 1; and
   Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol; and
   R₂₂₈ is H or alkyl;
   under the proviso that the metal binding moiety is not: 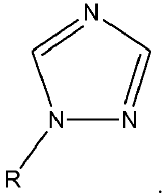
41. The metalloenzyme inhibitor of para 31, wherein the 5-membered heteroaromatic ring having four or five heteroatoms is selected from the group consisting of: 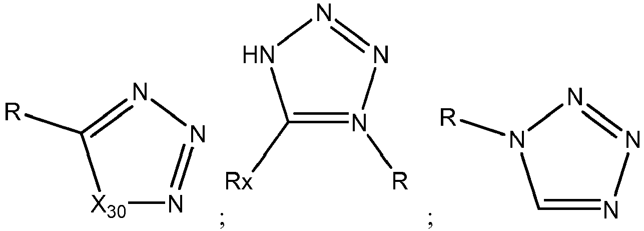 and 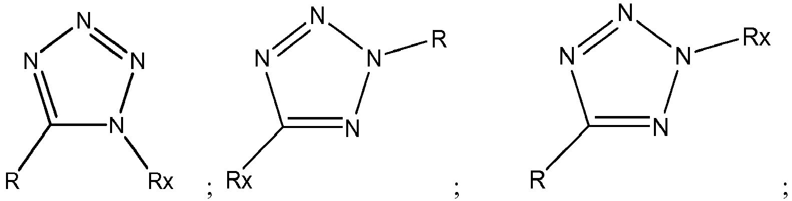 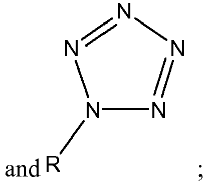 wherein:
   X₃₀ is selected from the group consisting of NH, O, and S;
   R is an attachment site through which the metal binding moiety can be attached to a targeting moiety, optionally through a linker, Lₙ, wherein n can be 0 or 1; and
   Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol.
42. The metalloenzyme inhibitor of para 31, wherein the 5-membered saturated or partially unsaturated heteroalkyl ring having one heteroatom is selected from the group consisting of: 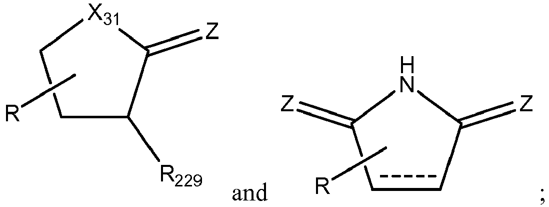 wherein:
   X₃₁ is selected from the group consisting of NH, NOH, O, and S; and
   each Z is independently selected from the group consisting of O, S, and NR₂₃₀, wherein R₂₃₀ is H or alkyl;
   R is an attachment site through which the metal binding moiety can be attached to a targeting moiety, optionally through a linker, Lₙ, wherein n can be 0 or 1; and
   R₂₂₉ is selected from the group consisting of H and OH; and
   wherein a dashed line indicates that a bond can be present or absent.
43. The metalloenzyme inhibitor of para 31, wherein the 5-membered saturated or partially unsaturated heteroalkyl ring having two heteroatoms is selected from the group consisting of: 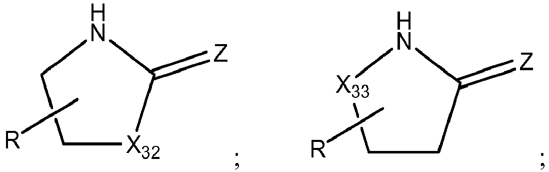 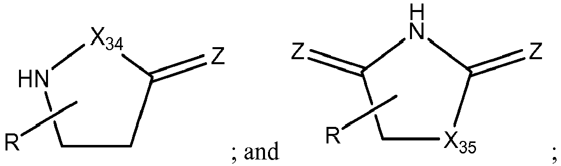 wherein:
   R is an attachment site through which the metal binding moiety can be attached to a targeting moiety, optionally through a linker, Lₙ, wherein n can be 0 or 1;
   X₃₂, X₃₃, and X₃₅ are each independently selected from the group consisting of NH, O, and S;
   X₃₄ is O or S; and
   each Z is independently selected from the group consisting of O, S, and NR₂₃₁, wherein R₂₃₁ is H or alkyl.
44. The metalloenzyme inhibitor of para 31, wherein the six-membered aromatic ring has the following formula: 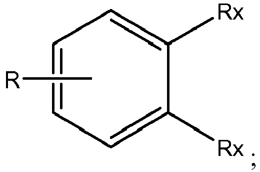 wherein:
   R is an attachment site through which the metal binding moiety can be attached to a targeting moiety, optionally through a linker, Lₙ, wherein n can be 0 or 1; and
   each Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol.
45. The metalloenzyme inhibitor of para 31, wherein the 6-membered heteroaromatic ring having one heteroatom has the following formula: 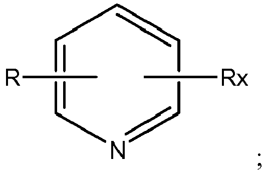 wherein:
   R is an attachment site through which the metal binding moiety can be attached to a targeting moiety, optionally through a linker, Lₙ, wherein n can be 0 or 1; and
   Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol.
46. The metalloenzyme inhibitor of para 31, wherein the 6-membered aromatic ring having two heteroatoms is selected from the group consisting of: 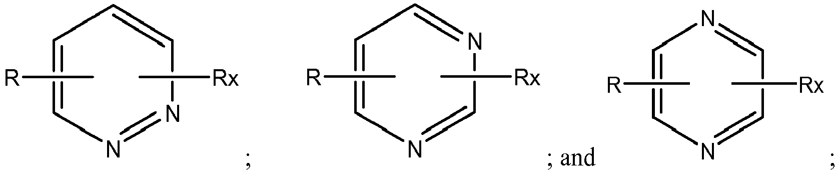 wherein:
   R is an attachment site through which the metal binding moiety can be attached to a targeting moiety, optionally through a linker, Lₙ, wherein n can be 0 or 1; and
   Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol.
47. The metalloenzyme inhibitor of para 31, wherein the 6-membered heteroaromatic ring having three or four heteroatoms is selected from the group consisting of: 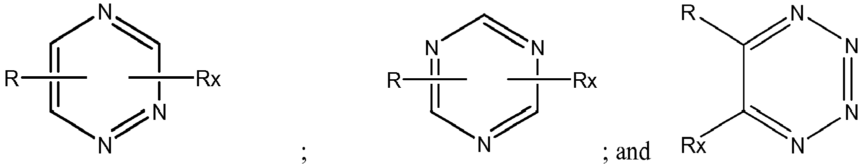 wherein:
   R is an attachment site through which the metal binding moiety can be attached to a targeting moiety, optionally through a linker, Lₙ, wherein n can be 0 or 1; and
   Rₓ is selected from the group consisting of hydrogen, alkyl, alcohol, aromatic, amino, amido, carbonyl, carboxyl, cyano, nitro, ethers, esters, aldehydes, sulfonyl, a silicon moiety, halogen, a sulfur-containing moiety, a phosphorus containing moiety, and an ethylene glycol.
48. The metalloenzyme inhibitor of para 31, wherein the 6-membered unsaturated or partially saturated heteroalkyl ring having one heteroatom is selected from the group consisting of: 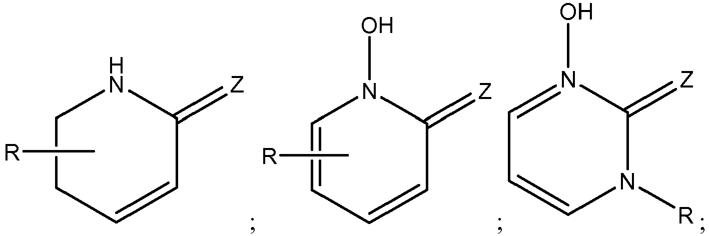 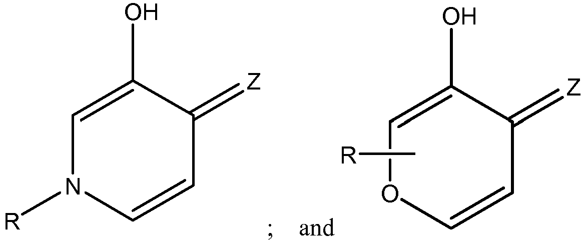 wherein:
   R is an attachment site through which the metal binding moiety can be attached to a targeting moiety, optionally through a linker, Lₙ, wherein n can be 0 or 1; and
   Z is selected from the group consisting of O, S, and NR₂₃₂, wherein R₂₃₂ is H or alkyl.
49. The metalloenzyme inhibitor of para 31, wherein the 6-membered unsaturated or partially saturated heteroalkyl ring having two heteroatoms is selected from the group consisting of: 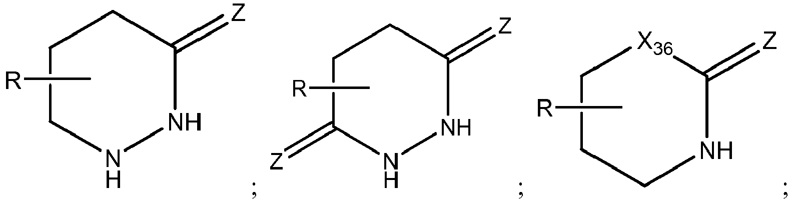 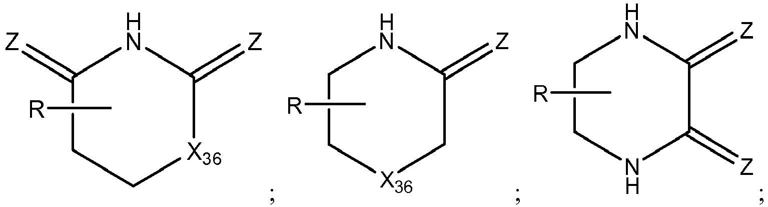 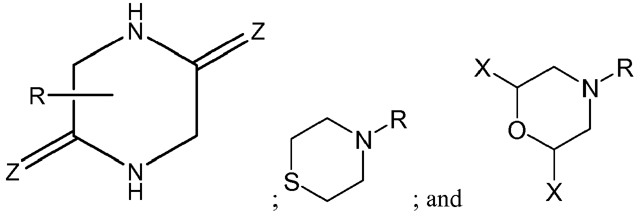 wherein:
   R is an attachment site through which the metal binding moiety can be attached to a targeting moiety, optionally through a linker, Lₙ, wherein n can be 0 or 1;
   each X₃₆ is NR₂₃₃, wherein R₂₃₃ is H or alkyl; and
   each Z is independently selected from the group consisting of O, S, and NR₂₃₄, wherein R₂₃₄ is H or alkyl.
50. The metalloenzyme inhibitor of any of paras 1-30, wherein the linker can be present or absent and when present is selected from the group consisting of alkylene, substituted alkylene, arylene, substituted arylene, heteroalkylene, and heteroarylene, and when absent is represented by a direct bond.
51. A method of screening for inhibitors of a metalloenzyme comprising:
   a) providing a candidate inhibitor comprising:
      i) a targeting moiety;
      ii) a metal binding moiety; and
      iii) optionally a linker;
   b) contacting the inhibitor candidate with a metalloenzyme; and
   c) determining the activity of the metalloenzyme.
52. The method of para 51, wherein the metalloenzyme is selected from the group consisting of carbonic anhydrase, catechol O-methyl transferase (COMT), farnesyl diphosphate synthase, farnesyl transferase, neprilysin (NEP), thromboxane synthase, anthrax lethal factor, endothelin converting enzyme, methionine aminopeptidase-2, peptide deformylase, HIV integrase, TNF-alpha converting enzyme (TACE), UDP-(3-O-acyl)-N-acetylglucosamine deacetylase (LpxC), histone deacetylase (HDAC), and matrix metalloproteinase (MMP).
53. The method of para 51, wherein the metal binding moiety is selected from the group consisting of the metal binding moieties of any of paras 31-50.
54. The method of para 51, further comprising contacting the metalloenzyme with a plurality of different candidate inhibitors.
55. The method of para 51, wherein the determining is done by measuring a substrate of the metalloenzyme.
56. The method of para 55, wherein the determining is done by measuring a product resulting from the hydrolysis of the substrate by at least one step.
57. The method of para 55, wherein the determining is done by measuring a product resulting from a demethylation of the substrate by more than one step.
58. A pharmaceutical composition comprising a pharmaceutical carrier and the composition of any of paras 1- 50, or a prodrug or salt thereof.
59. A method of treating a metalloenzyme related disorder comprising administering a composition of any of paras 1-50 or a prodrug or salt thereof to a patient in need thereof.
60. The method of para 59 wherein the disorder is selected from disorders associated with carbonic anhydrase, catechol O-methyl transferase (COMT), farnesyl diphosphate synthase, farnesyl transferase, neprilysin (NEP), thromboxane synthase, anthrax lethal factor, endothelin converting enzyme, methionine aminopeptidase-2, peptide deformylase, HIV integrase, TNF-alpha converting enzyme (TACE), UDP-(3-O-acyl)-N-acetylglucosamine deacetylase (LpxC), histone deacetylase (HDAC), and matrix metalloproteinase (MMP).
61. A method of inhibiting a metalloenzyme comprising contacting the metalloenzyme with an inhibitor of any of paras 1- 50.
62. The method of para 61 wherein the metalloenzyme is selected from the group consisting of carbonic anhydrase, catechol O-methyl transferase (COMT), farnesyl diphosphate synthase, farnesyl transferase, neprilysin (NEP), thromboxane synthase, anthrax lethal factor, endothelin converting enzyme, methionine aminopeptidase-2, peptide deformylase, HIV integrase, TNF-alpha converting enzyme (TACE), UDP-(3-O-acyl)-N-acetylglucosamine deacetylase (LpxC), histone deacetylase (HDAC), and matrix metalloproteinase (MMP).

## Claims

1. A metalloenzyme inhibitor, wherein the metalloenzyme inhibitor is a UDP-(3-O-acyl)-N-acetylglucosamine deacetylase (LpxC) inhibitor comprising a targeting moiety, a metal binding moiety, and optionally a linker, and wherein the inhibitor has the formula 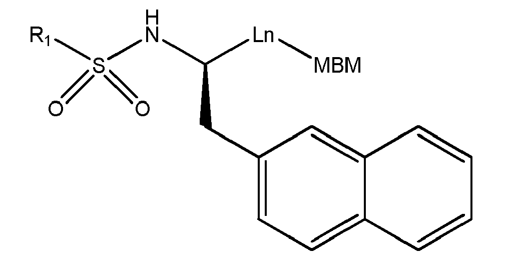 wherein:
MBM is a metal binding moiety;
Lₙ is a linker, wherein n is an integer from 0 to 1, and
R₁ is aryl or substituted aryl;
under the proviso that the metal binding moiety is not a metal binding moiety selected from the group consisting of: 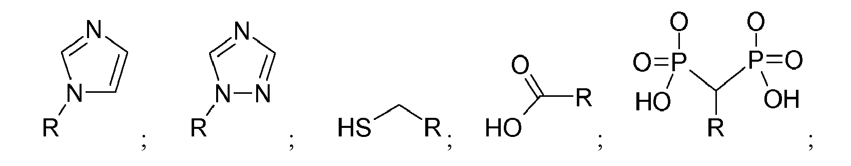 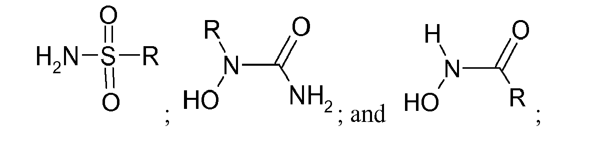 wherein:
R is an attachment site through which the metal binding moiety can be attached to the targeting moiety, optionally through linker, Lₙ.

2. The metalloenzyme inhibitor of claim 1, wherein R₁ is aryl or substituted aryl, which aryl or substituted aryl is polycyclic.

3. The metalloenzyme inhibitor of claim 1 or claim 2, wherein R₁ is a polycyclic aryl.

4. The metalloenzyme inhibitor of claim 1 or claim 2, wherein n is 0.

5. The metalloenzyme inhibitor of any one of claims 1 to 3, wherein the metal binding moiety is selected from the group consisting of a sulfonyl moiety, a carbonyl moiety, a boronic acid or boronic ester moiety, a sulfur-containing moiety, a nitrogen-containing moiety, a phosphorous-containing moiety, a 5-membered hetero aromatic ring having one heteroatom, a 5-membered aromatic ring having two heteroatoms, a 5-membered heteroaromatic ring having three heteroatoms, a 5-membered heteroaromatic ring having four or five heteroatoms, a 5-membered saturated or partially unsaturated heteroalkyl ring having one heteroatom, a 5-membered saturated or partially unsaturated heteroalkyl ring having two heteroatoms, a six-membered aromatic ring, a 6-membered heteroaromatic ring having one heteroatom, a 6-membered aromatic ring having two heteroatoms, a 6-membered heteroaromatic ring having three or four heteroatoms, a 6-membered unsaturated or partially saturated heteroalkyl ring having one heteroatom, and a 6-membered unsaturated or partially saturated heteroalkyl ring having two heteroatoms; under the proviso that the metal binding moiety is not a metal binding moiety selected from the group consisting of: 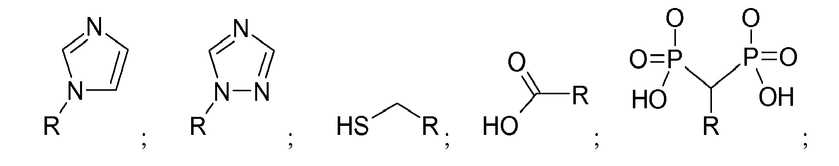 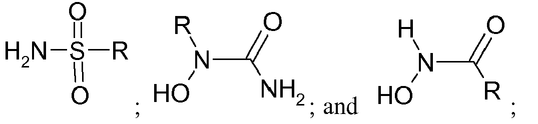 wherein:
R is an attachment site through which the metal binding moiety can be attached to the targeting moiety, optionally through linker, Lₙ, wherein the sulfonyl moiety has the following general formula: 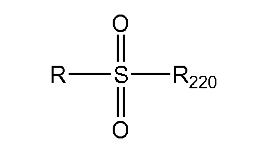 wherein:
R is an attachment site through which the metal binding moiety can be attached to a targeting moiety, optionally through a linker, Lₙ, wherein n can be 0 or 1; and
R₂₂₀ is selected from the group consisting of -OH, -SH, -NHNH₂, -NHOH, - NHOCH₃, -NHN(CH₃)₂, -NHNHC(=O)CH₃, -NHNHC(=O)NH₂, -NHNHC(=S)NH₂, - NHC≡N, -CH₂C≡N, -NHC(=O)CH₃, -NHC(=O)NH₂, -NHC(=S)NH₂, - NHC(=NH)NH₂, -CH₂C(=S)NH₂, -CH₂C(=O)NH₂, -CH₂P(=O)(OH)₂, -NHCH₂C≡N, - NHCH₂C(=O)-NH₂, -NHCH₂C(=NOH)-NH₂, -NHOCH₂C(=O)OH, 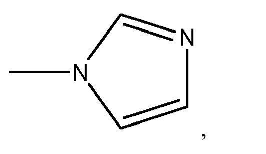 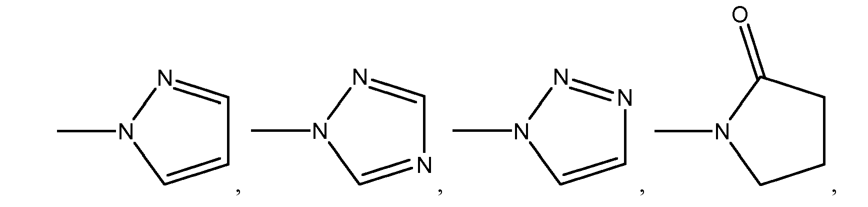 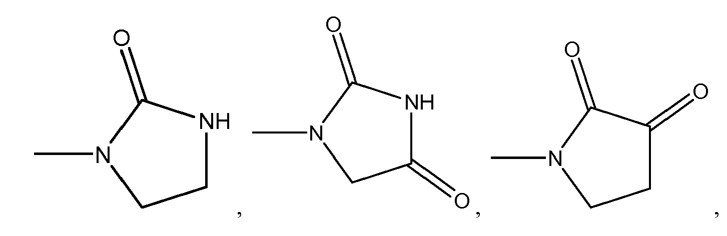 and 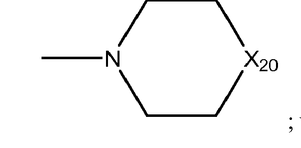 wherein X₂₀ is NH, O, or S.

6. The metalloenzyme inhibitor of claim 5, wherein the carbonyl moiety has the following general formula: 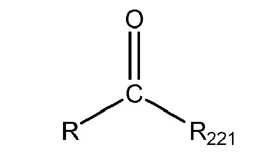 wherein:
R is an attachment site through which the metal binding moiety can be attached to a targeting moiety, optionally through a linker, Lₙ, wherein n can be 0 or 1; and
R₂₂₁ is selected from the group consisting of -NH₂, -SH, -NHNH₂, -N(OH)NH₂, - NHOH, -NCH₃OH, -NHOCH₃, -NCH₃OCH₃, -NHNHCH₃, -NHNHOH, - NHNHC(=O)CH₃, -NHNHC(=O)NH₂, -NHNHC(=S)NH₂, -NHC≡N, -NHC(=NH)NH₂, -CH₂C(=O)NH₂, -CH₂P(=O)(OH)₂, -NHCH₂C≡N, -NHCH₂C(=O)-NH₂, -NHC(=NOH)- 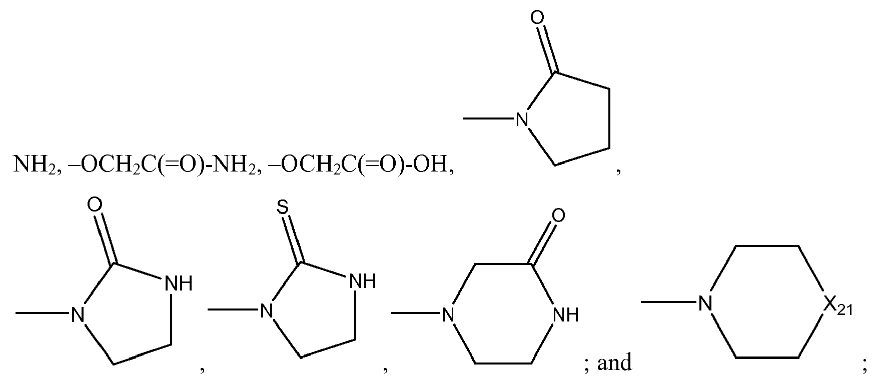 wherein X₂₁ is NH, S, or O; or R₂₂₁ is selected from the group consisting of:

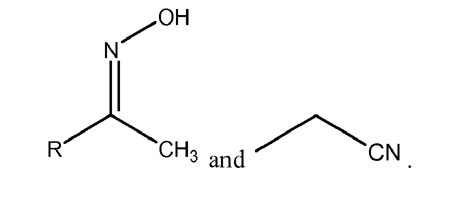

7. The metalloenzyme inhibitor of claim 6, wherein R₂₂₁ is -NHNH₂.

8. The metalloenzyme inhibitor of claim 1 or claim 2, wherein the linker can be present or absent and when present is selected from the group consisting of alkylene, substituted alkylene, arylene, substituted arylene, heteroalkylene, and heteroarylene, and when absent is represented by a direct bond.

9. A pharmaceutical composition comprising a pharmaceutical carrier and a metalloenzyme inhibitor as defined in any one of claims 1 to 8, or a prodrug or salt thereof.

10. A metalloenzyme inhibitor as defined in any one of claims 1 to 8, or a prodrug or salt thereof, for use in a method of treating a metalloenzyme related disorder comprising administering said metalloenzyme inhibitor, or said prodrug or salt thereof, to a patient in need thereof.

11. An *in vitro* method of inhibiting a UDP-(3-O-acyl)-N-acetylglucosamine deacetylase (LpxC) comprising contacting the UDP-(3-O-acyl)-N-acetylglucosamine deacetylase (LpxC) with an inhibitor as defined in any one of claims 1-8 or a prodrug or salt thereof.

12. A metalloenzyme inhibitor as defined in any one of claims 1 to 8, or a prodrug or salt thereof, for use in a method of inhibiting a metalloenzyme comprising administering said metalloenzyme inhibitor, or said prodrug or salt thereof, to a patient in need thereof.
